(19) Europäisches Patentamt
European Patent Office
Office européen des brevets

(11) EP 1 642 967 A2

(12) EUROPEAN PATENT APPLICATION

(43) Date of publication:
05.04.2006 Bulletin 2006/14

(21) Application number: 05019846.4

(22) Date of filing: 02.12.1999

(51) Int Cl.:
*C12N 9/10* (2006.01) *G01N 33/574* (2006.01)
*C12Q 1/68* (2006.01) *C12N 15/54* (2006.01)

(84) Designated Contracting States:
AT BE CH CY DE DK ES FI FR GB GR IE IT LI LU MC NL PT SE

(30) Priority: 08.03.1999 WOPCT/US99/05028
01.09.1999 WOPCT/US99/20111
29.10.1999 US 162506 P
30.11.1999 WOPCT/US99/28313
01.12.1999 WOPCT/US99/28634

(62) Document number(s) of the earlier application(s) in accordance with Art. 76 EPC:
99965090.6 / 1 159 422

(71) Applicant: GENENTECH, INC.
South San Francisco, CA 94080-4990 (US)

(72) Inventors:
- Botstein, David
Belmont, CA 94002 (US)
- Goddard, Audrey
San Francisco, CA 94131 (US)
- Gurney, Austin L.
San Francisco, CA 94114 (US)
- Roy, Margaret Ann
San Francisco, CA 94123 (US)
- Watanabe, Colin K.
Moraga, CA 94556 (US)
- Wood, William I.
Cupertino, CA 95014 (US)

(74) Representative: Denison, Christopher Marcus et al
Mewburn Ellis LLP
York House
23 Kingsway
London WC2B 6HP (GB)

Remarks:
This application was filed on 13 - 09 - 2005 as a divisional application to the application mentioned under INID code 62.

# (54) Composition and methods for the diagnosis of tumours

(57) The invention concerns compositions and methods for the diagnosis and treatment of neoplastic cell growth and proliferation in mammals, including humans. The invention is based upon the identification of genes that are amplified in the genome of tumor cells. Such gene amplification is expected to be associated with the overexpression of the gene product as compared to normal cells of the same tissue type and contribute to tumorigenesis. Accordingly, the proteins encoded by the amplified genes are believed to be useful targets for the diagnosis and/or treatment (including prevention) of certain cancers, and may act as predictors of the prognosis of tumor treatment. The present invention is directed to novel polypeptides and to nucleic acid molecules encoding those polypeptides. Also provided herein are vectors and host cells comprising those nucleic acid sequences, chimeric polypeptide molecules comprising the polypeptides of the present invention fused to heterologous polypeptide sequences, antibodies which bind to the polypeptides of the present invention and to methods for producing the polypeptides of the present invention.

EP 1 642 967 A2

## Description

Field of the Invention

The present invention relates to compositions and methods for the diagnosis and treatment of tumor.

Background of the Invention

Malignant tumors (cancers) are the second leading cause of death in the United States, after heart disease (Boring *et al.,* CA Cancel J. Clin., 43:7 [1993]).

Cancer is characterized by an increase in the number of abnormal, or neoplastic cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites (metastasis). In a cancerous state, a cell proliferates under conditions in which normal cells would not grow. Cancer manifests itself in a wide variety of forms, characterized by different degrees of invasiveness and aggressiveness.

Alteration of gene expression is intimately related to the uncontrolled cell growth and de-differentiation which are a common feature of all cancers. The genomes of certain well studied tumors have been found to show decreased expression of recessive genes, usually referred to as tumor suppression genes, which would normally function to prevent malignant cell growth, and/or overexpression of certain dominant genes, such as oncogenes, that act to promote malignant growth. Each of these genetic changes appears to be responsible for importing some of the traits that, in aggregate, represent the full neoplastic phenotype (Hunter, Cell, 64: 1129 [1991] and Bishop, Cell, 64:235-248 [1991]).

A well known mechanism of gene (*e.g.*, oncogene) overexpression in cancer cells is gene amplification. This is a process where in the chromosome of the ancestral cell multiple copies of a particular gene are produced. The process involves unscheduled replication of the region of chromosome comprising the gene, followed by recombination of the replicated segments back into the chromosome (Alitalo *et al.*, Adv. Cancer Res., 47:235-281 [1986]). It is believed that the overexpression of the gene parallels gene amplification, *i.e.*, is proportionate to the number of copies made.

Proto-oncogenes that encode growth factors and growth factor receptors have been identified to play important roles in the pathogenesis of various human malignancies, including breast cancer. For example, it has been found that the human ErbB2 gene (erbB2, also known as her2, or c-erbB-2), which encodes a 185-kd transmembrane glycoprotein receptor ($p185^{HER2}$; HER2) related to the epidermal growth factor receptor EGFR), is overexpressed in about 25% to 30% of human breast cancer (Slamon *et al.*, Science, 235:177-182 [1987]; Slamon *et al.*, Science, 244:707-712 [1989]).

It has been reported that gene amplification of a proto-oncogene is an event typically involved in the more malignant forms of cancer, and could act as a predictor of clinical outcome (Schwab *et al.*, Genes Chromosomes Cancer, 1:181-193 [1990]; Alitalo *et al., supra*). Thus, *erb*B2 overexpression is commonly regarded as a predictor of a poor prognosis, especially in patients with primary disease that involves axillary lymph nodes (Slamon *et al.*, [1987] and [1989], *supra*; Ravdin and Chamness, Gene, 159:19-27 [1995]; and Hynesand Stetn, Biochim. Biophys. Acta, 1198: 165-184 [1994]), and has been linked to sensitivity and/or resistance to hormone therapy and chemotherapeutic regimens, including CMF (cyclophosphamide, methotrexate, and fluoruracil) and anthracyclines (Baselga *et al.*, Oncology, 11 (3 Suppl 1):43-48 [1997]). However, despite the association of *erb*B2 overexpression with poor prognosis, the odds of HER2-positive patients responding clinically to treatment with taxanes were greater than three times those of HER2-negative patients (*Ibid*). A recombinant humanized anti-ErbB2 (anti-HER2) monoclonal antibody (a humanized version of the murine anti-ErbB2 antibody 4D5, referred to as rhuMAb HER2 or Herceptin™) has been clinically active in patients with ErbB2-overexpressing metastatic breast cancers that had received extensive prior anticancer therapy. (Baselga *et al.,* J. Clin. Oncol., 14:737-744 [1996]).

In light of the above, there is obvious interest in identifying novel methods and compositions which are useful for diagnosing and treating tumors which are associated with gene amplification.

Summary of the Invention

A. Embodiments

The present invention concerns compositions and methods for the diagnosis and treatment of neoplastic cell growth and proliferation in mammals, including humans. The present invention is based on the identification of genes that are amplified in the genome of tumor cells. Such gene amplification is expected to be associated with the overexpression of the gene product and contribute to tumorigenesis. Accordingly, the proteins encoded by the amplified genes are believed to be useful targets for the diagnosis and/or treatment (including prevention) ofcertain cancers, and may act as predictors of the prognosis of tumor treatment.

In one embodiment, the present invention concerns an isolated antibody which binds to a polypeptide designated herein as a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. In one aspect, the isolated antibody specifically binds to a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. In another aspect, the antibody induces the death of a cell which expresses a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. Often, the cell that expresses the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is a tumor cell that overexpresses the polypeptide as compared to a normal cell of the same tissue type. In yet another aspect, the antibody is a monoclonal antibody, which preferably has non-human complementarity determining region (CDR) residues and human framework region (FR) residues. The antibody may be labeled and may be immobilized on a solid support. In yet another aspect, the antibody is an antibody fragment, a single-chain antibody, or a humanized antibody which binds, preferably specifically, to a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

In another embodiment, the invention concerns a composition of matter which comprises an antibody which binds, preferably specifically, to a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in admixture with a pharmaceutically acceptable carrier. In one aspect, the composition of matter comprises a therapeutically effective amount of the antibody. In another aspect, the composition comprises a further active ingredient, which may, for example, be a further antibody or a cytotoxic or chemotherapeutic agent. Preferably, the composition is sterile.

In a further embodiment, the invention concerns isolated nucleic acid molecules which encode anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibodies, and vectors and recombinant host cells comprising such nucleic acid molecules.

In a still further embodiment, the invention concerns a method for producing an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody, wherein the methodcomprises culturing a host cell transformed with a nucleic acid molecule which encodes the antibody under conditions sufficient to allow expression of the antibody, and recovering the antibody from the cell culture.

The invention further concerns antagonists of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide that inhibit one or more of the biological and/or immunological functions or activities of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

In a further embodiment, the invention concerns an isolated nucleic acid molecule that hybridizes to a nucleic acid molecule encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide or the complement thereof. The isolated nucleic acid molecule is preferably DNA, and hybridization preferably occurs under stringent hybridization and wash conditions. Such nucleic acid molecules can act as antisense molecules of the amplified genes identified herein, which, in turn, can find use in the modulation of the transcription and/or translation of the respective amplified genes, or as antisense primers in amplification reactions. Furthermore, such sequences can be used as part of a ribozyme and/or a triple helix sequence which, in turn, may be used in regulation of the amplified genes.

In another embodiment, the invention provides a method for determining the presence of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in a sample suspected of containing a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, wherein the method comprises exposing the sample to an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407,anti-PRO1555,anti-PROI 096,anti-PRO2038 or anti-PRO2262antibody and determining binding of the antibody to a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in the sample. In another embodiment, the invention provides a method for determining the presence of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in a cell, wherein the method comprises exposing the cell to an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096,anti-PRO2018 or anti-PRO2262 antibody and determining binding of the antibody to the cell.

In yet another embodiment, the present invention concerns a method of diagnosing tumor in a mammal, comprising detecting the level of expression of a gene encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide (a) in a test sample of tissue cells obtained from the mammal, and (b) in a control sample of known normal tissue cells of the same cell type, wherein a higher expression level in the test sample as compared to the control sample, is indicative of the presence of tumor in the mammal from which the test tissue cells were obtained.

In another embodiment, the present invention concerns a method of diagnosing tumor in a mammal, comprising (a) contacting an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody with a test sample of tissue cells obtained from the mammal, and (b) detecting the formation of a complex between the anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody and a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in the test sample, wherein the formation of a complex is indicative of the presence of a tumor in said mammal. The detection may be qualitative or quantitative, and may be performed in comparison with monitoring the complex formation in a control sample of known normal tissue cells of the same cell type. A larger quantity of complexes formed in the test sample indicates the presence of tumor in the mammal from which the test tissue cells were obtained. The antibody preferably carries a detectable label. Complex formation can be monitored, for example, by light microscopy, flow cytometry, fluorimetry, or other techniques known in the art.

The test sample is usually obtained from an individual suspected to have neoplastic cell growth or proliferation (*e.g.* cancerous cells).

In another embodiment, the present invention concerns a cancer diagnostic kit comprising an anti-PRO381, anti-PRO1269, anti-PRO 1410, anti-PRO1755, anti-PRO1780, anti-PRO1788,anti-PRO3434,anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody and a carrier (*e.g.*, a buffer) in suitable packaging. The kit preferably contains instructions for using the antibody to detect the presence of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in a sample suspected of containing the same.

In yet another embodiment, the invention concerns a method for inhibiting the growth of tumor cells comprising exposing tumor cells which express a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide to an effective amount of an agent which inhibits a biological and/or immunological activity and/or the expression of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, wherein growth of the tumor cells is thereby inhibited. The agent preferably is an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody, a small organic and inorganic molecule, peptide, phosphopeptide, antisense or ribozyme molecule, or a triple helix molecule. In a specific aspect, the agent, *e.g.*, the anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555,anti-PRO1096,anti-PRO2038 or anti-PRO2262 antibody, induces cell death. In a further aspect, the tumor cells are further exposed to radiation treatment and/or a cytotoxic or chemotherapeutic agent.

In a further embodiment, the invention concerns an article of manufacture, comprising:

a container;
a label on the container; and
a composition comprising an active agent contained within the container; wherein the composition is effective for inhibiting the growth of tumor cells and the label on the container indicates that the composition can be used for treating conditions characterized by overexpression of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide as compared to a normal cell of the same tissue type. In particular aspects, the active agent in the composition is an agent which inhibits an activity and/or the expression of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. In preferred aspects, the active agent is an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody or an antisense oligonucleotide.

The invention also provides a method for identifying a compound that inhibits an activity of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, comprising contacting a candidate compound with a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide under conditions and for a time sufficient to allow these two components to interact and determining whether a biological and/or immunological activity of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is inhibited. In a specific aspect, either the candidate compound or the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is immobilized on a solid support. In another aspect, the non-immobilized component carries a detectable label. In a preferred aspect, this method comprises the steps of (a) contacting cells and a candidate compound to be screened in the presence ofthe PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide under conditions suitable for the induction of a cellular response normally induced by a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide and (b) determining the induction of said cellular response to determine if the test compound is an effective antagonist.

In another embodiment, the invention provides a method for identifying a compound that inhibits the expression of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in cells that express the polypeptide, wherein the method comprises contacting the cells with a candidate compound and determining whether the expression of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is inhibited. In a preferred aspect, this method comprises the steps of (a) contacting cells and a candidate compound to be screened under conditions suitable for allowing expression ofthe PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide and (b) determining the inhibition of expression ofsaid polypeptide.

B. Additional Embodiments

In other embodiments of the present invention, the invention provides an isolated nucleic acid molecule comprising a nucleotide sequence that encodes a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

In one aspect, the isolated nucleic acid molecule comprises a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule encoding a PRO381, PRO1269, PRO1410, PRO1755, PRO1780, PRO1788, PRO3434, PRO1927, PRO3567, PRO1295, PRO1293, PRO303, PRO4344, PRO4354, PRO4397, PRO4407, PRO555, PRO1096, PRO2038 or PRO2262 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91 % sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule comprising the coding sequence of a full-length PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide cDNA as disclosed herein, the coding sequence of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide lacking the signal peptide as disclosed herein, the coding sequence of an extracellular domain of a transmembrane PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule that encodes the same mature polypeptide encoded by any of the human protein cDNAs deposited with the ATCC as disclosed herein, or (b) the complement of the DNA molecule of (a).

Another aspect the invention provides an isolated nucleic acid molecule comprising a nucleotide sequence encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide which is either transmembrane domain-deleted or transmembrane domain-inactivated, or is complementary to such encoding nucleotide sequence, wherein the transmembranedomain(s)ofsuch polypeptide are disclosed herein. Therefore, soluble extracellular domains of the herein described PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptides are contemplated.

Another embodiment is directed to fragments of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide coding sequence, or the complement thereof, that may find use as, for example, hybridization probes, for encoding fragments of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide that may optionally encode a polypeptide comprising a bindingsite for an anti-PRO381, anti-PRO1269, anti-PRO1410, anti-PRO1755, anti-PRO1780, anti-PRO1788, anti-PRO3434, anti-PRO1927, anti-PRO3567, anti-PRO1295,anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody or as antisense oligonucleotide probes. Such nucleic acid fragments are usually at least about 20 nucleotides in length, preferably at least about 30 nucleotides in length, more preferably at least about 40 nucleotides in length, yet more preferably at least about 50 nucleotides in length, yet more preferably at least about 60 nucleotides in length, yet more preferably at least about 70 nucleotides in length, yet more preferably at least about 80 nucleotides in length, yet more preferably at least about 90 nucleotides in length, yet more preferably at least about 100 nucleotides in length, yet more preferably at least about 110 nucleotides in length, yet more preferably at least about 120 nucleotides in length, yet more preferably at least about 130 nucleotides in length, yet more preferably at least about 140 nucleotides in length, yet more preferably at least about 150 nucleotides in length, yet more preferably at least about 160 nucleotides in length, yet more preferably at least about 170 nucleotides in length, yet more preferably at least about 180 nucleotides in length, yet more preferably at least about 190 nucleotides in length, yet more preferably at least about 200 nucleotides in length, yet more preferably at least about 250 nucleotides in length, yet more preferably at least about 300 nucleotides in length, yet more preferably at least about 350 nucleotides in length, yet more preferably at least about 400 nucleotides in length, yet more preferably at least about 450 nucleotides in length, yet more preferably at least about 500 nucleotides in length, yet more preferably at least about 600 nucleotides in length, yet more preferably at least about 700 nucleotides in length, yet more preferably at least about 800 nucleotides in length, yet more preferably at least about 900 nucleotides in length and yet more preferably at least about 1000 nucleotides in length, wherein in this context the term "about" means the referenced nucleotide sequence length plus or minus 10% of that referenced length. It is noted that novel fragments of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide-encoding nucleotide sequence may be determined in a routine manner by aligning the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide-encoding nucleotide sequence with other known nucleotide sequences using any of a number of well known sequence alignment programs and determining which PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide-encoding nucleotide sequence fragment(s) are novel. All of such PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide-encoding nucleotide sequences are contemplated herein. Also contemplated are the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide fragments encoded by these nucleotide molecule fragments, preferably those PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide fragments that comprise a binding site for an anti-PRO381, anti-PRO 1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody.

In another embodiment, the invention provides isolated PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide encoded by any of the isolated nucleic acid sequences hereinabove identified.

In a certain aspect, the invention concerns an isolated PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, comprising an amino acid sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91 % sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptidecomprising an amino acid sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to an amino acid sequence encoded by any of the human protein cDNAs deposited with the ATCC as disclosed herein.

In a further aspect, the invention concerns an isolated PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptidecomprisingan amino acid sequence scoring at least about 80% positives, preferably at least about 81% positives, more preferably at least about 82% positives, yet more preferably at least about 83% positives, yet more preferably at least about 84% positives, yet more preferably at least about 85% positives, yet more preferably at least about 86% positives, yet more preferably at least about 87% positives, yet more preferably at least about 88% positives, yet more preferably at least about 89% positives, yet more preferably at least about 90% positives, yet more preferably at least about 91% positives, yet more preferably at least about 92% positives, yet more preferably at least about 93% positives, yet more preferably at least about 94% positives, yet more preferably at least about 95% positives, yet more preferably at least about 96% positives, yet more preferably at least about 97% positives, yet more preferably at least about 98% positives and yet more preferably at least about 99% positives when compared with the amino acid sequence of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a specific aspect, the invention provides an isolated PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vectorwhich comprises the appropriate encoding nucleic acid molecule under conditions suitable for expression of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide and recovering the PRO381, PRO1269, PRO410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide from the cell culture.

Another aspect the invention provides an isolated PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407,PRO555,PRO 1096,PRO2038orPRO2262 polypeptide which is eithertransmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO381, PRO1269, PRO1410, PRO1755, PRO1780, PRO1788,

PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide and recovering the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide from the cell culture.

In yet another embodiment, the invention concerns antagonists of a native PRO3 81, PRO 1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide as defined herein. In a particular embodiment, the antagonist is an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody or a small molecule.

In a further embodiment, the invention concerns a method of identifying antagonists to a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide which comprise contacting the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide with a candidate molecule and monitoring a biological activity mediated by said PRO381,PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. Preferably,the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is a native PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

In a still further embodiment, the invention concerns a composition of matter comprising a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, or an antagonist of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide as herein described, or an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody, in combination with a carrier. Optionally, the carrier is a pharmaceutically acceptable carrier.

Another embodiment of the present invention is directed to the use of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, or an antagonist thereof as hereinbefore described, or an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody, for the preparation of a medicament useful in the treatment of a condition which is responsive to the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, an antagonist thereof or an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody.

In other embodiments of the present invention, the invention provides vectors comprising DNA encoding any of the herein described polypeptides. Host cell comprising any such vector are also provided. By way of example, the host cells may be CHO cells, *E. coli,* yeast, or Baculovirus-infected insect cells. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

Brief Description of the Figures

Figure 1 shows the nucleotide sequence (SEQ ID NO:1) of a cDNA containing a nucleotide sequence encoding native sequence PRO381, wherein the nucleotide sequence (SEQ ID NO:1) is a clone designated herein as DNA44194-1317. Also presented in bold font and underlined are the positions of the respective start and stop codons.
Figure 2 shows the amino acid sequence (SEQ ID NO:2) of a native sequence PRO381 polypeptide as derived from the coding sequence of SEQ ID NO:1 shown in Figure 1.
Figure 3 shows the nucleotide sequence (SEQ ID NO:6) of a cDNA containing a nucleotide sequence encoding native sequence PRO1269, wherein the nucleotide sequence (SEQ ID NO:6) is a clone designated herein as DNA66520-1536. Also presented in bold font and underlined are the positions of the respective start and stop codons.
Figure 4 shows the amino acid sequence (SEQ ID NO:7) of a native sequence PRO1269 polypeptide as derived from the coding sequence of SEQ ID NO:6 shown in Figure 3.
Figure 5 shows the nucleotide sequence (SEQ ID NO:8) of a cDNA containing a nucleotide sequence encoding native sequence PRO1410, wherein the nucleotide sequence (SEQ ID NO:8) is a clone designated herein as DNA68874-1622. Also presented in bold font and underlined are the positions of the respective start and stop codons.
Figure 6 shows the amino acid sequence (SEQ ID NO:9) of a native sequence PRO141 polypeptide as derived from the coding sequence of SEQ ID NO:8 shown in Figure 5.
Figure 7 shows the nucleotide sequence (SEQ ID NO:10) of a cDNA containing a nucleotide sequence encoding native sequence PRO1755, wherein the nucleotide sequence (SEQ ID NO:10) is a clone designated herein as DNA76396-1698. Also presented in bold font and underlined are the positions of the respective start and stop codons.
Figure 8 shows the amino acid sequence (SEQ ID NO:11) of a native sequence PRO1755 polypeptide as derived from the coding sequence of SEQ ID NO:10 shown in Figure 7.
Figure 9 shows the nucleotide sequence (SEQ ID NO:12) of a cDNA containing a nucleotide sequence encoding native sequence PRO1780, wherein the nucleotide sequence (SEQ ID NO:12) is a clone designated herein as DNA71169-1709. Also presented in bold font and underlined are the positions of the respective start and stop codons.
Figure 10 shows the amino acid sequence (SEQ ID NO:13) of a native sequence PRO1780 polypeptide as derived from the coding sequence of SEQ ID NO:12 shown in Figure 9.

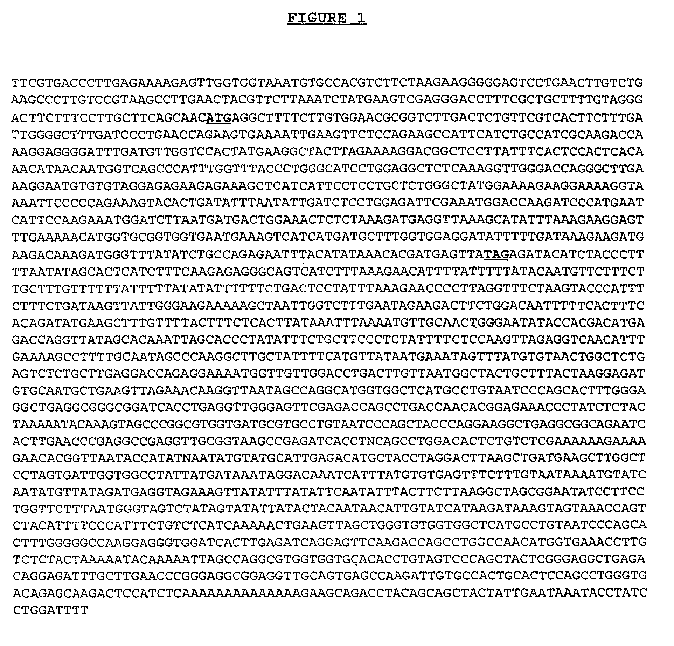

Figure 1l shows the nucleotide sequence (SEQ ID NO:17) of a cDNA containing a nucleotide sequence encoding native sequence PRO1788, wherein the nucleotide sequence (SEQ ID NO:17) is a clone designated herein as DNA77652-2505. Also presented in bold font and underlined are the positions of the respective start and stop codons.

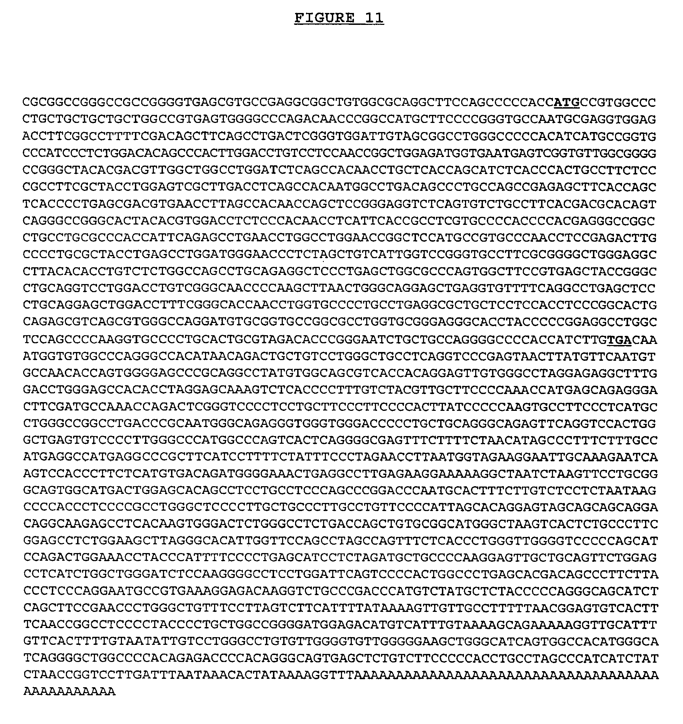

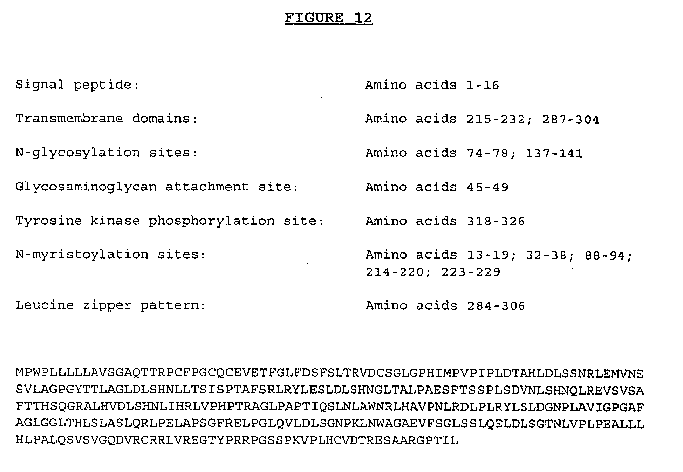

Figure 12 shows the amino acid sequence (SEQ ID NO:18) of a native sequence PRO1788 polypeptide as derived from the coding sequence of SEQ ID NO:17 shown in Figure 11.

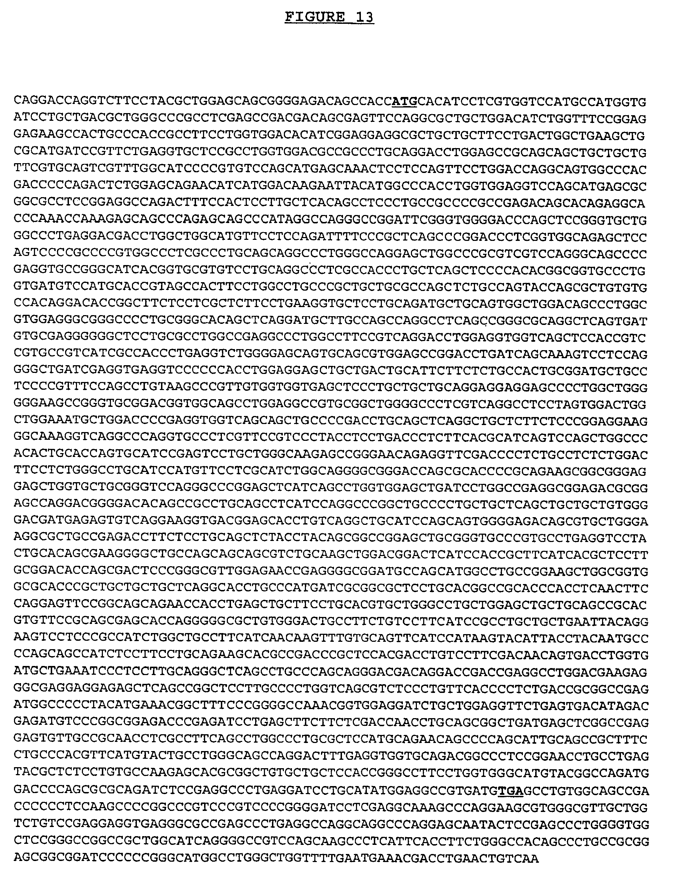

Figure 13 shows the nucleotide sequence (SEQ ID NO:22) of a cDNA containing a nucleotide sequence encoding native sequence PRO3434, wherein the nucleotide sequence (SEQ ID NO:22) is a clone designated herein as DNA77631-2537. Also presented in bold font and underlined are the positions of the respective start and stop codons.

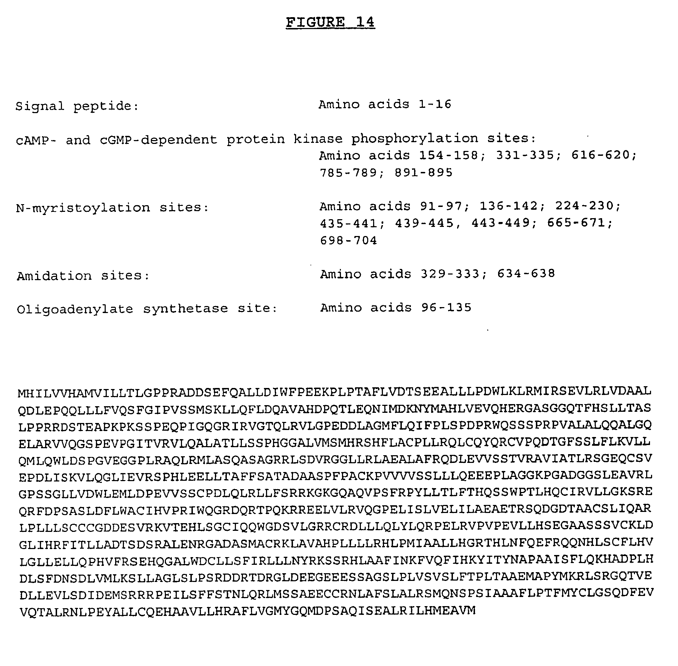

Figure 14 shows the amino acid sequence (SEQ ID NO:23) of a native sequence PRO3434 polypeptide as derived from the coding sequence of SEQ ID NO:22 shown in Figure 13.

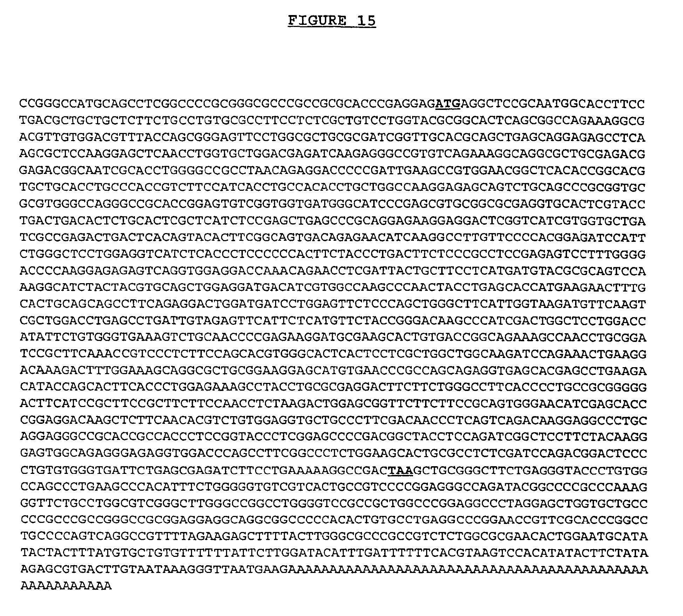

Figure. 15 shows the nucleotide sequence (SEQ ID NO:24) of a cDNA containing a nucleotide sequence encoding native sequence PRO1927, wherein the nucleotide sequence (SEQ ID NO:24) is a clone designated herein as DNA82307-2531. Also presented in bold font and underlined are the positions of the respective start and stop codons.

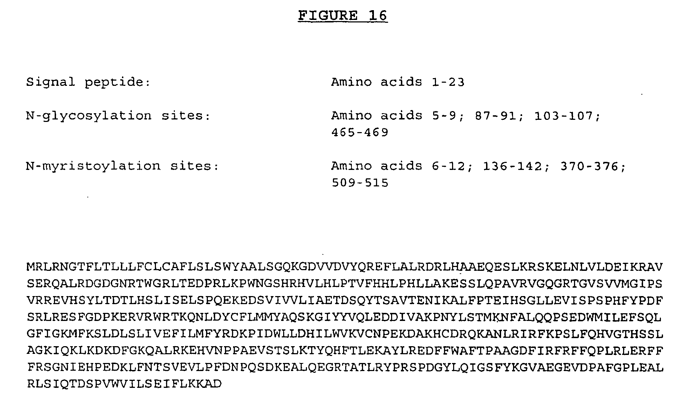

Figure 16 shows the amino acid sequence (SEQ ID NO:25) of a native sequence PRO1927 polypeptide as derived from the coding sequence of SEQ ID NO:24 shown in Figure 15.

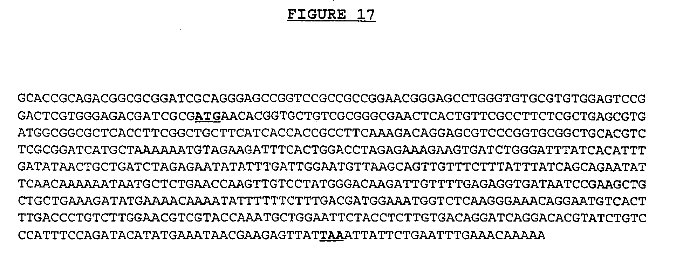

Figure 17 shows the nucleotide sequence (SEQ ID NO:26) of a cDNA containing a nucleotide sequence encoding native sequence PRO3567, wherein the nucleotide sequence (SEQ ID NO:26) is a clone designated herein as DNA56049-2543. Also presented in bold font and underlined are the positions of the respective start and stop codons.

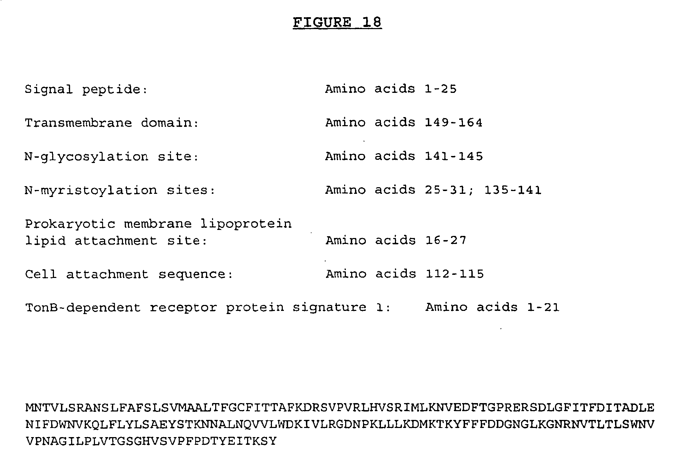

Figure 18 shows the amino acid sequence (SEQ ID NO:27) of a native sequence PRO3567 polypeptide as derived from the coding sequence of SEQ ID NO:26 shown in Figure 17.

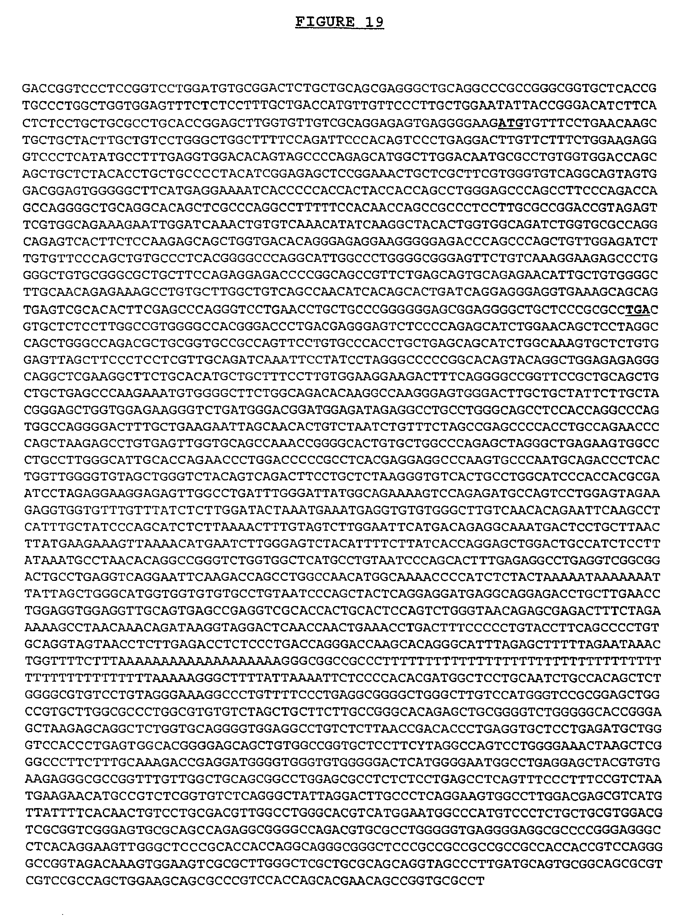

Figure 19 shows the nucleotide sequence (SEQ ID NO:28) of a cDNA containing a nucleotide sequence encoding native sequence PRO 1295, wherein the nucleotide sequence (SEQ ID NO:28) is a clone designated herein as DNA59218-1559. Also presented in bold font and underlined are the positions of the respective start and stop codons.

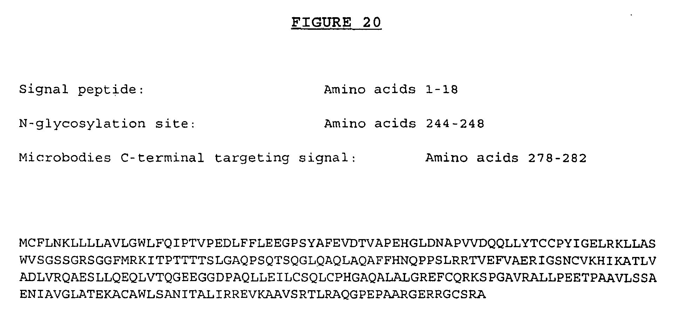

Figure 20 shows the amino acid sequence (SEQ ID NO:29) of a native sequence PRO1295 polypeptide as derived from the coding sequence of SEQ ID NO:28 shown in Figure 19.

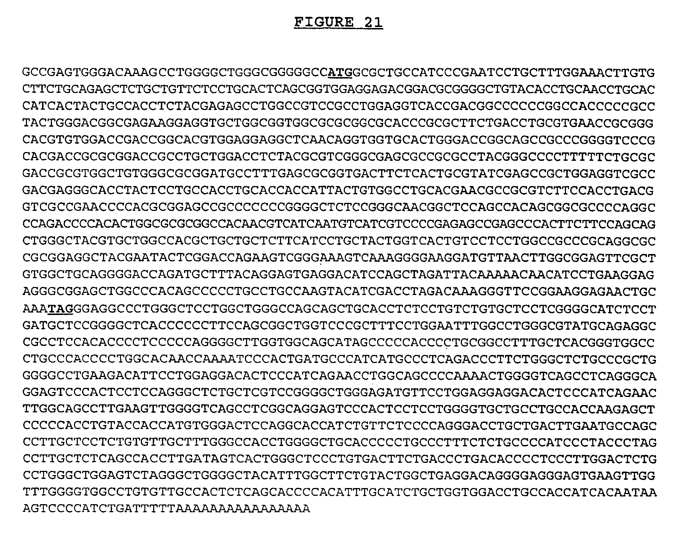

Figure 21 shows the nucleotide sequence (SEQ ID NO:30) of a cDNA containing a nucleotide sequence encoding native sequence PRO1293, wherein the nucleotide sequence (SEQ IDNO:30) is a clone designated herein as DNA60618-1557. Also presented in bold font and underlined are the positions of the respective start and stop codons.

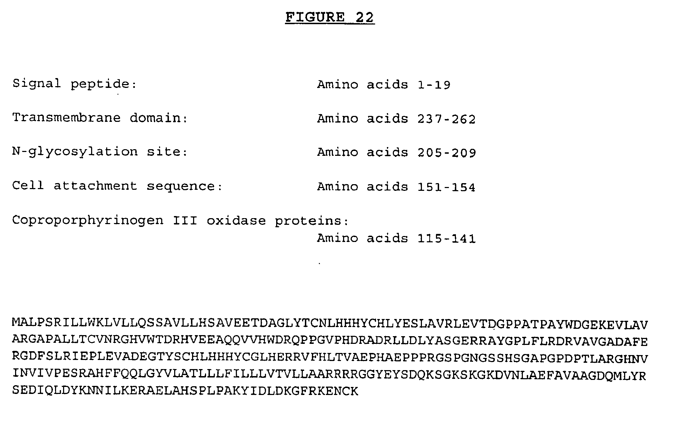

Figure 22 shows the amino acid sequence (SEQ ID NO:31) of a native sequence PRO1293 polypeptide as derived from the coding sequence of SEQ ID NO:30 shown in Figure 21.

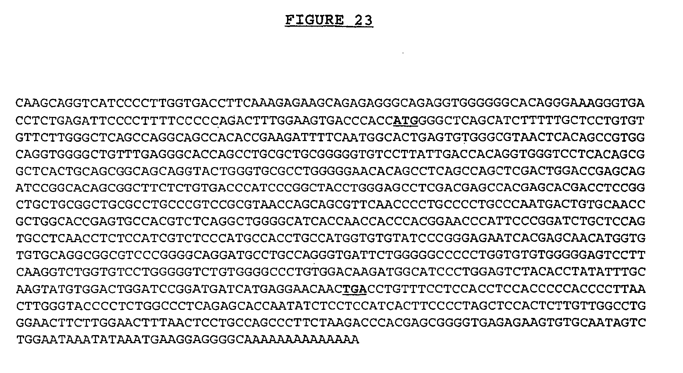

Figure 23 shows the nucleotide sequence (SEQ ID NO:32) of a cDNA containing a nucleotide sequence encoding native sequence PRO1303, wherein the nucleotide sequence (SEQ ID NO:32) is a clone designated herein as DNA65409-1566. Also presented in bold font and underlined are the positions of the respective start and stop codons.

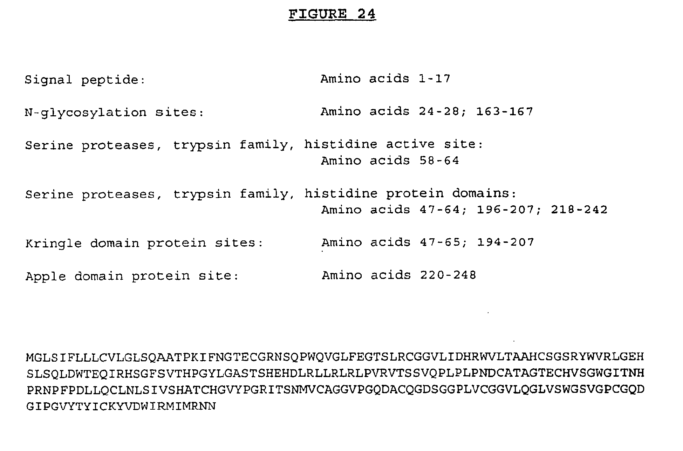

Figure 24 shows the amino acid sequence (SEQ ID NO:33) of a native sequence PRO1303 polypeptide as derived from the coding sequence of SEQ ID NO:32 shown in Figure 23.

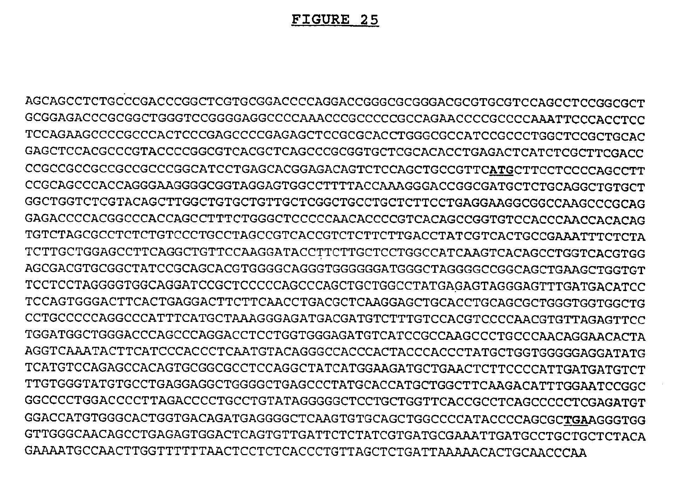

Figure 25 shows the nucleotide sequence (SEQ ID NO:34) of a cDNA containing a nucleotide sequence encoding native sequence PRO4344, wherein the nucleotide sequence (SEQ ID NO:34) is a clone designated herein as DNA84927-2585. Also presented in bold font and underlined are the positions of the respective start and stop codons.

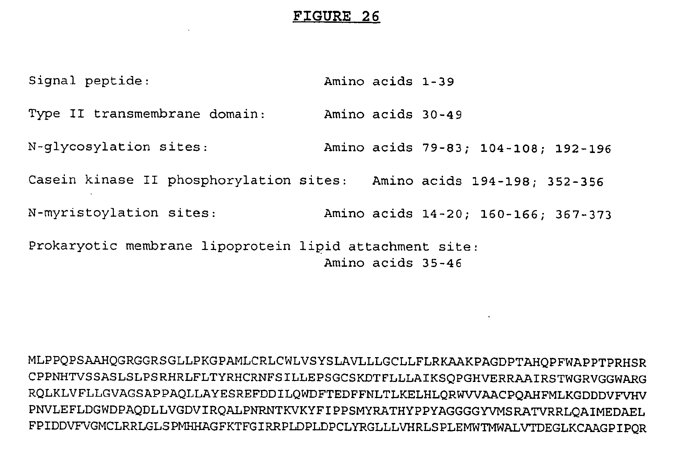

Figure 26 shows the amino acid sequence (SEQ ID NO:35) of a native sequence PRO4344 polypeptide as derived from the coding sequence of SEQ ID NO:34 shown in Figure 25.

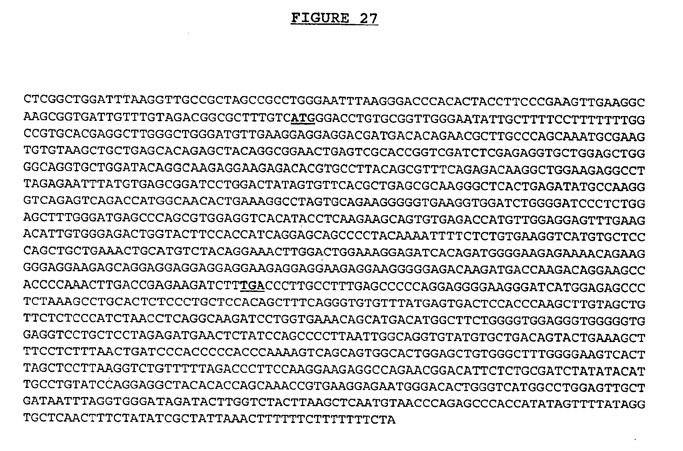

Figure 27 shows the nucleotide sequence (SEQ ID NO:39) of a cDNA containing a nucleotide sequence encoding native sequence PRO4354, wherein the nucleotide sequence (SEQ ID NO:39) is a clone designated herein as DNA92256-2596. Also presented in bold font and underlined are the positions of the respective start and stop codons.

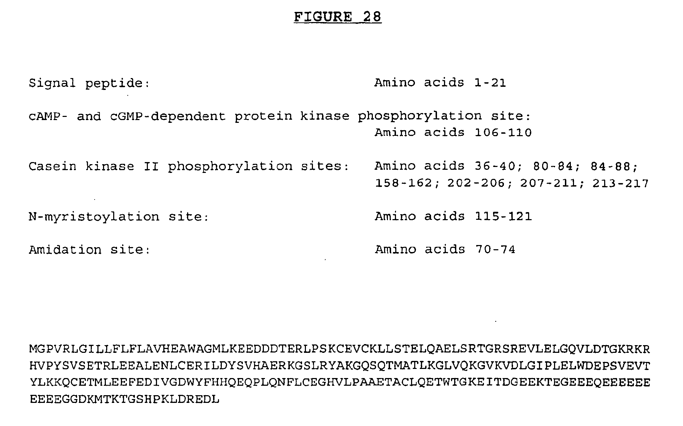

Figure 28 shows the amino acid sequence (SEQ ID NO:40) of a native sequence PRO4354 polypeptide as derived from the coding sequence of SEQ ID NO:39 shown in Figure 27.

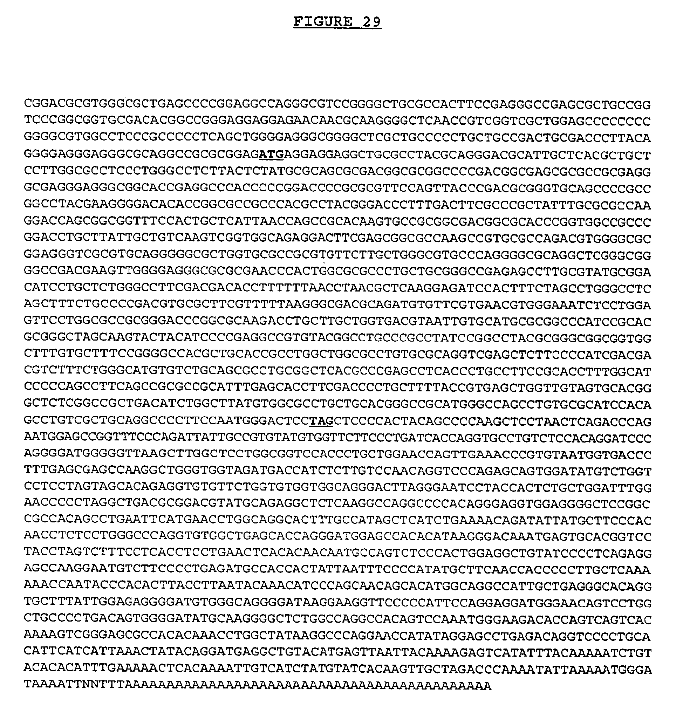

Figure 29 shows the nucleotide sequence (SEQ ID NO:41) of a cDNA containing a nucleotide sequence encoding native sequence PRO4397, wherein the nucleotide sequence (SEQ ID NO:41) is a clone designated herein as DNA83505-2606. Also presented in bold font and underlined are the positions of the respective start and stop codons.

Figure 30 shows the amino acid sequence (SEQ ID NO:42) of a native sequence PRO4397 polypeptide as derived from the coding sequence of SEQ ID NO:41 shown in Figure 29.

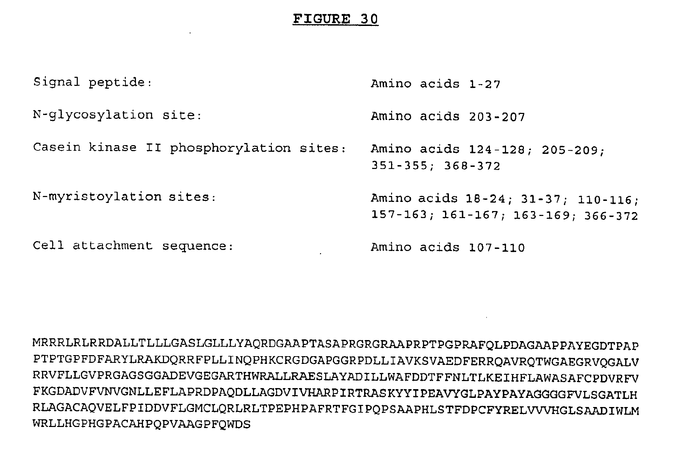

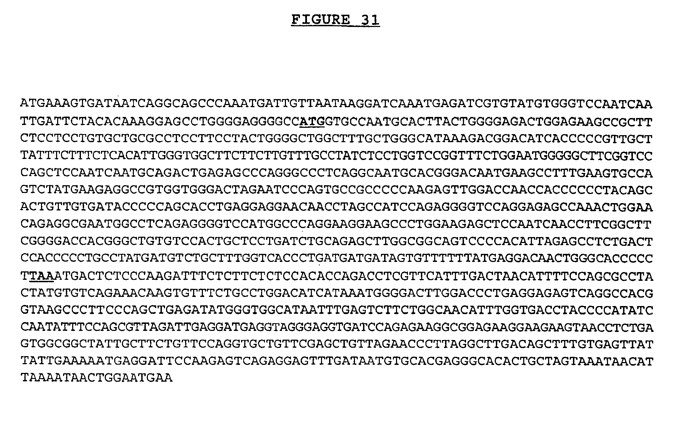

Figure 31 shows the nucleotide sequence (SEQ ID NO:46) of a cDNA containing a nucleotide sequence encoding native sequence PRO4407, wherein the nucleotide sequence (SEQ ID NO:46) is a clone designated herein as DNA92264-2616. Also presented in bold font and underlined are the positions of the respective start and stop codons.

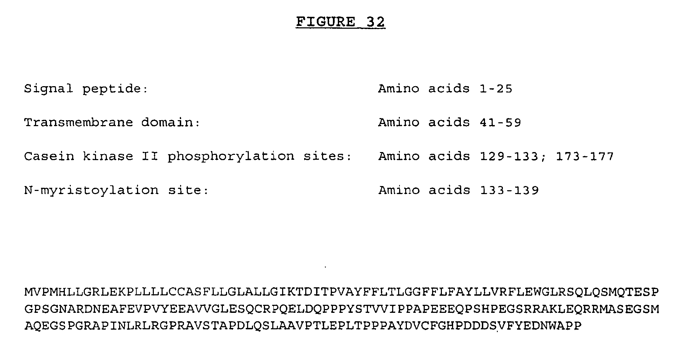

Figure 32 shows the amino acid sequence (SEQ ID NO:47) of a native sequence PRO4407 polypeptide as derived from the coding sequence of SEQ ID NO:46 shown in Figure 31.

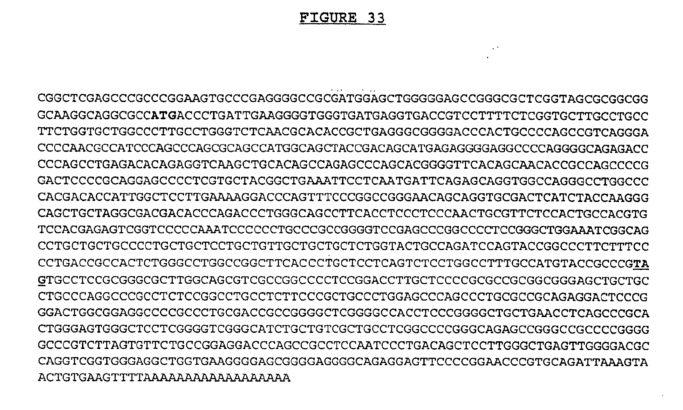

Figure 33 shows the nucleotide sequence (SEQ ID NO:48) of a cDNA containing a nucleotide sequence encoding native sequence PRO1555, wherein the nucleotide sequence (SEQ ID NO:48) is a clone designated herein as DNA73744-1665. Also presented in bold font and underlined are the positions of the respective start and stop codons.

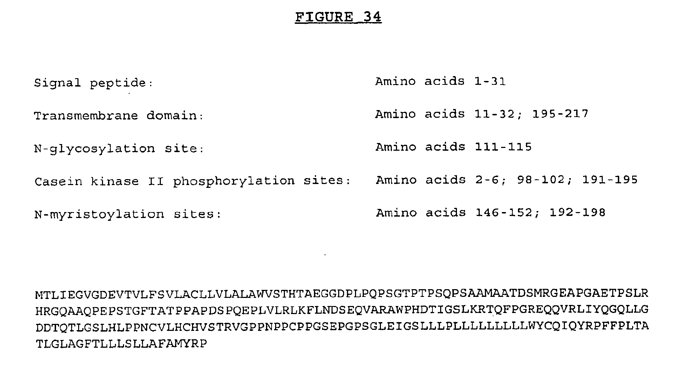

Figure 34 shows the amino acid sequence (SEQ ID NO:49) of a native sequence PRO1555 polypeptide as derived from the coding sequence of SEQ ID NO:48 shown in Figure 33.

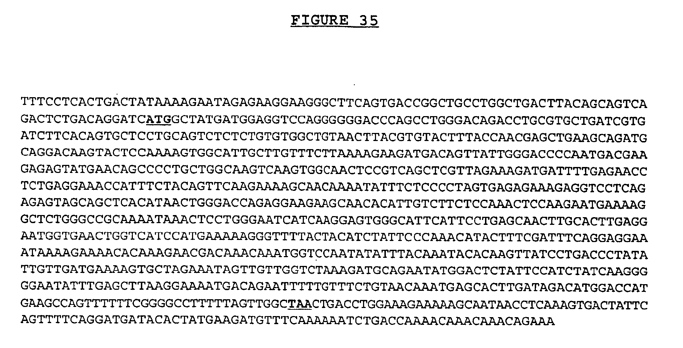

Figure 35 shows the nucleotide sequence (SEQ ID NO:50) of a cDNA containing a nucleotide sequence encoding native sequence PRO1096, wherein the nucleotide sequence (SEQ ID NO:50) is a clone designated herein as DNA61870. Also presented in bold font and underlined are the positions of the respective start and stop codons.

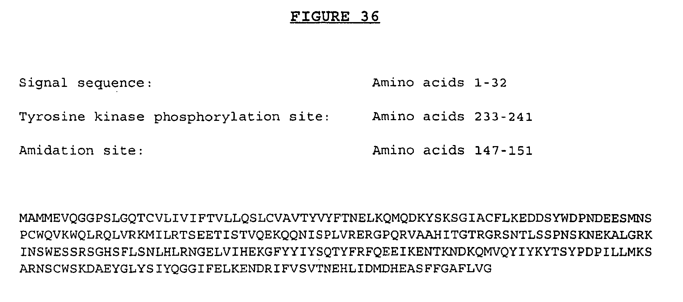

Figure 36 shows the amino acid sequence (SEQ ID NO:51) of a native sequence PRO1096 polypeptide as derived from the coding sequence of SEQ ID NO:50 shown in Figure 35.

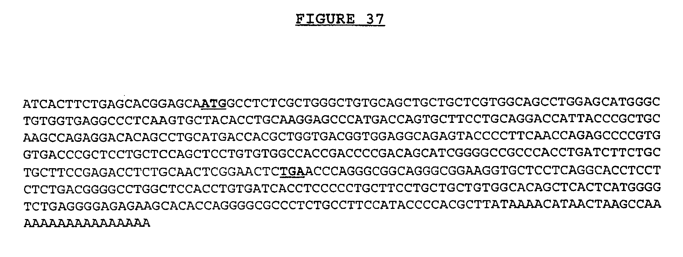

Figure 37 shows the nucleotide sequence (SEQ ID NO:52) of a cDNA containing a nucleotide sequence encoding native sequence PRO2038, wherein the nucleotide sequence (SEQ ID NO:52) is a clone designated herein as DNA83014. Also presented in bold font and underlined are the positions of the respective start and stop codons.

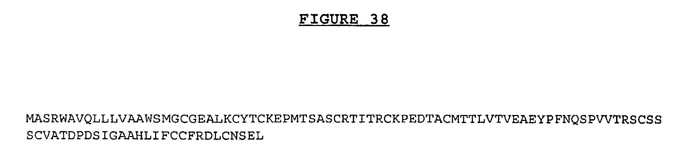

Figure 38 shows the amino acid sequence (SEQ ID NO:53) of a native sequence PRO2038 polypeptide as derived from the coding sequence of SEQ ID NO:52 shown in Figure 37.

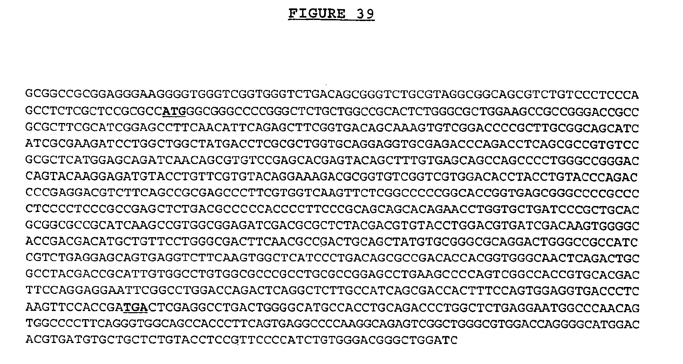

Figure 39 shows the nucleotide sequence (SEQ ID NO:54) of a cDNA containing a nucleotide sequence encoding native sequence PRO2262, wherein the nucleotide sequence (SEQ ID NO:54) is a clone designated herein as DNA88273. Also presented in bold font and underlined are the positions of the respective start and stop codons.

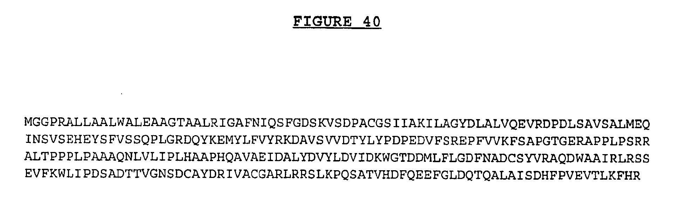

Figure 40 shows the amino acid sequence (SEQ ID NO:55) of a native sequence PRO2262 polypeptide as derived from the coding sequence of SEQ ID NO:54 shown in Figure 39.

## Detailed Description of the Invention

### I. Definitions

The phrases "gene amplification" and "gene duplication" are used interchangeably and refer to a process by which multiple copies of a gene or gene fragment are formed in a particular cell or cell line. The duplicated region (a stretch of amplified DNA) is often referred to as "amplicon." Usually, the amount ofthe messenger RNA (mRNA) produced, *i.e.,* the level of gene expression, also increases in the proportion ofthe number of copies made of the particular gene expressed.

"Tumor", as used herein, refers to all neoplastic cell growth and proliferation,whether malignant or benign, and all pre-cancerous and cancerous cells and tissues.

The terms "cancer" and "cancerous" refer to or describe the physiological.condition in mammals that is typically characterized by unregulated cell growth. Examples of cancer include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, liver cancer, vulval cancer, thyroid cancer, hepatic carcinoma and various types of head and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor (*e.g.*, cancer) treatment, a therapeutic agent may directly decrease the pathology of tumor cells, or render the tumor cells more susceptible to treatment by other therapeutic agents, *e.g.*, radiation and/or chemotherapy.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

"Mammal" for purposes of treatment refers to any animal classified as a mammal, including humans, domestic and farm animals, and zoo, sports, or pet animals, such as dogs, horses, cats, cattle, pigs, sheep, etc. Preferably, the mammal is human.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

Administration "in combination with" one or more further therapeutic agents includes simultaneous (concurrent) and consecutive administration in any order.

The term "cytotoxic agent" as used herein refers to a substance that inhibits or prevents the function of cells and/or causes destruction of cells. The term is intended to include radioactive isotopes (*e.g.*, $I^{131}$, $I^{125}$, $Y^{90}$ and $Re^{186}$), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g.*, paclitaxel (Taxol, Bristol-Myers Squibb Oncology,Princeton,NJ), and doxetaxel (Taxotere, Rhone-Poulenc Rorer, Antony, Rnace), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), 5-FU, 6-thioguanine, 6-mercaptopurine, actinomycin D, VP-16, chlorambucil, melphalan, and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo If inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami *et al.,* (WB Saunders: Philadelphia, 1995), especially p. 13.

"Doxorubicin" is an anthracycline antibiotic. The full chemical name of doxorubicin is (8S-cis)-10-[(3-amino-2,3,6-trideoxy- α-L-lyxo-hexapyranosyl) oxy]- 7,8,9,10-tetrahydro- 6,8,11-trihydroxy- 8-(hydroxyacetyl)- 1-methoxy-5,12-naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon -α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL- 1a, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy", Biochemical Society Transactions, 14:375-382, 615th Meeting, Belfast (1986), and Stella *et al.*, "Prodrugs: A Chemical Approach to Targeted Drug Delivery", Directed Drug Delivery, Borchardt *et al.*, (ed.), pp. 147-267, Humana Press (1985). The prodrugs of this invention include, but are not limited to, phosphate-containing prodrugs, thiophosphate-containing prodrugs, sulfate-containing prodrugs, peptide-containingprodrugs, D-amino acid-modified prodrugs, glysocylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of evtotoxic drugs that can be derivatized into a prodrugs form for use in this invention include, but are not limited to, those chemotherapeutic agents described above.

An "effective amount" of a polypeptide disclosed herein or an antagonist thereof, in reference to inhibition of neoplastic cell growth, tumor growth or cancer cell growth, is an amount capable of inhibiting, to some extent, the growth of target cells. The term includes an amount capable of invoking a growth inhibitory, cytostatic and/or cytotoxic effect and/or apoptosis of the target cells. An "effective amount" of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide antagonist for purposes of inhibiting neoplastic cell growth, tumor growth or cancer cell growth, may be determined empirically and in a routine manner.

A "therapeutically effective amount", in reference to the treatment of tumor, refers to an amount capable of invoking one or more of the following effects: (1) inhibition, to some extent, of tumor growth, including, slowing down and complete growth arrest; (2) reduction in the number of tumor cells; (3) reduction in tumor size; (4) inhibition (*i.e.*, reduction, slowing down or complete stopping) of tumor cell infiltration into peripheral organs; (5) inhibition (*i.e.*, reduction, slowing down or complete stopping) of metastasis; (6) enhancement of anti-tumor immune response, which may, but does not have to, result in the regression or rejection of the tumor; and/or (7) relief, to some extent, of one or more symptoms associated with the disorder. A "therapeutically effective amount" of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide antagonist for purposes of treatment of tumor may be determined empirically and in a routine manner.

A "growth inhibitory amount" of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 antagonist is an amount capable of inhibiting the growth of a a cell, especially tumor, e.g., cancer cell, either *in vitro* or *in vivo.* A "growth inhibitory amount" of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 antagonist for purposes of inhibiting neoplastic cell growth may be determined empirically and in a routine manner.

A "cytotoxic amount" of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 antagonist is an amount capable of causing the destruction of a cell, especially tumor, *e.g.*, cancer cell, either *in vitro* or *in vivo.* A "cytotoxic amount" of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397,PRO4407,PRO 1555, PRO 1096, PRO2038 or PRO2262 antagonist for purposes of inhibiting neoplastic cell growth may be determined empirically and in a routine manner.

The terms "PRO381", "PRO1269", "PRO1410", "PRO1755", "PRO1780", "PRO1788", "PRO3434", "PRO1927", "PRO3567", "PRO1295", "PRO1293", "PRO1303", "PRO4344", "PRO4354", "PRO4397", "PRO4407", "PRO1555", "PRO1096", "PRO2038" or " PRO2262" polypeptide or protein when used herein encompassnative sequence PRO381, PRO1269, PRO1410, PRO1755, PRO1780, PRO1788. PRO3434,PRO1927, PRO3567, PRO1295, PRO1293, PRO1303, PRO4344, PRO4354, PRO4397, PRO4407, PRO1555, PRO1096, PRO2038 or PRO2262 polypeptides and PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide variants (which are further defined herein). The PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide may be isolated from a variety of sources, such as from human tissue types or from another source, or prepared by recombinant and/or synthetic methods.

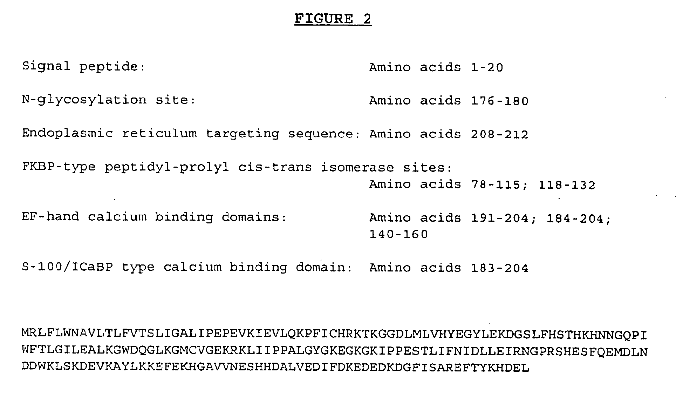

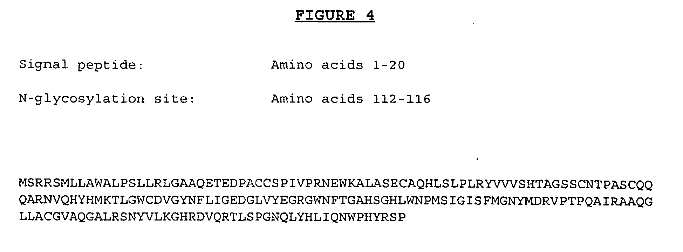

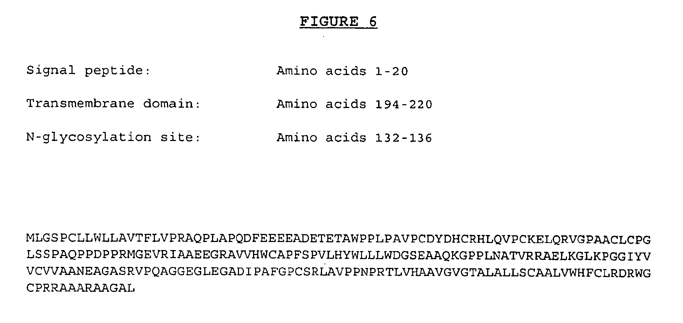

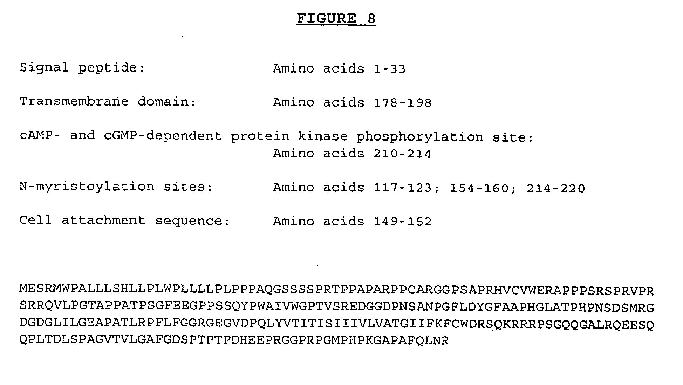

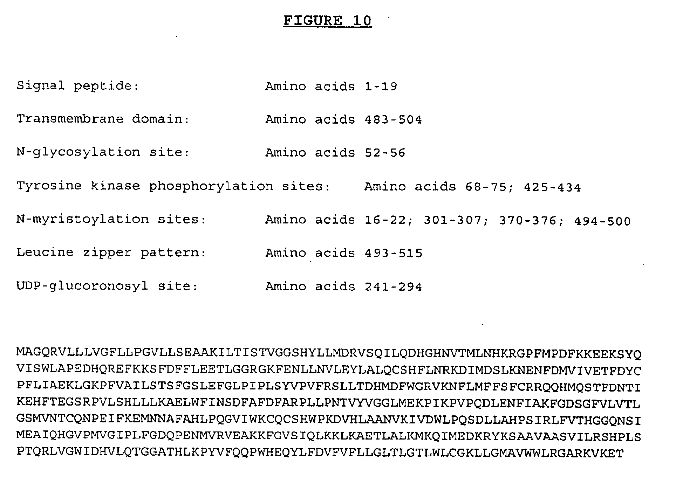

A "nativesequencePRO381 ","native sequence PRO1269", "native sequence PRO1410", "nativesequence PRO1755", "native sequence PRO1780", "native sequence PRO1788", "native sequence PRO3434", "native sequence PRO1927", "native sequence PRO3567", "native sequence PRO1295", "native sequence PRO1293", "native sequence PRO1303", "native sequence PRO4344", "native sequence PRO4354", "native sequence PRO4397", "native sequence PRO4407", "native sequence PRO1555", "native sequence PRO1096", "native sequence PRO203 8" or "native sequence PRO2262" comprises a polypeptide having the same amino acid sequence as the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide as derived from nature. Such native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide can be isolated from nature or can be produced by recombinant and/or synthetic means. The term "native sequence" PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO 1555, PRO1096, PRO2038 or PRO2262 specifically encompasses naturally-occurring truncated or secreted forms (*e.g.*, an extracellular domain sequence), naturally-occurring variant forms (*e.g.*, alternatively spliced forms) and naturally-occurring allelic variants of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptides. In one embodiment of the invention, the native sequence PRO381, PRO1269, PRO1410, PRO1755, PRO 1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is a mature or full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide as shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), respectively. Also, while the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptides disclosed in Figure 2 (SEQ ID NO:2), Figure 4 (SEQ ID NO:7), Figure 6 (SEQ ID NO:9), Figure 8 (SEQ ID NO: 11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO: 31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), respectively, are shown to begin with the methionine residue designated therein as amino acid position 1, it is conceivable and possible that another methionine residue located either upstream or downstream from amino acid position 1 in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), respectively, may be employed as the starting amino acid residue for the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

The "extracellular domain" or "ECD" of a polypeptide disclosed herein refers to a form of the polypeptide which is essentially free of the transmembrane and cytoplasmic domains. Ordinarily, a polypeptide ECD will have less than about 1% of such transmembrane and/or cytoplasmic domains and preferably, will have less than about 0.5% of such domains. It will be understood that any transmembrane domain(s) identified for the polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified and as shown in the appended figures. As such, in one embodiment of the present invention, the extracellular domain of a polypeptide of the present invention comprises amino acids 1 to X of the mature amino acid sequence, wherein X is any amino acid within 5 amino acids on either side of the extracellular domain/transmembrane domain boundary.

The approximate location of the "signal peptides" of the various PRO polypeptides disclosed herein are shown in the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (*e.g.*, Nielsen *et al.*, Prot. Eng., 10:1-6 (1997) and von Heinje *et al.*, Nucl. Acids. Res., 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO381 polypeptide variant", "PRO1269 polypeptide variant", "PRO1410 polypeptide variant", "PRO1755 polypeptide variant", "PRO1780 polypeptide variant", "PRO1788 polypeptide variant", "PRO3434 polypeptide variant", "PRO1927 polypeptide variant", "PRO3567 polypeptide variant", "PRO1295 polypeptide variant", ''PRO1293 polypeptide variant", "PRO1303 polypeptide variant", "PRO4344 polypeptide variant", "PRO4354 polypeptide variant", "PRO4397 polypeptide variant", "PRO4407 polypeptide variant", "PRO1555 polypeptide variant", "PRO1096 polypeptide variant", "PRO2038 polypeptide variant" or "PRO2262 polypeptide variant" means an active PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide as defined above or below having at least about 80% amino acid sequence identity with a full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide sequence as disclosed herein, a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide sequence lacking the signal peptide as disclosed herein, an extracellular domain of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, with or without the signal peptide, as disclosed herein or any other fragment of a full-length PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide sequence as disclosed herein. Such PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide variants include, for instance, PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptides wherein one or more amino acid residues are added, or deleted, at the N- or C-terminus of the full-length native amino acid sequence. Ordinarily, a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide variant will have at least about 80% amino acid sequence identity, preferably at least about 81 % amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and most preferably at least about 99% amino acid sequence identity with a full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide sequence as disclosed herein, a PRO381, PRO1269, PRO1410, PRO1755, PRO1780, PRO1788, PRO3434, PRO927, PRO3567, PRO1295, PRO1293, PRO303, PRO4344, PRO4354, PRO4397, PRO4407, PRO1555, PRO1096, PRO2038 or PRO2262 polypeptide sequence lacking the signal peptide as disclosed herein, an extracellular domain of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, with or without the signal peptide, as disclosed herein or any other fragment of a full-length PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide sequence as disclosed herein. Ordinarily, PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 300 amino acids in length, or more.

As shown below, Table 1 provides the complete source code for the ALIGN-2 sequence comparison computer program. This source code may be routinely compiled for use on a UNIX operating system to provide the ALIGN-2 sequence comparison computer program.

In addition, Tables 2A-2B show hypothetical exemplifications for using the below described method to determine % amino acid sequence identity (Tables 2A-2B) and % nucleic acid sequence identity (Tables 2C-2D) using the ALIGN-2 sequence comparison computer program, wherein "PRO" represents the amino acid sequence of a hypothetical PRO381, PRO1269, PRO1410, PRO1755, PRO1780,PRO1788,PRO3434,PRO1927,PRO3567, PRO1295, PRO1293, PRO1303, PRO4344, PRO4354, PRO4397, PRO4407, PRO1555, PRO1096, PRO2038 or PRO2262 polypeptide of interest, "Comparison Protein" represents the amino acid sequence of a polypeptide against which the "PRO" polypeptide of interest is being compared, "PRO-DNA" represents a hypothetical PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding nucleic acid sequence of interest, "Comparison DNA" represents the nucleotide sequence of a nucleic acid molecule against which the "PRO-DNA" nucleic acid molecule of interest is being compared, "X", "Y", and "Z" each represent different hypothetical amino acid residues and "N", "L" and "V" each represent different hypothetical nucleotides.

## Table 1

```
/*
 *
 * C-C increased from 12 to 15
 * Z is average of EQ
 * B is average of ND
 * match with stop is _M; stop-stop = 0; J (joker) match = 0
 */
#define   _M        -8          /* value of a match with a stop */

int       _day[26][26] = {
/*      A  B  C  D  E  F  G  H  I  J  K  L  M  N  O  P  Q  R  S  T  U  V  W  X  Y  Z */
/* A */   { 2, 0,-2, 0, 0,-4, 1,-1,-1, 0,-1,-2,-1, 0,_M, 1, 0,-2, 1, 1, 0, 0,-6, 0,-3, 0},
/* B */   { 0, 3,-4, 3, 2,-5, 0, 1,-2, 0, 0,-3,-2, 2,_M,-1, 1, 0, 0, 0, 0,-2,-5, 0,-3, 1},
/* C */   {-2,-4,15,-5,-5,-4,-3,-3,-2, 0,-5,-6,-5,-4,_M,-3,-5,-4, 0,-2, 0,-2,-8, 0, 0,-5},
/* D */   { 0, 3,-5, 4, 3,-6, 1, 1,-2, 0, 0,-4,-3, 2,_M,-1, 2,-1, 0, 0, 0,-2,-7, 0,-4, 2},
/* E */   { 0, 2,-5, 3, 4,-5, 0, 1,-2, 0, 0,-3,-2, 1,_M,-1, 2,-1, 0, 0, 0,-2,-7, 0,-4, 3},
/* F */   {-4,-5,-4,-6,-5, 9,-5,-2, 1, 0,-5, 2, 0,-4,_M,-5,-5,-4,-3,-3, 0,-1, 0, 0, 7,-5},
/* G */   { 1, 0,-3, 1, 0,-5, 5,-2,-3, 0,-2,-4,-3, 0,_M,-1,-1,-3, 1, 0, 0,-1,-7, 0,-5, 0},
/* H */   {-1, 1,-3, 1, 1,-2,-2, 6,-2, 0, 0,-2,-2, 2,_M, 0, 3, 2,-1,-1, 0,-2,-3, 0, 0, 2},
/* I */   {-1,-2,-2,-2,-2, 1,-3,-2, 5, 0,-2, 2, 2,-2,_M,-2,-2,-2,-1, 0, 0, 4,-5, 0,-1,-2},
/* J */   { 0, 0, 0, 0, 0, 0, 0, 0, 0, 0, 0, 0, 0, 0,_M, 0, 0, 0, 0, 0, 0, 0, 0, 0, 0, 0},
/* K */   {-1, 0,-5, 0, 0,-5,-2, 0,-2, 0, 5,-3, 0, 1,_M,-1, 1, 3, 0, 0, 0,-2,-3, 0,-4, 0},
/* L */   {-2,-3,-6,-4,-3, 2,-4,-2, 2, 0,-3, 6, 4,-3,_M,-3,-2,-3,-3,-1, 0, 2,-2, 0,-1,-2},
/* M */   {-1,-2,-5,-3,-2, 0,-3,-2, 2, 0, 0, 4, 6,-2,_M,-2,-1, 0,-2,-1, 0, 2,-4, 0,-2,-1},
/* N */   { 0, 2,-4, 2, 1,-4, 0, 2,-2, 0, 1,-3,-2, 2,_M,-1, 1, 0, 1, 0, 0,-2,-4, 0,-2, 1},
/* O */   {_M,_M,_M,_M,_M,_M,_M,_M,_M,_M,_M,_M,_M,_M, 0,_M,_M,_M,_M,_M,_M,_M,_M,_M,_M,_M},
/* P */   { 1,-1,-3,-1,-1,-5,-1, 0,-2, 0,-1,-3,-2,-1,_M, 6, 0, 0, 1, 0, 0,-1,-6, 0,-5, 0},
/* Q */   { 0, 1,-5, 2, 2,-5,-1, 3,-2, 0, 1,-2,-1, 1,_M, 0, 4, 1,-1,-1, 0,-2,-5, 0,-4, 3},
/* R */   {-2, 0,-4,-1,-1,-4,-3, 2,-2, 0, 3,-3, 0, 0,_M, 0, 1, 6, 0,-1, 0,-2, 2, 0,-4, 0},
/* S */   { 1, 0, 0, 0, 0,-3, 1,-1,-1, 0, 0,-3,-2, 1,_M, 1,-1, 0, 2, 1, 0,-1,-2, 0,-3, 0},
/* T */   { 1, 0,-2, 0, 0,-3, 0,-1, 0, 0, 0,-1,-1, 0,_M, 0,-1,-1, 1, 3, 0, 0,-5, 0,-3, 0},
/* U */   { 0, 0, 0, 0, 0, 0, 0, 0, 0, 0, 0, 0, 0, 0,_M, 0, 0, 0, 0, 0, 0, 0, 0, 0, 0, 0},
/* V */   { 0,-2,-2,-2,-2,-1,-1,-2, 4, 0,-2, 2, 2,-2,_M,-1,-2,-2,-1, 0, 0, 4,-6, 0,-2,-2},
/* W */   {-6,-5,-8,-7,-7, 0,-7,-3,-5, 0,-3,-2,-4,-4,_M,-6,-5, 2,-2,-5, 0,-6,17, 0, 0,-6},
/* X */   { 0, 0, 0, 0, 0, 0, 0, 0, 0, 0, 0, 0, 0, 0,_M, 0, 0, 0, 0, 0, 0, 0, 0, 0, 0, 0},
/* Y */   {-3,-3, 0,-4,-4, 7,-5, 0,-1, 0,-4,-1,-2,-2,_M,-5,-4,-4,-3,-3, 0,-2, 0, 0,10,-4},
/* Z */   { 0, 1,-5, 2, 3,-5, 0, 2,-2, 0, 0,-2,-1, 1,_M, 0, 3, 0, 0, 0, 0,-2,-6, 0,-4, 4}
};
```

```
/*
 */
#include <stdio.h>
#include <ctype.h>

#define  MAXJMP      16      /* max jumps in a diag */
#define  MAXGAP      24      /* don't continue to penalize gaps larger than this */
#define  JMPS        1024    /* max jmps in an path */
#define  MX          4       /* save if there's at least MX-1 bases since last jmp */

#define  DMAT        3       /* value of matching bases */
#define  DMIS        0       /* penalty for mismatched bases */
#define  DINS0       8       /* penalty for a gap */
#define  DINS1       1       /* penalty per base */
#define  PINS0       8       /* penalty for a gap */
#define  PINS1       4       /* penalty per residue */

struct jmp {
        short            n[MAXJMP];    /* size of jmp (neg for dely) */
        unsigned short   x[MAXJMP];    /* base no. of jmp in seq x */
};                                     /* limits seq to 2^16 -1 */

struct diag {
        int              score;        /* score at last jmp */
        long             offset;       /* offset of prev block */
        short            ijmp;         /* current jmp index */
        struct jmp       jp;           /* list of jmps */
};

struct path {
        int     spc;            /* number of leading spaces */
        short   n[JMPS];/* size of jmp (gap) */
        int     x[JMPS];/* loc of jmp (last elem before gap) */
};

char            *ofile;                 /* output file name */
char            *namex[2];              /* seq names: getseqs() */
char            *prog;                  /* prog name for err msgs */
char            *seqx[2];               /* seqs: getseqs() */
int             dmax;                   /* best diag: nw() */
int             dmax0;                  /* final diag */
int             dna;                    /* set if dna: main() */
int             endgaps;                /* set if penalizing end gaps */
int             gapx, gapy;             /* total gaps in seqs */
int             len0, len1;             /* seq lens */
int             ngapx, ngapy;           /* total size of gaps */
int             smax;                   /* max score: nw() */
int             *xbm;                   /* bitmap for matching */
long            offset;                 /* current offset in jmp file */
struct  diag    *dx;                    /* holds diagonals */
struct  path    pp[2];                  /* holds path for seqs */

char            *calloc(), *malloc(), *index(), *strcpy();
char            *getseq(), *g_calloc();
```

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"

static    _dbval[26] = {
          1,14,2,13,0,0,4,11,0,0,12,0,3,15,0,0,0,5,6,8,8,7,9,0,10,0
};

static    _pbval[26] = {
          1, 2|(1<<('D'-'A'))|(1<<('N'-'A')), 4, 8, 16, 32, 64,
          128, 256, 0xFFFFFFF, 1<<10, 1<<11, 1<<12, 1<<13, 1<<14,
          1<<15, 1<<16, 1<<17, 1<<18, 1<<19, 1<<20, 1<<21, 1<<22,
          1<<23, 1<<24, 1<<25|(1<<('E'-'A'))|(1<<('Q'-'A'))
};

main(ac, av)                                                              main
          int       ac;
          char      *av[];
{
          prog = av[0];
          if (ac != 3) {
                    fprintf(stderr,"usage: %s file1 file2\n", prog);
                    fprintf(stderr,"where file1 and file2 are two dna or two protein sequences.\n");
                    fprintf(stderr,"The sequences can be in upper- or lower-case\n");
                    fprintf(stderr,"Any lines beginning with ';' or '<' are ignored\n");
                    fprintf(stderr,"Output is in the file \"align.out\"\n");
                    exit(1);
          }
          namex[0] = av[1];
          namex[1] = av[2];
          seqx[0] = getseq(namex[0], &len0);
          seqx[1] = getseq(namex[1], &len1);
          xbm = (dna)? _dbval : _pbval;

          endgaps = 0;                      /* 1 to penalize endgaps */
          ofile = "align.out";              /* output file */

          nw();             /* fill in the matrix, get the possible jmps */
          readjmps();       /* get the actual jmps */
          print();          /* print stats, alignment */

          cleanup(0);       /* unlink any tmp files */
}
```

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                              nw
{
        char            *px, *py;           /* seqs and ptrs */
        int             *ndely, *dely;      /* keep track of dely */
        int             ndelx, delx;        /* keep track of delx */
        int             *tmp;               /* for swapping row0, row1 */
        int             mis;                /* score for each type */
        int             ins0, ins1;         /* insertion penalties */
        register        id;                 /* diagonal index */
        register        ij;                 /* jmp index */
        register        *col0, *col1;       /* score for curr, last row */
        register        xx, yy;             /* index into seqs */

        dx = (struct diag *)g_calloc("to get diags", len0+len1+1, sizeof(struct diag));

        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;

        smax = -10000;
        if (endgaps) {
                for (col0[0] = dely[0] = -ins0, yy = 1; yy <= len1; yy++) {
                        col0[yy] = dely[yy] = col0[yy-1] - ins1;
                        ndely[yy] = yy;
                }
                col0[0] = 0;        /* Waterman Bull Math Biol 84 */
        }
        else
                for (yy = 1; yy <= len1; yy++)
                        dely[yy] = -ins0;

        /* fill in match matrix
         */
        for (px = seqx[0], xx = 1; xx <= len0; px++, xx++) {
                /* initialize first entry in col
                 */
                if (endgaps) {
                        if (xx == 1)
                                col1[0] = delx = -(ins0+ins1);
                        else
                                col1[0] = delx = col0[0] - ins1;
                        ndelx = xx;
                }
                else {
                        col1[0] = 0;
                        delx = -ins0;
                        ndelx = 0;
                }
```

...nw

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

...nw

```
                    id = xx - yy + len1 - 1;
                    if (mis >= delx && mis >= dely[yy])
                            col1[yy] = mis;
                    else if (delx >= dely[yy]) {
                            col1[yy] = delx;
                            ij = dx[id].ijmp;
                            if (dx[id].jp.n[0] && (!dna || (ndelx >= MAXJMP
                            && xx > dx[id].jp.x[ij]+MX) || mis > dx[id].score+DINS0)) {
                                    dx[id].ijmp++;
                                    if (++ij >= MAXJMP) {
                                            writejmps(id);
                                            ij = dx[id].ijmp = 0;
                                            dx[id].offset = offset;
                                            offset += sizeof(struct jmp) + sizeof(offset);
                                    }
                            }
                            dx[id].jp.n[ij] = ndelx;
                            dx[id].jp.x[ij] = xx;
                            dx[id].score = delx;
                    }
                    else {
                            col1[yy] = dely[yy];
                            ij = dx[id].ijmp;

    if (dx[id].jp.n[0] && (!dna || (ndely[yy] >= MAXJMP
                            && xx > dx[id].jp.x[ij]+MX) || mis > dx[id].score+DINS0)) {
                                    dx[id].ijmp++;
                                    if (++ij >= MAXJMP) {
                                            writejmps(id);
                                            ij = dx[id].ijmp = 0;
                                            dx[id].offset = offset;
                                            offset += sizeof(struct jmp) + sizeof(offset);
                                    }
                            }
                            dx[id].jp.n[ij] = -ndely[yy];
                            dx[id].jp.x[ij] = xx;
                            dx[id].score = dely[yy];
                    }
                    if (xx == len0 && yy < len1) {
                            /* last col
                             */
                            if (endgaps)
                                    col1[yy] -= ins0+ins1*(len1-yy);
                            if (col1[yy] > smax) {
                                    smax = col1[yy];
                                    dmax = id;
                            }
                    }
            }
            if (endgaps && xx < len0)
                    col1[yy-1] -= ins0+ins1*(len0-xx);
            if (col1[yy-1] > smax) {
                    smax = col1[yy-1];
                    dmax = id;
            }
            tmp = col0; col0 = col1; col1 = tmp;
    }
    (void) free((char *)ndely);
    (void) free((char *)dely);
    (void) free((char *)col0);
    (void) free((char *)col1);
}
```

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC      3
#define P_LINE   256      /* maximum output line */
#define P_SPC    3        /* space between name or num and seq */

extern   _day[26][26];
int      olen;            /* set output line length */
FILE     *fx;             /* output file */

print()                                                              print
{
        int     lx, ly, firstgap, lastgap;      /* overlap */

        if ((fx = fopen(ofile, "w")) == 0) {
                fprintf(stderr,"%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {          /* leading gap in x */
                pp[0].spc = firstgap = len1 - dmax - 1;
                ly -= pp[0].spc;
        }
        else if (dmax > len1 - 1) {   /* leading gap in y */
                pp[1].spc = firstgap = dmax - (len1 - 1);
                lx -= pp[1].spc;
        }
        if (dmax0 < len0 - 1) {         /* trailing gap in x */
                lastgap = len0 - dmax0 -1;
                lx -= lastgap;
        }
        else if (dmax0 > len0 - 1) { /* trailing gap in y */
                lastgap = dmax0 - (len0 - 1);
                ly -= lastgap;
        }
        getmat(lx, ly, firstgap, lastgap);
        pr_align();
}
```

```
/*
 * trace back the best path, count matches
 */
static
getmat(lx, ly, firstgap, lastgap)                                        getmat
        int     lx, ly;                         /* "core" (minus endgaps) */
        int     firstgap, lastgap;              /* leading trailing overlap */
{
        int             nm, i0, i1, siz0, siz1;
        char            outx[32];
        double          pct;
        register        n0, n1;
        register char   *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, "< %d match%s in an overlap of %d: %.2f percent similarity\n",
                nm, (nm == 1)? "" : "es", lx, pct);
```

...getmat

```
        fprintf(fx, "<gaps in first sequence: %d", gapx);
        if (gapx) {
                (void) sprintf(outx, " (%d %s%s)",
                        ngapx, (dna)? "base":"residue", (ngapx == 1)? "":"s");
                fprintf(fx,"%s", outx);

        fprintf(fx, ", gaps in second sequence: %d", gapy);
        if (gapy) {
                (void) sprintf(outx, " (%d %s%s)",
                        ngapy, (dna)? "base":"residue", (ngapy == 1)? "":"s");
                fprintf(fx,"%s", outx);
        }
        if (dna)
                fprintf(fx,
                "\n<score: %d (match = %d, mismatch = %d, gap penalty = %d + %d per base)\n",
                smax, DMAT, DMIS, DINS0, DINS1);
        else
                fprintf(fx,
                "\n<score: %d (Dayhoff PAM 250 matrix, gap penalty = %d + %d per residue)\n",
                smax, PINS0, PINS1);
        if (endgaps)
                fprintf(fx,
                "<endgaps penalized. left endgap: %d %s%s, right endgap: %d %s%s\n",
                firstgap, (dna)? "base" : "residue", (firstgap == 1)? "" : "s",
                lastgap, (dna)? "base" : "residue", (lastgap == 1)? "" : "s");
        else
                fprintf(fx, "<endgaps not penalized\n");
}

static          nm;                 /* matches in core -- for checking */
static          lmax;               /* lengths of stripped file names */
static          ij[2];              /* jmp index for a path */
static          nc[2];              /* number at start of current line */
static          ni[2];              /* current elem number -- for gapping */
static          siz[2];
static char     *ps[2];             /* ptr to current element */
static char     *po[2];             /* ptr to next output char slot */
static char     out[2][P_LINE];     /* output line */
static char     star[P_LINE];       /* set by stars() */

/*
 * print alignment of described in struct path pp[]
 */
static
pr_align()
{
        int             nn;         /* char count */
        int             more;
        register        i;

        for (i = 0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];
        }
```

pr_align

...pr_align

```
        for (nn = nm = 0, more = 1; more; ) {
                for (i = more = 0; i < 2; i++) {
                        /*
                         * do we have more of this sequence?
                         */
                        if (!*ps[i])
                                continue;

                        more++;

                        if (pp[i].spc) {        /* leading space */
                                *po[i]++ = ' ';
                                pp[i].spc--;
                        }
                        else if (siz[i]) {      /* in a gap */
                                *po[i]++ = '-';
                                siz[i]--;
                        }
                        else {                  /* we're putting a seq element
                                                 */
                                *po[i] = *ps[i];
                                if (islower(*ps[i]))
                                        *ps[i] = toupper(*ps[i]);
                                po[i]++;
                                ps[i]++;

                                /*
                                 * are we at next gap for this seq?
                                 */
                                if (ni[i] == pp[i].x[ij[i]]) {
                                        /*
                                         * we need to merge all gaps
                                         * at this location
                                         */
                                        siz[i] = pp[i].n[ij[i]++];
                                        while (ni[i] == pp[i].x[ij[i]])
                                                siz[i] += pp[i].n[ij[i]++];
                                }
                                ni[i]++;
                        }
                }
                if (++nn == olen || !more && nn) {
                        dumpblock();
                        for (i = 0; i < 2; i++)
                                po[i] = out[i];
                        nn = 0;
                }
        }
}

/*
 * dump a block of lines, including numbers, stars: pr_align()
 */
static
dumpblock()
{
        register  i;

        for (i = 0; i < 2; i++)
                *po[i]-- = '\0';
```

dumpblock

...dumpblock

```
		(void) putc('\n', fx);
		for (i = 0; i < 2; i++) {
			if (*out[i] && (*out[i] != ' ' || *(po[i]) != ' ')) {
				if (i == 0)
					nums(i);
				if (i == 0 && *out[1])
					stars();
				putline(i);
				if (i == 0 && *out[1])
					fprintf(fx, star);
				if (i == 1)
					nums(i);
			}
		}
}

/*
 * put out a number line: dumpblock()
 */
static
nums(ix)
		int		ix;		/* index in out[] holding seq line */
{
		char			nline[P_LINE];
		register		i, j;
		register char		*pn, *px, *py;

		for (pn = nline, i = 0; i < lmax+P_SPC; i++, pn++)
			*pn = ' ';
		for (i = nc[ix], py = out[ix]; *py; py++, pn++) {
			if (*py == ' ' || *py == '-')
				*pn = ' ';
			else {
				if (i%10 == 0 || (i == 1 && nc[ix] != 1)) {
					j = (i < 0)? -i : i;
					for (px = pn; j; j /= 10, px--)
						*px = j%10 + '0';
					if (i < 0)
						*px = '-';
				}
				else
					*pn = ' ';
				i++;
			}
		}
		*pn = '\0';
		nc[ix] = i;
		for (pn = nline; *pn; pn++)
			(void) putc(*pn, fx);
		(void) putc('\n', fx);
}

/*
 * put out a line (name, [num], seq, [num]): dumpblock()
 */
static
putline(ix)
		int		ix;
{
```

nums

putline

**...putline**

```
        int                 i;
        register char ,     *px;

        for (px = namex[ix], i = 0; *px && *px != ':'; px++, i++)
                (void) putc(*px, fx);
        for (; i < lmax+P_SPC; i++)
                (void) putc(' ', fx);

        /* these count from 1:
         * ni[] is current element (from 1)
         * nc[] is number at start of current line
         */
        for (px = out[ix]; *px; px++)
                (void) putc(*px&0x7F, fx);
        (void) putc('\n', fx);
}


/*
 * put a line of stars (seqs always in out[0], out[1]): dumpblock()
 */
static
stars()
{
        int                 i;
        register char       *p0, *p1, cx, *px;

        if (!*out[0] || (*out[0] == ' ' && *(po[0]) == ' ') ||
           !*out[1] || (*out[1] == ' ' && *(po[1]) == ' '))
                return;
        px = star;
        for (i = lmax+P_SPC; i; i--)
                *px++ = ' ';

        for (p0 = out[0], p1 = out[1]; *p0 && *p1; p0++, p1++) {
                if (isalpha(*p0) && isalpha(*p1)) {

                        if (xbm[*p0-'A']&xbm[*p1-'A']) {
                                cx = '*';
                                nm++;
                        }
                        else if (!dna && _day[*p0-'A'][*p1-'A'] > 0)
                                cx = '.';
                        else
                                cx = ' ';
                }
                else
                        cx = ' ';
                *px++ = cx;
        }
        *px++ = '\n';
        *px = '\0';
}
```

**stars**

```
/*
 * strip path or prefix from pn, return len: pr_align()
 */
static
stripname(pn)                                                        stripname
        char    *pn;    /* file name (may be path) */
{
        register char   *px, *py;

        py = 0;
        for (px = pn; *px; px++)
                if (*px == '/')
                        py = px + 1;
        if (py)
                (void) strcpy(pn, py);
        return(strlen(pn));

}
```

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char    *jname = "/tmp/homgXXXXXX";         /* tmp file for jmps */
FILE    *fj;

int     cleanup();                          /* cleanup tmp file */
long    lseek();

/*
 * remove any tmp file if we blow
 */
cleanup(i)                                                          cleanup
        int     i;
{
        if (fj)
                (void) unlink(jname);
        exit(i);
}

/*
 * read, return ptr to seq, set dna, len, maxlen
 * skip lines starting with ';', '<', or '>'
 * seq in upper or lower case
 */
char    *
getseq(file, len)                                                   getseq
        char    *file;  /* file name */
        int     *len;   /* seq len */
{
        char            line[1024], *pseq;
        register char   *px, *py;
        int             natgc, tlen;
        FILE            *fp;

        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

...getseq

```
        py = pseq + 4;
        *len = tlen;
        rewind(fp);

        while (fgets(line, 1024, fp)) {
                if (*line == ';' || *line == '<' || *line == '>')
                        continue;
                for (px = line; *px != '\n'; px++) {
                        if (isupper(*px))
                                *py++ = *px;
                        else if (islower(*px))
                                *py++ = toupper(*px);
                        if (index("ATGCU",*(py-1)))
                                natgc++;
                }
        }
        *py++ = '\0';
        *py = '\0';
        (void) fclose(fp);
        dna = natgc > (tlen/3);
        return(pseq+4);
}
```

g_calloc

```
char    *
g_calloc(msg, nx, sz)
        char    *msg;           /* program, calling routine */
        int     nx, sz;         /* number and size of elements */
{
        char            *px, *calloc();

        if ((px = calloc((unsigned)nx, (unsigned)sz)) == 0) {
                if (*msg) {
                        fprintf(stderr, "%s: g_calloc() failed %s (n=%d, sz=%d)\n", prog, msg, nx, sz);
                        exit(1);
                }
        }
        return(px);
}
```

readjmps

```
/*
 * get final jmps from dx[] or tmp file, set pp[], reset dmax: main()
 */
readjmps()
{
        int             fd = -1;
        int             siz, i0, i1;
        register  i, j, xx;

        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;
```

**...readjmps**

```
			if (j < 0 && dx[dmax].offset && fj) {
				(void) lseek(fd, dx[dmax].offset, 0);
				(void) read(fd, (char *)&dx[dmax].jp, sizeof(struct jmp));
				(void) read(fd, (char *)&dx[dmax].offset, sizeof(dx[dmax].offset));
				dx[dmax].ijmp = MAXJMP-1;
			}
			else
				break;
		}
		if (i >= JMPS) {
			fprintf(stderr, "%s: too many gaps in alignment\n", prog);
			cleanup(1);
		}
		if (j >= 0) {
			siz = dx[dmax].jp.n[j];
			xx = dx[dmax].jp.x[j];
			dmax += siz;
			if (siz < 0) {		/* gap in second seq */
				pp[1].n[i1] = -siz;
				xx += siz;

				/* id = xx - yy + len1 - 1
				 */
				pp[1].x[i1] = xx - dmax + len1 - 1;
				gapy++;
				ngapy -= siz;
/* ignore MAXGAP when doing endgaps */
				siz = (-siz < MAXGAP || endgaps)? -siz : MAXGAP;
				i1++;
			}
			else if (siz > 0) { /* gap in first seq */
				pp[0].n[i0] = siz;
				pp[0].x[i0] = xx;
				gapx++;
				ngapx += siz;
/* ignore MAXGAP when doing endgaps */
				siz = (siz < MAXGAP || endgaps)? siz : MAXGAP;
				i0++;
			}
		}
		else
			break;
	}

	/* reverse the order of jmps
	 */
	for (j = 0, i0--; j < i0; j++, i0--) {
		i = pp[0].n[j]; pp[0].n[j] = pp[0].n[i0]; pp[0].n[i0] = i;
		i = pp[0].x[j]; pp[0].x[j] = pp[0].x[i0]; pp[0].x[i0] = i;
	}
	for (j = 0, i1--; j < i1; j++, i1--) {
		i = pp[1].n[j]; pp[1].n[j] = pp[1].n[i1]; pp[1].n[i1] = i;
		i = pp[1].x[j]; pp[1].x[j] = pp[1].x[i1]; pp[1].x[i1] = i;
	}
	if (fd >= 0)
		(void) close(fd);
	if (fj) {
		(void) unlink(jname);
		fj = 0;
		offset = 0;
	}
}
```

```
/*
 * write a filled jmp struct offset of the prev one (if any): nw()
 */
writejmps(ix)
        int     ix;
{
        char    *mktemp();

        if (!fj) {
                if (mktemp(jname) < 0) {
                        fprintf(stderr, "%s: can't mktemp() %s\n", prog, jname);
                        cleanup(1);
                }
                if ((fj = fopen(jname, "w")) == 0) {
                        fprintf(stderr, "%s: can't write %s\n", prog, jname);
                        exit(1);
                }
        }
        (void) fwrite((char *)&dx[ix].jp, sizeof(struct jmp), 1, fj);
        (void) fwrite((char *)&dx[ix].offset, sizeof(dx[ix].offset), 1, fj);
}
```

**writejmps**

Table 2A

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity =

(the number of identically matching amino acid residues between the two polypeptide sequences as determined by ALIGN-2) divided by (the total number of amino acid residues of the PRO polypeptide) =

5 divided by 15 = 33.3%

Table 2B

| | | |
|---|---|---|
| PRO | XXXXXXXXXX | (Length = 10 amino acids) |
| Comparison Protein | XXXXXYYYYYYZZYZ | (Length = 15 amino acids) |

% amino acid sequence identity =

(the number of identically matching amino acid residues between the two polypeptide sequences as determined by ALIGN-2) divided by (the total number of amino acid residues of the PRO polypeptide) =

5 divided by 10 = 50%

Table 2C

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

6 divided by 14 = 42.9%

Table 2D

| | | |
|---|---|---|
| PRO-DNA | NNNNNNNNNNNN | (Length = 12 nucleotides) |
| Comparison DNA | NNNNLLLVV | (Length = 9 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

4 divided by 12 = 33.3%

"Percent (%) amino acid sequence identity" with respect to the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide sequences identified herein is defined as the percentage of amino acid residues in a candidate sequence that are identical with the amino acid residues in a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table I has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table I. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations, Tables 2A-2B demonstrate how to calculate the % am ino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However, % amino acid sequence identity may also be determined using the sequence comparison program NCBI-BLAST2 (Altschul *et al.,* Nucleic Acids Res., 25:3389-3402 (1997)). The NCBI-BLAST2 sequence comparison program may be downloaded from http://www.ncbi.nlm.nih.gov. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity ofB to A.

In addition, % amino acid sequence identity may also be determined using the WU-BLAST-2 computer program (Altschul *et al*., Methods in Enzymology, 266:460-480 (1996)). Most of the WU-BLAST-2 search parameters are set to the default values. Those not set to default values, *i.e.*, the adjustable parameters, are set with the following values: overlap span = 1, overlap fraction = 0.125, word threshold (T) = 11, and scoring matrix = BLOSUM62. For purposes herein, a % amino acid sequence identity value is determined by dividing (a) the number of matching identical amino acids residues between the amino acid sequence of the PRO polypeptide of interest having a sequence derived from the native PRO polypeptide and the comparison amino acid sequence of interest (*i.e.*, the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

"PRO381 variant polypeptide", "PRO1269 variant polypeptide", "PRO1410 variant polypeptide", "PRO1755 variant polypeptide", "PRO1780 variant polypeptide", "PRO1788 variant polypeptide", "PRO3434 variant polypeptide", "PRO1927 variant polypeptide", "PRO3567 variant polypeptide", "PRO1295 variant polypeptide", "PRO1293 variant polypeptide", "PRO1303 variant polypeptide", "PRO4344 variant polypeptide", "PRO4354 variant polypeptide", "PRO4397 variant polypeptide", "PRO4407 variant polypeptide", "PRO1555 variant polypeptide", "PRO1096 variant polypeptide", "PRO2038 variant polypeptide" and "PRO2262 variant polynucleotide" or "PRO381 variant nucleic acid sequence", "PRO1269 variant nucleic acid sequence", "PRO1410 variant nucleic acid sequence", "PRO1755 variant nucleic acid sequence", "PRO1780 variant nucleic acid sequence", "PRO1788 variant nucleic acid sequence", "PRO3434 variant nucleic acid sequence", "PRO1927 variant nucleic acid sequence", "PRO3567 variant nucleic acid sequence", "PRO1295 variant nucleic acid sequence", "PRO1293 variant nucleic acid sequence", "PRO1303 variant nucleic acid sequence", "PRO4344 variant nucleic acid sequence", "PRO4354 variant nucleic acid sequence", "PRO4397 variant nucleic acid sequence", "PRO4407 variant nucleic acid sequence", "PRO1555 variant nucleic acid sequence", "PRO1096 variant nucleic acid sequence", "PRO2038 variant nucleic acid sequence" and "PRO2262 variant nucleic acid sequence" means a nucleic acid molecule which encodes an active PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide as defined below and which has at least about 80% nucleic acid sequence identity with a nucleotide acid sequence encoding a full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide sequence as disclosed herein, a full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide sequence lacking the signal peptide as disclosed herein, an extracellular domain of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide, with or without the signal peptide, as disclosed herein or any other fragment of a full-length PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide sequence as disclosed herein. Ordinarily, a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with the nucleic acid sequence encoding a full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide sequence as disclosed herein, a full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide sequence lacking the signal peptide as disclosed herein, an extracellulardomain of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide, with or without the signal sequence, as disclosed herein or any other fragment of a full-length PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide sequence as disclosed herein. Variants do not encompass the native nucleotide sequence.

Ordinarily, PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 variant polynucleotides are at least about 30 nucleotides in length, often at least about 60 nucleotides in length, more often at least about 90 nucleotides in length, more often at least about 120 nucleotides in length, more often at least about 150 nucleotides in length, more often at least about 180 nucleotides in length, more often at least about 210 nucleotides in length, more often at least about 240 nucleotides in length, more often at least about 270 nucleotides in length, more often at least about 300 nucleotides in length, more often at least about 450 nucleotides in length, more often at least about 600 nucleotides in length, more often at least about 900 nucleotides in length, or more.

"Percent(%) nucleic acid sequence identity" with respect to the PRO381, PRO 1269, PRO1410, PRO 1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide-encoding nucleic acid sequences identified herein is defined as the percentage of nucleotides in a candidate sequence that are identical with the nucleotides in a PRO381, PRO1269, PRO1410, PRO1755, PRO1780, PRO1788, PRO3434, PRO1927. PRO3567, PRO1295, PRO1293, PRO1303, PRO4344, PRO4354, PRO4397, PRO4407, PRO1555, PRO1096, PRO2038 or PRO2262 polypeptide-encoding nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % nucleic acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table I has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of% nucleic acid sequence identity calculations, Tables 2C-2D demonstrate how to calculate the % nucleic acid sequence identity of the nucleic acid sequence designated "Comparison DNA" to the nucleic acid sequence designated "PRO-DNA".

Unless specifically stated otherwise, all % nucleic acid sequence identity values used herein are obtained as described above using the ALIGN-2 sequence comparison computer program. However, % nucleic acid sequence identity may also be determined using the sequence comparison program NCBI-BLAST2 (Altschul *et al.*, Nucleic Acids Res., 25:3389-3402 (1997)). The NCBI-BLAST2 sequence comparison program may be downloaded from http://www.ncbi.nlm.nih.gov. NCBI-BLAST2 uses several search parameters, wherein all of those search parameters are set to default values including, for example, unmask = yes, strand = all, expected occurrences = 10, minimum low complexity length = 15/5, multi-pass e-value =0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In addition, % nucleic acid sequence identity values may also be generated using the WU-BLAST-2 computer program (Altschul *et al.*, Methods in Enzymology, 266:460-480 (1996)). Most of the WU-BLAST-2 search parameters are set to the default values. Those not set to default values, *i.e.*, the adjustable parameters, are set with the following values: overlap span = 1, overlap fraction = 0.125, word threshold (T) = 11, and scoring matrix = BLOSUM62. For purposes herein, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (*i.e.*, the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

In other embodiments, PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 variant polynucleotides are nucleic acid molecules that encode an active PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide and which are capable of hybridizing, preferably under stringent hybridization and wash conditions, to nucleotide sequences encoding the full-length PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO: 33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID N0:53) or Figure 40 (SEQ ID NO:55), respectively. PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 variant polypeptides may be those that are encoded by a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 variant polynucleotide.

The term "positives", in the context of the amino acid sequence identity comparisons performed as described above, includes amino acid residues in the sequences compared that are not only identical, but also those that have similar properties. Amino acid residues that score a positive value to an amino acid residue of interest are those that are either identical to the amino acid residue of interest or are a preferred substitution (as defined in Table 3 below) of the amino acid residue of interest.

For purposes herein, the % value of positives of a given amino acid sequence A to, with, or against a given amino acid sequence B (which can alternatively be phrased as a given amino acid sequence A that has or comprises a certain % positives to, with, or against a given amino acid sequence B) is calculated as follows:

100 times the fraction X/Y

where X is the number of amino acid residues scoring a positive value as defined above by the sequence alignment program ALIGN-2 in that program's alignment of A and B, and where Y is the total number of amino acid residues in B. It will be appreciated that where the length of amino acid sequence A is not equal to the length of amino acid sequence B, the % positives of A to B will not equal the % positives of B to A.

"Isolated," when used to describe the various polypeptides disclosed herein, means polypeptide that has been identified and separated and/or recovered from a component of its natural environment. Preferably, the isolated polypeptide is free of association with all components with which it is naturally associated. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassic blue or, preferably, silver stain. Isolated polypeptide includes polypeptide *in situ* within recombinant cells, since at least one component of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 natural environment will not be present. Ordinarily, however, isolated polypeptide will be prepared by at least one purification step.

An "isolated" nucleic acid molecule encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO 1555, PRO 1096, PRO2038 or PRO2262 polypeptide or an "isolated" nucleic acid encoding an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody, is a nucleic acid molecule that is identified and separated from at least one contaminant nucleic acid molecule with which it is ordinarily associated in the natural source of the PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-,PRO4407-,PRO 1555-, PRO 1096-, PRO203 8- or PRO2262-encoding nucleic acid or the anti-PRO381-, anti-PRO1269-, anti-PRO410-,anti-PRO1755-,anti-PRO1780-,anti-PRO1788-,anti-PRO3434-, anti-PRO1927-, anti-PRO3567-,anti-PRO1295-,anti-PRO1293-, anti-PRO1303-, anti-PRO4344-,anti-PRO4354-,anti-PRO4397-, anti-PRO4407-, anti-PRO1555-, anti-PRO1096-, anti-PRO2038- or anti-PRO2262-encoding nucleic acid. Preferably, the isolated nucleic acid is free of association with all components with which it is naturally associated. An isolated PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding nucleic acid molecule or an anti-PRO381-, anti-PRO1269-, anti-PRO1410-, anti-PRO1755-, anti-PRO1780-, anti-PRO1788-,anti-PRO3434-,anti-PRO1927-,anti-PRO3567-,anti-PRO1295-,anti-PRO1293-,anti-PRO1303-,anti-PRO4344-, anti-PRO4354-, anti-PRO4397-, anti-PRO4407-,anti-PRO1555-, anti-PRO1096-, anti-PRO2038- or anti-PRO2262-encoding nucleic acid molecule is other than in the form or setting in which it is found in nature. Isolated nucleic acid molecules therefore are distinguished from the PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding nucleic acid molecule or the anti-PRO381-,anti-PRO1269-,anti-PRO1410-,anti-PRO1755-,anti-PRO1780-, anti-PRO1788-, anti-PRO3434-,anti-PRO1927-,anti-PRO3567-,anti-PRO1295-,anti-PRO1293-,anti-PRO1303-,anti-PRO4344-,anti-PRO4354-,anti-PRO4397-, anti-PRO4407-, anti-PRO1555-,anti-PRO1096-, anti-PRO2038- or anti-PRO2262-encoding nucleic acid molecule as it exists in natural cells. However, an isolated nucleic acid molecule encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide or an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody includes PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-nucleic acid molecules and anti-PRO381-, anti-PRO 1269-,anti-PRO1410-, anti-PRO 1755-, anti-PRO 1780-, anti-PRO 1788-, anti-PRO3434-, anti-PRO1927-, anti-PRO3567-,anti-PRO1295-,anti-PRO1293-,anti-PRO1303-,anti-PRO4344-, anti-PRO4354-, anti-PRO4397-, anti-PRO4407-, anti-PRO1555-, anti-PRO1096-, anti-PRO2038- or anti-PRO2262-encoding nucleic acid molecules contained in cells that ordinarily express PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptides or express anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibodies where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO 1555, anti-PRO 1096, anti-PRO2038 or anti-PRO2262 monoclonal antibodies (including antagonist, and neutralizing antibodies),anti-PRO381, anti-PRO1269, anti-PRO1410, anti-PRO1755, anti-PRO1780,anti-PRO1788, anti-PRO3434, anti-PRO1927, anti-PRO3567, anti-PRO 1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody compositions with polyepitopic specificity, single chain anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibodies, and fragments of anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, *see* Ausubel *et al.*, Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook *et al.,* Molecular Cloning: A Laboratory Manual, New York: Cold Spring Harbor Press, 1989, and include the use of washing solution and hybridization conditions (*e.g.*, temperature, ionic strength and % SDS) less stringent than those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 35 °C-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" for the purposes herein refers to form(s) of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptides which retain a biological and/or an immunological activity/property of a native or naturally-occurring PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, wherein "biological" activity refers to a function (either inhibitory or stimulatory) caused by a native or naturally-occurring PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide other than the ability to induce the production of an antibody against an antigenic epitope possessed by a a native or naturally-occurring PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide and an "immunological" activity refers to the ability to induce the production of an antibody against an antigenic epitope possessed by a native or naturally-occurring PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

"Biological activity" in the context of an antibody or another antagonist molecule that can be identified by the screening assays disclosed herein (*e.g.*, an organic or inorganic small molecule, peptide, etc.) is used to refer to the ability of such molecules to bind or complex with the polypeptides encoded by the amplified genes identified herein, or otherwise interfere with the interaction of the encoded polypeptides with other cellular proteins or otherwise interfere with the transcription or translation of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. A preferred biological activity is growth inhibition of a target tumor cell. Another preferred biological activity is cytotoxic activity resulting in the death of the target tumor cell.

The term "biological activity" in the context of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide means the ability of a PRO381, PRO1269, PRO1410,

PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide to induce neoplastic cell growth or uncontrolled cell growth.

The phrase "immunological activity" means immunological cross-reactivity with at least one epitope of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

"Immunological cross-reactivity" as used herein means that the candidate polypeptide is capable of competitively inhibiting the qualitative biological activity of a PRO381, PRO1269,PRO1410,PRO1755,PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide having this activity with polyclonal antisera raised against the known active PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. Such antisera are prepared in conventional fashion by injecting goats or rabbits, for example, subcutaneously with the known active analogue in complete Freund's adjuvant, followed by booster intraperitoneal or subcutaneous injection in incomplete Freunds. The immunological cross-reactivity preferably is "specific", which means that the binding affinity of the immunologically cross-reactive molecule (*e.g.*, antibody) identified, to the corresponding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is significantly higher (preferably at least about 2-times, more preferably at least about 4-times, even more preferably at least about 8-times, most preferably at least about 10-times higher) than the binding affinity of that molecule to any other known native polypeptide.

The term "antagonist" is used in the broadest sense, and includes any molecule that partially or fully blocks, inhibits, or neutralizes a biological activity of a native PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide disclosed herein or the transcription or translation thereof. Suitable antagonist molecules specifically include antagonist antibodies orantibodyfragments, fragments, peptides, small organic molecules, anti-sense nucleic acids, etc. Included are methods for identifying antagonists of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide with a candidate antagonist molecule and measuring a detectable change in one or more biological activities normally associated with the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

A "small molecule" is defined herein to have a molecular weight below about 500 Daltons.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas. The term "antibody" is used in the broadest sense and specifically covers, without limitation, intact monoclonal antibodies, polyclonal antibodies, multispecific antibodies (*e.g.*, bispecific antibodies) formed from at least two intact antibodies, and antibody fragments so long as they exhibit the desired biological activity.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR) regions. The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (*see* Kabat *et al*., NIH Publ. No.91-3242, Vol. 1, pages 647-669 (1991)). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from a "complementarity determining region" or "CDR" (i.e., residues 24-34 (L1), 50-56 (L2) and 89-97 (L3) in the light chain variable domain and 31-35 (H1), 50-65 (H2) and 95-102 (H3) in the heavy chain variable domain; Kabat *et al*., Sequences of Proteins of Immunological Interest, 5th Ed. Public Health Service, National Institute of Health, Bethesda, MD. [1991]) and/or those residues from a "hypervariable loop" (*i.e*., residues 26-32 (L1), 50-52 (L2) and 91-96 (L3) in the light chain variable domain and 26-32 (H1), 53-55 (H2) and 96-101 (H3) in the heavy chain variable domain ; Clothia and Lesk, J. Mol. Biol., 196:901-917 [1987]). "Framework" or "FR" residues are those variable domain residues other than the hypervariable region residues as herein defined.

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies (Zapata *et al*., Protein Eng., 8(10):1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an $F(ab')_2$ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH 1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa ($\kappa$) and lambda ($\lambda$), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), *e.g*., IgG1, IgG2, IgG3, IgG4, IgA, and IgA2. The heavy-chain constant domains that correspond to the different classes of immunoglobulins are called $\alpha$, $\delta$, $\in$, $\gamma$, and $\mu$, respectively. The subunit structures and three-dimensional configurations of different classes of immunoglobulins are well known.

The term "monoclonal antibody" as used herein refers to an antibody obtained from a population of substantially homogeneous antibodies, *i.e*., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler *et al*., Nature, 256:495 [1975], or may be made by recombinant DNA methods (*see*, *e.g.,* U.S. Patent No. 4,816,567). The "monoclonal antibodies" may also be isolated from phage antibody libraries using the techniques described in Clackson *et al.,* Nature, 352:624-628 [1991] and Marks *et al.,* J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison *et al*., Proc. Natl. Acad. Sci. USA, 81:6851-6855 [1984]).

"Humanized" forms of non-human (*e.g.*, murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, *see,* Jones *et al*., Nature, 321:522-525 (1986); Reichmann *et al*., Nature, 332: 323-329 [1988]; and Presta, Curr. Op. Struct. Biol., 2:593-596 (1992). The humanized antibody includes a PRIMATIZED™ antibody wherein the antigen-bindingregion of the antibody is derived from an antibody produced by immunizing macaque monkeys with the antigen of interest.

"Single-chain Fv" or "sFv" antibody fragments comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the sFv to form the desired structure for antigen binding. For a review of sFv *see* Pluckthun in The Phannacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain ($V_H$) connected to a light-chain variable domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger *et al*., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (*e.g.*, radioisotope labels or fluorescent labels) or, in the case of an enzymatic label, may catalyze chemical alteration of a substrate compound or composition which is detectable. Radionuclides that can serve as detectable labels include, for example, I-131, I-123, I-125, Y-90, Re-188, Re-186, At-211, Cu-67, Bi-212, and Pd-109. The label may also be a non-detectable entity such as a toxin.

By "solid phase" is meant a non-aqueous matrix to which the antibody ofthe present invention can adhere. Examples of solid phases encompassed herein include those formed partially or entirely of glass (*e.g.*, controlled pore glass), polysaccharides (*e.g.*, agarose), polyacrylamides, polystyrene, polyvinyl alcohol and silicones. In certain embodiments, depending on the context, the solid phase can comprise the well of an assay plate; in others it is a purification column (*e.g.*, an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide or antibody thereto and, optionally, a chemotherapeutic agent) to a mammal. The components of the liposome are commonly arranged in a bilayer formation, similar to the lipid arrangement of biological membranes.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody *(i.e.,* is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

II. Compositions and Methods of the Invention

A. Full-length PRO381, PRO1269, PRO1410, PRO1755, PRO1780, PRO1788, PRO3434, PRO1927, PRO3567, PRO1295. PRO1293, PRO1303, PRO4344, PRO4354, PRO4397, PRO4407, PRO1555, PRO1096, PRO2038 and PRO2262 polypeptides

The present invention provides newly identified and isolated nucleotide sequences encoding polypeptides referred to in the present application as PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262. In particular, cDNA encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptides has been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the proteins encoded by the herein disclosed nucleic acid sequences as well as all further native homologues and variants included in the foregoing definition of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 will be referred to as "PRO381", "PRO1269", "PRO1410", "PRO1755", "PRO1780", "PRO1788", "PRO3434", "PRO1927", "PRO3567", "PRO1295", "PRO1293", "PRO1303", "PRO4344", "PRO4354", "PRO4397", "PRO4407", "PRO1555", "PRO1096", "PRO2038" or "PRO2262", regardless of their origin or mode of preparation.

As disclosed in the Examples below, cDNA clones have been deposited with the ATCC with the exception of known clones PRO1096, PRO2038 and PRO2262, respectively. The actual nucleotide sequence of the clones can readily be determined by the skilled artisan by sequencing of the deposited clone using routine methods in the art. The predicted amino acid sequences can be determined from the nucleotide sequences using routine skill. For the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptides and encoding nucleic acid described herein, Applicants have identified what are believed to be the reading frames best identifiable with the sequence information available at the time.

B. PRO381, PRO1269 PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 Variants

In addition to the full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptides described herein, it is contemplated that PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 variants can be prepared. PRO381, PRO1269, PRO1410, PRO1755, PRO1780, PRO1788, PRO3434, PRO1927, PRO3567, PRO295, PRO1293, PRO1303, PRO4344, PRO4354, PRO4397, PRO4407, PRO1555, PRO1096, PRO2038 and PRO2262 variants can be prepared by introducing appropriate nucleotide changes into the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 DNA, and/or by synthesis of the desired PRO381, PRO1269, PRO1410, PRO1755, PRO1780, PRO1788, PRO3434, PRO1927, PRO3567, PRO1295, PRO1293, PRO303, PRO4344, PRO4354, PRO4397, PRO4407, PRO1555, PRO1096, PRO2038 or PRO2262 polypeptide. Those skilled in the art will appreciate that amino acid changes may alter post-translational processes of the PRO381, PRO1269, PRO1410, PRO1755, PRO1780, PRO1788, PRO3434, PRO1927, PRO3567, PRO1295, PRO1293, PRO303, PRO4344, PRO4354, PRO4397, PRO4407, PRO1555, PRO1096, PRO2038 or PRO2262, such as changing the number or position of glycosylation sites or altering the membrane anchoring characteristics.

Variations in the native full-length sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 or in various domains of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 that results in a change in the amino acid sequence of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 as compared with the native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. Optionally the variation is by substitution of at least one amino acid with any other amino acid in one or more of the domains of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. Guidance in determining which amino acid residue may be inserted, substituted or deleted without adversely affecting the desired activity may be found by comparing the sequence of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, *i.e.,* conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 polypeptide fragments are provided herein. Such fragments may be truncated at the N-terminus or C-terminus, or may lack internal residues, for example, when compared with a full-length native protein. Certain fragments lack amino acid residues that are not essential for a desired biological activity of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 fragments may be prepared by any of a number of conventional techniques. Desired peptide fragments may be chemically synthesized. An alternative approach involves generating PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 fragments by enzymatic digestion, *e.g.*, by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide fragments share at least one biological and/or immunological activity with thenativePRO381, PRO1269, PRO1410, PRO1755, PRO1780, PRO1788. PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

In particular embodiments, conservative substitutions of interest are shown in Table 3 under the heading of preferred substitutions. If such substitutions result in a change in biological activity, then more substantial changes, denominated exemplary substitutions in Table 3, or as further described below in reference to amino acid classes, are introduced and the products screened.

Table 3

| Original Residue | Exemplary Substitutions | Preferred Substitutions |
|---|---|---|
| Ala (A) | val; leu; ile | val |

Table continued

| Original Residue | Exemplary Substitutions | Preferred Substitutions |
|---|---|---|
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu: val: met: ala; phe: norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter *et al.*, Nucl. Acids Res., 13: 4331 (1986); Zoller *et* al., Nucl. Acids Res., 10:6487 (1987)], cassette mutagenesis [Wells *et al.*, Gene, 34:315 (1985)], restriction selection mutagenesis [Wells *et al.*, Philos. Trans. R. Soc. London SerA 317:415 (1986)] or other known techniques can be performed on the cloned DNA to produce the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

C. Modifications of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262

Covalent modifications of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 are included within the scope of this invention. One type of covalent modification includes reacting targeted amino acid residues of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide with an organic derivatizing agent that is capable of reacting with selected side chains or the N- or C- terminal residues of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. Derivatization with bifunctional agents is useful, for instance, for crosslinking PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 to a water-insoluble support matrix or surface for use in the method for purifying anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibodies, and vice-versa. Commonly used crosslinking agents include, *e.g.*, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis (succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide included within the scope of this invention comprises altering the native glycosylation pattern of the polypeptide. "Altering the native glycosylation pattern" is intended for purposes herein to mean deleting one or more carbohydrate moieties found in native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 (either by removing the underlying glycosylation site or by deleting the glycosylation by chemical and/or enzymatic means), and/or adding one or more glycosylation sites that are not present in the native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. In addition, the phrase includes qualitative changes in the glycosylation of the native proteins, involving a change in the nature and proportions of the various carbohydrate moieties present.

Addition of glycosylation sites to the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO555, PRO1096, PRO2038 or PRO2262 polypeptide may be accomplished by altering the amino acid sequence. The alteration may be made, for example, by the addition of, or substitution by, one or more serine or threonine residues to the native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 (for O-linked glycosylation sites). The PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 amino acid sequence may optionally be altered through changes at the DNA level, particularly by mutating the DNA encoding the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide at preselected bases such that codons are generated that will translate into the desired amino acids.

Another means of increasing the number of carbohydrate moieties on the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is by chemical or enzymatic coupling of glycosides to the polypeptide. Such methods are described in the art, *e.g.*, in WO 87/05330 published I September 1987, and in Aplin and Wriston, CRC Crit. Rev. Biochem., pp. 259-306 (1981).

Removal of carbohydrate moieties present on the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide may be accomplished chemically or enzymatically or by mutational substitution of codons encoding for amino acid residues that serve as targets for glycosylation. Chemical deglycosylation techniques are known in the art and described, for instance, by Hakimuddin, *et al*., Arch. Biochem. BioPhys., 259:52 (1987) and by Edge *et al.,* Anal. Biochem., 118:131 (1981). Enzymatic cleavage of carbohydrate moieties on polypeptides can be achieved by the use of a variety of endo- and exo-glycosidases as described by Thotakura *et al*., Meth. Enzymol., 138:350 (1987).

Another type of covalent modification of PRO381, PRO1269, PRO1410, PRO1755, PRO1780,PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 comprises linking the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide to one of a variety of nonproteinaceous polymers, *e.g.*, polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 of the present invention may also be modified in a way to form a chimeric molecule comprising PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 fused to another, heterologous polypeptide or amino acid sequence.

In one embodiment, such a chimeric molecule comprises a fusion of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 with a tag polypeptide which provides an epitope to which an anti-tag antibody can selectively bind. The epitope tag is generally placed at the amino- or carboxyl-terminus of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. The presence of such epitope-tagged forms of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can be detected using an antibody against the tag polypeptide. Also, provision of the epitope tag enables the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 to be readily purified by affinity purification using an anti-tag antibody or another type of affinity matrix that binds to the epitope tag. Various tag polypeptides and their respective antibodies are well known in the art. Examples include poly-histidine (poly-His) or poly-histidine-glycine (poly-His-gly) tags; the flu HA tag polypeptide and its antibody 12CA5 [Field *et al*., Mol. Cell. Biol., 8:2159-2165 (1988)]; the c-myc tag and the 8F9, 3C7, 6E10, G4, B7 and 9E10 antibodies thereto [Evan *et al*., Molecular and Cellular Biology, 5: 3610-3616 (1985)]; and the Herpes Simplex virus glycoprotein D (gD) tag and its antibody [Paborsky *et al*., Protein Engineering, 3(6):547-553 (1990)]. Other tag polypeptides include the Flag-peptide [Hopp *el al*., BioTechnology, 6: 1204-1210 (1988)]; the KT3 epitope peptide [Martin *et al*., Science, 255:192-194 (1992)]; an $\alpha$-tubulin epitope peptide [Skinner *et al*., J. Biol. Chem., 266:15163-15166 (1991)]; and the T7 gene 10 protein peptide tag [Lutz-Freyermuth *et al*., Proc. Natl. Acad. Sci. USA, 87:6393-6397 (1990)].

In an alternative embodiment, the chimeric molecule may comprise a fusion of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions *see* also, US Patent No. 5,428,130 issued June 27, 1995.

D. Preparation of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 and PRO2262 Polypeptides

The description below relates primarily to production of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 by culturing cells transformed or transfected with a vector containing PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 nucleic acid. It is, of course, contemplated that alternative methods, which are well known in the art, may be employed to prepare PRO381, PRO1269, PRO1410, PRO1755, PRO1780, PRO1788, PRO3434, PRO1927, PRO3567, PRO1295, PRO1293, PRO303, PRO4344, PRO4354, PRO4397, PRO4407, PRO1555, PRO1096, PRO2038 or PRO2262. For instance, the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 sequence, or portions thereof, may be produced by direct peptide synthesis using solid-phase techniques [*see, e.g.,* Stewart *et al.*, Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 may be chemically synthesized separately and combined using chemical or enzymatic methods to produce the full-length PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262.

a. Isolation of DNA Encoding a PRO381, PRO1269, PRO1410, PRO1755, PRO1780, PRO1788, PRO3434, PRO1927 PRO3567 PRO1295, PRO1293, PRO1303, PRO4344, PRO4354, PRO4397, PRO4407, PRO1555, PRO1096, PRO2038 or PRO2262 Polypeptide

DNA encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 may be obtained from acDNA library prepared from tissue believed to possess the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 mRNA and to express it at a detectable level. Accordingly, human PRO381, human PRO1269, human PRO1410, human PRO1755, human PRO1780, human PRO1788, human PRO3434, human PRO1927, human PRO3567, human PRO1295, human PRO1293, human PRO1303, human PRO4344, human PRO4354, human PRO4397,human PRO4407, human PRO1555, human PRO1096, human PRO2038 or human PRO2262 DNA can be conveniently obtained from a cDNA library prepared from human tissue, such as described in the Examples. PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding gene may also be obtained from a genomic library or by oligonucleotide synthesis.

Libraries can be screened with probes (such as antibodies to the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, or oligonucleotides of at least about 20-80 bases) designed to identify the gene of interest or the protein encoded by it. Screening the cDNA or genomic library with the selected probe may be conducted using standard procedures, such as described in Sambrook *et al.*, Molecular Cloning: A Laboratory Manual (New York: Cold Spring Harbor Laboratory Press, 1989). An alternative means to isolate the gene encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 is to use PCR methodology [Sambrook *et al.*, *supra*; Dieffenbach *et al.*, PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}$P-labeled ATP, biotinylation or enzyme labeling. Hybridization conditions, including moderate stringency and high stringency, are provided in Sambrook *et al., supra*.

Sequences identified in such library screening methods can be compared and aligned to other known sequences deposited and available in public databases such as GenBank or other private sequence databases. Sequence identity (at either the am ino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al.*, *supra*, to detect precursors and processing intermediates of mRNA that may not have been reverse-transcribed into cDNA.

b. Selection and Transformation of Host Cells

Host cells are transfected or transformed with expression or cloning vectors described herein for PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach M. Butler, ed. (IRL Press, 1991) and Sambrook *el al.*, *supra*.

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook *et al., supra*, or electroporation is generally used for prokaryotes. Infection with *Agrobacterium tumefaciens* is used for transformation of certain plant cells, as described by Shaw *et al.*, Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen *et al.*, J. Bact., 130:946 (1977) and Hsiao *et al.*, Proc. Natl. Acad. Sci. (USA), 76:3829 (1979). However, other methods for introducing DNA into cells, such as by nuclear microinjection, electroporation, bacterial protoplast fusion with intact cells, or polycations, *e.g.*, polybrene, polyornithine, may also be used. For various techniques for transforming mammalian cells, *see*, Keown *et al.*, Methods in Enzymology, 185:527-537 (1990) and Mansour *et al.*, Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and *E. coli* strain K5 772 (ATCC 53,635). Other suitable prokaryotic host cells include Enterobacteriaceae such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli such* as *B. subtilis* and *B. licheniformis (e.g., B. licheniformis* 41P disclosed in DD 266,710 published 12 April 1989), *Pseudomonas* such as *P. aeruginosa*, and *Streptomyces*. These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3*; *E. coli* W31 10 strain 27C7 (ATCC 55,244), which has the complete genotype *tonA ptr3 phoA E15 (argF-lac)169 degP ompT karl*; *E. coli* W3110 strain 37D6, which has the complete genotype *tonA ptr3 phoA E15 (argF-lac) 169 degP ompTrbs7 ilvG* $kan^r$; *E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, *e.g.,* PCR or other nucleic acid polymerase reactions, are suitable.

In addition to prokaryotes, eukaryotic microbes such as filamentous fungi or yeast are suitable cloning or expression hosts for PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding vectors. *Saccharomyces cerevisiae* is a commonly used lower eukaryotic host microorganism. Others include *Schizosaccharoniycespombe* (Beach and Nurse, Nature, 290: 140 [1981]; EP 139,383 published 2 May 1985); *Kluyveromyces* hosts (U.S. Patent No. 4,943,529; Fleer *et al.*, Bio/Technology, 9: 968-975 (1991)) such as, *e.g., K. lactis* (MW98-8C, CBS683, CBS4574; Louvencourt *et al.*, J. Bacteriol., 737 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Vanden Berg *et al.*, Bio/Technolopy, 8:135 (1990)), *K. thermotolerans*, and *K. marxianus; yarrowia* (EP 402,226); *Pichia pastoris* (EP 183,070; Sreekrishna *et al.*, J. Basic Microbiol., 28:265-278 [1988]); *Candida; Trichoderma reesia* (EP 244,234); *Neurospora crassa* (Case *et al.*, Proc. Natl. Acad. Sci. USA, 76:5259-5263 [1979]); *Schwanniomyces* such as *Schwanniomyces occidentalis* (EP 394,538 published 31 October 1990); and filamentous fungi such as, *e.g., Neurospora, Penicillium, Tolypocladium* (WO 91/00357 published 10 January 1991), and *Aspergillus* hosts such as *A. nidulans* (Ballance *et al.*, Biochem. Biophys. Res. Commun., 112:284-289 [1983]; Tilburn *et al.*, Gene, 26: 205-221 [1983]; Yelton *et al.*, Proc. Natl. Acad. Sci. USA 81:1470-1474 [1984]) *and A. niger* (Kelly and Hynes, EMBO J., 4:475-479 [1985]). Methylotropic yeasts are suitable herein and include, but are not limited to, yeast capable of growth on methanol selected from the genera consisting of *Nansenula, Candida, Kloeckera, Pichia, Saccharomyces. Torulopsis,* and *Rhodotorula.* A list of specific species that are exemplary of this class of yeasts may be found in C. Anthony, The Biochemistry of Methylotrophs, 269 (1982).

Suitable host cells for the expression ofglycosylated PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV 1 line transformed by SV40 (COS-7, ATCC CRL 1651); human embryonic kidney line (293 or 293 cells subcloned for growth in suspension culture, Graham *et al.*, J. Gen Virol., 36:59 (1977)); Chinese hamster ovary cells/-DHFR (CHO), Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

c. Selection and Use of a Replicable Vector

The nucleic acid (*e.g.*, cDNA or genomic DNA) encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding DNA that is inserted into the vector. The signal sequence may be a prokaryotic signal sequence selected, for example, from the group of the alkaline phosphatase, penicillinase, lpp, or heat-stable enterotoxin II I leaders. For yeast secretion the signal sequence may be, *e.g.*, the yeast invertase leader, alpha factor leader (including *Saccharomyces* and *Kluyveromyces* $\alpha$-factor leaders, the latter described in U.S. Patent No. 5,010,182), or acid phosphatase leader, the C. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2$\mu$ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.*, ampicillin, neomycin, methotrexate, or tetracycline, (b) complement auxotrophic deficiencies, or (c) supply critical nutrients not available from complex media, *e.g.*, the gene encoding D-alanine racemase for *Bacilli.*

An example of suitable selectable markers for mammalian cells are those that enable the identification of cells competent to take up the PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1535-, PRO1096-, PRO2038- or PRO2262-encoding nucleic acid, such as DHFR or thymidine kinase. An appropriate host cell when wild-type DHFR is employed is the CHO cell line deficient in DHFR activity, prepared and propagated as described by Urlaub *et al.*, Proc. Natl. Acad. Sci. USA, 77:4216 (1980). A suitable selection gene for use in yeast is the *trp*1 gene present in the yeast plasmid YRp7 [Stinchcomb *et al.*, Nature, 282:39 (1979); Kingsman *et al.*, Gene, 7:141 (1979); Tschemper *et al.*, Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding nucleic acid sequence to direct mRNA synthesis. Promoters recognized by a variety of potential host cells are well known. Promoters suitable for use with prokaryotic hosts include the β-lactamase and lactose promoter systems [Chang *et al.*, Nature, 275:615 (1978); Goeddel *et al.*, Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer *et al.*, Proc. Natl. Acad. Sci. USA, 80:21-25 (1983)]. Promoters for use in bacterial systems also will contain a Shine-Dalgarno (S.D.) sequence operably linked to the DNA encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262.

Examples of suitable promoting sequences for use with yeast hosts include the promoters for 3-phosphoglycerate kinase [Hitzeman *et al.*, J. Biol. Chem., 255:2073 (1980)] or other glycolytic enzymes [Hess *et al.*, J. Adv. Enzyme Reg., 7:149(1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, and from heat-shock promoters, provided such promoters are compatible with the host cell systems.

Transcription of a DNA encoding the PRO381, PRO1269, PRO1410, PRO1755. PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions ofeukaryoticor viral DNAs or cDNAs. These regions contain nucleotide segments transcribed as polyadenylated fragments in the untranslated portion of the mRNA encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262.

Still other methods, vectors, and host cells suitable for adaptation to the synthesis of PRO381, PRO1269, PRO1410, PRO1755, PRO1780, PRO1788, PRO3434, PRO1927, PRO3567, PRO1295, PRO1293, PRO1303,

PRO4344, PRO4354, PRO4397, PRO4407, PRO1555, PRO1096, PRO2038 or PRO2262 in recombinant vertebrate cell culture are described in Gething *et al.*, Nature, 293:620-625 (1981); Mantei *et al.*, Nature, 281:40-46 (1979); EP 117,060; and EP 117,058.

d. Detecting Gene Amplification/Expression

Gene amplification and/or expression may be measured in a sample directly, for example, by conventional Southern blotting, Northern blotting to quantitate the transcription ofmRNA [Thomas, Proc. Natl. Acad. Sci. USA. 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide or against a synthetic peptide based on the DNA sequences provided herein or against an exogenous sequence fused to PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 DNA and encoding a specific antibody epitope.

e. Purification of Polypeptide

Forms of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 may be recovered from culture medium or from host cell lysates. If membrane-bound, it can be released from the membrane using a suitable detergent solution (*e.g.*, Triton-X 100) or by enzymatic cleavage. Cells employed in expression of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can be disrupted by various physical or chemical means, such as freeze-thaw cycling, sonication, mechanical disruption, or cell lysing agents.

It may be desired to purify PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms ofthe PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 produced.

E. Amplification of Genes Encoding the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 Polypeptides in Tumor Tissues and Cell Lines

The present invention is based on the identification and characterization of genes that are amplified in certain cancer cells.

The genome of prokaryotic and eukaryotic organisms is subjected to two seemingly conflicting requirements. One is the preservation and propagation of DNA as the genetic information in its original form, to guarantee stable inheritance through multiple generations. On the other hand, cells or organisms must be able to adapt to lasting environmental changes. The adaptive mechanisms can include qualitative or quantitative modifications of the genetic material. Qualitative modifications include DNA mutations, in which coding sequences are altered resulting in a structurally and/or functionally different protein. Gene amplification is a quantitative modification, whereby the actual number of complete coding sequence, *i.e.*, a gene, increases, leading to an increased number of available templates for transcription, an increased number of translatable transcripts, and, ultimately, to an increased abundance of the protein encoded by the amplified gene.

The phenomenon of gene amplification and its underlying mechanisms have been investigated *in vitro* in several prokaryotic and eukaryotic culture systems. The best-characterized example of gene amplification involves the culture of eukaryotic cells in medium containing variable concentrations of the cytotoxic drug methotrexate (MTX). MTX is a folic acid analogue and interferes with DNA synthesis by blocking the enzyme dihydrofolate reductase (DHFR). During the initial exposure to low concentrations of MTX most cells (>99.9%) will die. A small number of cells survive, and are capable of growing in increasing concentrations of MTX by producing large amounts of DHFR-RNA and protein. The basis of this overproduction is the amplification of the single DHFR gene. The additional copies of the gene are found as extrachromosomal copies in the form of small, supernumerary chromosomes (double minutes) or as integrated chromosomal copies.

Gene amplification is most commonly encountered in the development of resistance to cytotoxic drugs (antibiotics for bacteria and chemotherapeutic agents for eukaryotic cells) and neoplastic transformation. Transfomiation of a eukaryotic cell as a spontaneous event or due to a viral or chemical/environmental insult is typically associated with changes in the genetic material of that cell. One of the most common genetic changes observed in human malignancies are mutations of the p53 protein. p53 controls the transition of cells from the stationary (G1) to the replicative (S) phase and prevents this transition in the presence of DNA damage. In other words, one of the main consequences of disabling p53 mutations is the accumulation and propagation of DNA damage, *i.e.*, genetic changes. Common types of genetic changes in neoplastic cells are, in addition to point mutations, amplifications and gross, structural alterations, such as translocations.

The amplification of DNA sequences may indicate a specific functional requirement as illustrated in the DHFR experimental system. Therefore, the amplification of certain oncogenes in.malignancies points toward a causative role of these genes in the process of malignant transformation and maintenance of the transformed phenotype. This hypothesis has gained support in recent studies. For example, the *bcl-2* protein was found to be amplified in certain types ofnon-Hodgkin's lymphoma. This protein inhibits apoptosis and leads to the progressive accumulation of neoplastic cells. Members of the gene family of growth factor receptors have been found to be amplified in various types of cancers suggesting that overexpression of these receptors may make neoplastic cells less susceptible to limiting amounts of available growth factor. Examples include the amplification ofthe androgen receptor in recurrent prostate cancer during androgen deprivation therapy and the amplification of the growth factor receptor homologue ERB2 in breast cancer. Lastly, genes involved in intracellular signaling and control of cell cycle progression can undergo amplification during malignant transformation. This is illustrated by the amplification of the *bcl-l* and *ras* genes in various epithelial and lymphoid neoplasms.

These earlier studies illustrate the feasibility of identifying amplified DNA sequences in neoplasms, because this approach can identify genes important for malignant transformation. The case of ERB2 also demonstrates the feasibility from a therapeutic standpoint, since transforming proteins may represent novel and specific targets for tumor therapy.

Several different techniques can be used to demonstrate amplified genomic sequences. Classical cytogenetic analysis of chromosome spreads prepared from cancer cells is adequate to identify gross structural alterations, such as translocations, deletions and inversions. Amplified genomic regions can only be visualized, if they involve large regions with high copy numbers or are present as extrachromosomal material. While cytogenetics was the first technique to demonstrate the consistent association of specific chromosomal changeswith particular neoplasms, it is inadequate for the identification and isolation of manageable DNA sequences. The more recently developed technique of comparative genomic hybridization (CGH) has illustrated the widespread phenomenon of genomic amplification in neoplasms. Tumor and normal DNA are hybridized simultaneously onto metaphases of normal cells and the entire genome can be screened by image analysis for DNA sequences that are present in the tumor at an increased frequency. (WO 93/18,186; Gray *et al.*, Radiation Res., 137:275-289 [1994]). As a screening method, this type of analysis has revealed a large number of recurring amplicons (a stretch of amplified DNA) in a variety of human neoplasms. Although CGH is more sensitive than classical cytogenetic analysis in identifying amplified stretches of DNA, it does not allow a rapid identification and isolation of coding sequences within the amplicon by standard molecular genetic techniques.

The most sensitive methods to detect gene amplification are polymerase chain reaction (PCR)-based assays. These assays utilize very small amount of tumor DNA as starting material, are exquisitely sensitive, provide DNA that is amenable to further analysis, such as sequencing and are suitable for high-volume throughput analysis.

The above-mentioned assays are not mutually exclusive, but are frequently used in combination to identify amplifications in neoplasms. While cytogenetic analysis and CGH represent screening methods to survey the entire genome for amplified regions, PCR-based assays are most suitable for the final identification ofcoding sequences, *i.e.*, genes in amplified regions.

According to the present invention, such genes have been identified by quantitative PCR (S. Gelmini *et al.*, Clin. Chem., 43:752 [1997]), by comparing DNA from a variety of primary tumors, including breast, lung, colon, prostate, brain, liver, kidney, pancreas, spleen, thymus, testis, ovary, uterus, etc., tumor, or tumor cell lines, with pooled DNA from healthy donors. Quantitative PCR was performed using a TaqMan instrument (ABI). Gene-specific primers and fluorogenic probes were designed based upon the coding sequences of the DNAs.

Human lung carcinoma cell lines include A549 (SRCC768), Calu-1 (SRCC769),Calu-6(SRCC770),H 157 (SRCC771), H441 (SRCC772), H460 (SRCC773), SKMES-1 (SRCC774), SW900 (SRCC775), H522 (SRCC832),and H810 (SRCC833), all available from ATCC. Primary human lung tumor cells usually derive from adenocarcinomas, squamous cell carcinomas, large cell carcinomas, non-small cell carcinomas, small cell carcinomas, and broncho alveolar carcinomas, and include, for example, SRCC724 (adenocarcinoma, abbreviated as "AdenoCa")(LT1), SRCC725 (squamous cell carcinoma, abbreviated as "SqCCa)(LT1a), SRCC726 (adenocarcinoma)(LT2), SRCC727 (adenocarcinoma)(LT3), SRCC728 (adenocarcinoma)(LT4), SRCC729 (squamous cell carcinoma)(LT6), SRCC730 (adeno/squamous cell carcinoma)(LT7), SRCC731 (adenocarcinoma)(LT9), SRCC732 (squamous cell carcinoma)(LT10), SRCC733 (squamous cell carcinoma)(LT11), SRCC734 (adenocarcinoma)(LT12), SRCC735 (adeno/squamous cell carcinoma) (LT13), SRCC736 (squamous cell carcinoma)(LT15), SRCC737 (squamous cell carcinoma)(LT16), SRCC738 (squamous cell carcinoma)(LT17), SRCC739 (squamous cell carcinoma)(LT 18), SRCC740(squamouscell carcinoma)(LT19), SRCC741 (lung cell carcinoma, abbreviated as "LCCa")(LT21), SRCC811 (adenocarcinoma)(LT22), SRCC825 (adenocarcinoma)(LT8), SRCC886 (adenocarcinoma)(LT25), SRCC887 (squamous cell carcinoma) (LT26), SRCC888 (adeno-BAC carcinoma) (LT27), SRCC889 (squamous cell carcinoma) (LT28), SRCC890 (squamous cell carcinoma) (LT29), SRCC891 (adenocarcinoma) (LT30), SRCC892 (squamous cell carcinoma) (LT31), SRCC894 (adenocarcinoma) (LT33). Also included are human lung tumors designated SRCC1125[HF-000631], SRCC1127 [HF-000641], SRCC1129 [HF-000643], SRCC1133 [HF-000840], SRCC1135 [HF-000842], SRCC1227 [HF-001291], SRCC1229 [HF-001293], SRCC1230 [HF-001294], SRCC1231 [HF-001295], SRCC1232 [HF-001296], SRCC1233 [HF-001297], SRCC1235 [HF-001299], and SRCC1236 [HF-001300].

Colon cancer cell lines include, for example, ATCC cell lines SW480 (adenocarcinoma, SRCC776), SW620 (lymph node metastasis of colon adenocarcinoma, SRCC777), Colo320 (carcinoma, SRCC778), HT29 (adenocarcinoma, SRCC779), HM7 (a high mucin producing variant of ATCC colon adenocarcinoma cell line, SRCC780, obtained from Dr. Robert Warren, UCSF), Ca WiDr (adenocarcinoma,SRCC781), HCT116 (carcinoma, SRCC782), SKCO1 (adenocarcinoma, SRCC783), SW403 (adenocarcinoma, SRCC784), LS174T (carcinoma, SRCC785),Colo205 (carcinoma, SRCC828), HCT15 (carcinoma, SRCC829), HCC2998 (carcinoma, SRCC830), and KM12 (carcinoma, SRCC831). Primary colon tumors include colon adenocarcinomas designated CT2 (SRCC742), CT3 (SRCC743),CT8 (SRCC744), CT10 (SRCC745), CT12 (SRCC746), CT14 (SRCC747), CT15 (SRCC748), CT16 (SRCC749), CT17 (SRCC750), CT1 (SRCC751), CT4 (SRCC752), CT5 (SRCC753), CT6 (SRCC754), CT7 (SRCC755), CT9 (SRCC756), CT11 (SRCC757), CT18 (SRCC758), CT19 (adenocarcinoma, SRCC906), CT20 (adenocarcinoma, SRCC907), CT21 (adenocarcinoma, SRCC908), CT22 (adenocarcinoma, SRCC909), CT23 (adenocarcinoma, SRCC910), CT24 (adenocarcinoma, SRCC911), CT25 (adenocarcinoma, SRCC912), CT26 (adenocarcinoma, SRCC913), CT27 (adenocarcinoma, SRCC914),CT28 (adenocarcinoma, SRCC915), CT29 (adenocarcinoma, SRCC916), CT30 (adenocarcinoma, SRCC917), CT31 (adenocarcinoma, SRCC918), CT32 (adenocarcinoma, SRCC919), CT33 (adenocarcinoma, SRCC920), CT35 (adenocarcinoma, SRCC921), and CT36 (adenocarcinoma, SRCC922). Also included are human colon tumor centers designated SRCC1051 [HF-000499], SRCC1052 [HF-000539], SRCC1053 [HF-000575], SRCC1054 [HF-000698], SRCC1142 [HF-000762], SRCC1144 [HF-000789], SRCC1146 [HF-000795] and SRCC1148 [HF-000811].

Human breast carcinoma cell lines include, for example, HBL100 (SRCC759), MB435s (SRCC760), T47D (SRCC761), MB468(SRCC762), MB175 (SRCC763), MB361 (SRCC764), BT20 (SRCC765), MCF7 (SRCC766), and SKBR3 (SRCC767), and human breast tumor center designated SRCC1057 [HF-000545]. Also included are human breast tumors designated SRCC1094, SRCC1095, SRCC1096, SRCC1097, SRCC1098, SRCC1099, SRCC1100, SRCC1101, and human breast-met-lung-NS tumor designated SRCC893 [LT 32].

Human kidney tumor centers include SRCC989 [HF-000611] and SRCC1014 [HF-000613].

Human testis tumor center includes SRCC1001 [HF-000733] and testis tumor margin SRCC999 [HF-000716].

Human parathyroid tumor includes SRCC1002 [HF-000831] and SRCC1003 [HF-000832].

F. Tissue Distribution

The results of the gene amplification assays herein can be verified by further studies, such as, by determining mRNA expression in various human tissues.

As noted before, gene amplification and/or gene expression in various tissues may be measured by conventional Southern blotting, Northern blotting to quantitate the transcription of mRNA (Thomas, Proc. Natl. Acad.Sci.USA, 77: 5201-5205 [1980]), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochem ical staining and/or assay of sample fluids may be either monoclonal or polyclonal, and may be prepared in any mammal. Conveniently, the antibodies may be prepared against a native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide or against a synthetic peptide based on the DNA sequences provided herein or against exogenous sequence fused to sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 DNA and encoding a specific antibody epitope. General techniques for generating antibodies, and special protocols for Northern blotting and *in situ* hybridization are provided hereinbelow.

G. Chromosome Mapping

If the amplification of a given gene is functionally relevant, then that gene should be amplified more than neighboring genomic regions which are not important for tumor survival. To test this, the gene can be mapped to a particular chromosome, *e.g.*, by radiation-hybrid analysis. The amplification level is then determined at the location identified, and at the neighboring genomic region. Selective or preferential amplification at the genomic region to which the gene has been mapped is consistent with the possibility that the gene amplification observed promotes tumor growth or survival. Chromosome mapping includes both framework and epicenter mapping. For further details *see. e.g.*, Stewart *et al.*, Genome Research. 7:422-433 (1997).

H. Antibody Binding Studies

The results of the gene amplification study can be further verified by antibody binding studies, in which the ability of anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibodies to inhibit the expression of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptides on tumor (cancer) cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein (encoded by a gene amplified in a tumor cell) in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. *See*, *e.g.*, U.S. Patent No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tumor sample may be fresh or frozen or may be embedded in paraffin and fixed with a preservative such as formalin, for example.

I. Cell-Based Tumor Assays

Cell-based assays and animal models for tumors (*e.g.*, cancers) can be used to verify the findings of the gene amplification assay, and further understand the relationship between the genes identified herein and the development and pathogenesis of neoplastic cell growth. The role of gene products identified herein in the development and pathology of tumor or cancer can be tested by using primary tumor cells or cells lines that have been identified to amplify the genes herein. Such cells include, for example, the breast, colon and lung cancer cells and cell lines listed above.

In a different approach, cells of a cell type known to be involved in a particular tumor are transfected with the cDNAs herein, and the ability of these cDNAs to induce excessive growth is analyzed. Suitable cells include, for example, stable tumor cells lines such as, the B104-1-I cell line (stable NIH-3T3 cell line transfected with the neu protooncogene) and *ras*-transfected NIH-3T3 cells, which can be transfected with the desired gene, and monitored for tumorogenic growth. Such transfected cell lines can then be used to test the ability of poly- or monoclonal antibodies or antibody compositions to inhibit tumorogenic cell growth by exerting cytostatic or cytotoxic activity on the growth of the transformed cells, or by mediating antibody-dependent cellular cytotoxicity (ADCC). Cells transfected with the coding sequences of the genes identified herein can further be used to identify drug candidates for the treatment of cancer.

In addition, primary cultures derived from tumors in transgenic animals (as described below) can be used in the cell-based assays herein, although stable cell lines are preferred. Techniques to derive continuous cell lines from transgenic animals are well known in the art *(see, e.g.,* Small *et al.,* Mol. Cell. Biol., 5:642-648 [1985]).

J. Animal Models

A variety of well known animal models can be used to further understand the role of the genes identified herein in the development and pathogenesis of tumors, and to test the efficacy of candidate therapeutic agents, including antibodies, and other antagonists of the native polypeptides, including small molecule antagonists. The *in vivo* nature of such models makes them particularly predictive of responses in human patients. Animal models of tumors and cancers (*e.g.,* breast cancer, colon cancer, prostate cancer, lung cancer, etc.) include both non-recombinant and recombinant (transgenic) animals. Non-recombinant animal models include, for example, rodent, *e.g.*, murine models. Such models can be generated by introducing tumor cells into syngeneic mice using standard techniques, *e.g.*, subcutaneous injection, tail vein injection, spleen implantation, intraperitoneal implantation, implantation under the renal capsule, or orthopin implantation, *e.g.*, colon cancer cells implanted in colonic tissue. (*See, e.g.,* PCT publication No. WO 97/33551, published September 18, 1997).

Probably the most often used animal species in oncological studies are immunodeficient mice and, in particular, nude mice. The observation that the nude mouse with hypo/aplasia could successfully act as a host for human tumor xenografts has lead to its widespread use for this purpose. The autosomal recessive *nu* gene has been introduced into a very large number of distinct congenic strains of nude mouse, including, for example, ASW, A/He, AKR, BALB/c, B10.LP, C17, C3H, C57BL, C57, CBA, DBA, DDD, I/st, NC, NFR, NFS, NFS/N, NZB, NZC, NZW, P, RIII and SJL. In addition, a wide variety of other animals with inherited immunological defects other than the nude mouse have been bred and used as recipients of tumor xenografts. For further details *see, e.g.*, The Nude Mouse in Oncology Research, E. Boven and B. Winograd, eds., CRC Press, Inc., 1991.

The cells introduced into such animals can be derived from known tumor/cancer cell lines, such as, any of the above-listed tumor cell lines, and, for example, the B 104-1-1 cell line (stable NIH-3T3 cell line transfected with the *neu* protooncogene); *ras*-transfected NIH-3T3 cells; Caco-2 (ATCC HTB-37); a moderately well-differentiated grade II human colon adenocarcinoma cell line, HT-29 (ATCC HTB-38), or from tumors and cancers. Samples of tumor or cancer cells can be obtained from patients undergoing surgery, using standard conditions, involving freezing and storing in liquid nitrogen (Karmali *et al.*, Br. J. Cancer, 48:689-696 [1983]).

Tumor cells can be introduced into animals, such as nude mice, by a variety of procedures. The subcutaneous (s.c.) space in mice is very suitable for tumor implantation. Tumors can be transplanted s.c. as solid blocks, as needle biopsies by use of a trochar, or as cell suspensions. For solid block or trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue. Boven and Winograd (1991), *supra.*

Animal models of breast cancer can be generated, for example, by implanting rat neuroblastoma cells (from which the *neu* oncogen was initially isolated), or *neu*-transformed NIH-3T3 cells into nude mice, essentially as described by Drebin *et al.,* PNAS USA, 83:9129-9133 (1986).

Similarly, animal models of colon cancer can be generated by passaging colon cancer cells in animals, *e.g.*, nude mice, leading to the appearance of tumors in these animals. An orthotopic transplant model of human colon cancer in nude mice has been described, for example, by Wang *et al.,* Cancer Research. 54:4726-4728 (1994) and Too *et al.,*

Cancer Research. 55:681-684 (1995). This model is based on the so-called "METAMOUSE" sold by AntiCancer, Inc., (San Diego, California).

Tumors that arise in animals can be removed and cultured *in vitro.* Cells from the *in vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.

For example, Meth A, CMS4, CMS5, CMS21, and WEH1-164 are chemically induced fibrosarcomas of BALB/c female mice (DeLeo *et al.,* J. Exp. Med., 146:720 [1977]), which provide a highly controllable model system for studying the anti-tumor activities of various agents (Palladino *et al.,* J. Immunol., 138:4023-4032 [1987]). Briefly, tumor cells are propagated *in vitro* in cell culture. Prior to injection into the animals, the cell lines are washed and suspended in buffer, at a cell density of about $10x10^6$ to $10x10^7$ cells/ml. The animals are then infected subcutaneously with 10 to 100 μl of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small cell carcinoma of the lung (SCCL). This tumor can be introduced in normal mice upon injection of tumor fragments from an affected mouse or of cells maintained in culture (Zupi *et at.,* Br. J. Cancer, 41 auppl. 4:309 [1980]), and evidence indicates that tumors can be started from injection of even a single cell and that a very high proportion of infected tumor cells survive. For further information about this tumor model *see*, Zacharski, Haemostasis, 16:300-320 [1986]).

One way of evaluating the efficacy of a test compound in an animal model on an implanted tumor is to measure the size of the tumor before and after treatment. Traditionally, the size of implanted tumors has been measured with a slide caliper in two or three dimensions. The measure limited to two dimensions does not accurately reflect the size of the tumor, therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen, Proc. 6th Int. Workshop on Immune-Deficient Animals Wu and Sheng eds., Basel, 1989, 301. It is noted, however, that necrosis and inflammatory responses following treatment may actually result in an increase in tumor size, at least initially. Therefore, these changes need to be carefully monitored, by a combination of a morphometric method and flow cytometric analysis.

Recombinant (transgenic) animal models can be engineered by introducing the coding portion ofthe genes identified herein into the genome of animals ofinterest, using standard techniques for producing transgenic animals. Animals that can serve as a target for transgenic manipulation include, without limitation, mice, rats, rabbits, guinea pigs, sheep, goats, pigs, and non-human primates, e.g., baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e.g.*, Van der Putten *et al.,* Proc. Natl. Acad. Sci. USA, 82:6148-615 [1985]); gene targeting in embryonic stem cells (Thompson *et al.,* Cell, 56:313-321 [1989]); electroporation of embryos (Lo, Mol. Cell Biol., 3:1803-1814 [1983]); sperm-mediated gene transfer (Lavitrano *et al.*, Cell, 57:717-73 [1989]). For review, *see,* for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g.*, head-to-head or head-to-tail tandems. Selective introduction of a transgene into a particular cell type is also possible by following, for example, the technique of Lasko *et al.*, Proc. Natl. Acad. Sci. USA 89:6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals are further examined for signs of tumor or cancer development.

Alternatively, "knock out" animals can be constructed which have a defective or altered gene encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide identified herein, as a result of homologous recombination between the endogenous gene encoding the polypeptide and altered genomic DNA encoding the same polypeptide introduced into an embryonic cell of the animal. For example, cDNA encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide can be used to clone genomic DNA encoding that polypeptide in accordance with established techniques. A portion of the genomic DNA encoding a particular PRO381, PRO1269, PRO1410, PRO1755, PRO1780, PRO1788, PRO3434, PRO1927, PRO3567, PRO1295, PRO1293, PRO1303, PRO4344, PRO4354,

PRO4397, PRO4407, PRO1555, PRO1096, PRO2038 or PRO2262 polypeptide can be deleted or replaced with another gene, such as a gene encoding a selectable marker which can be used to monitor integration. Typically, several kilobases of unaltered flanking DNA (both at the 5' and 3' ends) are included in the vector [*see, e.g.,* Thomas and Capecchi, Cell, 51:503 (1987) for a description of homologous recombination vectors]. The vector is introduced into an embryonic stem cell line (*e.g.*, by electroporation) and cells in which the introduced DNA has homologously recombined with the endogenous DNA are selected [*see*, *e.g.,* Li *et al.,* Cell, 69:915 (1992)]. The selected cells are then injected into a blastocyst of an animal (*e.g.*, a mouse or rat) to form aggregation chimeras [*see, e.g.,* Bradley, in Teratocarcinomas and Embryonic Stem Cells: A Practical Approach, E.J. Robertson, ed. (IRL, Oxford, 1987), pp. 113-152] A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, by their ability to defend against certain pathological conditions and by their development of pathological conditions due to absence of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

The efficacy of antibodies specifically binding the polypeptides identified herein and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because ofthe anatomy of the feline oral cavity. At present, there is no effective treatment for this tumor. Prior to entry into the study, each cat undergoes complete clinical examination, biopsy, and is scanned by computed tomography (CT). Cats diagnosed with sublingual oral squamous cell tumors are excluded from the study. The tongue can become paralyzed as a result of such tumor, and even if the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CT scan. CT scans and thoracic radiograms are evaluated every 8 weeks thereafter. The data are evaluated for differences in survival, response and toxicity as compared to control groups. Positive response may require evidence of tumor regression, preferably with improvement of quality of life and/or increased life span.

In addition, other spontaneous animal tumors, such as fibrosarcoma, adenocarcinoma, lymphoma, chrondroma, leiomyosarcoma of dogs, cats, and baboons can also be tested. Of these mammary adenocarcinoma in dogs and cats is a preferred model as its appearance and behavior are very similar to those in humans. However, the use of this model is limited by the rare occurrence of this type of tumor in animals.

K. Screening Assays for Drug Candidates

Screening assays for drug candidates are designed to identify compounds that bind or complex with the polypeptides encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulinfusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art.

All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g.*, on a microtiter plate, by covalent or non-covalent attachments. Non-covalent attachment generally is accomplished by coating the solid surface with a solution of the polypeptide and drying. Alternatively, an immobilized antibody, *e.g.*, a monoclonal antibody, specific for the polypeptide to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide encoded by a gene identi fied herein, its interaction with that polypeptide can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature, 340:245-246 (1989); Chien *et al.,* Proc. Natl. Acad. Sci. USA, 88: 9578-9582 (1991)] as disclosed by Chevray and Nathans, Proc. Natl. Acad. Sci. USA 89:5789-5793 (199)]. Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GALI-*lacZ* reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding gene identified herein and other intra- or extracellular components can be tested as follows: usually a reaction mixture is prepared containing the product of the amplified gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide may be added to a cell along with the compound to be screened for a particular activity and the ability of the compound to inhibit the activity of interest in the presence of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide indicates that the compound is an antagonist to the PRO381, PRO 1269, PRO 1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. Alternatively, antagonists may be detected by combining the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide and a potential antagonist with membrane-bound PRO381, PRO1269, PRO 1410, PRO1755, PRO1780, PRO1788, PRO3434, PRO927, PRO3567, PRO1295, PRO1293, PRO303, PRO4344, PRO4354, PRO4397, PRO4407, PRO1555, PRO1096, PRO2038 or PRO2262 polypeptide receptors or recombinant receptors under appropriate conditions for a competitive inhibition assay. The PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide can be labeled, such as by radioactivity. such that the number of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide molecules bound to the receptor can be used to determine the effectiveness of the potential antagonist. The gene encoding the receptor can be identified by numerous methods known to those of skill in the art, for example, ligand panning and FACS sorting. Coligan *et al.*, Current Protocols in Immun., 1(2): Chapter 5 (1991). Preferably, expression cloning is employed wherein polyadenylated RNA is prepared from a cell responsive to the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide and a cDNA library created from this RNA is divided into pools and used to transfect COS cells or other cells that are not responsive to the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. Transfected cells that are grown on glass slides are exposed to labeled PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. The PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, labeled PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro-sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in the presence of the candidate compound. The ability of the compound to enhance or block this interaction could then be measured.

More specific examples of potential antagonists include an oligonucleotide that binds to the fusions of immunoglobulin with the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PRO381, PRO1269, PRO1410, PRO1755, PRO1780, PRO1788, PRO3434, PRO1927, PRO3567, PRO1295, PRO1293, PRO 1303, PRO4344, PRO4354, PRO4397, PRO4407, PRO1555, PRO1096, PRO2038 or PRO2262 polypeptide that recognizes the receptor but imparts no effect, thereby competitively inhibiting the action of the PRO381, PRO1269, PRO1410, PRO1755, PRO1780, PRO1788, PRO3434, PRO927, PRO3567, PRO1295, PRO1293, PRO1303, PRO4344, PRO4354, PRO4397, PRO4407, PRO1555, PRO1096, PRO2038 or PRO2262 polypeptide.

Anotherpotential PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide herein, is used to design an antisense RNA oligonucleotide of from about 10 to 40 base pairs in length. A DNA oligonucleotide is designed to be complementary to a region of the gene involved in transcription (triple helix -*see*, Lee *et al.*, Nucl. Acids Res., 6:3073 (1979); Cooney *et al.*, Science, 241: 456 (1988); Dervan *et al.*, Science, 251:1360 (1991)), thereby preventing transcription and the production of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. The antisense RNA oligonucleotide hybridizes to the mRNA *in vivo* and blocks translation of the mRNA molecule into the PRO381, PRO1269, PRO1410, PRO1755, PRO1780, PRO1788, PRO3434, PRO927, PRO3567, PRO1295, PRO1293, PRO1303, PRO4344, PRO4354, PRO4397, PRO4407, PRO1555, PRO1096, PRO2038 or PRO2262 polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the PRO381,PRO1269,PRO1410, PRO1755, PRO1780, PRO1788, PRO3434, PRO1927,

PRO3567, PRO1295, PRO1293, PRO1303, PRO4344, PRO4354, PRO4397, PRO4407, PRO1555, PRO1096, PRO2038 or PRO2262 polypeptide. When antisense DNA is used, oligodeoxyribonucleotides derived from the translation-initiation site, *e.g.*, between about -10 and +10 positions of the target gene nucleotide sequence, are preferred.

Antisense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

Potential antagonists include small molecules that bind to the active site, the receptor binding site, or growth factor or other relevant binding site of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, thereby blocking the normal biological activity of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details *see*, *e.g.*, Rossi, Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details *see, e.g.,* PCT publication No. WO 97/33551, *supra.*

These small molecules can be identified by any one or more of the screening assays discussed hereinabove and/or by any other screening techniques well known for those skilled in the art.

## L. Compositions and Methods for the Treatment of Tumors

The compositions useful in the treatment of tumors associated with the amplification of the genes identified herein include, without limitation, antibodies, small organic and inorganic molecules, peptides, phosphopeptides, antisense and ribozyme molecules, triple helix molecules, etc., that inhibit the expression and/or activity of the target gene product.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e.g.*, between about -10 and +10 positions of the target gene nucleotide sequence, are preferred.

Ribozymesare enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details *see, e.g.,* Rossi, Current Biology, 4:469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details *see, e.g.*, PCT publication No. WO 97/33551, *supra*.

These molecules can be identified by any or any combination of the screening assays discussed hereinabove and/or by any other screening techniques well known for those skilled in the art.

## M. Antibodies

Some of the most promising drug candidates according to the present invention are antibodies and antibody fragments which may inhibit the production or the gene product of the amplified genes identified herein and/or reduce the activity of the gene products.

### 1. Polyclonal Antibodies

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti- PRO4397, anti-PRO4407, anti-PRO1555, anti-PRO1096, anti-PRO2038 or anti-PRO2262 antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, including fragments, or a fusion protein of such protein or a fragment thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection (ATCC), Manassas, Virginia. Human myeloma and mouse-human heteromyeloma cell lines also have been described for the production of human monoclonal antibodies [Kozbor, J. Immunol., 133:3001 (1984); Brodeur *et al.*, Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, *supra*]. Suitable culture media for this purpose include, for example, Dulbecco's Modified Eagle's Medium and RPMI-I 640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (*e.g.*, by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison *et al.*, *supra*] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

3. Human and Humanized Antibodies

The anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti- PRO4397, anti-PRO4407, anti-PRO1555, anti-PRO1096, anti-PRO2038 or anti-PRO2262 antibodies may further comprise humanized antibodies or human antibodies. Humanized forms ofnon-human (*e.g.*, murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-humanimmunoglobulin. Humanized antibodies include human immunoglobulins (recipientantibody)in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones *et al.*, Nature, 321:522-525 (1986); Riechmann *et al.*, Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones *el al.,* Nature, 321: 522-525 (1986); Riechmann *et al.*, Nature, 332:323-327 (1988); Verhoeyen *et al.,* Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol. 227:381 (1991); Marks *et al.*, J. Mol. Biol. 222:581 (1991)]. The techniques of Cole *et al.*, and Boerner *et al.*, are also available for the preparation of human monoclonal antibodies (Cole *et al.*, Monoclonal Antibodies and Cancer Therapy, Alan R. Liss, p. 77 (1985) and Boerner *et al.*, J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks *et al.*, Bio/Technology, 10:779-783 (1992); Lonberg *et al.*, Nature, 368:856-859 (1994); Morrison, Nature, 368:812-13 (1994); Fishwild *et al.*, Nature Biotechnology, 14:845-51 (1996); Neuberger, Nature Biotechnology, 14:826 (1996);

Lonberg and Huszar, Intern. Rev. Immunol., 13:65-93 (1995).

4. Antibody Dependent Enzyme Mediated Prodrug Therapy (ADEPT)

The antibodies of the present invention may also be used in ADEPT by conjugating the antibody to a prodrug-activating enzyme which converts a prodrug (*e.g.*, a peptidyl chemotherapeutic agent, *see* WO 81/01145) to an active anti-cancer drug. *See,* for example, WO 88/07378 and U. S. Patent No. 4,975,278.

The enzyme component of the immunoconjugate useful for ADEPT includes any enzyme capable of acting on a prodrug in such as way so as to convert it into its more active, cytotoxic form.

Enzymes that are useful in the method of this invention include, but are not limited to, glycosidase, glucose oxidase, human lysosyme, human glucuronidase, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug 5-fluorouracil; proteases, such as serratia protease, thermolysin, subtilisin, carboxypeptidases (*e.g.*, carboxypeptidase G2 and carboxypeptidase A) and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin Vamidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes" can be used to convert the prodrugs of the invention into free active drugs (*see*, *e.g.,* Massey, Nature, 328:457-458 (1987)). Antibody-abzyme conjugates can be prepared as described herein for delivery of the abzyme to a tumor cell population.

The enzymes ofthis inventioncan be covalently bound to the anti-PRO381, anti-PRO1269, anti-PRO1410, anti-PRO1755, anti-PRO1780, anti-PRO1788, anti-PRO3434, anti-PRO1927,anti-PRO3567,anti-PRO1295, anti-PRO1293, anti-PRO1303, anti-PRO4344, anti-PRO4354, anti- PRO4397, anti-PRO4407, anti-PRO1555, anti-PRO1096, anti-PRO2038 or anti-PRO2262 antibodies by techniques well known in the art such as the use of the heterobifunctional cross-linking agents discussed above. Alternatively, fusion proteins comprising at least the antigen binding region of the antibody of the invention linked to at least a functionally active portion of an enzyme of the invention can be constructed using recombinant DNA techniques well known in the art (*see, e.g.,* Neuberger *et al*., Nature, 312: 604-608 (1984)).

5. Bispecific Antibodies

Bispecific antibodies are monoclonal, preferably human or humanized, antibodies that have binding specificities for at least two different antigens. In the present case, one of the binding specificities is for the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 [1983]). Because of the random assortment of immunoglobulin heavy and light chains, these hybridomas (quadromas) produce a potential mixture often different antibody molecules, of which only one has the correct bispecific structure. The purification of the correct molecule is usually accomplished by affinity chromatography steps. Similar procedures are disclosed in WO 93/08829, published 13 May 1993, and in Traunecker *et al*., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details ofgenerating bispecific antibodies *see*, for example, Suresh *et al*., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (*e.g.*, tyrosine or tryptophan). Compensatory "cavities" of identical or similar size to the large side chain(s) are created on the interface of the second antibody molecule by replacing large amino acid side chains with smaller ones (*e.g.*, alanine or threonine). This provides a mechanism for increasing the yield of the heterodimer over other unwanted end-products such as homodimers.

Bispecific antibodies can be prepared as full length antibodies or antibody fragments (*e.g.*, $F(ab')_2$ bispecific antibodies). Techniques for generating bispecific antibodies from antibody fragments have been described in the literature. For example, bispecific antibodies can be prepared using chemical linkage. Brennan *et al.*, Science, 229:81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-T'NB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby *et al.,* J. Exp. Med., 175:217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny *et al.*, J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger *et al.*, Proc. Natl. Acad. Sci. USA 90:6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. *See*, Gruber *et al.*, J. Immunol., 152:5368 (1994).

Antibodies with more than two valencies are contemplated. For example, trispecific antibodies can be prepared. Tutt *et al.*, J. Immunol., 147:60 (1991).

Exemplary bispecific antibodies may bind to two different epitopes on a given polypeptide herein. Alternatively, an anti-polypeptide arm may be combined with an arm which binds to a triggering molecule on a leukocyte such as a T-cell receptor molecule (*e.g.*, CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular polypeptide. Bispecific antibodies may also be used to localize cytotoxic agents to cells which express a particular polypeptide. These antibodies possess a polypeptide-binding arm and an arm which binds a cytotoxic agent or a radionuclide chelator, such as EOTUBE, DPTA, DOTA, or TETA. Another bispecific antibody of interest binds the polypeptide and further binds tissue factor (TF).

6. Heteroconiugate Antibodies

Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

7. Effector function engineering

It may be desirable to modify the antibody of the invention with respect to effector function, so as to enhance the effectiveness of the antibody in treating cancer, for example. For example, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). *See*, Caron *et al.*, J. Exp Med., 176:1191-1195 (1992) and Shopes, J.

Immunol., 148:2918-2922 (1992). Homodimeric antibodies with enhanced anti-tumor activity may also be prepared using heterobifunctional cross-linkers as described in Wolff *et al.*, Cancer Research, 53:2560-2565 (1993). Alternatively, an antibody can be engineered which has dual Fc regions and may thereby have enhanced complement lysis and ADCC capabilities. *See*, Stevenson *et al.*, Anti-Cancer Drug Design, 3:219-230 (1989).

8. Immunoconjugates

The invention also pertains to immunoconjugates comprising an antibody conjugated to a cytotoxic agent such as a chemotherapeutic agent, toxin (*e.g.*, an enzymatically active toxin of bacterial, fungal, plant or animal origin, or fragments thereof, or a small molecule toxin), or a radioactive isotope (*i.e.*, a radioconjugate).

Chemotherapeutic agents useful in the generation of such immunoconjugates have been described above. Enzymatically active protein toxins and fragments thereof which can be used include diphtheria A chain, nonbinding active fragments of diphtheria toxin, cholera toxin, botulinus toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII,and PAP-S), momordica charantia inhibitor, curcin, crotin, sapaonaria officinalis inhibitor, gelonin, saporin, mitogellin, restrictocin, phenomycin, enomycin and the tricothecenes. Small molecule toxins include, for example, calicheamicins, maytansinoids, palytoxin and CC 1065. A variety of radionuclides are available for the production of radioconjugated antibodies. Examples include $^{212}$Bi, $^{131}$I, $^{131}$In, $^{90}$Y and $^{186}$Re.

Conjugates of the antibody and cytotoxic agent are made using a variety of bifunctional protein coupling agents such as N-succinimidyl-3-(2-pyridyldithiol) propionate (SPDP), iminothiolane (IT), bifunctionalderivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta *et al.*, Science, 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See,* WO94/11026.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) which is conjugated to a cytotoxic agent (*e.g.*, a radionucleotide).

9. Immunoliposomes

The antibodies disclosed herein may also be formulated as immunoliposomes. Liposomes containing the antibody are prepared by methods known in the art, such as described in Epstein *et al.*, Proc. Natl. Acad. Sci. USA, 82: 3688 (1985); Hwang *et al.*, Proc. Natl. Acad. Sci. USA, 77:4030 (1980); and U.S. Patent Nos. 4,485,045 and 4,544,545. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin *et al.*, J. Biol. Chem., 257:286-288 (1982) via a disulfide interchange reaction. A chemotherapeutic agent (such as Doxorubicin) is optionally contained within the liposome. *See,* Gabizon *et al.*, J. National Cancer Inst., 81(19):1484 (1989).

N. Pharmaceutical Compositions

Antibodies specifically binding the product of an amplified gene identified herein, as well as other molecules identified by the screening assays disclosed hereinbefore, can be administered for the treatment of tumors, including cancers, in the form of pharmaceutical compositions.

If the protein encoded by the amplified gene is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (*see, e.g.*, Marasco *et al.*, Proc. Natl. Acad. Sci. USA, 90:7889-7893 [1993]).

Therapeutic formulations of the antibody are prepared for storage by mixing the antibody having the desired degree of purity with optional pharmaceutically acceptable carriers, excipients or stabilizers (Remineton's Pharmaceutical Sciences, 16th edition, Osol, A. ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and *m*-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins;chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as TWEEN™, PLURONICS™ or polyethylene glycol (PEG).

Non-antibody compounds identified by the screening assays of the present invention can be formulated in an analogous manner, using standard techniques well known in the art.

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.*, films or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT ™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intemiolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

## O. Methods of Treatment

It is contemplated that the antibodies and other anti-tumor compounds of the present invention may be used to treat various conditions, including those characterized by overexpression and/or activation of the amplified genes identified herein. Exemplary conditions or disorders to be treated with such antibodies and other compounds, including, but not limited to, small organic and inorganic molecules, peptides, antisense molecules, etc., include benign or malignant tumors (*e.g.*, renal, liver, kidney, bladder, breast, gastric, ovarian, colorectal, prostate, pancreatic, lung, vulval, thyroid, hepatic carcinomas; sarcomas; glioblastomas; and various head and neck tumors); leukemias and lymphoid malignancies; other disorders such as neuronal, glial, astrocytal, hypothalamic and other glandular, macrophagal, epithelial, stromal and blastocoelic disorders; and inflammatory, angiogenic and immunologic disorders.

The anti-tumor agents of the present invention, *e.g.*, antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration ofthe antibody is preferred.

Other therapeutic regimens may be combined with the administration of.the anti-cancer agents, *e.g.*, antibodies of the instant invention. For example, the patient to be treated with such anti-cancer agents may also receive radiation therapy. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD(1992). The chemotherapeutic agent may precede, or follow administration ofthe anti-tumor agent, *e.g.*, antibody, or may be given simultaneously therewith. The antibody may be combined with an anti-oestrogen compound such as tamoxifen or an anti-progesterone such as onapristone (*see*, EP 616812) in dosages known for such molecules.

It may be desirable to also administer antibodies against other tumor associated antigens, such as antibodies which bind to the ErbB2, EGFR, ErbB3, ErbB4, or vascular endothelial factor (VEGF). Alternatively, or in addition, two or more antibodies binding the same or two or more different antigens disclosed herein may be co-administered to the patient. Sometimes, it may be beneficial to also administer one or more cytokines to the patient. In a preferred embodiment, the antibodies herein are co-administered with a growth inhibitory agent. For example, the growth inhibitory agent may be administered first, followed by an antibody of the present invention. However, simultaneous administration or administration of the antibody of the present invention first is also contemplated. Suitable dosages for the growth inhibitory agent are those presently used and may be lowered due to the combined action (synergy) of the growth inhibitory agent and the antibody herein.

For the prevention or treatment of disease, the appropriate dosage of an anti-tumor agent, e.g., an antibody herein will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments.

For example, depending on the type and severity of the disease, about 1 $\mu$g/kg to 15 mg/kg (*e.g.*, 0.1-20 mg/kg) of antibody is an initial candidate dosage for administration to the patient, whether, for example, by one or more separate administrations, or by continuous infusion. A typical daily dosage might range from about 1 $\mu$g/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

P. Articles of Manufacture

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually an anti-tumor agent capable of interfering with the activity of a gene product identified herein, e.g., an antibody. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

Q. Diagnosis and Prognosis of Tumors

While cell surface proteins, such as growth receptors overexpressed in certain tumors are excellent targets for drug candidates or tumor (*e.g.*, cancer) treatment, the same proteins along with secreted proteins encoded by the genes amplified in tumor cells find additional use in the diagnosis and prognosis of tumors. For example, antibodies directed against the protein products of genes amplified in tumor cells can be used as tumor diagnostics or prognostics.

For example, antibodies, including antibody fragments, can be used to qual itatively or quantitatively detect the expression of proteins encoded by the amplified genes ("marker gene products"). The antibody preferably is equipped with a detectable, *e.g.*, fluorescent label, and binding can be monitored by light microscopy, flow cytometry, fluorimetry, or other techniques known in the art. These techniques are particularly suitable, if the amplified gene encodes a cell surface protein, *e.g.*, a growth factor. Such binding assays are performed essentially as described in section 5 above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

All patent and literature references cited in the present specification are hereby incorporated by reference in their entirety.

EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, 10801 University Blvd., Manassas, VA 20110-2209. All original deposits referred to in the present application were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc., and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

Unless otherwise noted, the present invention uses standard procedures of recombinant DNA technology, such as those described hereinabove and in the following textbooks: Sambrook *et al.*, Molecular Cloning: A Laboratory Manual, Cold Spring Harbor Press N.Y., 1989; Ausubel *et al.*, Current Protocols in Molecular Biology, Green Publishing Associates and Wiley Interscience, N.Y., 1989; Innis *et al.*, PCR Protocols: A Guide to Methods and Applications, Academic Press, Inc., N.Y., 1990; Harlow *et al.*, Antibodies: A Laboratory Manual, Cold Spring Harbor Press, Cold Spring Harbor, 1988; Gait, Oligonucleotide Synthesis, IRL Press, Oxford, 1984; R.I. Freshney, Animal Cell Culture, 1987; Coligan *et al.*, Current Protocols in Immunology, 1991.

EXAMPLE 1: Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e.g.*, Dayhoff, GenBank), and proprietary databases (*e.g.* LIFESEQ®, Incyte Pharmaceuticals, Palo Alto, CA). The search was performed using the computer program BLAST or BLAST-2 (Altschul *et al.,* Methods in Enzymology, 266:460-480 (1996)) as a comparison of the ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green, University of Washington, Seattle, Washington).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5 kbp. In order to screen several libraries for a full-length clone, DNA from the libraries was screened by PCR amplification, as per Ausubel *et al.*, Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes *et al.*, Science, 253:1278-1280 (1991)) in the unique XhoI and NotI sites.

EXAMPLE 2: Isolation of cDNA Clones Usine Signal Algorithm Analysis

Various polypeptide-encoding nucleic acid sequences were identified by applying a proprietary signal sequence finding algorithm developed by Genentech, Inc., (South San Francisco, CA) upon ESTs as well as clustered and assembled EST fragments from public (*e.g.*, GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

EXAMPLE 3: Isolation of cDNA clones Encoding Human PRO381

A consensus DNA sequence was assembled relative to other EST sequences using phrap as described in Example 1 above. This consensus sequence is designated herein as DNA39651. Based on the DNA39651 consensus sequence, oligonucleotides were synthesized: 1) to identify by PCR a cDNA library that contained the sequence of interest, and 2) for use as probes to isolate a clone of the full-length coding sequence for PRO381.

A pair of PCR primers (forward and reverse) were synthesized:

forward PCR primer (39651.f1):

5'-CTTTCCTTGCTTCAGCAACATGAGGC-3' (SEQ ID NO:3)

reverse PCR primer (39651.r1):

5'-GCCCAGAGCAGGAGGAATGATGAGC-3' (SEQ ID NO:4)

Additionally, a synthetic oligonucleotide hybridization probe was constructed from the consensus DNA39651 sequence which had the following nucleotide sequence:

hybridization probe (39651.p1):

5'-GTGGAACGCGGTCTTGACTCTGTTCGTCACTTCTTTGATTGGGGCTTTG-3' (SEQ ID NO:5)

In order to screen several libraries for a source of a full-length clone, DNA from the libraries was screened by PCR amplification with the PCR primer pair identified above. A positive library was then used to isolate clones encoding the PRO381 gene using the probe oligonucleotide and one of the PCR primers. RNA for construction of the cDNA libraries was isolated from human fetal kidney tissue (LIB227).

DNA sequencing of the isolated clones isolated as described above gave the full-length DNA sequence for DNA44194-1317 [Figure I, SEQ ID NO:1]; and the derived protein sequence for PRO381.

The entire coding sequence of DNA44194-1317 is included in Figure 1 (SEQ ID NO:1). Clone DNA44194-1317 contains a single open reading frame with an apparent translational initiation site at nucleotide positions 174-176, and an apparent stop codon at nucleotide positions 807-809. The predicted polypeptide precursor is 211 amino acids long. Analysis of the full-length PRO381 sequence shown in Figure 2 (SEQ ID NO:2) evidences the presence of a variety of important polypeptide domains, wherein the locations given for those important polypeptide domains are approximate as described above. Analysis of the full-length PRO381 polypeptide shown in Figure 2 evidences the presence of the following: a signal peptide from about amino acid I to about amino acid 20; a potential N-glycosylation site from about amino acid 176 to about amino acid 180; an endoplasmic reticulum targeting sequence from about amino acid 208 to about amino acid 212; FKBP-type peptidyl-prolyl cis-trans isomerase sites from about amino acid 78 to about amino acid 115, and from about amino acid 118 to about amino acid 132; EF-hand calcium binding domains from about amino acid 191 to about amino acid 204, from about amino acid 184 to about amino acid 204, and from about amino acid 140 to about amino acid 160; and an S-100/ICaBP type calcium binding domain from about amino acid 183 to about amino acid 204. Clone DNA44194-1317 has been deposited with the ATCC on April 28, 1998 and is assigned ATCC deposit no. 209808. The full-length PRO381 protein shown in Figure 2 has an estimated molecular weight of about 24,172 daltons and a pl of about 5.99.

Analysis of the amino acid sequence of the full-length PRO381 polypeptide suggests that it possesses sugnificant sequence similarity to FKBP immunophilin proteins, thereby indicating that PRO381 may be a novel FKBP immunophilin homolog. More specifically, an analysis of the Dayhoff database (version 35.45 SwissProt 35), using a WU-BLAST2 sequence alignment analysis of the full-length sequence shown in Figure 2 (SEQ ID NO:2), revealed sequence identity between the PRO381 amino acid sequence and the following Dayhoffsequences: AF040252_1, 149669, P_R93551, S71238, CELC05C8_1, CEU27353_1, MIP_TRYCR, CEZC455_3, FKB4_HUMAN and 140718.

EXAMPLE 4: Isolation of cDNA clones Encoding Human PRO1269

DNA66520-1536 was identified by applying the proprietary signal sequence finding algorithm described in Example 2 above. Use of the above described signal sequence algorithm allowed identification of an EST cluster sequence from the LIFESEQ®database, designated Incyte EST cluster no. 101920. This ESTcluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul *et al.*, Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56509.

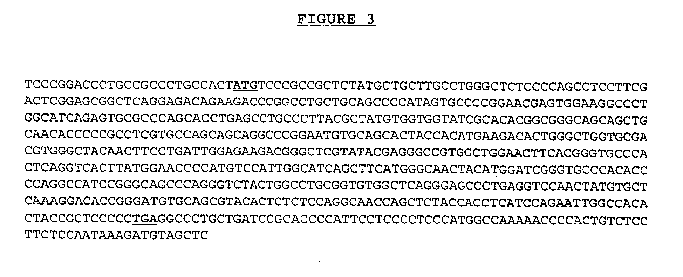

In light of the sequence homology between the DNA56509 sequence and Incyte EST no. 103157, Incyte EST no. 103 157 was purchased and the cDNA insert was obtained and sequenced. The sequence of this cDNA insert is shown in Figure 3 (SEQ ID NO:6) and is herein designated as DNA66520-1536.

Clone DNA66520-1536 contains a single open reading frame with an apparent translational initiation site at nucleotide positions 26-28 and ending at the stop codon at nucleotide positions 614-616 (Figure 3). The predicted polypeptide precursor is 196 amino acids long (Figure 4; SEQ ID NO:7). The full-length PRO1269 protein shown in Figure 4 has an estimated molecular weight of about 21,731 daltons and a pI of about 8.97. Analysis of the full-length PRO1269 sequence shown in Figure 4 (SEQ ID NO:7) evidences the presence of a variety of important polypeptide domains, wherein the locations given for those important polypeptide domains are approximate as described above. Analysis of the full-length PRO1269 sequence shown in Figure 4 evidences the presence of the following: a signal peptide from about amino acid 1 to about amino acid 20; and a potential N-glycosylation site from about amino acid 112 to about amino acid 116. Clone DNA66520-1536 has been deposited with ATCC on September 15, 1998 and is assigned ATCC deposit no. 203226.

An analysis of the Dayhoff database (version 35.45 SwissProt 35), using a WU-BLAST2 sequence alignment analysis of the full-length sequence shown in Figure 4 (SEQ ID NO:7), evidenced significant homology between the PRO1269 amino acid sequence and the amino acid sequence of Dayhoff sequence P_W23722. In addition, sequence homology was found between the PRO1269 amino acid sequence and the amino acid sequences of the following Dayhoff sequences: MMTAG7_1, MTV026_16, NAAA_BPT3, S75616_1, and NCP_P1G.

EXAMPLE 5: Isolation of cDNA clones Encoding Human PRO1410

DNA68874-1622 was identified by applying the proprietary signal sequence finding algorithm described in Example 2 above. Use of the above described signal sequence algorithm allowed identification of an ESTcluster sequence from the LIFESEQ® database, designated Incyte EST cluster sequence no. 98502. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul *et al.,* Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56451.

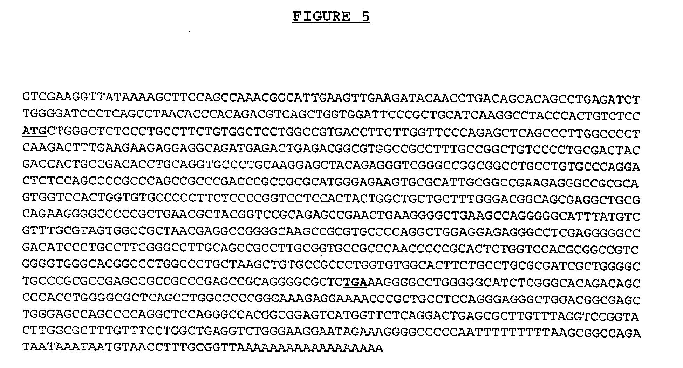

In light of the sequence homology between the DNA56451 sequence and the Incyte EST clone no. 1257046, the Incyte EST clone no. 1257046 was purchased and the cDNA insert was obtained and sequenced. The sequence of this cDNA insert is shown in Figure 5 (SEQ ID NO:8) and is herein designated as DNA68874-1622.

Clone DNA68874-1622 contains a single open reading frame with an apparent translational initiation site at nucleotide positions 152-154 and ending at the stop codon at nucleotide positions 866-868 (Figure 5). The predicted polypeptide precursor is 238 amino acids long (Figure 6; SEQ ID NO:9). The full-length PRO1410 protein shown in Figure 6 has an estimated molecular weight of about 25,262 daltons and a p1 of about 6.44. Analysis of the full-length PRO1410 sequence shown in Figure 6 (SEQ ID NO:9) evidences the presence of a variety of important polypeptide domains, wherein the locations given for those important polypeptide domains are approximate as described above.

Analysis of the full-length PRO1410 sequence shown in Figure 6 evidences the presence of the following: a signal peptide from about amino acid 1 to about amino acid 20; a transmembrane domain from about amino acid 194 to about amino acid 220; and a potential N-glycosylation site from about amino acid 132 to about am ino acid 136. Clone DNA68874-1622 has been deposited with ATCC on September 22, 1998 and is assigned ATCC deposit no. 203277.

An analysis of the Dayhoff database (version 35.45 SwissProt 35), using a WU-BLAST2 sequence alignment analysis of the full-length sequence shown in Figure 6 (SEQ ID NO:9), evidenced significant homology between the PRO1410 amino acid sequence and the following Dayhoff sequences: 148652, P_R76466, HSMHC3W36A_2, EPB4_HUMAN, P_R14256, EPA8_MOUSE, P_R77285, P_W13569, AF000560_1, and ASF1_HELAN.

EXAMPLE 6: Isolation of cDNA clones Encoding Human PRO1755

DNA76396-1698 was identified by applying the proprietary signal sequence finding algorithm described in Example 2 above. Use of the above described signal sequence algorithm allowed identification of an EST cluster sequence from the LIFESEQ® database, designated Incyte EST cluster no. 141872. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul *et al.*, Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA55731.

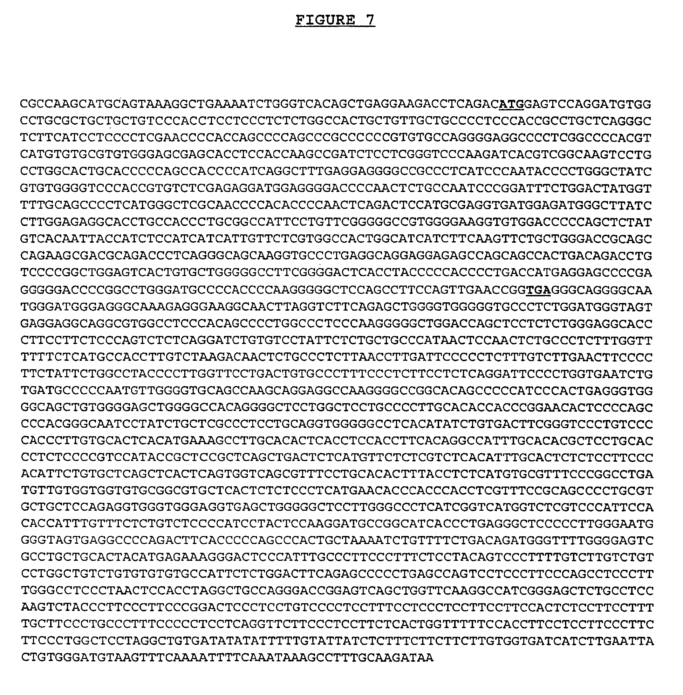

In light of the sequence homology between the DNA55731 sequence and Incyte EST no. 257323, Incyte EST no. 257323 was purchased and the cDNA insert was obtained and sequenced. The Incyte ESTclone no. 257323 was derived from a library constructed using RNA isolated from the hNT2 cell line (Stratagene library no. STR9372310), which was derived from a human teratocarcinoma that exhibited properties characteristic of a committed neuronal precursor at an early stage of development. The sequence of this cDNA insert is shown in Figure 7 (SEQ ID NO:10) and is herein designated as DNA76396-1698. Alternatively, the DNA76396 sequence can be obtained by preparing oligonucleotide probes and primers and isolating the sequence from an appropriate library (*e.g.*, STR9372310).

The entire coding sequence of DNA76396-1698 is included in Figure 7 (SEQ ID NO:10). Clone DNA76396-1698 contains a single open reading frame with an apparent translational initiation site at nucleotide positions 58-60 and ending at the stop codon at nucleotide positions 886-888 (Figure 7). The predicted polypeptide precursor is 276 amino acids long (Figure 8; SEQ ID NO: 11). The full-length PRO1755 protein shown in Figure 8 has an estimated molecular weight of about 29,426 daltons and a pl of about 9.40. Analysis of the full-length PRO1755 sequence shown in Figure 8 (SEQ ID NO:11) evidences the presence of a variety of important polypeptide domains, wherein the locations given for those important polypeptide domains are approximate as described above. Analysis of the full-length PRO1755 sequence shown in Figure 8 evidences the presence of the following: a signal peptide from about amino acid I to about amino acid 33; a transmembrane domain from about amino acid 178 to about amino acid 198; a cAMP- and cGMP-dependent protein kinase phosphorylation site from about amino acid 210 to about amino acid 214; potential N-myristoylation sites from about amino acid 117 to about amino acid 123, from about amino acid 154 to about amino acid 160, and from about amino acid 214 to about amino acid 220; and a cell attachment sequence from about amino acid 149 to about amino acid 152. Clone DNA76396-1698 has been deposited with ATCC on November 17, 1998 and is assigned ATCC deposit no. 203471.

An analysis of the Dayhoff database (version 35.45 SwissProt 35), using a WU-BLAST2 sequence alignment analysis of the full-length sequence shown in Figure 8 (SEQ ID NO:11), evidenced some homology between the PRO1755 amino acid sequence and the following Dayhoff sequences: APG-BRANA, P_R37743, NAU88587_1, YHL1_EBV, P_W31855, CET10B10_4, AF039404_1, PRP1_HUMAN, AF038575_1, and AF053091_1.

EXAMPLE 7: Isolation of cDNA clones Encoding Human PRO1780

A consensus DNA sequence was assembled relative to other EST sequences using phrap as described in Example I above. Incyte EST no. 3349314, designated herein as DNA63837.init, from the LIFESEQ® database was identified as a sequence of interest having a BLAST score of 70 or greater that did not encode a known protein. The DNA63837.init sequence was extended using repeated cycles of BLAST and the program "phrap" to extend the consensus sequence as far as possible using the sources of EST sequences discussed above. This consensus sequence is designated herein as DNA63837.

Based on the DNA63837 consensus sequence, oligonucleotides were synthesized: 1) to identify by PCR a cDNA library that contained the sequence of interest, and 2) for use as probes to isolate a clone of the full-length coding sequence for PRO1780.

A pair of PCR primers (forward and reverse) were synthesized:

forward PCR primer (63837.f1):

5'-TGCCTTTGCTCACCTACCCCAAGG-3' (SEQ ID NO:14)

reverse PCR primer (63837.r1):

5'-TCAGGCTGGTCTCCAAAGAGAGGG-3' (SEQ ID NO:15)

Additionally, a synthetic oligonucleotide hybridization probe was constructed from the consensus DNA63837 sequence which had the following nucleotide sequence:

hybridization probe (63837.p1):

5'-CCCAAAGATGTCCACCTGGCTGCAAATGTGAAAATTGTGGACTGG-3' (SEQ ID NO:16)

In order to screen several libraries for a source of a full-length clone, DNA from the libraries was screened by PCR amplification with the PCR primer pair identified above. A positive library was then used to isolate clones encoding the PRO1780 gene using the probe oligonucleotide and one of the PCR primers. RNA for construction of the cDNA libraries was isolated from human fetal kidney tissue.

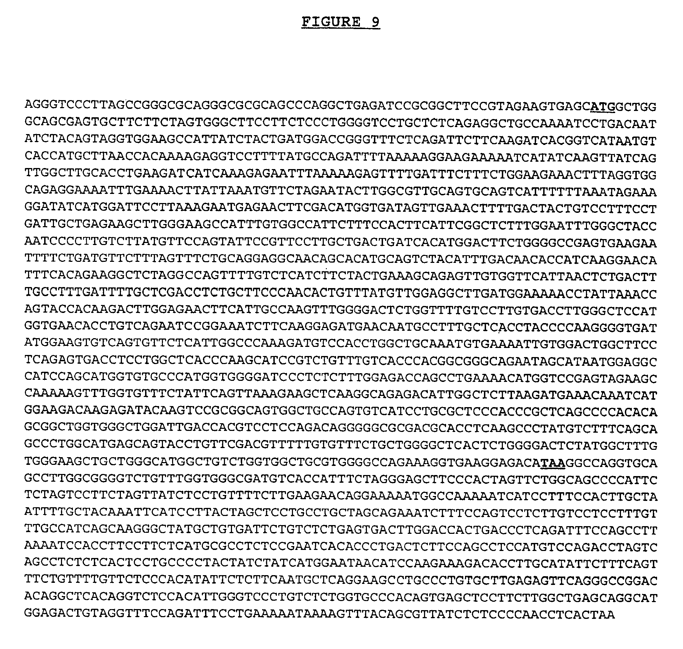

DNA sequencing of the isolated clones isolated as described above gave the full-length DNA sequence for DNA71169-1709 [Figure 9, SEQ ID NO:12]; and the derived protein sequence for PRO1780.

The entire coding sequence of DNA71169-1709 is included in Figure 9 (SEQ ID NO:12). Clone DNA71169-1709 contains a single open reading frame with an apparent translational initiation site at nucleotide positions 68-70, and an apparent stop codon at nucleotide positions 1637-1639. The predicted polypeptide precursor is 523 amino acids long. Analysis of the full-length PRO1780 sequence shown in Figure 10 (SEQ ID NO:13) evidences the presence of a variety of important polypeptide domains, wherein the locations given for those important polypeptide domains are approximate as described above. Analysis of the full-length PRO1780 polypeptide shown in Figure 10 evidences the presence of the following: a signal peptide from about amino acid 1 to about amino acid 19; a transmembrane domain from about amino acid 483 to about amino acid 504; an N-glycosylation site from about amino acid 52 to about amino acid 56; tyrosine kinase phosphorylation sites from about amino acid 68 to about amino acid 75, and from about amino acid 425 to about amino acid 434; N-myristoylation sites from about amino acid 16 to about amino acid 22, from about amino acid 301 to about amino acid 307, from about amino acid 370 to about amino acid 376, and from about amino acid 494 to about amino acid 500; a leucine zipper pattern from about amino acid 493 to about amino acid 515; and an UDP-glucoronosyl site from about amino acid 241 to about amino acid 294. Clone DNA71169-1709 has been deposited with the ATCC on November 17, 1998 and is assigned ATCC deposit no. 203467. The full-length PRO1780 protein shown in Figure 10 has an estimated molecular weight of about 59,581 daltons and a pl of about 8.68.

An analysis of the Dayhoff database (version 35.45 SwissProt 35), using a WU-BLAST2 sequence alignment analysis of the full-length sequence shown in Figure 10 (SEQ ID NO:13), revealed significant sequence identity between the PRO1780 amino acid sequence and the following Dayhoff sequences: UDA2_RABIT, CGT_HUMAN, UD11_HUMAN, P_R26153, UDB1_RAT, HSU59209_1, ABO10872_1, UDB5_MOUSE, UDB8_HUMAN, and UD14_HUMAN.

EXAMPLE 8: Isolation of cDNA clones Encoding Human PRO1788

A consensus DNA sequence was assembled relative to other EST sequences using phrap as described in Example 1 above. Incyte clone no. 2968304 was identified as a sequence of interest having a BLAST score of 70 or greater that did not encode known proteins. In addition, the sequence was extended using repeated cycles of BLAST and the program "phrap" to extend the sequence as far as possible using the sources of EST sequences discussed above. This consensus sequence is designated herein as DNA49648. Based on the DNA49648 consensus sequence, oligonucleotides were synthesized: 1) to identify by PCR a cDNA library that contained the sequence of interest, and 2) for use as probes to isolate a clone of the full-length coding sequence for PRO1788.

A pair of PCR primers (forward and reverse) were synthesized:

forward PCR primer (49648.f1):

5'-CCCTGCCAGCCGAGAGCTTCACC-3' (SEQ ID NO:19)

reverse PCR primer (49648.r1):

5'-GGTTGGTGCCCGAAAGGTCCAGC-3' (SEQ ID NO:20)

Additionally, a synthetic oligonucleotide hybridization probe was constructed from the consensus DNA49648 sequence which had the following nucleotide sequence:

hybridization probe (49648.p1):

5'-CAACCCCAAGCTTAACTGGGCAGGAGCTGAGGTGTTTTCAGGCC-3' (SEQ ID NO:21)

In order to screen several libraries for a source of a full-length clone, DNA from the libraries was screened by PCR amplification with the PCR primer pair identified above. A positive library was then used to isolate clones encoding the PRO1788 gene using the probe oligonucleotide and one of the PCR primers. RNA for construction of the cDNA libraries was isolated from human fetal kidney tissue.

DNA sequencing of the isolated clones isolated as described above gave the full-length DNA sequence for DNA77652-2505 [Figure 11, SEQ ID NO:17]; and the derived protein sequence for PRO1788.

The entire coding sequence of DNA77652-2505 is included in Figure I 1 (SEQ ID NO:17). Clone DNA77652-2505 contains a single open reading frame with an apparent translational initiation site at nucleotide positions 64-66, and an apparent stop codon at nucleotide positions 1123-1125. The predicted polypeptide precursor is 353 amino acids long. Analysis of the full-length PRO1788 sequence shown in Figure 12 (SEQ ID NO:18) evidences the presence of a variety of important polypeptide domains, wherein the locations given forthose important polypeptide domains are approximate as described above. Analysis of the full-length PRO1788 polypeptide shown in Figure 12 evidences the presence of the following: a signal peptide from about amino acid 1 to about amino acid 16; transmembrane domains from about amino acid 215 to about amino acid 232, and from about amino acid 287 to about amino acid 304; potential N-glycosylation sites from about amino acid 74 to about amino acid 78, and from about amino acid 137 to about amino acid 141; a glycosaminoglycan atachment site from about amino acid 45 to about amino acid 49; a tyrosine kinase phosphorylation site from about amino acid 318 to about amino acid 326; N-myristoylation sites from about amino acid 13 to about amino acid 19, from about amino acid 32 to about amino acid 38, from about amino acid 88 to about amino acid 94, from about amino acid 214 to about amino acid 220, and from about amino acid 223 to about amino acid 229; and a leucine zipper pattern from about amino acid 284 to about amino acid 306. Clone DNA77652-2505 has been deposited with the ATCC on November 17, 1998 and is assigned ATCC deposit no. 203480. The full-length PRO1788 protein shown in Figure 12 has an estimated molecular weight of about 37,847 daltons and a pI of about 6.80.

An analysis of the Dayhoff database (version 35.45 SwissProt 35), using a WU-BLAST2 sequence alignment analysis of the full-length sequence shown in Figure 12 (SEQ ID NO:18), revealed significant sequence identity between the PRO1788 amino acid sequence and the following Dayhoff sequences: AF030435_1; AF062006_1: DMTARTAN_ 1; GARP_HUMAN; S42799; P_R71294; HSU88879_1; DROWHEELER_1; A58532; and AF068920_1.

EXAMPLE 9: Isolation of cDNA clones Encoding Human PRO3434

DNA77631-2537 was identified by applying the proprietary signal sequence finding algorithm described in Example 2 above. Use of the above described signal sequence algorithm allowed identification of an EST cluster sequence from the LIFESEQ® database. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul *et al.,* Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56099.

In light of the sequence homology between the DNA56099 sequence and Incyte EST clone no. 3327089, Incyte EST clone no. 3327089 was purchased and the cDNA insert was obtained and sequenced. The sequence of this cDNA insert is shown in Figure 13 (SEQ ID NO:22) and is herein designated as DNA77631-2537.

Clone DNA77631-2537 contains a single open reading frame with an apparent translational initiation site at nucleotide positions 46-48 and ending at the stop codon at nucleotide positions 3133-3135 (Figure 13). The predicted polypeptide precursor is 1029 amino acids long (Figure 14; SEQ ID NO:23). The full-length PRO3434 protein shown in Figure 14 has an estimated molecular weight of about 114,213 daltons and a pI of about 6.42. Analysis of the full-length PRO3434 sequence shown in Figure 14 (SEQ ID NO:23) evidences the presence of a variety of important polypeptide domains, wherein the locations given for those importanCpolypeptide domains are approximate as described above. Analysis of the full-length PRO3434 sequence shown in Figure 14 evidences the presence of the following: a signal peptide from about amino acid 1 to about amino acid 16; cAMP- and cGMP-dependent protein kinase phosphorylation sites from about amino acid 154 to about amino acid 158, from about amino acid 331 to about amino acid 335, from about amino acid 616 to about amino acid 620, from about amino acid 785 to about amino acid 789, and from about amino acid 891 to about amino acid 895; potential N-myristoylation sites from about amino acid 91 to about amino acid 97, from about amino acid 136 to about amino acid 142, from about amino acid 224 to about amino acid 230, from about amino acid 435 to about amino acid 441, from about amino acid 439 to about amino acid 445, from about amino acid 443 to about amino acid 449, from about amino acid 665 to about amino acid 671, and from about amino acid 698 to about amino acid 704; amidation sites from about amino acid 329 to about amino acid 333, and from about amino acid 634 to about amino acid 638; and an oligoadenylate synthetase site from about amino acid 96 to about amino acid 135. Clone DNA77631-2537 has been deposited with ATCC on February 9, 1999 and is assigned ATCC deposit no. 203651.

An analysis of the Dayhoff database (version 35.45 SwissProt 35), using a WU-BLAST2 sequence alignment analysis of the full-length sequence shown in Figure 14 (SEQ ID NO:23), revealed significant sequence identity between the PRO3434 amino acid sequence and the following Dayhoff sequences: VATX_YEAST, P_R51171, POLS_1BDVP, IBDVORF_2, JC5043, IBDVPIV_1, VE7_HPV 11, GEN 14220, MUTS_THETH and COAC_CHICK.

EXAMPLE 10: Isolation of cDNA clones Encoding Human PRO1927

DNA82307-253 was identified by applying the proprietary signal sequence finding algorithm described in Example 2 above. Use of the above described signal sequence algorithm allowed identification of an ESTcluster sequence from the LIFESEQ® database, designated EST cluster no. 1913. This EST cluster sequence was then compared to a variety of expressed sequence tag (EST) databases which included public EST databases (*e.g.*, GenBank), a proprietary EST DNA database (LIFESEQ®, Incyte Pharmaceuticals, Palo Alto, CA), and an additional proprietary EST DNA database (Genentech, Inc., South San Francisco, CA) to identify existing homologies. The homology search was performed using the computer program BLAST or BLAST2 (Altshul *et al.*, Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The assembly included an EST from the Genentech database which is herein designated "DNA20168". The consensus sequence obtained therefrom is herein designated as DNA73896.

In light of the sequence homology between the DNA73896 sequence and EST clone no. 3326981H1, [obtained from a library constructed from RNA isolated from aortic tissue], EST clone no. 3326981H1 was purchased and the cDNA insert was obtained and sequenced. The sequence of this cDNA insert is shown in Figure 15 (SEQ ID NO:24) and is herein designated as DNA82307-2531.

Clone DNA82307-2531 contains a single open reading frame with an apparent translational initiation site at nucleotide positions 51-53 and ending at the stop codon at nucleotide positions 1695-1697 (Figure 15). The predicted polypeptide precursor is 548 amino acids long (Figure 16; SEQ ID NO:25). The full-length PRO1927 protein shown in Figure 16 has an estimated molecular weight of about 63,198 daltons and a pI of about 8.10. Analysis of the full-length PRO1927 sequence shown in Figure 16 (SEQ ID NO:25) evidences the presence of a variety of important polypeptide domains, wherein the locations given for those important polypeptide domains are approximate as described above. Analysis of the full-length PRO1927 sequence shown in Figure 16 evidences the presence of the following: a signal peptide from about amino acid I to about amino acid 23; potential N-glycosylation sites from about amino acid 5 to about amino acid 9, from about amino acid 87 to about amino acid 91, from about amino acid 103 to about amino acid 107, and from about amino acid 465 to about amino acid 469; and potential N-myristoylation sites from about amino acid 6 to about amino acid 12, from about amino acid 136 to about amino acid 142, from about amino acid 370 to about amino acid 376, and from about amino acid 509 to about amino acid 515. Clone DNA82307-2531 has been deposited with ATCC on December 15, 1998 and is assigned ATCC deposit no. 203537.

An analysis of the Dayhoff database (version 35.45 SwissProt 35), using a WU-BLAST2 sequence alignment analysis of the full-length sequence shown in Figure 16 (SEQ ID NO:25), evidenced significant homology between the PRO1927 amino acid sequence and the Dayhoff sequence AB000628_1. Homology was also revealed between the PRO1927 amino acid sequence and the following additional Dayhoff sequences: HGS_A251, HGS_A197, CELC50H11_2, CPXM_BACSU,VF03_VACCC, VF03_VACCV, DYHA_CHLRE, C69084, and A64315.

EXAMPLE 11: Isolation of cDNA clones Encoding Human PRO3567

A consensus DNA sequence was assembled relative to other EST sequences using phrap as described in Example 1 above. This consensus sequence is designated herein as DNA52711. Based on the DNA52711 consensus sequence and Merck EST clone no. AA082750, Merck EST clone no. AA082750 was purchased and its insert obtained and sequenced. The entire nucleotide sequence thereof is shown in Figure 17 (SEQ ID NO:26) and is herein designated DNA56049-2543.

Clone DNA56049-2543 contains a single open reading frame with an apparent translational initiation site at nucleotide positions 97-99, and an apparent stop codon at nucleotide positions 637-639. The predicted polypeptide precursor is 180 amino acids long. Analysis of the full-length PRO3567 sequence shown in Figure 18 (SEQ ID NO:27) evidences the presence of a variety of important polypeptide domains, wherein the locations given for those important polypeptide domains are approximate as described above. Analysis of the full-length PRO3567 polypeptide shown in Figure 18 evidences the presence of the following: a signal peptide from about amino acid 1 to about amino acid 25; a transmembrane domain from about amino acid 149 to about amino acid 164; an N-glycosylation site from about amino acid 141 to about amino acid 145; N-myristoylation sites from about amino acid 25 to about amino acid 31, and from about amino acid 135 to about amino acid 141; a prokaryotic membrane lipoprotein lipid attachment site from about amino acid 16 to about amino acid 27; a cell attachment sequence from about amino acid 112 to about amino acid 115; and a TonB-dependent receptor protein signature 1 from about amino acid 1 to about amino acid 21. Clone DNA56049-2543 has been deposited with the ATCC on February 9, 1999 and is assigned ATCC deposit no. 203662. The full-length PRO3567 protein shown in Figure 18 has an estimated molecular weight of about 20,313 daltons and a pI of about 8.91.

An analysis of the Dayhoff database (version 35.45 SwissProt 35), using a WU-BLAST2 sequence alignment analysis of the full-length sequence shown in Figure 18 (SEQ ID NO:27), revealed significant sequence identity between the PRO3567 amino acid sequence and the following Dayhoff sequences: SPC2_CANFA, SPC2_CHICK, SPC2_CAEEL, AF057144_1, YD2B_SCHPO, S61639, SPC3_YEAST, AMU21992_1, EPU22004_1, and CMU20539_1.

EXAMPLE 12: Isolation of cDNA clones Encoding Human PRO1295

DNA59218-1559 was identified by applying the proprietary signal sequence finding algorithm described in Example 2 above. Use of the above described signal sequence algorithm allowed identification of an ESTcluster sequence from the LIFESEQ® database. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. One or more of the ESTs was derived from a thymus tissue library. The homology search was performed using the computer program BLAST or BLAST2 (Altshul *et al.*, Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56262.

In light of the sequence homology between the DNA56262 sequence and Incyte EST no. 3743334, the clone including this EST was purchased and the cDNA insert was obtained and sequenced. The sequence of this cDNA insert is shown in Figure 19 (SEQ ID NO:28) and is herein designated as DNA59218-1559.

Clone DNA59218-1559 contains a single open reading frame with an apparent translational initiation site at nucleotide positions 207-209 and ending at the stop codon at nucleotide positions 1047-1049 (Figure 19). The predicted polypeptide precursor is 280 amino acids long (Figure 20; SEQ ID NO:29). The full-length PRO1295 protein shown in Figure 20 has an estimated molecular weight of about 30,163 daltons and a pI of about 6.87. Analysis of the full-length PRO1295 sequence shown in Figure 20 (SEQ ID NO:29) evidences the presence of a variety of important polypeptide domains, wherein the locations given for those important polypeptide domains are approximate as described above. Analysis of the full-length PRO1295 sequence shown in Figure 20 evidences the presence of the following: a signal peptide from about amino acid 1 to about amino acid 18; an N-glycosylation site from about amino acid 244 to about amino acid 248; and a microbodies C-terminal targeting signal from about amino acid 278 to about amino acid 282. Clone DNA59218-1559 has been deposited with ATCC on September 29, 1998 and is assigned ATCC deposit no. 203287.

An analysis of the Dayhoff database (version 35.45 SwissProt 35), using a WU-BLAST2 sequence alignment analysis of the full-length sequence shown in Figure 20 (SEQ ID NO:29), evidenced sequence identity between the PRO1295 amino acid sequence and the following Dayhoff sequences: AB011099_1, IL VE_MYCTU, ATTECR_2, AF010496_27, P_R15346, S37191, PER_DROMS, L2MU_ADECC and P_W34238.

EXAMPLE 13: Isolation of cDNA clones Encoding Human PRO1293

DNA60618-1557 was identified by applying the proprietary signal sequence finding algorithm described in Example 2 above. Use of the above described signal sequence algorithm allowed identification of an EST cluster sequence from the LIFESEQ® database, designated Incyte EST cluster sequence no. 115204. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul *et al.*, Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56522.

In light of the sequence homology between the DNA56522 sequence and Incyte EST clone no. 2966119, Incyte EST clone no. 2966119 was purchased and the cDNA insert was obtained and sequenced. The sequence of this cDNA insert is shown in Figure 21 (SEQ ID NO:30) and is herein designated as DNA60618-1557.

Clone DNA60618-1557 contains a single open reading frame with an apparent translational initiation site at nucleotide positions 37-39 and ending at the stop codon at nucleotide positions 1060-1062 (Figure 21). The predicted polypeptide precursor is 341 amino acids long (Figure 22; SEQ ID NO:31). The full-length PRO1293 protein shown in Figure 22 has an estimated molecular weight of about 38,070 daltons and a pI of about 6.88. Analysis of the full-length PRO1293 sequence shown in Figure 22 (SEQ ID NO:31) evidences the presence of a variety of important polypeptide domains, wherein the locations given for those important polypeptide domains are approximate as described above. Analysis of the full-length PRO1293 sequence shown in Figure 22 evidences the presence of the following: a signal peptide from about amino acid 1 to about amino acid 19; a transmembrane domain from about amino acid 237 to about amino acid 262; a potential N-glycosylation site from about amino acid 205 to about amino acid 209; a cell attachment sequence from about amino acid 151 to about amino acid 154; and an amino acid sequence block having homology to coproporphyrinogen III oxidase proteins from about amino acid 115 to about amino acid 141. Clone DNA60618-1557 has been deposited with ATCC on September 29, 1998 and is assigned ATCC deposit no. 203292.

An analysis of the Dayhoff database (version 35.45 SwissProt 35), using a WU-BLAST2 sequence alignment analysis of the full-length sequence shown in Figure 22 (SEQ ID NO:31), evidenced significant homology between the PRO1293 amino acid sequence and the following Dayhoff sequences: HSVCD54_1, A33_HUMAN, AF009220_1, HSU82279_1, AF004230_1, P_R13272, AF004231_1, AF043644_1, S44125 and HSIGGHC85_1.

EXAMPLE 14: Isolation of cDNA clones Encoding Human PRO1303

A consensus DNA sequence was assembled relative to other EST sequences using phrap as described in Example 1 above. This consensus sequence is designated herein as DNA47347. Based on the DNA47347 consensus sequence and its homology to an Incyte EST within the assembly from which DNA47347 was derived, Incyte clone no. 1430305 was purchased and its insert obtained and sequenced in full. The entire nucleotide sequence thereof is shown in Figure 23 (SEQ ID NO:32) and is herein designated DNA65409-1566.

Clone DNA65409-1566 contains a single open reading frame with an apparent translational initiation site at nucleotide positions 121-123, and an apparent stop codon at nucleotide positions 865-867. The predicted polypeptide precursor is 248 amino acids long. Analysis of the full-length PRO1303 sequence shown in Figure 24 (SEQ ID NO:33) evidences the presence of a variety of important polypeptide domains, wherein the locations given for those important polypeptide domains are approximate as described above. Analysis of the full-length PRO1303 polypeptide shown in Figure 24 evidences the presence of the following: a signal peptide from about amino acid 1 to about amino acid 17; N-glycosylation sites from about amino acid 24 to about amino acid 28, and from about amino acid 163 to about amino acid 167; a serine proteases, trypsin family, histidine active site from about amino acid 58 to about amino acid 64; serine proteases, trypsin family, histidine protein domains from about amino acid 47 to about amino acid 64, from about amino acid 196 to about amino acid 207, and from about amino acid 218 to about amino acid 242; kringle domain protein sites from about amino acid 47 to about amino acid 65, and from about amino 194 to about amino acid 207; and an apple domain site from about amino acid 220 to ablout amino acid 248. Clone DNA65409-1566 has been deposited with the ATCC on September 15, 1998 and is assigned ATCC deposit no. 203232. The full-length PRO1303 protein shown in Figure 24 has an estimated molecular weight of about 26,734 daltons and a pI of about 7.90.

An analysis of the Dayhoff database (version 35.45 SwissProt 35), using a WU-BLAST2 sequence alignment analysis of the full-length sequence shown in Figure 24 (SEQ ID NO:33), revealed homology between the PRO1303 amino acid sequence and the following Dayhoff sequences: AB009849_1, P_W08475, AF024605_1, A42048_1, TRY3_RAT, MMAE00066414, TRY1_RAT, MMAE000663_4, MMAE000665_2, and MMAE00066412.

EXAMPLE 15: Isolation of cDNA clones Encoding Human PRO4344

A consensus DNA sequence was assembled relative to other EST sequences using phrap as described in Example 1 above. This consensus sequence is designated herein as DNA80203. Based on the DNA80203 consensus sequence, oligonucleotides were synthesized: 1) to identify by PCR a cDNA library that contained the sequence of interest, and 2) for use as probes to isolate a clone of the full-length coding sequence for PRO4344.

A pair of PCR primers (forward and reverse) were synthesized:

forward PCR primer:

5'-CTTGCTCCTGGCCATCAAGTCAC-3' (SEQ ID NO:36)

reverse PCR primer:

5'-GTTGAAGAAGTCCTCAGTGAAGTCCCAC-3' (SEQ ID NO:37)

Additionally, a synthetic oligonucleotide hybridization probe was constructed from the consensus DNA80203 sequence which had the following nucleotide sequence:

hybridization probe:

5'-CAGCTGAAGCTGGTGTTCCTCCTAGGGGTGGCAGGATCCG-3' (SEQ ID NO:38)

In order to screen several libraries for a source of a full-length clone, DNA from the libraries was screened by PCR amplification with the PCR primer pair identified above. A positive library was then used to isolate clones encoding the PRO4344 gene using the probe oligonucleotide and one of the PCR primers. RNA for construction of the cDNA libraries was isolated from human aortic endothelial cells.

DNA sequencing of the isolated clones isolated as described above gave the full-length DNA sequence for DNA84927-2585 [Figure 25, SEQ ID NO:34]; and the derived protein sequence for PRO4344.

The entire coding sequence of DNA84927-2585 is included in Figure 25 (SEQ ID NO:34). Clone DNA84927-2585 contains a single open reading frame with an apparent translational initiation site at nucleotide positions 3S7-359, and an apparent stop codon at nucleotide positions 1491-1493. The predicted polypeptide precursor is 378 amino acids long. Analysis of the full-length PRO4344 sequence shown in Figure 26 (SEQ ID NO:35) evidences the presence of a variety of important polypeptide domains, wherein the locations given for those important polypeptide domains are approximate as described above. Analysis of the full-length PRO4344 polypeptide shown in Figure 26 evidences the presence of the following: a signal peptide from about amino acid I to about amino acid 39; a Type II transmembrane domain from about amino acid 30 to about amino acid 49; N-glycosylation sites from about amino acid 79 to about amino acid 83, from about amino acid 104 to about amino acid 108, and from about amino acid 192 to about amino acid 196; casein kinase 11 phosphorylation sites from about amino acid 194 to about amino acid 198, and from about amino acid 352 to about amino acid 356; N-myristoylation sites from about amino acid 14 to about amino acid 20, from about amino acid 160 to about amino acid 166, and from about amino acid 367 to about amino acid 373; and a prokaryotic membrane lipoprotein lipid attachment site from about amino acid 35 to about amino acid 46. Clone DNA84927-2585 has been deposited with the ATCC on March 23, 1999 and is assigned ATCC deposit no. 203865. The full-length PRO4344 protein shown in Figure 26 has an estimated molecular weight of about 42,310 daltons and a pl of about 9.58.

An analysis of the Dayhoff database (version 35.45 SwissProt 35), using a WU-BLAST2 sequence alignment analysis of the full-length sequence shown in Figure 26 (SEQ ID NO:35), revealed homology between the PRO4344 amino acid sequence and the following Dayhoff sequences: P_W64558, P_W80212, AF029790_1, P_R57433,AB003478_1,MMHC425O1814,DMU41449_1,DMSEG0007_10,DMC65G3_4,andFNG_DROME.

EXAMPLE 16: Isolation of cDNA clones Encoding Human PRO4354

DNA92256-2596 was identified by applying the proprietary signal sequence finding algorithm described in Example 2 above. Use of the above described signal sequence algorithm allowed identification of an ESTcluster (92909) sequence also referred to herein as "DNA 10195". This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul *et al.*, Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56063.

Based upon the cluster sequence and the sequence alignments, DNA92264-2596 was identified and sequenced in full. The entire coding sequence is included in Figure 27 (SEQ ID NO:39).

Clone DNA92256-2596 contains a single open reading frame with an apparent translational initiation site at nucleotide positions 108-110 and ending at the stop codon at nucleotide positions 852-854 (Figure 27). The predicted polypeptide precursor is 248 amino acids long (Figure 28; SEQ ID NO:40). The full-length PRO4354 protein shown in Figure 28 has an estimated molecular weight of about 28,310 daltons and a pl of about 4.63. Analysis of the full-length PRO4354 sequence shown in Figure 28 (SEQ ID NO:40) evidences the presence of a variety of important polypeptide domains, wherein the locations given for those important polypeptide domains are approximate as described above. Analysis of the full-length PRO4354 sequence shown in Figure 28 evidences the presence or the following: a signal peptide from about amino acid 1 to about amino acid 21; a cAMP- and cGMP-dependent protein kinase phosphorylation site from about amino acid 106 to about amino acid 110; casein kinase II phosphorylation sites from about amino acid 36 to about amino acid 40, from about amino acid 80 to about amino acid 84, from about amino acid 84 to about amino acid 88, from about amino acid 158 to about amino acid 162, from about amino acid 202 to about amino acid 206, from about amino acid 207 to about amino acid 211, and from about amino aacid 213 to about amino acid 217; an N-myristoylation site from about amino acid 115 to about amino acid 121; and an am idation site from about amino acid 70 to about amino acid 74. Clone DNA92256-2596 has been deposited with ATCC on March 30, 1999 and is assigned ATCC deposit no. 203891.

An analysis of the Dayhoff database (version 35.45 SwissProt 35), using a WU-BLAST2 sequence alignment analysis of the full-length sequence shown in Figure 28 (SEQ ID NO:40), revealed homology between the PRO4354 amino acid sequence and the following Dayhoff sequences: HGS_RF300, CEVK04G11_2, CEC11H1_7, HSU80744_1, CEF09E8_2, RNAJ2967_1, DDICOI_1, AB020648_1, P_W33887 and A64319.

EXAMPLE 17: Isolation of cDNA clones Encoding Human PRO4397

A consensus DNA sequence was assembled relative to other EST sequences using phrap as described in Example 1 above. This consensus sequence is designated herein as DNA79196. Based on the DNA79196 consensus sequence, oligonucleotides were synthesized: 1) to identify by PCR a cDNA library that contained the sequence of interest, and 2) for use as probes to isolate a clone of the full-length coding sequence for PRO4397.

A pair of PCR primers (forward and reverse) were synthesized:

forward PCR primer:

5'-ACCTAACGCTCAAGGAGATCCACTTTC-3' (SEQ ID NO:43)

reverse PCR primer:

5'-GGCTCCATTCTGGGTCTGAGTTAGG-3' (SEQ ID NO:44)

Additionally, a synthetic oligonucleotide hybridization probe was constructed from the consensus DNA79196 sequence which had the following nucleotide sequence:

hybridization probe:

5'-GCCTCAGCTTTCTGCCCCGACGTGCGCTTCGTTTTTAAGG-3' (SEQ ID NO:45)

In order to screen several libraries for a source of a full-length clone, DNA from the libraries was screened by PCR amplification with the PCR primer pair identified above. A positive library was then used to isolate clones encoding the PRO4397 gene using the probe oligonucleotide and one of the PCR primers. RNA for construction of the cDNA libraries was isolated from a human adenocarcinoma cell line.

DNA sequencing of the isolated clones isolated as described above gave the full-length DNA sequence for DNA83505-2606 [Figure 29, SEQ ID NO:41]; and the derived protein sequence for PRO4397.

The entire coding sequence of DNA83505-2606 is included in Figure 29 (SEQ ID NO:41). Clone DNA83505-2606 contains a single open reading frame with an apparent translational initiation site at nucleotide positions 254-256, and an apparent stop codon at nucleotide positions 1460-1462. The predicted polypeptide precursor is 402 amino acids long. Analysis of the full-length PRO4397 sequence shown in Figure 30 (SEQ ID NO:42) evidences the presence of a variety of important polypeptide domains, wherein the locations given forthose important polypeptide domains are approximate as described above. Analysis of the full-length PRO4397 polypeptide shown in Figure 30 evidences the presence of the following: a signal peptide from about amino acid 1 to about amino acid 27; an N-glycosylation site from about amino acid 203 to about amino acid 207; casein kinase II phosphorylation sites from about amino acid 124 to about amino acid 128, from about amino acid 205 to about amino acid 209, from about amino acid 351 to about amino acid 355, and from about amino acid 368 to about amino acid 372; N-myristoylation sites from about amino acid 18 to about amino acid 24, from about amino acid 31 to about amino acid 37, from about amino acid 110 to about amino acid 116, from about amino acid 157 to about amino acid 163, from about amino acid 161 to about amino acid 167, from about amino acid 163 to about amino acid 169, and from about amino acid 366 to about amino acid 372; and a cell attachment sequence from about amino acid 107 to about amino acid 110. Clone DNA83505-2606 has been deposited with the ATCC on May 25, 1999 and is assigned ATCC deposit no. 132-PTA. The full-length PRO4397 protein shown in Figure 30 has an estimated molecular weight of about 43,751 daltons and a pI of about 9.42.

An analysis of the Dayhoff database (version 35.45 SwissProt 35), using a WU-BLAST2 sequence alignment analysis of the full-length sequence shown in Figure 30 (SEQ ID NO:42), revealed homology between the PRO4397 amino acid sequence and the following Dayhoff sequences: P_W64558, P_W80212, HSGALT2_1, P_R57433, AF100956_7, HS1033B10_2, AF029792_1, DMU41449_1, DMSEG0007_10, and AF092051_1.

EXAMPLE 18: Isolation of cDNA clones Encoding Human PRO4407

DNA92264-2616 was identified by applying the proprietary signal sequence finding algorithm described in Example 2 above. Use ofthe above described signal sequence algorithm allowed identification of an ESTcluster sequence from the LIFESEQ® database. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul *et al.*, Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). Based upon the cluster sequence and the sequence alignments, DNA92264-2616 was identified and sequenced.

The entire coding sequence of DNA92264-2616 is included in Figure 31 (SEQ ID NO:46). Clone DNA92264-2616 contains a single open reading frame with an apparent translational initiation site at nucleotide positions 109-111 and ending at the stop codon at nucleotide positions 757-759 (Figure 31). The predicted polypeptide precursor is 216 amino acids long (Figure 32; SEQ ID NO:47). The full-length PRO4407 protein shown in Figure 32 has an estimated molecular weight of about 23,729 daltons and a pI of about 4.73. Analysis of the full-length PRO4407 sequence shown in Figure 32 (SEQ ID NO:47) evidences the presence of a variety of important polypeptide domains, wherein the locations given for those important polypeptide domains are approximate as described above. Analysis of the full-length PRO4407 sequence shown in Figure 32 evidences the presence of the following: a signal peptide from about amino acid I to about amino acid 25; a transmembrane domain from about amino acid 4 to about amino acid 59; casein kinase II phosphorylation sites from about amino acid 129 to about amino acid 133, and from about amino acid 173 to about amino acid 177; and an N-myristoylation site from about amino acid 133 to about amino acid 139. Clone DNA92264-2616 has been deposited with ATCC on April 27, 1999 and is assigned ATCC deposit no. 203969.

An analysis of the Dayhoff database (version 35.45 SwissProt 35), using a WU-BLAST2 sequence alignment analysis of the full-length sequence shown in Figure 32 (SEQ ID NO:47), revealed homology between the PRO4407 amino acid sequence and the following Dayhoff sequences: SC1E6_12, D80003_1, HMGA_SOYBN, DROTRO12_1, HSU91934_1, GEN14338, AF051945_1, A45644, P_W60213, and P_W33807.

EXAMPLE 19: Isolation of cDNA clones Encoding Human PRO1555

DNA73744-1665 was identified by applying the proprietary signal sequence finding algorithm described in Example 2 above. Use of the above described signal sequence algorithm allowed identification of an ESTcluster sequence from the LIFESEQ® database, designated EST cluster no. 521, and also referred to herein as "DNA10316". This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul *et al.*, Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence is herein designated DNA56374.

In light of the sequence homology between the DNA56374 sequence and Incyte EST no. 2855769, Incyte EST no. 2855769 was purchased and the cDNA insert was obtained and sequenced. Incyte EST no. 2855769 was derived from a library constructed from female breast fat tissue. The sequence of this cDNA insert is herein designated as DNA73744-1665.

The entire coding sequence is included in Figure 33 (SEQ ID NO:48). Clone DNA73744-1665 contains a single open reading frame with an apparent translational initiation site at nucleotide positions 90-92 and ending at the stop codon at nucleotide positions 828-830 (Figure 33). The predicted polypeptide precursor is 246 amino acids long (Figure 34; SEQ ID NO:49). The full-length PRO1555 protein shown in Figure 34 has an estimated molecular weight of about 26,261 daltons and a pl of about 5.65. Analysis of the full-length PRO1555 sequence shown in Figure 34 (SEQ ID NO: 49) evidences the presence of a variety of important polypeptide domains, wherein the locations given forthose important polypeptide domains are approximate as described above. Analysis of the full-length PRO1555 sequence shown in Figure 34 evidences the presence of the following: a signal peptide from about amino acid 1 to about amino acid 31; transmembrane domains from about amino acid 11 to about amino acid 32 and from about amino acid 195 to about amino acid 217; a potential N-glycosylation site from about amino acid 111 to about amino acid 115; potential casein kinase II phosphorylation sites from about amino acid 2 to about amino acid 6, from about amino acid 98 to about amino acid 102, and from about amino acid 191 to about amino acid 195; and potential N-myristoylation sites from about amino acid 146 to about amino acid 152, and from about amino acid 192 to about amino acid 198. Clone DNA73744-1665 has been deposited with ATCC on October 6, 1998 and is assigned ATCC deposit no. 203322.

An analysis of the Dayhoff database (version 35.45 SwissProt 35), using a WU-BLAST2 sequence alignment analysis of the full-length sequence shown in Figure 34 (SEQ ID NO:49), evidenced some homology between the PRO1555 amino acid sequence and the following Dayhoff sequences: YKA4_CAEEL, AB014541_1, HVSX99518_2, SSU63019_1, GEN14286, MMU68267_1, XP2_XENLA, ICP4_HSV11, P_W40200, and AE001360_1.

EXAMPLE 20

Gene Amplification

This example shows that the PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding genes are amplified in the genome of certain human lung, colon and/or breast cancers and/or cell lines. Amplification is associated with overexpression of the gene product, indicating that the polypeptides are useful targets for therapeutic intervention in certain cancers such as colon, lung, breast and other cancers. Therapeutic agents may take the form of antagonists of PRO381, PRO1269, PRO1410, PRO1755, PRO1780, PRO1788, PRO3434, PRO1927, PRO3567, PRO1295, PRO1293, PRO1303, PRO4344, PRO4354,PRO4397, PRO4407, PRO1555, PRO1096, PRO2038 or PRO2262 polypeptides, for example, murine-human chimeric, humanized or human antibodies against a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

The starting material for the screen was genomic DNA isolated from a variety of cancers. The DNA is quantitated precisely, *e.g.*, fluorometrieally. As a negative control, DNA was isolated from the cells of ten normal healthy individuals which was pooled and used as assay controls for the gene copy in healthy individuals (not shown). The 5' nuclease assay (for example, TaqMan™) and real-time quantitative PCR (for example, ABI Prizm 7700 Sequence Detection System™ (Perkin Elmer, Applied Biosystems Division, Foster City, CA)), were used to find genes potentially amplified in certain cancers. The results were used to determine whether the DNA encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 is over-represented in any of the primary lung or colon cancers or cancer cell lines or breast cancer cell lines that were screened. The primary lung cancers were obtained from individuals with tumors of the type and stage as indicated in Table 4. An explanation of the abbreviations used for the designation of the primary tumors listed in Table 4 and the primary tumors and cell lines referred to throughout this example has been given hereinbefore.

The results of the TaqMan™ are reported in delta (Δ) Ct units. One unit corresponds to 1 PCR cycle or approximately a 2-fold amplification relative to normal, two units corresponds to 4-fold, 3 units to 8-fold amplification and so on. Quantitation was obtained using primers and a TaqMan™ fluorescent probe derived from the PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-encoding gene. Regions of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 which are most likely to contain unique nucleic acid sequences and which are least likely to have spliced out introns are preferred for the primer and probe derivation, *e.g.*, 3'-untranslated regions. The sequences for the primers and probes (forward, reverse and probe) used for the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 gene amplification analysis were as follows:

PRO381 (DNA44194-1317)

44194.tm.f:
5'-CTTTGAATAGAAGACTTCTGGACAATTT-3' (SEQ ID NO:56)

44194.tm.p:
5'-TTGCAACTGGGAATATACCACGACATGAGA-3' (SEQ ID NO:57)

44194.tm.r:
5'-TAGGGTGCTAATTTGTGCTATAACCT-3' (SEQ ID NO:58)

44194.tm.f2:
5'-GGCTCTGAGTCTCTGCTTGA-3' (SEQ ID NO:59)

44194.tm.p2:
5'-TCCAACAACCATTTTCCTCTGGTCC-3' (SEQ ID NO:60)

44194.tm.r2:
5'-AAGCAGTAGCCATTAACAAGTCA-3' (SEQ ID NO:61)

PRO1269 (DNA66520-1536)

66520.tm.f1:
5'-AAAGGACACCGGGATGTG-3' (SEQ ID NO:62)

66520.tm.p1:
5'-AGCGTACACTCTCTCCAGGCAACCAG-3' (SEQ ID NO:63)

66520.tm.r1:
5'-CAATTCTGGATGAGGTGGTAGA-3' (SEQ ID NO:64)

PRO1410 (DNA68874-1622)

68874.tm.f1:

5'-CAGGACTGAGCGCTTGTTTA-3' (SEQ ID NO:65)

68874.tm.p1:

5'-CAAAGCGCCAAGTACCGGACC-3' (SEQ ID NO:66)

68874.tm.r1:

5'-CCAGACCTCAGCCAGGAA-3' (SEQ ID NO:67)

PRO1755 (DNA76396-1698)

76396.tm.f1:

5'-TCATGGTCTCGTCCCATTC-3' (SEQ ID NO:68)

76396.tm.p1:

5'-CACCATTTGTTTCTCTGTCTCCCCATC-3' (SEQ ID NO:69)

76396.tm.r1:

5'-CCGGCATCCTTGGAGTAG-3' (SEQ ID NO:70)

PRO1780 (DNA71169-1709)

71169.tm.f1:

5'-CTCTGGTGCCCACAGTGA-3' (SEQ ID NO:71)

71169.tm.p1:

5'-CCATGCCTGCTCAGCCAAGAA-3' (SEQ ID NO:72)

71169.tm.r1:

5'-CAGGAAATCTGGAAACCTACAGT-3' (SEQ ID NO:73)

PRO1788 (DNA77652-2505)

77652.tm.f1:

5'-TCCCCATTAGCACAGGAGTA-3' (SEQ ID NO:74)

77652.tm.p1:

5'-AGGCTCTTGCCTGTCCTGCTGCT-3' (SEQ ID NO:75)

77652.tm.r1:

5'-GCCCAGAGTCCCACTTGT-3' (SEQ ID NO:76)

PRO3434 (DNA77631-2537)

77631.tm.f1:

5'-GTCCAGCAAGCCCTCATT-3' (SEQ ID NO:77)

77631.tm.p1:

5'-CTTCTGGGCCACAGCCCTGC-3' (SEQ ID NO:78)

77631.tm.r1:

5'-CAGTTCAGGTCGTTTCATTCA-3' (SEQ ID NO:79)

PRO1927 (DNA82307-2531)

82307.tm.f1:

5'-CCAGTCAGGCCGTTTTAGA-3' (SEQ ID NO:80)

82307.tm.p1:

5'-CGGGCGCCCAAGTAAAAGCTC-3' (SEQ ID NO:81)

82307.tm.r1:

5'-CATAAAGTAGTATATGCATTCCAGTGTT-3' (SEQ ID NO:82)

PRO3567 (DNA56049-2543)

56049.tm.f1:

5'-GGAAATGGTCTCAAGGGAAA-3' (SEQ ID NO:83)

56049.tm.p1:

5'-TCACTTTGACCCTGTCTTGGAACGTC-3' (SEQ ID NO:84)

56049.tm.r1:

5'-GGTAGAATTCCAGCATTTGGTA-3' (SEQ ID NO:85)

PRO1295 (DNA59218-1559)

59218.tm.f1:

5'-AGGACTTGCCCTCAGGAA-3' (SEQ ID NO:86)

59218.tm.r1:

5'-CGCAGGACAGTTGTGAAAATA-3' (SEQ ID NO:87)

59218.tm.p1:

5'-ATGACGCTCGTCCAAGGCCAC-3' (SEQ ID NO:88)

PRO1293 (DNA60618-1557)

60618.tm.f1:

5'-CCCACCTGTACCACCATGT-3' (SEQ ID NO:89)

60618.tm.p1:

5'-ACTCCAGGCACCATCTGTTCTCCC-3' (SEQ ID NO:90)

60618.tm.r1:

5'-AAGGGCTGGCATTCAAGTU-3' (SEQ ID NO:91)

PRO1303 (DNA65409-1566)

65409.tm.f1:

5'-CTGGCCCTCAGAGCACCAAT-3' (SEQ ID NO:92)

65409.tm.p1:

5'-TCCTCCATCACTTCCCCTAGCTCCA-3' (SEQ ID NO:93)

65409.tm.r1:

5'-CTGGCAGGAGTTAAAGTTCCAAGA-3' (SEQ ID NO:94)

PRO4344 (DNA84927-2585)

84927.tm.f1:

5'-GGGCAACAGCCTGAGAGT-3' (SEQ ID NO:95)

84927.tm.p1:

5'-ACTCAGTGTTGATTCTCTATCGTGATGCG-3' (SEQ ID NO:96)

84927.tm.r1:

5'-GAGCAGCAGGCATCAATTT-3' (SEQ ID NO:97)

PRO4354 (DNA92256-2596)

92256.tm.f1:

5'-GGCCTGGAGTTGCTGATAA-3' (SEQ ID NO:98)

92256.tm.p1:

5'-TTGAGCTTAAGTAGACCAAGTATCTATCCCACCTAAA-3' (SEQ ID NO:99)

92256.tm.r1

5'-GGTGGGCTCTGGGTTACA-3' (SEQ ID NO:100)

PRO4397 (DNA83505-2606)

83505.tm.f1:

5'-AGCCGGTTTCCCAGATTAT-3' (SEQ ID NO:101)

83505.tm.p1:

5'-TGCCGTGTATGTGGTTCTTCCCTG-3' (SEQ ID NO:102)

83505.tm.r1:

5'-GAGACAGGCACCTGGTGAT-3' (SEQ ID NO:103)

PRO4407 (DNA92264-2616)

92264.tm.f1:

5'-TGTTTCTGCCTGGACATCA-3' (SEQ ID NO:104)

92264.tm.r1:

5'-GCTTACCGTGGCCTGACT-3' (SEQ ID NO:105)

92264.tm.p1:

5'-TCCTCAGGGTCCAAGTCCCCAT-3' (SEQ ID NO:106)

PRO1555 (DNA73744-1665)

73744.tm.f1:

5'-CCTTGAAAAGGACCCAGTTT-3' (SEQ ID NO:107)

73744.tm.p1:

5'-ATGAGTCGCACCTGCTGTTCCC-3' (SEQ ID NO:108)

73744.tm.r1:

5'-TAGCAGCTGCCCTTGGTA-3' (SEQ ID NO:109)

73744.tm.f2:

5'-AACAGCAGGTGCGACTCATCTA-3' (SEQ ID NO:110)

73744.tm.p2:

5'-TGCTAGGCGACGACACCCAGACC-3' (SEQ ID NO:111)

73744.tm.r2 :

5'-TGGACACGTGGCAGTGGA-3' (SEQ ID NO:112)

PRO1096 (DNA61870)

61870.tm.f1:

5'-TGGACCATGAAGCCAGTTT-3' (SEQ ID NO:113)

61870.tm.p1:

5'-CCTTTTTAGTTGGCTAACTGACCTGGAAAGAA-3' (SEQ ID NO:114)

61870.tm.r1:

5'-TGAATAGTCACTTTGAGGTTATTGC-3' (SEQ ID NO:115)

PRO2038 (DNA83014)

83014.tm.f1:

5'-CCTGGCTCCACCTGTGAT-3' (SEQ ID NO:116)

83014.tm.p1:

5'-ACCTCCCCCTGCTTCCTGCTG-3' (SEQ ID NO:117)

83014.tm.r1:

5'-CCTCAGACCCCATGAGTGA-3' (SEQ ID NO:118)

PRO2262 (DNA88273)

88273.tm.f1:

5'-GAGGAATGGCCCAACAGT-3' (SEQ ID NO:119)

88273.tm.p1:

5'-TGGCAGCCACCCTTCAGTGAG-3' (SEQ ID NO:120)

88273.tm.r1:

5'-CAGCACATCACGTGTCCA-3' (SEQ ID NO:121)

88273.tm.f2:

5'-GAGGAATGGCCCAACAGT-3' (SEQ ID NO:122)

88273.tm.p2:

5'-TGTCCATGCCCCTGGTCCAC-3' (SEQ ID NO:123)

88273.tm.r2 :

5'-GAGGTACAGAGCAGCACATCA-3' (SEQ ID NO:124)

The 5' nuclease assay reaction is a fluorescent PCR-based technique which makes use of the 5' exonuclease activity of Taq DNA polymerase enzyme to monitor amplification in real time. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

The 5' nuclease procedure is run on a real-time quantitative PCR device such as the ABI Prism 7700TM Sequence Detection. The system consists of a thermocycler, laser, charge-coupled device (CCD) camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5' Nuclease assay data are initially expressed as Ct, or the threshold cycle. This is defined as the cycle at which the reporter signal accumulates above the background level of fluorescence. The ΔCt values are used as quantitative measurement of the relative number of starting copies of a particular target sequence in a nucleic acid sample when comparing cancer DNA results to normal human DNA results.

Table 4 describes the stage, T stage and N stage of various primary tumors which were used to screen the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 compounds of the invention.

Table 4 Primary Lung and Colon Tumor Profiles

| Primary Tumor | Stage | Other Stage | Dukes Stage | T Stage | N Stage |
|---|---|---|---|---|---|
| Human lung tumor AdenoCa (SRCC724) [LT1] | IIA | | | T1 | N I |
| Human lung tumor SqCCa (SRCC725) [LT1a] | IIB | | | T3 | N0 |
| Human lung tumor AdenoCa (SRCC726) [LT2] | IB | | | T2 | N0 |
| Human lung tumor AdenoCa (SRCC727) [LT3] | IIIA | | | T1 | N2 |
| Human lung tumor AdenoCa (SRCC728) [LT4] | IB | | | T2 | N0 |
| Human lung tumor SqCCa (SRCC729) [LT6] | IB | | | T2 | N0 |
| Human lung tumor Aden/SqCCa (SRCC730) [LT7] | IA | | | T1 | N0 |
| Human lung tumor AdenoCa (SRCC731)[LT9] | IB | | | T2 | N0 |
| Human lung tumor SqCCa (SRCC732) [LT10] | IIB | | | T2 | N I |
| Human lung tumor SqCCa (SRCC733) [LT11] | IIA | | | T1 | N1 |
| Human lung tumor AdenoCa (SRCC734) [LT12] | IV | | | T2 | N0 |
| Human lung tumor AdenoSqCCa (SRCC735) [LT13] | IB | | | T2 | N0 |
| Human lung tumor SqCCa (SRCC736) [LT15] | IB | | | T2 | N0 |
| Human lung tumor SqCCa (SRCC737) [LT16] | IB | | | T2 | N0 |
| Human lung tumor SqCCa (SRCC738) [LT17] | IIB | | | T2 | N 1 |
| Human lung tumor SqCCa (SRCC739) [LT18] | IB | | | T2 | N0 |
| Human lung tumor SqCCa (SRCC740) [LT19] | IB | | | T2 | N0 |
| Human lung tumor LCCa (SRCC741) [LT21] | IIB | | | T3 | N 1 |
| Human lung AdenoCa (SRCC811) [LT22] | 1A | | | T1 | N0 |
| Human colon AdenoCa (SRCC742) [CT2] | | M1 | D | pT4 | N0 |
| Human colon AdenoCa (SRCC743) [CT3] | | | B | pT3 | N0 |
| Human colon AdenoCa (SRCC 744) [CT8] | | | B | T3 | N0 |
| Human colon AdenoCa (SRCC745) [CT10] | | | A | pT2 | N0 |
| Human colon AdenoCa (SRCC746) [CT12] | | MO, R1 | B | T3 | N0 |
| Human colon AdenoCa (SRCC747) [CT14] | | pMO, RO | B | pT3 | pN0 |
| Human colon AdenoCa (SRCC748) [CT15] | | M1, R2 | D | T4 | N2 |
| Human colon AdenoCa (SRCC749) [CT16] | | pMO | B | pT3 | pN0 |
| Human colon AdenoCa (SRCC750) [CT17] | | | C 1 | pT3 | pN1 |
| Human colon AdenoCa (SRCC751) [CT1] | | MO, R1 | B | pT3 | N0 |
| Human colon AdenoCa (SRCC752) [CT4] | | | B | pT3 | MO |
| Human colon AdenoCa (SRCC753) [CT5] | | G2 | C 1 | pT3 | pN0 |
| Human colon AdenoCa (SRCC754) [CT6] | | pMO, RO | B | pT3 | pN0 |
| Human colon AdenoCa (SRCC755)[CT7] | | G I | A | pT2 | pN0 |
| Human colon AdenoCa (SRCC756) [CT9] | | G3 | D | pT4 | pN2 |
| Human colon AdenoCa (SRCC757) [CT11] | | | B | T3 | N0 |
| Human colon AdenoCa (SRCC758) [CT18] | | MO, RO | B | pT3 | pN0 |

DNA Preparation:

DNA was prepared from cultured cell lines, primary tumors, and normal human blood. The isolation was performed using purification kit, buffer set and protease and all from Quiagen, according to the manufacturer's instructions and the description below.

*Cell culture lysis:*

Cells were washed and trypsinized at a concentration of 7.5 x $10^8$ per tip and pelleted by centrifuging at 1000 rpm for 5 minutes at 4°C, followed by washing again with 1/2 volume of PBS and recentrifugation. The pellets were washed a third time, the suspended cells collected and washed 2x with PBS. The cells were then suspended into 10 ml PBS. Buffer C1 was equilibrated at 4°C. Qiagen protease #19155 was diluted into 6.25 ml cold $ddH_2O$ to a final concentration of 20 mg/ml and equilibrated at 4°C. 10 ml of G2 Buffer was prepared by diluting Qiagen RNAse A stock (100 mg/ml) to a final concentration of 200 $\mu$g/ml.

Buffer C 1 (10 ml, 4°C) and ddH2O (40 ml, 4°C) were then added to the 10 ml of cell suspension, mixed by inverting and incubated on ice for 10 minutes. The cell nuclei were pelleted by centrifuging in a Beckman swinging bucket rotor at 2500 rpm at 4°C for 15 minutes. The supernatant was discarded and the nuclei were suspended with a vortex into 2 ml Buffer C 1 (at 4°C) and 6 ml $ddH_2O$, followed by a second 4°C centrifugation at 2500 rpm for 15 minutes. The nuclei were then resuspended into the residual buffer using 200 $\mu$l per tip. G2 buffer (10 ml) was added to the suspended nuclei while gentle vortexing was applied. Upon completion of buffer addition, vigorous vortexing was applied for 30 seconds. Quiagen protease (200 $\mu$l, prepared as indicated above) was added and incubated at 50°C for 60 minutes. The incubation and centrifugation were repeated until the lysates were clear (*e.g.*, incubating additional 30-60 minutes, pelleting at 3000 x g for 10 min., 4°C).

*Solid human tumor sample preparation and lysis:*

Tumor samples were weighed and placed into 50 ml conical tubes and held on ice. Processing was limited to no more than 250 mg tissue per preparation (1 tip/preparation). The protease solution was freshly prepared by diluting into 6.25 ml cold $ddH_2O$ to a final concentration of 20 mg/ml and stored at 4°C. G2 buffer (20 ml) was prepared by diluting DNAse A to a final concentration of 200 mg/ml (from 100 mg/ml stock). The tumor tissue was homogenated in 19 ml G2 buffer for 60 seconds using the large tip of the polytron in a laminar-flow TC hood in order to avoid inhalation of aerosols, and held at room temperature. Between samples, the polytron was cleaned by spinning at 2 x 30 seconds each in 2L $ddH_2O$, followed by G2 buffer (50 ml). If tissue was still present on the generator tip, the apparatus was disassembled and cleaned.

Quiagen protease (prepared as indicated above, 1.0 ml) was added, followed by vortexing and incubation at 50°C for 3 hours. The incubation and centrifugation were repeated until the lysates were clear (*e.g.,* incubating additional 30-60 minutes, pelleting at 3000 x g for 10 min., 4°C).

*Human blood preparation and lysis:*

Blood was drawn from healthy volunteers using standard infectious agent protocols and citrated into 10 ml samples per tip. Quiagen protease was freshly prepared by dilution into 6.25 ml cold $ddH_2O$ to a final concentration of 20 mg/ml and stored at 4°C. G2 buffer was prepared by diluting RNAse A to a final concentration of 200 $\mu$g/ml from 100 mg/ml stock. The blood (10 ml) was placed into a 50 ml conical tube and 10 ml C 1 buffer and 30 ml $ddH_2O$ (both previously equilibrated to 4°C) were added, and the components mixed by inverting and held on ice for 10 minutes. The nuclei were pelleted with a Beckman swinging bucket rotor at 2500 rpm, 4°C for 15 minutes and the supernatant discarded. With a vortex, the nuclei were suspended into 2 ml C 1 buffer (4°C) and 6 ml $ddH_2O$ (4°C). Vortexing was repeated until the pellet was white. The nuclei were then suspended into the residual buffer using a 200 $\mu$l tip. G2 buffer (10 ml) was added to the suspended nuclei while gently vortexing, followed by vigorous vortexing for 30 seconds. Quiagen protease was added (200 $\mu$l) and incubated at 50°C for 60 minutes. The incubation and centrifugation were repeated until the lysates were clear (*e.g.*, incubating additional 30-60 minutes, pelleting at 3000 x g for 10 min., 4°C).

*Purification of cleared lysates:*

(1) Isolation of genomic DNA:

Genomic DNA was equilibrated (1 sample per maxi tip preparation) with 10 ml QBT buffer. QF elution buffer was equilibrated at 50°C. The samples were vortexed for 30 seconds, then loaded onto equilibrated tips and drained by gravity. The tips were washed with 2 x 15 ml QC buffer. The DNA was eluted into 30 ml silanized, autoclaved 30 ml Corex tubes with 15 ml QF buffer (50°C). Isopropanol (10.5 ml) was added to each sample, the tubes covered with parafin and mixed by repeated inversion until the DNA precipitated. Samples were pelleted by centrifugation in the SS-34 rotor at 15,000 rpm for 10 minutes at 4°C. The pellet location was marked, the supernatant discarded, and 10 ml 70% ethanol (4°C) was added. Samples were pelleted again by centrifugation on the SS-34 rotor at 10,000 rpm for 10 minutes at 4°C. The pellet location was marked and the supernatant discarded. The tubes were then placed on their side in a drying rack and dried 10 minutes at 37°C, taking care not to overdry the samples.

After drying, the pellets were dissolved into 1.0 ml TE (pH 8.5) and placed at 50°C for 1-2 hours. Samples were held overnight at 4°C as dissolution continued. The DNA solution was then transferred to 1.5 ml tubes with a 26 gauge needle on a tuberculin syringe. The transfer was repeated 5x in order to shear the DNA. Samples were then placed at 50°C for 1-2 hours.

(2) Quantitation of genomic DNA and preparation for gene amplification assay:

The DNA levels in each tube were quantified by standard $A_{260}/A_{280}$ spectrophotometry on a 1:20 dilution (5 μl DNA + 95 μl $ddH_2O$) using the 0.1 ml quartz cuvettes in the Beckman DU640 spectrophotometer. $A_{260}/A_{280}$ ratios were in the range of 1.8-1.9. Each DNA sample was then diluted further to approximately 200 ng/ml in TE (pH 8.5). If the original material was highly concentrated (about 700 ng/μl), the material was placed at 50°C for seceral hours until resuspended.

Fluorometric DNA quantitation was then performed on the diluted material (20-600 ng/ml) using the manufacturer's guidelines as modified below. This was accomplished by allowing a Hoeffer DyNA Quant 200 fluorometer to warm-up for about 15 minutes. The Hoechst dye working solution (#H33258, 10 μl, prepared within 12 hours of use) was diluted into 100 ml 1 x TNE buffer. A 2 ml cuvette was filled with the fluorometer solution, placed into the machine, and the machine was zeroed. pGEM 3Zf(+) (2 μl, lot #360851026) was added to 2 ml of fluorometer solution and calibrated at 200 units. An additional 2 μl ofpGEM 3Zf(+) DNA was then tested and the reading confirmed at 400 +/- 10 units. Each sample was then read at least in triplicate. When 3 samples were found to be within 10% of each other, their average was taken and this value was used as the quantification value.

The fluorometricly determined concentration was then used to dilute each sample to 10 ng/μl in $ddH_2O$. This was done simultaneously on all template samples for a single TaqMan plate assay, and with enough material to run 500-1000 assays. The samples were tested in triplicate with Taqman™ primers and probe both B-actin and GAPDH on a single plate with normal human DNA and no-template controls. The diluted samples were used provided that the CT value of normal human DNA subtracted from test DNA was +/- 1 Ct. The diluted, lot-qualified genomic DNA was stored in 1.0 ml aliquots at -80°C. Aliquots which were subsequently to be used in the gene amplification assay were stored at 4°C. Each 1 ml aliquot is enough for 8-9 plates or 64 tests.

*Gene amplification assay:*

The PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 compounds of the invention were screened in the following primary tumors and the resulting ΔCt values are reported in Tables 5A-5B.

Table 5A

$\Delta Ct$ values in lung and colon primary tumors and cell line models

| Primary Tumors or Cell lines | PRO381 | PRO1269 | PRO1410 | PRO1755 | PRO1780 | PRO1788 | PRO3434 | PRO1927 | PRO3567 | PRO1295 |
|---|---|---|---|---|---|---|---|---|---|---|
| LT1 | --- | --- | --- | --- | --- | --- | --- | --- | --- | --- |
| LT1-a | --- | --- | --- | --- | --- | --- | --- | --- | --- | --- |
| LT2 | --- | --- | --- | --- | --- | --- | --- | --- | --- | --- |
| LT3 | --- | --- | --- | --- | --- | --- | --- | --- | --- | --- |
| LT4 | --- | --- | --- | --- | 1.16 | --- | --- | --- | --- | --- |
| LT6 | --- | --- | --- | --- | --- | --- | --- | --- | --- | --- |
| LT7 | --- | --- | --- | --- | 1.02 | --- | --- | --- | --- | --- |
| LT9 | --- | --- | --- | --- | --- | --- | --- | --- | --- | --- |
| LT10 | --- | --- | --- | --- | --- | --- | --- | --- | --- | --- |
| LT11 | --- | --- | ---- | --- | --- | --- | --- | --- | --- | --- |
| LT12 | --- | --- | --- | --- | --- | --- | --- | --- | --- | --- |
| LT13 | --- | --- | 1.12<br>1.42 | --- | --- | --- | 5.24<br>4.47 | 4.38<br>4.80 | --- | --- |
| LT15 | --- | 1.22 | 2.10<br>1.82 | --- | --- | --- | 1.24 | --- | --- | --- |

Table 5A Continued

ΔCt values in lung and colon primary tumors and cell line models

| Primary Tumors or Cell lines | PRO381 | PRO1269 | PRO1410 | PRO1755 | PRO1780 | PRO1788 | PRO3434 | PRO1927 | PRO3567 | PRO1295 |
|---|---|---|---|---|---|---|---|---|---|---|
| LT16 | --- | 1.14 | 1.44<br>1.45 | 1.36 | --- | --- | 3.65<br>3.19 | 2.73<br>2.74 | --- | --- |
| LT17 | --- | 1.26 | --- | --- | --- | --- | --- | --- | --- | --- |
| LT18 | --- | --- | --- | 1.18 | --- | --- | --- | --- | --- | --- |
| LT19 | --- | --- | --- | --- | --- | --- | --- | --- | --- | --- |
| LT21 | --- | --- | --- | --- | --- | --- | --- | --- | --- | --- |
| CT2 | --- | --- | 2.36 | 2.35 | --- | --- | --- | --- | --- | --- |
| CT3 | --- | --- | 1.09 | --- | --- | 1.35 | --- | --- | --- | --- |
| CT8 | --- | --- | --- | 1.64 | --- | 1.26 | --- | --- | --- | --- |
| CT10 | --- | --- | 1.41 | 2.05 | --- | 1.37 | --- | --- | --- | --- |
| CT12 | --- | --- | --- | 1.15 | --- | 1.24 | --- | --- | --- | --- |
| CT14 | --- | --- | 1.46 | 1.40 | --- | 2.58 | --- | --- | --- | --- |
| CT15 | --- | --- | --- | --- | --- | --- | 1.19<br>1.40 | 1.10<br>1.30 | --- | --- |
| CT16 | --- | --- | --- | --- | --- | --- | --- | --- | --- | --- |
| CT17 | --- | --- | --- | --- | --- | --- | --- | --- | --- | --- |
| CT1 | --- | --- | --- | --- | --- | 1.09 | --- | --- | --- | --- |

Table 5A Continued

ΔCt values in lung and colon primary tumors and cell line models

| Primary Tumors or Cell lines | PRO381 | PRO1269 | PRO1410 | PRO1755 | PRO1780 | PRO1788 | PRO3434 | PRO1927 | PRO3567 | PRO1295 |
|---|---|---|---|---|---|---|---|---|---|---|
| CT4 | --- | --- | --- | --- | --- | 1.22 | --- | --- | --- | --- |
| CT5 | --- | --- | 2.14 | --- | --- | --- | --- | --- | --- | --- |
| CT6 | --- | --- | --- | --- | --- | --- | --- | --- | --- | --- |
| CT7 | --- | --- | --- | --- | --- | --- | --- | --- | --- | --- |
| CT9 | --- | --- | --- | --- | --- | 1.52 | --- | --- | --- | --- |
| CT11 | --- | --- | 1.29 | --- | --- | --- | --- | --- | --- | --- |
| CT18 | --- | --- | --- | --- | --- | --- | --- | --- | --- | --- |
| HBL100 | --- | --- | --- | --- | --- | --- | --- | --- | --- | --- |
| MB435s | --- | --- | --- | --- | --- | --- | --- | --- | --- | --- |
| T47D | --- | --- | --- | --- | --- | --- | --- | --- | --- | --- |
| MB468 | --- | --- | --- | --- | --- | --- | --- | --- | --- | --- |
| MB175 | --- | --- | --- | --- | --- | --- | --- | --- | --- | --- |
| MB361 | --- | --- | --- | --- | --- | --- | --- | --- | --- | --- |
| BT20 | --- | --- | --- | --- | --- | --- | --- | --- | --- | --- |
| MCF7 | --- | --- | --- | --- | --- | --- | --- | --- | --- | --- |
| SKBR3 | --- | --- | --- | --- | --- | --- | --- | --- | --- | --- |

Table 5A Continued

ΔCt values in lung and colon primary tumors and cell line models

| Primary Tumors or Cell lines | PRO381 | PRO1269 | PRO1410 | PRO1755 | PRO1780 | PRO1788 | PRO3434 | PRO1927 | PRO3567 | PRO1295 |
|---|---|---|---|---|---|---|---|---|---|---|
| A549 | --- | --- | --- | --- | --- | --- | 1.09<br>1.51 | --- | --- | --- |
| Calu-1 | --- | --- | --- | --- | --- | --- | --- | --- | --- | --- |
| Calu-6 | --- | --- | --- | --- | --- | --- | 1.60<br>1.22 | --- | --- | --- |
| H157 | --- | --- | --- | --- | --- | --- | 1.61 | --- | --- | --- |
| H441 | --- | --- | --- | --- | --- | --- | 1.07<br>1.15 | --- | --- | --- |
| H460 | --- | --- | --- | --- | --- | --- | 1.01 | --- | --- | --- |
| SKMES1 | --- | --- | --- | --- | --- | --- | 1.02 | --- | --- | --- |
| SW900 | --- | --- | --- | --- | --- | --- | --- | --- | --- | --- |
| SW480 | --- | --- | --- | --- | --- | --- | --- | --- | --- | --- |
| SW620 | --- | --- | --- | --- | --- | --- | 1.30 | --- | --- | --- |
| Colo320 | --- | --- | --- | --- | --- | --- | 1.78<br>1.76<br>1.74 | 1.51 | 2.31 | --- |
| HT29 | --- | --- | 1.22 | --- | --- | --- | 1.64 | --- | --- | --- |
| HM7 | --- | --- | --- | --- | --- | --- | --- | --- | --- | --- |
| WiDr | --- | --- | --- | --- | --- | --- | --- | --- | --- | --- |

Table 5A Continued

ΔCt values in lung and colon primary tumors and cell line models

| Primary Tumors or Cell lines | PRO381 | PRO1269 | PRO1410 | PRO1755 | PRO1780 | PRO1788 | PRO3434 | PRO1927 | PRO3567 | PRO1295 |
|---|---|---|---|---|---|---|---|---|---|---|
| HCT116 | --- | --- | --- | --- | --- | --- | 2.15<br>2.22 | 1.41<br>1.47 | --- | --- |
| SKC01 | --- | --- | --- | --- | --- | --- | --- | --- | --- | --- |
| SW403 | --- | --- | --- | --- | --- | --- | 1.75 | --- | 1.07 | --- |
| LS174T | --- | --- | --- | --- | --- | --- | 1.42 | --- | 1.17 | --- |
| LT22 | --- | --- | --- | 1.26 | 1.07 | --- | --- | --- | --- | --- |
| LT8 | --- | --- | --- | --- | --- | --- | --- | --- | --- | --- |
| Colo205 | --- | --- | --- | --- | --- | --- | --- | --- | --- | --- |
| HCT15 | --- | --- | --- | --- | --- | --- | --- | --- | --- | --- |
| HCC2998 | --- | --- | --- | --- | --- | --- | 1.15 | --- | --- | --- |
| KM12 | --- | --- | --- | --- | --- | --- | --- | --- | --- | --- |
| H522 | --- | --- | --- | --- | --- | --- | --- | --- | --- | --- |
| H810 | --- | --- | --- | --- | --- | --- | 1.20<br>1.54 | --- | --- | --- |
| LT26 | --- | --- | --- | --- | --- | --- | --- | --- | --- | --- |
| LT27 | --- | --- | --- | --- | --- | --- | --- | --- | --- | --- |
| LT28 | --- | --- | --- | --- | --- | --- | --- | --- | --- | --- |

Table 5A Continued

ΔCt values in lung and colon primary tumors and cell line models

| Primary Tumors or Cell lines | PRO381 | PRO1269 | PRO1410 | PRO1755 | PRO1780 | PRO1788 | PRO3434 | PRO1927 | PRO3567 | PRO1295 |
|---|---|---|---|---|---|---|---|---|---|---|
| LT29 | --- | --- | --- | --- | --- | --- | --- | --- | --- | --- |
| LT30 | --- | --- | --- | --- | --- | --- | --- | --- | --- | --- |
| LT31 | --- | --- | --- | --- | --- | --- | --- | --- | --- | --- |
| LT33 | --- | --- | --- | --- | --- | --- | --- | --- | --- | --- |
| CT25 | --- | --- | --- | --- | --- | --- | --- | --- | --- | --- |
| CT28 | --- | --- | --- | --- | --- | --- | --- | --- | --- | --- |
| CT35 | --- | --- | --- | --- | --- | --- | --- | --- | --- | --- |
| HF-000716 | --- | --- | --- | --- | --- | --- | --- | --- | --- | --- |
| HF-000733 | --- | --- | --- | --- | --- | --- | --- | --- | --- | --- |
| HF-000831 | --- | --- | --- | --- | --- | --- | --- | --- | --- | --- |
| HF-000832 | --- | --- | --- | --- | --- | --- | --- | --- | --- | --- |
| HF-000613 | --- | --- | --- | --- | --- | --- | --- | --- | --- | --- |
| HF-000499 | --- | --- | --- | --- | --- | --- | --- | --- | --- | --- |
| HF-000539 | --- | --- | --- | --- | --- | --- | --- | --- | --- | 1.49 |
| HF-000575 | --- | --- | --- | --- | --- | --- | --- | --- | --- | --- |
| HF-000698 | --- | --- | --- | --- | --- | --- | --- | --- | --- | 1.09 |

Table 5A Continued

ΔCt values in lung and colon primary tumors and cell line models

| Primary Tumors or Cell lines | PRO381 | PRO1269 | PRO1410 | PRO1755 | PRO1780 | PRO1788 | PRO3434 | PRO1927 | PRO3567 | PRO1295 |
|---|---|---|---|---|---|---|---|---|---|---|
| HF-000545 | --- | --- | --- | --- | --- | --- | --- | --- | --- | 1.11 |
| SRCC1094 | --- | --- | --- | --- | --- | --- | --- | --- | --- | --- |
| SRCC1095 | --- | --- | --- | --- | --- | --- | --- | --- | --- | --- |
| SRCC1096 | --- | ---- | --- | --- | --- | --- | --- | --- | --- | --- |
| SRCC1097 | --- | --- | --- | --- | --- | --- | --- | --- | --- | --- |
| SRCC1098 | --- | --- | --- | --- | --- | --- | --- | --- | --- | --- |
| SRCC1099 | --- | --- | --- | --- | --- | --- | --- | --- | --- | --- |
| SRCC1100 | --- | --- | --- | --- | --- | --- | --- | --- | --- | --- |
| SRCC1101 | --- | --- | --- | --- | --- | --- | --- | --- | --- | --- |
| HF-000631 | --- | --- | --- | --- | --- | --- | --- | --- | --- | 1.27 |
| HF-000641 | --- | --- | --- | --- | --- | --- | --- | --- | --- | --- |
| HF-000643 | 4.83 | --- | --- | --- | --- | --- | --- | --- | --- | --- |
| HF-000840 | 1.08 | --- | --- | --- | --- | --- | --- | --- | --- | 1.97 |
| HF-000842 | --- | --- | --- | --- | --- | --- | 2.20 | 2.41 | --- | --- |
| HF-000762 | --- | --- | --- | --- | --- | --- | --- | --- | --- | --- |
| HF-000789 | --- | --- | --- | --- | --- | --- | --- | --- | --- | --- |

Table 5A Continued

ΔCt values in lung and colon primary tumors and cell line models

| Primary Tumors or Cell lines | PRO381 | PRO1269 | PRO1410 | PRO1755 | PRO1780 | PRO1788 | PRO3434 | PRO1927 | PRO3567 | PRO1295 |
|---|---|---|---|---|---|---|---|---|---|---|
| HF-000795 | --- | --- | --- | --- | --- | --- | --- | --- | --- | --- |
| HF-000811 | 2.09<br>3.15 | --- | --- | --- | --- | --- | --- | --- | --- | --- |
| HF-001294 | 1.14<br>1.08 | --- | --- | --- | --- | --- | 1.17 | 2.31 | --- | --- |
| HF-001293 | --- | --- | --- | --- | --- | --- | --- | --- | --- | --- |
| HF-001300 | --- | --- | --- | --- | --- | --- | --- | --- | --- | --- |
| HF-001297 | --- | --- | --- | --- | --- | --- | --- | --- | --- | --- |
| HF-001295 | --- | --- | --- | --- | --- | --- | --- | --- | --- | --- |
| HF-001299 | --- | --- | --- | --- | --- | --- | 1.11 | 2.40 | --- | --- |
| HF-001296 | 3.18<br>3.53 | --- | --- | --- | --- | --- | 4.64 | 5.14 | --- | --- |
| HF-001291 | 1.17 | --- | --- | --- | --- | --- | --- | --- | --- | --- |
| HF-000842 | --- | --- | --- | --- | --- | --- | --- | --- | --- | --- |

Table 5B

$\Delta$Ct values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| LT1 | --- | --- | --- | --- | --- | --- | --- | --- | --- | --- |
| LT1-a | --- | --- | --- | --- | --- | --- | --- | --- | 1.25 | --- |
| LT2 | --- | --- | --- | --- | --- | --- | --- | --- | --- | --- |
| LT3 | --- | --- | --- | --- | --- | --- | --- | --- | --- | --- |
| LT4 | --- | --- | --- | --- | --- | --- | --- | --- | --- | --- |
| LT6 | --- | --- | --- | --- | --- | --- | --- | --- | 1.20 | --- |
| LT7 | --- | --- | --- | --- | --- | --- | --- | --- | 1.66 | --- |
| LT9 | --- | --- | --- | --- | --- | --- | --- | --- | --- | --- |
| LT10 | --- | --- | --- | --- | --- | --- | --- | --- | --- | --- |
| LT11 | --- | --- | --- | --- | --- | --- | --- | --- | --- | --- |
| LT12 | --- | --- | --- | --- | --- | --- | --- | --- | 1.27 | --- |
| LT13 | --- | 1.42 | --- | --- | --- | --- | 4.20<br>4.45 | --- | --- | --- |
| LT15 | --- | 1.17 | --- | --- | --- | --- | 1.36<br>1.15 | --- | --- | --- |
| LT16 | --- | 1.42 | --- | --- | --- | --- | 3.71<br>3.99 | --- | --- | --- |
| LT17 | --- | --- | --- | --- | --- | --- | --- | --- | --- | --- |

Table 5B

ΔCt values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| LT18 | --- | --- | --- | --- | --- | --- | --- | --- | --- | --- |
| LT19 | --- | --- | --- | --- | --- | --- | --- | --- | --- | --- |
| LT21 | --- | --- | --- | --- | --- | --- | --- | --- | --- | --- |
| CT2 | --- | --- | --- | --- | --- | --- | --- | --- | --- | --- |
| CT3 | --- | --- | --- | --- | --- | --- | --- | --- | --- | --- |
| CT8 | --- | --- | --- | --- | --- | --- | --- | --- | --- | --- |
| CT10 | --- | --- | --- | --- | --- | --- | --- | --- | --- | --- |
| CT12 | --- | --- | --- | --- | --- | --- | --- | --- | --- | --- |
| CT14 | --- | --- | --- | --- | --- | --- | --- | --- | --- | --- |
| CT15 | --- | --- | --- | --- | --- | --- | 1.34<br>1.62 | --- | --- | --- |
| CT16 | --- | 1.13 | --- | --- | --- | --- | 1.04<br>1.05 | --- | --- | --- |
| CT17 | --- | --- | --- | --- | --- | --- | 1.16 | --- | --- | --- |
| CT1 | --- | --- | --- | --- | --- | --- | --- | --- | --- | --- |
| CT4 | --- | --- | --- | --- | --- | --- | --- | --- | --- | --- |
| CT5 | --- | --- | --- | --- | --- | --- | --- | --- | --- | --- |
| CT6 | --- | --- | --- | --- | --- | --- | --- | --- | --- | --- |

Table 5B Continued

ΔCt values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| CT7 | --- | --- | --- | --- | --- | --- | --- | --- | --- | --- |
| CT9 | --- | --- | --- | --- | --- | --- | --- | --- | --- | --- |
| CT11 | --- | --- | --- | --- | --- | --- | --- | --- | --- | --- |
| CT18 | --- | --- | --- | --- | --- | --- | --- | --- | --- | --- |
| HBL100 | --- | --- | --- | --- | --- | --- | --- | 1.10 | --- | --- |
| MB435s | --- | --- | --- | --- | --- | --- | --- | --- | --- | --- |
| T47D | --- | --- | --- | --- | --- | --- | --- | 1.37 | --- | --- |
| MB468 | --- | --- | --- | --- | --- | --- | --- | --- | --- | --- |
| MB175 | --- | --- | --- | --- | --- | --- | --- | --- | --- | --- |
| MB361 | --- | --- | --- | --- | --- | --- | --- | --- | --- | --- |
| BT20 | --- | --- | --- | --- | --- | --- | --- | --- | --- | --- |
| MCF7 | --- | --- | --- | --- | --- | --- | --- | --- | --- | --- |
| SKBR3 | --- | --- | --- | --- | --- | --- | --- | --- | --- | --- |
| A549 | --- | 1.20 | --- | --- | --- | --- | 2.17<br>2.11 | --- | --- | --- |
| Calu-1 | --- | --- | --- | --- | --- | --- | 1.39 | --- | --- | --- |
| Calu-6 | --- | --- | --- | --- | --- | --- | 1.12 | --- | --- | --- |
| H157 | --- | --- | --- | --- | --- | --- | --- | --- | --- | --- |

Table 5B Continued

ΔCt values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| H441 | --- | --- | --- | --- | --- | --- | 2.06 | --- | --- | --- |
| H460 | --- | --- | --- | --- | --- | --- | 1.88 | --- | --- | --- |
| SKMES1 | --- | --- | --- | --- | --- | --- | 1.90 | --- | --- | --- |
| SW900 | --- | --- | --- | --- | --- | --- | --- | --- | --- | --- |
| SW480 | --- | --- | --- | --- | --- | --- | --- | 1.41 | --- | --- |
| SW620 | --- | --- | --- | --- | --- | --- | 2.24 | --- | --- | --- |
| Colo320 | --- | --- | --- | --- | --- | --- | 2.21<br>2.24 | 1.72 | --- | --- |
| HT29 | --- | --- | --- | --- | --- | --- | --- | 1.32 | --- | --- |
| HM7 | --- | --- | --- | --- | --- | --- | --- | --- | --- | --- |
| WiDr | --- | --- | --- | --- | --- | --- | --- | 1.57 | --- | --- |
| HCT116 | --- | --- | --- | --- | --- | --- | 2.46<br>2.66 | 1.06 | --- | --- |
| SKC01 | --- | --- | --- | --- | --- | --- | --- | 1.01 | --- | --- |
| SW403 | --- | --- | --- | --- | --- | --- | --- | 1.43 | --- | --- |
| LS174T | --- | --- | --- | --- | --- | --- | --- | --- | --- | --- |
| LT22 | --- | --- | --- | --- | --- | --- | --- | --- | --- | --- |

Table 5B Continued

ΔCt values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| LT8 | --- | --- | --- | --- | --- | --- | --- | --- | 1.74 | --- |
| Colo205 | --- | --- | --- | --- | --- | --- | --- | --- | --- | --- |
| HCT15 | --- | --- | --- | --- | --- | --- | --- | --- | --- | --- |
| HCC2998 | --- | --- | --- | --- | --- | --- | --- | --- | --- | --- |
| KM12 | --- | --- | --- | --- | --- | --- | --- | --- | --- | --- |
| H522 | --- | --- | --- | --- | --- | --- | --- | --- | --- | --- |
| H810 | --- | --- | --- | --- | --- | --- | --- | --- | --- | --- |
| LT25 | --- | --- | --- | --- | --- | --- | --- | --- | --- | --- |
| LT26 | --- | --- | --- | --- | --- | --- | --- | --- | 1.01 | --- |
| LT27 | --- | --- | --- | --- | --- | --- | --- | --- | --- | --- |
| LT28 | --- | --- | --- | --- | --- | --- | --- | --- | 1.32 | --- |
| LT29 | --- | --- | --- | --- | --- | --- | --- | --- | --- | --- |
| LT30 | --- | --- | --- | --- | --- | --- | --- | --- | --- | --- |
| LT31 | --- | --- | --- | --- | --- | --- | --- | --- | --- | --- |
| LT32 | --- | --- | --- | --- | --- | --- | --- | --- | --- | --- |
| LT33 | --- | --- | --- | --- | --- | --- | --- | --- | --- | --- |
| CT19 | --- | --- | --- | --- | --- | --- | --- | --- | --- | --- |

Table 5B Continued

ΔCt values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| CT20 | --- | --- | --- | --- | --- | --- | --- | --- | --- | --- |
| CT21 | --- | --- | --- | --- | --- | --- | --- | --- | --- | --- |
| CT22 | --- | --- | --- | --- | --- | --- | --- | --- | --- | --- |
| CT23 | --- | --- | --- | --- | --- | --- | --- | --- | --- | --- |
| CT24 | --- | --- | --- | --- | --- | --- | --- | --- | --- | --- |
| CT25 | --- | --- | --- | --- | --- | --- | --- | --- | --- | --- |
| CT26 | --- | --- | --- | --- | --- | --- | --- | --- | --- | --- |
| CT27 | --- | --- | --- | --- | --- | --- | --- | --- | --- | --- |
| CT28 | --- | --- | -- | --- | --- | --- | --- | --- | --- | --- |
| CT29 | --- | --- | --- | --- | --- | --- | --- | --- | --- | --- |
| CT30 | --- | --- | --- | --- | --- | --- | --- | --- | --- | --- |
| CT31 | --- | --- | --- | --- | --- | --- | --- | --- | --- | --- |
| CT32 | --- | --- | --- | --- | --- | --- | --- | --- | --- | --- |
| CT33 | --- | --- | --- | --- | --- | --- | --- | --- | --- | --- |
| CT35 | --- | --- | --- | --- | --- | --- | --- | --- | --- | --- |
| CT36 | --- | --- | --- | --- | --- | --- | --- | --- | --- | --- |
| HF-000716 | --- | --- | --- | --- | --- | --- | 2.63<br>2.73 | --- | --- | 1.68 |

Table 5B Continued

ΔCt values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| HF-000733 | --- | --- | --- | --- | --- | --- | 2.58<br>2.71<br>1.39 | --- | --- | 2.04 |
| HF-000831 | --- | --- | --- | --- | --- | --- | --- | --- | --- | --- |
| HF-000832 | --- | --- | --- | 1.10 | --- | 1.31<br>1.18 | --- | --- | --- | 1.13 |
| HF-000611 | --- | --- | --- | --- | --- | --- | 4.99 | --- | --- | --- |
| HF-000613 | --- | --- | --- | --- | --- | --- | --- | --- | --- | --- |
| HF-000499 | --- | --- | --- | --- | --- | --- | --- | --- | --- | --- |
| HF-000539 | 2.33 | --- | 1.32 | 1.39<br>1.09 | --- | 1.79<br>1.61 | 3.13<br>2.55 | --- | --- | 2.03<br>1.86 |
| HF-000575 | --- | --- | 1.21 | 1.14 | --- | 1.48<br>1.25 | 1.32 | --- | --- | 1.16 |
| HF-000698 | --- | --- | --- | 1.14 | --- | 1.14 | --- | --- | --- | 1.40<br>1.22 |
| HF-000545 | --- | --- | 1.62 | 2.12<br>1.72 | --- | 1.52<br>1.63 | 1.59<br>1.68 | --- | --- | 1.17 |
| SRCC1094 | --- | --- | --- | --- | --- | --- | --- | --- | --- | --- |
| SRCC1095 | --- | --- | --- | --- | --- | --- | --- | --- | --- | --- |
| SRCC1096 | --- | --- | --- | --- | --- | --- | --- | --- | --- | --- |
| SRCC1097 | --- | --- | --- | --- | --- | --- | --- | --- | --- | --- |

Table 5B Continued

ΔCt values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| SRCC1098 | --- | --- | --- | --- | --- | --- | --- | --- | 1.24 | --- |
| SRCC1099 | --- | --- | --- | --- | --- | --- | --- | --- | --- | --- |
| SRCC1100 | --- | --- | --- | --- | --- | --- | --- | --- | --- | --- |
| SRCC1101 | --- | --- | --- | --- | --- | --- | --- | --- | --- | --- |
| HF-000631 | --- | --- | --- | --- | -- | --- | 1.37 | --- | --- | --- |
| HF-000641 | --- | --- | --- | --- | --- | --- | --- | --- | --- | --- |
| HF-000643 | --- | --- | --- | --- | --- | --- | --- | --- | --- | --- |
| HF-000840 | 1.71 | --- | 1.03 | --- | 2.09 | 1.50<br>1.42 | 3.63 | --- | --- | 1.49<br>1.49 |
| HF-000842 | --- | --- | --- | --- | --- | 1.24 | 1.99 | - | --- | --- |
| HF-000762 | --- | --- | --- | 1.39<br>1.15 | --- | --- | --- | --- | --- | --- |
| HF-000789 | --- | --- | --- | --- | --- | --- | --- | --- | --- | --- |
| HF-000795 | 1.13 | --- | 1.12 | --- | --- | 1.02<br>1.04 | --- | --- | --- | --- |
| HF-000811 | --- | --- | --- | --- | --- | --- | --- | --- | --- | --- |
| HF-001291 | --- | --- | --- | --- | --- | --- | --- | --- | --- | --- |
| HF-001293 | --- | --- | --- | --- | --- | --- | --- | --- | --- | --- |

Table 5B Continued

ΔCt values in lung and colon primary tumors and cell line models

| Primary Tumors or Cell Lines | PRO1293 | PRO1303 | PRO4344 | PRO4354 | PRO4397 | PRO4407 | PRO1555 | PRO1096 | PRO2038 | PRO2262 |
|---|---|---|---|---|---|---|---|---|---|---|
| HF-001294 | --- | --- | --- | --- | --- | --- | --- | --- | --- | --- |
| HF-001295 | --- | --- | --- | --- | --- | --- | --- | --- | --- | --- |
| HF-001296 | --- | --- | --- | 2.11 | --- | 2.21 | --- | --- | --- | 2.18 |
| HF-001297 | --- | --- | --- | --- | --- | --- | --- | --- | --- | --- |
| HF-001299 | --- | --- | --- | --- | --- | --- | --- | --- | --- | --- |
| HF-001300 | --- | --- | --- | --- | --- | --- | --- | --- | --- | --- |

PRO3434:

PRO3434 (DNA77631-2537) was reexamined along with selected tumors from the above initial screen with epicenter mapping. Table 6 describes the epicenter markers that were employed in association with PRO3434 (DNA77631-2537). These markers are located in close proximity to DNA77631 and are used to assess the amplification status of the region of Chromosome 7 in which DNA77631 is located. The distance between markers is measured in centirays (cR), which is a radiation breakage unit approximately equal to a 1 % chance of a breakage between two markers. One cR is very roughly equivalent to 20 kilobases. The marker sWSS918 is the marker found to be the closest to the location on Chromosome 7 where DNA77631-2537 closely maps.

Table 7 indicates the ΔCt values for results of epicenter mapping relative to DNA77631, indicating the relative amplification in the region more immediate to the actual location of DNA77631 along Chromosome 7.

Table 6 Epicenter Markers Along Chromosome 7 Used for DNA77631

| **Map Position on Chromosome 7** | **Stanford Human Genome Center Marker Name** | **Distance to Next Marker (cR)** |
|---|---|---|
| G1 | SHGC-34913 | 17 |
| G2 | AFMa090xg 1 | 25 |
| G3 | SHGC-10715 | 16 |
| G4 | SHGC-34866 | 5 |
| G5 | SHGC-32510 | 48 |
| DNA 77631 | - | - |
| G6 | sWSS918 | 19 |
| G7 | AFMc027xb5 | - |

Table 7

Amplification of Epicenter Markers Relative to DNA77631 (ΔCt)

| **Tumor** | **G1** | **G2** | **G3** | **G4** | **G5** | **DNA 77631** | **G6** | **G7** |
|---|---|---|---|---|---|---|---|---|
| **HF-000613** | 1.31 | 0.12 | 0.61 | 0.46 | 0.38 | -0.28 | 0.48 | 0.58 |
| **HF-000545** | 1.55 | 0.55 | 0.09 | 0.16 | 0.27 | 0.69 | 0.26 | 0.25 |
| **HF-000539** | 1.53 | -1.68 | 0.88 | 0.76 | 0.79 | 2.75 | 0.98 | 0.96 |
| **HF-000575** | 1.47 | 1.02 | 0.02 | -0.17 | 0.15 | 0.48 | 0.20 | 0.33 |
| **HF-000698** | 0.73 | -0.31 | -0.08 | -0.27 | -0.07 | -0.26 | -0.21 | -0.10 |
| **HF-000499** | 0.67 | -0.05 | 0.04 | 0.12 | 0.23 | -0.30 | -0.14 | 0.21 |
| **HF-000733** | 1.08 | 1.19 | 0.41 | 0.39 | 0.46 | 3.00 | 0.51 | 0.68 |
| **HF-000716** | 0.65 | 0.56 | -0.41 | -0.02 | -0.13 | 2.59 | -0.23 | 0.01 |

DISCUSSION AND CONCLUSION:

PRO381 (DNA44194-1317):

The ΔCt values for DNA44194-1317 in a variety of tumors are reported in Table 5A. A ΔCt of >1 was typically used as the threshold value for amplification scoring, as this represents a doubling of gene copy. Table 5A indicates that significant amplification of nucleic acid DNA44194-1317 encoding PRO381 occurred: (1) in primary lung tumors: HF-000643, HF-000840, HF-001291, HF-001294, and HF-001296; and (2) in colon tumor center HF-000811. Because amplification of DNA44194-1317 occurs in various tumors, it is highly probable to play a significant role in tumor formation or growth. As a result, antagonists (*e.g.*, antibodies) directed against the protein encoded by DNA44194-1317 (PRO381) would be expected to have utility in cancer therapy.

PRO1269 (DNA66520-1536):

The ΔCt values for DNA66520-1536 in a variety of tumors are reported in Table 5A. A ΔCt of > 1 was typically used as the threshold value for amplification scoring, as this represents a doubling of gene copy. Table 5A indicates that significant amplification of nucleic acid DNA66520-1536 encoding PRO1269 occurred in primary lung tumors: LT15, LT16 and LT17. Because amplification of DNA66520-1536 occurs in various lung tumors, it is highly probable to play a significant role in tumor formation or growth. As a result, antagonists (*e.g.*, antibodies) directed against the protein encoded by DNA66520-1536 (PRO1269) would be expected to have utility in cancer therapy.

PRO1410 (DNA68874-1622):

The ΔCt values for DNA68874-1622 in a variety of tumors are reported in Table 5A. A ΔCt of >1 was typically used as the threshold value for amplification scoring, as this represents a doubling of gene copy. Table 5A indicates that significant amplification of nucleic acid DNA68874-1622 encoding PRO1410 occurred: (1) in primary lung tumors: LT13, LT15 and LT16; (2) in primary colon tumors: CT2, CT3, CT5, CT10, CT11, and CT14; and (3) in colon cell line HT29. Because amplification of DNA68874-1622 occurs in various lung and colon tumors, it is highly probable to play a significant role in tumor formation or growth. As a result, antagonists (*e.g.*, antibodies) directed against the protein encoded by DNA68874-1622 (PRO1410) would be expected to have utility in cancer therapy.

PRO1755 (DNA76396-1698):

The ΔCt values for DNA76396-1698 in a variety of tumors are reported in Table 5A. A ΔCt of >1 was typically used as the threshold value for amplification scoring, as this represents a doubling of gene copy. Table 5A indicates that significant amplification of nucleic acid DNA76396-1698 encoding PRO1755 occurred: (1) in primary lung tumors: LT16, LT18 and LT22; and (2) in primary colon tumors: CT2, CT8, CT10, CT12, and CT14. Because amplification of DNA76396-1698 occurs in various lung and colon tumors, it is highly probable to play a significant role in tumor formation or growth. As a result, antagonists (*e.g.*, antibodies) directed against the protein encoded by DNA76396-1698 (PRO1755) would be expected to have utility in cancer therapy.

PRO1780 (DNA71169-1709):

The ΔCt values for DNA71169-1709 in a variety of tumors are reported in Table 5A. A ΔCt of >1 was typically used as the threshold value for amplification scoring, as this represents a doubling of gene copy. Table 5A indicates that significant amplification of nucleic acid DNA71169-1709 encoding PRO1780 occurred in primary lung tumors: LT4, LT7 and LT22. Because amplification of DNA71169-1709 occurs in various lung tumors, it is highly probable to play a significant role in tumor formation or growth. As a result, antagonists (*e.g.*, antibodies) directed against the protein encoded by DNA71169-1709 (PRO1780) would be expected to have utility in cancer therapy.

PRO1788 (DNA77652-2505):

The ΔCt values for DNA77652-2505 in a variety of tumors are reported in Table 5A. A ΔCt of > 1 was typically used as the threshold value for amplification scoring, as this represents a doubling of gene copy. Table 5A indicates that significant amplification of nucleic acid DNA77652-2505 encoding PRO1788 occurred in primary colon tumors: CT1, CT3, CT4, CT8, CT9, CT10, CT12, and CT14. Because amplification of DNA77652-2505 occurs in various colon tumors, it is highly probable to play a significant role in tumor formation or growth. As a result, antagonists (*e.g.*, antibodies) directed against the protein encoded by DNA77652-2505 (PRO1788) would be expected to have utility in cancer therapy.

PRO3434 (DNA77631-2537):

The ΔCt values for DNA77631-2537 in a variety of tumors are reported in Table 5A. A ΔCt of > 1 was typically used as the threshold value for amplification scoring, as this represents a doubling of gene copy. Table 5A indicates that significant amplification of nucleic acid DNA77631-2537 encoding PRO3434 occurred: (1) in primary lung tumors: LT13, LT15, LT16, HF-000842, HF-001294, HF-001296, and HF-001299; (2) in lung cell lines: A549, Calu-6, H 157, H441, H460, SKMES1, and H810; (3) in primary colon tumor CT15; and (4) in colon cell lines: SW620, Colo320, HT29, HCT116, SW403, LS174T, and HCC2998.

Amplification has been confirmed by epicenter mapping (Table 7) for DNA77631-2537 and resulted in significant amplification: (1) in primary colon tumor HF-000539; and (2) in testis tumor center HF-000733 and testis tumor margin HF-000716. In contrast, the amplification of the closest known epicenter markers does not occur to a greater extent than that of DNA77631 (Table 7). This strongly suggests that DNA77631-1317 is the gene responsible for the amplification of the particular region on Chromosome 7.

Because amplification of DNA77631 occurs in various tumors including colon and testis tumors, it is highly probable to play a significant role in tumor formation or growth. As a result, antagonists (*e.g.*, antibodies) directed against the protein encoded by DNA77631-2537 (PRO3434) would be expected to have utility in cancer therapy.

PRO1927 (DNA82307-2531):

The ΔCt values for DNA82307-2531 in a variety of tumors are reported in Table 5A. A ΔCt of >1 was typically used as the threshold value for amplification scoring, as this represents a doubling of gene copy. Table 5A indicates that significant amplification of nucleic acid DNA82307-2531 encoding PRO1927 occurred: (1) in primary lung tumors: LT13, LT16, HF-000842, FIF-001294, HF-001296, and HF-001299; (2) in primary colon tumor CT15; and (3) in colon cell lines: Colo320 and HCT116. Because amplification of DNA occurs in various lung and colon tumors, it is highly probable to play a significant role in tumor formation or growth. As a result, antagonists (*e.g.*, antibodies) directed against the protein encoded by DNA82307-2531 (PRO1927) would be expected to have utility in cancer therapy.

PRO3567 (DNA56049-2543):

The ΔCt values for DNA56049-2543 in a variety of tumors are reported in Table 5A. A ΔCt of >1 was typically used as the threshold value for amplification scoring, as this represents a doubling of gene copy. Table 5A indicates that significant amplification of nucleic acid DNA56049-2543 encoding PRO3567 occurred in colon cell lines: Colo320, SW403 and LS174T. Because amplification of DNAS6049-2543 occurs in various colon cell lines, it is highly probable to play a significant role in tumor formation or growth. As a result, antagonists (*e.g.*, antibodies) directed against the protein encoded by DNA56049-2543 (PRO3567)would be expected to have utility in cancer therapy.

PRO1295 (DNA59218-1559):

The ΔCt values for DNA59218-1559 in a variety of tumors are reported in Table 5A. A ΔCt of >1 was typically used as the threshold value for amplification scoring, as this represents a doubling of gene copy. Table 5A indicates that significant amplification of nucleic acid DNA59218-1559 encoding PRO1295 occurred: (1) in primary lung tumors: HF-000631 and HF-000840; (2) colon tumor centers: HF-000539 and HF-000698; and (3) in breast tumor center HF-000545. Because amplification of DNA59218-1559 occurs in various tumors, it is highly probable to play a significant role in tumor formation or growth. As a result, antagonists (*e.g.*, antibodies) directed against the protein encoded by DNA59218-1559 (PRO1295) would be expected to have utility in cancer therapy.

PRO1293 (DNA60618-1557):

The ΔCt values for DNA60618-1557 in a variety of tumors are reported in Table 5B. A ΔCt of >1 was typically used as the threshold value for amplification scoring, as this represents a doubling of gene copy. Table 5B indicates that significant amplification of nucleic acid DNA60618-1557 encoding PRO1293 occurred: (1) in primary lung tumor HF-000840; and (2) in colon tumor centers: HF-000539 and HF-000795. Because amplification of DNA60618-1557 occurs in various lung and colon tumors, it is highly probable to play a significant role in tumor formation or growth. As a result, antagonists (*e.g.*, antibodies) directed against the protein encoded by DNA60618-1557 (PRO1293) would be expected to have utility in cancer therapy.

PRO1303 (DNA65409-1566):

The ΔCt values for DNA65409-1566 in a variety of tumors are reported in Table 5B. A ΔCt of >1 was typically used as the threshold value for amplification scoring, as this represents a doubling of gene copy. Table 5B indicates that significant amplification of nucleic acid DNA65409-1566 encoding PRO1303 occurred: (1) in primary lung tumors: LT13, LT15 and LT16; (2) in lung cell line A549; and (3) in colon tumor CT16. Because amplification of DNA65409-1566 occurs in various tumors, it is highly probable to play a significant role in tumor formation or growth. As a result, antagonists (*e.g.*, antibodies) directed against the protein encoded by DNA65409-1566 (PRO1566) would be expected to have utility in cancer therapy.

PRO4344 (DNA84927-2585):

The ΔCt values for DNA84927-2585 in a variety of tumors are reported in Table 5B. A ΔCt of > 1 was typically used as the threshold value for amplification scoring, as this represents a doubling of gene copy. Table 5B indicates that significant amplification of nucleic acid DNA84927-2585 encoding PRO4344 occurred: (1) in primary lung tumor HF-000840; (2) in colon tumor centers: HF-000539, HF-000575 and HF-000795; and (3) in breast tumor center HF-000545. Because amplification of DNA84927-2585 occurs in various tumors, it is highly probable to play a significant role in tumor formation or growth. As a result, antagonists (*e.g.*, antibodies) directed against the protein encoded by DNA84927-2585 (PRO4344) would be expected to have utility in cancer therapy.

PRO4354 (DNA92256-2596):

The ΔCt values for DNA92256-2596 in a variety of tumors are reported in Table 5B. A ΔCt of > 1 was typically used as the threshold value for amplification scoring, as this represents a doubling of gene copy. Table 5B indicates that significant amplification of nucleic acid DNA92256-2596 encoding PRO4354 occurred: (1) in primary lung tumor HF-001296; (2) in colon tumor centers: HF-000539, HF-000575, HF-000698, and HF-000762; (3) in breast tumor center HF-000545; and (4) in parathyroid tumor HF-000832. Because amplification of DNA92256-2596 occurs in various tumors, it is highly probable to play a significant role in tumor formation or growth. As a result, antagonists (*e.g.*, antibodies) directed against the protein encoded by DNA92256-2596 (PRO4354) would be expected to have utility in cancer therapy.

PRO4397 (DNA83505-2606):

The ΔCt values for DNA83505-2606 in a variety of tumors are reported in Table 5B. A ΔCt of > 1 was typically used as the threshold value for amplification scoring, as this represents a doubling of gene copy. Table 5B indicates that significant amplification of nucleic acid DNA83505-2606 encoding PRO4397occurred in primary lung tumor HF-000840. Because amplification of DNA83505-2606 occurs in lung tumor, it is highly probable to play a significant role in tumor formation or growth. As a result, antagonists (*e.g.*, antibodies) directed against the protein encoded by DNA83505-2606 (PRO4397) would be expected to have utility in cancer therapy.

PRO4407 (DNA92264-26161):

The ΔCt values for DNA92264-2616 in a variety of tumors are reported in Table 5B. A ΔCt of >1 was typically used as the threshold value for amplification scoring, as this represents a doubling of gene copy. Table 58 indicates that significant amplification of nucleic acid DNA92264-2616 encoding PRO4407 occurred: (I) in primary lung tumorsHF-000840, HF-000842 and HF-001296; (2) in colon tumor centers: HF-000539, HF-000575, HF-000698, and HF-000795; (3) in breast tumor HF-000545; and (4) in parathyroid tumor HF-000832. Because amplification of DNA92264-2616 occurs in various tumors, it is highly probable to play a significant role in tumor formation or growth. As a result, antagonists (*e.g.*, antibodies) directed against the protein encoded by DNA92264-2616 (PRO2616) would be expected to have utility in cancer therapy.

PRO1555 (DNA73744-1665):

The ΔCt values for DNA73744-1665 in a variety of tumors are reported in Table 5B. A ΔCt of >1 was typically used as the threshold value for amplification scoring, as this represents a doubling of gene copy. Table 5B indicates that significant amplification of nucleic acid DNA73744-1665 encoding PRO1555 occurred: (1) in primary lung tumors: LT13, LT15, LT16, HF-000631, HF-000840, and HF-000842; (2) in lung cell lines: A549, Calu-1, Calu-6, H441, H460, and SKMES1; (3) in primary colon tumors: CT15, CT16, CT17, and colon tumor centers HF-000539 and HF-000575; (4) in colon cell lines: SW620, Colo320 and HCT116; (5) in breast tumor center HF-000545; (6) in kidney tumor center HF-000611; and (7) in testis tumor margin HF-000716 and testis tumor center HF-000733. Because amplification of DNA73744-1665 occurs in various tumors, it is highly probable to play a significant role in tumor formation or growth. As a result, antagonists (*e.g.*, antibodies) directed against the protein encoded by DNA73744-1665 (PRO1555) would be expected to have utility in cancer therapy.

PRO1096 (DNA61870):

The ΔCt values for DNA61870 in a variety of tumors are reported in Table 5B. A ΔCt of > 1 was typically used as the threshold value for amplification scoring, as this represents a doubling of gene copy. Table 5B indicates that significant amplification of nucleic acid DNA61870 encoding PRO1096 occurred: (1) in colon cell lines: SW480, Colo320, HT29, WiDr, HCT116, SKCO1, and SW403; and (2) in breast cell lines HBL100 and T47D. Because amplification of DNA61870 occurs in various tumors, it is highly probable to play a significant role in tumor formation or growth. As a result, antagonists (*e.g.*, antibodies) directed against the protein encoded by DNA61870 (PRO1096) would be expected to have utility in cancer therapy.

PRO2038 (DNA83014):

The ΔCt values for DNA83014 in a variety of tumors are reported in Table 5B. A ΔCt of > 1 was typically used as the threshold value for amplification scoring, as this represents a doubling of gene copy. Table 5 B indicates that significant amplification of nucleic acid DNA83014 encoding PRO2038 occurred: (1) in primary lung tumors: LT1a, LT6, LT7, LT8, LT12, LT26, and LT28; and (2) in breast tumor SRCC1098. Because amplification of DNA83014 occurs in various tumors, it is highly probable to play a significant role in tumor formation or growth. As a result, antagonists (*e.g.*, antibodies) directed against the protein encoded by DNA83014 (PRO2038) would be expected to have utility in cancer therapy.

PRO2262 (DNA88273):

The ΔCt values for DNA88273 in a variety of tumors are reported in Table 5B. A ΔCt of >1 was typically used as the threshold value for amplification scoring, as this represents a doubling of gene copy. Table 5B indicates that significant amplification of nucleic acid DNA88273 encoding PRO2262 occurred: (1) in primary lung tumors: HF-000840 and HF-001296; (2) in primary colon tumors: HF-000539, HF-000575 and HF-000698; (3) in breast tumor center HF-000545; (4) in testis tumor margin HF-000716 and testis tumor center HF-000733; and (5) in parathyroid tumor HF-000832. Because amplification of DNA88273 occurs in various tumors, it is highly probable to play a significant role in tumor formation or growth. As a result, antagonists (*e.g.*, antibodies) directed against the protein encoded by DNA88273 (PRO2262) would be expected to have utility in cancer therapy.

EXAMPLE 21

Use of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 as a hybridization probe

The following method describes use of a nucleotide sequence encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide as a hybridization probe.

DNA comprising the coding sequence of a full-length or mature "PRO" polypeptide as disclosed herein and/or fragments thereof may be employed as a probe to screen for homologous DNAs (such as those encoding naturally-occurring variants of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262) in human tissue cDNA libraries or human tissue genomic libraries.

Hybridization and washing offilters containing either library DNAs is performed under the following high stringency conditions. Hybridization of radiolabeled PRO381-, PRO1269-, PRO1410-, PRO1755-, PRO1780-, PRO1788-, PRO3434-, PRO1927-, PRO3567-, PRO1295-, PRO1293-, PRO1303-, PRO4344-, PRO4354-, PRO4397-, PRO4407-, PRO1555-, PRO1096-, PRO2038- or PRO2262-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1 x SSC and 0.1% SDS at 42°C.

DNAs having a desired sequence identity with the DNA encoding full-length native sequence PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can then be identified using standard techniques known in the art.

EXAMPLE 22

Expression of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 Polypeptides in *E. coli.*

This example illustrates preparation of an unglycosylated form of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 by recombinant expression in *E. coli.*

The DNA sequence encoding the PRO polypeptide of interest is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli*; *see* Bolivar *et al.*, Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a poly-His leader (including the first six STII codons, poly-His sequence, and enterokinase cleavage site), the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 coding region, lambda transcriptional terminator, and an argU gene.

The ligation mixture is then used to transform a selected *E. coli* strain using the methods described in Sambrook *et al., supra.* Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 protein can then be purified using a metal chelating column under conditions that allow tight binding of the protein.

PRO1788 and PRO1555 were successfully expressed in *E. coli* in a poly-His tagged form using the following procedure. The DNA encoding PRO1788 or PRO1555 was initially amplified using selected PCR primers. The primers contained restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences were then ligated into an expression vector, which was used to transform an *E. coli* host based on strain 52 (W3110 fuhA(tonA) lon galE rpoHts(htpRts) clpP(lacIq). Transformants were first grown in LB containing 50 mg/ml carbenicillin at 30°C with shaking until an O.D. of 3-5 at 600 nm was reached. Cultures were then diluted 50-100 fold into CRAP media (prepared by mixing 3.57 g $(NH_4)_2SO_4$, 0.71 g sodium citrate·$2H_2O$, 1.07 g KCl, 5.36 g Difco yeast extract, 5.36 g Sheffield hycase SF in 500 ml water, as well as 110 mM MPOS, pH 7.3, 0.55% (w/v) glucose and 7 mM $MgSO_4$) and grown for approximately 20-30 hours at 30°C with shaking. Samples were removed to verify expression by SDS-PAGE analysis, and the bulk culture was centrifuged to pellet the cells. Cell pellets were frozen until purification and refolding.

*E. coli* paste from 0.5 to l L fermentations (6-10 g pellets) was resuspended in 10 volumes (w/v) in 7 M guanidine, 20 mM Tris, pH 8 buffer. Solid sodium sulfite and sodium tetrathionate were added to make final concentrations of 0. 1M and 0.02 M, respectively, and the solution was stirred overnight at 4 °C. This step results in a denatured protein with all cysteine residues blocked by sulfitolization. The solution was centrifuged at 40,000 rpm in a Beckman Ultracentifuge for 30 min. The supernatant was diluted with 3-5 volumes of metal chelate column buffer (6 M guanidine, 20 mM Tris, pH 7.4) and filtered through 0.22 micron filters to clarify. The clarified extract was loaded onto a 5 ml Qiagen Ni $^{2+}$-NTA metal chelate column equilibrated in the metal chelate column buffer. The column was washed with additional buffer containing 50 mM imidazole (Calbiochem, Utrol grade), pH 7.4. The proteins were eluted with buffer containing 250 mM imidazole. Fractions containing the desired protein were pooled and stored at 4°C. Protein concentration was estimated by its absorbance at 280 nm using the calculated extinction coefficient based on its amino acid sequence.

The proteins were refolded by diluting sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes were chosen so that the final protein concentration was between 50 to 100 micrograms/ml. The refolding solution was stirred gently at 4 °C for 12-36 hours. The refolding reaction was quenched by the addition of TFA to a final concentration of 0.4% (pH of approximately 3). Before further purification of the protein, the solution was filtered through a 0.22 micron filter and acetonitrile was added to 2-10% final concentration. The refolded protein was chromatographed on a Poros R1/H reversed phase column using a mobile buffer of 0.1% TFA with elution with a gradient of acetonitrile from 10 to 80%. Aliquots of fractions with $A_{280}$ absorbance were analyzed on SDS polyacrylamide gels and fractions containing homogeneous refolded protein were pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO1788 and PRO1555 proteins were pooled and the acetonitrile removed using a gentle stream of nitrogen directed at the solution. Proteins were formulated into 20 mM Hepes, pH 6.8 with 0.14 M sodium chloride and 4% mannitol by dialysis or by gel filtration using G25 Superfine (Pharmacia) resins equilibrated in the fonnuiation buffer and sterile filtered.

EXAMPLE 23

Expression of PRO381, PRO1269 PRO1410 PRO1755, PRO1780 PRO1788, PRO3434, PRO1927, PRO3567. PRO1295. PRO1293. PRO1303, PRO4344, PRO4354, PRO4397, PRO4407, PRO1555, PRO1096, PRO2038 or PRO2262 in mammalian cells

This example illustrates preparation of a potentially glycosylated form of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 by recombinant expression in mammalian cells.

The vector, pRK5 *(see* EP 307,247, published March 15, 1989), is employed as the expression vector. Optionally, the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 DNA is ligated into pRK5 with selected restriction enzymes to allow insertion of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 DNA using ligation methods such as described in Sambrook *et al., supra.* The resulting vector is called pRK5-PRO381, pRK5-PRO1269, pRK5-PRO1410, pRK5-PRO1755, pRK5-PRO1780, pRK5-PRO1788, pRK5-PRO3434, pRK5-PRO1927, pRK5-PRO3567, pRK5-PRO1295, pRK5-PRO1293, pRK5-PRO1303, pRK5-PRO4344, pRK5-PRO4354, pRK5-PRO4397, pRK5-PRO4407, pRK5-PRO1555, pRK5-PRO1096, pRK5-PRO2038 or pRK5-PRO2262.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 $\mu$g pRK5-PRO381, pRK5-PRO1269, pRK5-PRO1410, pRK5-PRO1755, pRK5-PRO1780, pRK5-PRO1788, pRK5-PRO3434, pRK5-PRO1927, pRK5-PRO3567, pRK5-PRO1295, pRK5-PRO1293, pRK5-PRO1303, pRK5-PRO4344, pRK5-PRO4354, pRK5-PRO4397, pRK5-PRO4407, pRK5-PRO1555, pPK5-PRO1096, pRK5-PRO2038 or pRK5-PRO2262 DNA is mixed with about 1 $\mu$g DNA encoding the VA RNA gene [Thimmappaya *et al.*, Cell, 31:543 (1982)] and dissolved in 500 $\mu$l of 1 mM Tris-HCl, 0.1 mM EDTA, 0.227 M $CaCl_2$. To this mixture is added, dropwise, 500 $\mu$l of 50 mM HEPES (pH 7.35), 280 mM NaCl, 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 $\mu$Ci/ml $^{35}$S-cysteine and 200 $\mu$Ci/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide. The cultures containing transfected cells may undergo further incubation (in serum free medium) and the medium is tested in selected bioassays.

In an alternative technique, PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 DNA may be introduced into 293 cells transiently using the dextran sulfate method described by Somparyrac *et al.*, Proc. Natl. Acad. Sci., 12:7575 (1981). 293 cells are grown to maximal density in a spinner flask and 700 $\mu$g pRK5-PRO381, pRK5-PRO1269, pRK5-PRO1410, pRK5-PRO1755, pRK5-PRO1780, pRK5-PRO1788, pRK5-PRO3434, pRK5-PRO1927, pRK5-PRO3567, pPK5-PRO1295, pRK5-PRO1293, pRK5-PRO1303, pRK5-PRO4344, pRK5-PRO4354, pRK5-PRO4397, pRK5-PRO4407, pRK5-PRO1555, pRK5-PRO1096, pRK5-PRO2038 or pRK5-PRO2262 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 $\mu$g/ml bovine insulin and 0.1 $\mu$g/ml bovine transferrin. After about four days, the conditioned media is centrifuged and filtered to remove cells and debris. The sample containing expressed PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can then be concentrated and purified by any selected method, such as dialysis and/or column chromatography.

In another embodiment PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can be expressed in CHO cells. The pRK5-PRO381, pRK5-PRO1269, pRK5-PRO1410, pRK5-PRO1755, pRKS-PRO1780, pRK5-PRO1788, pRK5-PRO3434, pRK5-PRO1927, pRK5-PRO3567, pRK5-PRO1295, pRK5-PRO1293, pRK5-PRO1303, pRK5-PRO4344, pRK5-PRO4354, pRK5-PRO4397, pRK5-PRO4407, pRK5-PRO1555, pRKS-PRO1096, pRK5-PRO2038 or pRK5-PRO2262 vector can be transfected into CHO cells using known reagents such as $CaPO_4$ or DEAE-dextran. As described above, the cell cultures can be incubated, and the medium replaced with culture medium (alone) or medium containing a radiolabel such as $^{35}$S-methionine. After determining the presence of the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, the culture medium may be replaced with serum free medium. Preferably, the cultures are incubated for about 6 days, and then the conditioned medium is harvested. The medium containing the expressed PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can then be concentrated and purified by any selected method.

Epitope-tagged PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 may also be expressed in host CHO cells. The PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 may be subcloned out of the pRK5 vector. The subclone insert can undergo PCR to fuse in frame with a selected epitope tag such as a poly-His tag into a Baculovirus expression vector. The poly-His tagged PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can then be concentrated and purified by any selected method, such as by $Ni^{2+}$-chelate affinity chromatography. Expression in CHO and/or COS cells may also be accomplished by a transient expression procedure.

PRO381, PRO1410 and PRO1303 were expressed in CHO cells by a stable expression procedure. Stable expression in CHO cells was performed using the following procedure. The proteins were expressed as an IgG construct (immunoadhesin), in which the coding sequences for the soluble forms (*e.g.*, extracellular domains) of the respective proteins were fused to an IgG1 constant region sequence containing the hinge, CH2 and CH2 domains and/or in a poly-His tagged form.

Following PCR amplification, the respective DNAs were subcloned in a CHO expression vector using standard techniques as described in Ausubel *et al.*, <u>Current Protocols of Molecular Biology</u>, Unit 3.16, John Wiley and Sons (1997). CHO expression vectors are constructed to have compatible restriction sites 5' and 3' of the DNA of interest to allow the convenient shuttling of cDNA's. The vector used for expression in CHO cells is as described in Lucas *et al.*, <u>Nucl. Acids Res., 24:9</u> (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelvemicrograms ofthe desired plasmid DNA were introduced into approximately 10 million CHO cells using commercially available transfection reagents Superfect® (Quiagen), Dosper® or Fugene® (Boehringer Mannheim). The cells were grown as described in Lucas *et al., supra.* Approximately 3 x $10^{-7}$ cells are frozen in an ampule for further growth and production as described below.

The ampules containing the plasmid DNA were thawed by placement into a water bath and mixed by vortexing. The contents were pipetted into a centrifuge tube containing 10 mls of media and centrifuged at 1000 rpm for 5 minutes. The supernatant was aspirated and the cells were resuspended in 10 ml of selective media (0.2 $\mu$m filtered PS20 with 5% 0.2 $\mu$m diafiltered fetal bovine serum). The cells were then aliquoted into a 100 ml spinner containing 90 ml of selective media. After 1-2 days, the cells were transferred into a 250 ml spinner filled with 150 ml selective growth medium and incubated at 37°C. After another 2-3 days, 250 ml, 500 ml and 2000 ml spinners were seeded with 3 x $10^5$ cells/ml. The cell media was exchanged with fresh media by centrifugation and resuspension in production medium. Although any suitable CHO media may be employed, a production medium described in US Patent No. 5,122,469, issued June 16, 1992 was actually used. 3L production spinner was seeded at 1.2 x $10^6$ cells/ml. On day 0, the cell number and pH were determined. On day 1, the spinner was sampled and sparging with filtered air was commenced. On day 2, the spinner was sampled, the temperature shifted to 33°C, and 30 ml of 500 g/L glucose and 0.6 ml of 10% antifoam (*e.g.*, 35% polydimethylsiloxane emulsion, Dow Coming 365 Medical Grade Emulsion) added. Throughout the production, the pH was adjusted as necessary to keep at around 7.2. After 10 days, or until viability dropped below 70%, the cell culture was harvested by centrifugation and filtered through a 0.22 μm filter. The filtrate was either stored at 4°C or immediately loaded onto columns for purification.

For the poly-His tagged constructs, the proteins were purified using a Ni $^{2+}$-NTA column (Qiagen). Before purification, imidazole was added to the conditioned media to a concentration of 5 mM. The conditioned media was pumped onto a 6 ml Ni $^{2+}$-NTA column equilibrated in 20 mM Hepes, pH 7.4, buffer containing 0.3 M NaCl and 5 mM imidazole at a flow rate of 4-5 ml/min. at 4°C. After loading, the column was washed with additional equilibration buffer and the protein eluted with equilibration buffer containing 0.25 M imidazole. The highly purified protein was subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs were purified from the conditioned media as follows. The conditioned medium was pumped onto a 5 ml Protein A column (Pharmacia) which had been equilibrated in 20 mM Na phosphate buffer, pH 6.8. After loading, the column was washed extensively with equilibration buffer before elution with 100 mM citric acid, pH 3.5. The eluted protein was immediately neutralized by collecting 1 ml fractions into tubes containing 275 μl of 1 M Tris buffer, pH 9. The highly purified protein was subsequently desalted into storage buffer as described above for the poly-His tagged proteins. The homogeneity was assessed by SDS polyacrylamide gels and by N-terminal amino acid sequencing by Edman degradation.

EXAMPLE 24

Expression of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 in Yeast

The following method describes recombinant expression of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 in yeast.

First, yeast expression vectors are constructed for intracellular production or secretion of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 from the ADH2/GAPDH promoter. DNA encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 and the promoter is inserted into suitable restriction enzyme sites in the selected plasmid to direct intracellular expression of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. For secretion, DNA encoding PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO 1555, PRO1096, PRO2038 or PRO2262 can be cloned into the selected plasmid, together with DNA encoding the ADH2/GAPDI-1 promoter, a native PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 signal peptide or other mammalian signal peptide, or, for example, a yeast alpha-factor or invertase secretory signal/leader sequence, and linker sequences (if needed) for expression of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO381, PRO1269, PRO410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can subsequently be isolated and purified by removing the yeast cells from the fermentation medium by centrifugation and then concentrating the medium using selected cartridge filters. The concentrate containing PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 may further be purified using selected column chromatography resins.

EXAMPLE 25

Expression of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096 PRO2038 or PRO2262 in Baculovirus-infected Insect Cells

The following method describes recombinant expression in Baculovirus-infected insect cells.

The sequence coding for PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 is fused upstream of an epitope tag contained within a baculovirus expression vector. Such epitope tags include poly-His tags and immunoglobulin tags (like Fc regions of I9G). A variety of plasmids may be employed, including plasmids derived from commercially available plasmids such as pVL1393 (Novagen). Briefly, the sequence encoding PRO381, PRO1269, PRO1410, PRO1755, PRO1780, PRO1788, PRO3434, PRO1927, PRO3567, PRO1295, PRO1293, PRO303, PRO4344, PRO4354, PRO4397, PRO4407, PRO1555, PRO1096, PRO2038 or PRO2262 or the desired portion of the coding sequence of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 [such as the sequence encoding the extracellular domain of a transmembrane protein or the sequence encoding the mature protein if the protein is extracellular] is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodopterafrugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley *et al*., Baculovirus expression vectors: A Laboratory Manual, Oxford: Oxford University Press (1994).

Expressed poly-His tagged PRO381, PRO1269, PRO1410, PRO1755, PRO1780, PRO1788, PRO3434, PRO1927, PRO3567, PRO1295, PRO1293, PRO303, PRO4344, PRO4354, PRO4397, PRO4407, PRO1555, PRO1096, PRO2038 or PRO2262 can then be purified, for example, by $Ni^{2+}$-chelate affinity chromatography as follows. Extracts are prepared from recombinant virus-infected Sf9 cells as described by Rupert *et al*., Nature, 362:175-179 (1993). Briefly, Sf9 cells are washed, resuspended in sonication buffer (25 ml Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 $\mu$m filter. A $Ni^{2+}$-NTA agarose column (commercially available from Qiagen) is prepared with a bed volume of 5 ml, washed with 25 ml of water and equilibrated with 25 ml of loading buffer. The filtered cell extract is loaded onto the column at 0.5 ml per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM imidazole gradient in the secondary wash buffer. One ml fractions are collected and analyzed by SDS-PAGE and silver staining or Western blot with $Ni^{2+}$-NTA-conjugated to alkaline phosphatase (Qiagen). Fractions containing the eluted $His_{10}$-tagged PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262, respectively, are pooled and dialyzed against loading buffer.

Alternatively, purification of the IgG tagged (or Fc tagged) PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 can be performed using known chromatography techniques, including for instance, Protein A or protein G column chromatography.

While expression is actually performed in a 0.5-2 L scale, it can be readily scaled up for larger (*e.g.*, 8 L) preparations. The proteins are expressed as an IgG construct (immunoadhesin), in which the protein extracellular region is fused to an IgG I constant region sequence containing the hinge, CH2 and CH3 domains and/or in poly-His tagged forms.

Following PCR amplification,therespectivecoding sequences are subcloned into a baculovirus expression vector (pb.PH.IgG for IgG fusions and pb.PH.His.c for poly-His tagged proteins), and the vector and Baculogold® baculovirus DNA (Pharmingen) are co-transfected into 105 *Spodoptera frugiperda* ("Sf9") cells (ATCC CRL 1711), using Lipofectin (Gibco BRL). pb.PH.IgG and pb.PH.His are modifications of the commercially available baculovirus expression vector pVL1393 (Pharmingen), with modified polylinker regions to include the His or Fc tag sequences. The cells are grown in Hink's TNM-FH medium supplemented with 10% FBS (Hyclone). Cells are incubated for 5 days at 28°C. The supernatant is harvested and subsequently used for the first viral amplification by infecting Sf9 cells in Hink's TNM-FH medium supplemented with 10% FBS at an approximate multiplicity of infection (MOI) of 10. Cells are incubated for 3 days at 28 °C. The supernatant is harvested and the expression of the constructs in the baculovirus expression vector is determined by batch binding of 1 ml of supernatant to 25 ml of $Ni^{2+}$-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The first viral amplification supernatant is used to infect a spinner culture (500 ml) of Sf9 cells grown in ESF-921 medium (Expression Systems LLC) at an approximate MOI of 0.1. Cells are incubated for 3 days at 28 °C. The supernatant is harvested and filtered. Batch binding and SDS-PAGE analysis are repeated, as necessary, until expression of the spinner culture is confirmed.

The conditioned medium from the transfected cells (0.5 to 3 L) is harvested by centrifugation to remove the cells and filtered through 0.22 micron filters. For the poly-His tagged constructs, the protein construct is purified using a Ni $^{2+}$-NTA column (Qiagen). Before purification, imidazole is added to the conditioned media to a concentration of 5 mM. The conditioned media is pumped onto a 6 ml Ni $^{2+}$-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of proteins are purified from the conditioned media as follows. The conditioned media is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting I ml fractions into tubes containing 275 ml of 1 M Tris buffer, pH 9. The highly purified protein is subsequently desalted into storage buffer as described above for the poly-His tagged proteins. The homogeneity of the proteins is verified by SDS polyacrylamide gel (PEG) electrophoresis and N-terminal amino acid sequencing by Edman degradation.

Alternatively, a modified baculovirus procedure may be used incorporating high 5 cells. In this procedure, the DNA encoding the desired sequence is amplified with suitable systems, such as Pfu (Stratagene), or fused upstream (5'-of) of an epitope tag contained with a baculovirus expression vector. Such epitope tags include poly-His tags and immunoglobulin tags (like Fc regions of IgG). A variety of plasmids may be employed, including plasmids derived from commercially available plasmids such as pIE1-1 (Novagen). The pIE1-1 and pIE1-2 vectors are designed for constitutive expression of recombinant proteins from the baculovirus iel promoter in stably-transformed insect cells. The plasmids differ only in the orientation of the multiple cloning sites and contain all promoter sequences known to be important for iel-mediated gene expression in uninfected insect cells as well as the hr5 enhancer element. pIE1-1 and pIE1-2 include the translation initiation site and can be used to produce fusion proteins. Briefly, the desired sequence or the desired portion of the sequence (such as the sequence encoding the extracellular domain of a transmembrane protein) is amplified by PCR with primers complementary to the 5' and 3' regions. The 5' primer may incorporate flanking (selected) restriction enzyme sites. The product is then digested with those selected restriction enzymes and subcloned into the expression vector. Forexample, derivatives ofpIE 1-l can include the Fc region of human IgG (pb.PH.IgG) or an 8 histidine (pb.PH.His) tag downstream (3'-of) the desired sequence. Preferably, the vector construct is sequenced for confirmation.

High 5 cells are grown to a confluency of 50% under the conditions of 27°C, no $CO_2$, NO pen/strep. For each 150 mm plate, 30 $\mu$g of pIE based vector containing the sequence is mixed with 1 ml Ex-Cell medium (Media: Ex-Cell 401 + 1/100 L-Glu JRH Biosciences # 14401-78P (note: this media is light sensitive)), and in a separate tube, 100 $\mu$l of CellFectin (CellFECTIN (GibcoBRL # 10362-010)(vortexed to mix)) is mixed with 1 ml of Ex-Cell medium. The two solutions are combined and allowed to incubate at room temperature for 15 minutes. 8 ml of Ex-Cell media is added to the 2 ml of DNA/CellFECTIN mix and this is layered on high 5 cells that has been washed once with Ex-Cell media. The plate is then incubated in darkness for 1 hour at room temperature. The DNA/CellFECTIN mix is then aspirated, and the cells are washed once with Ex-Cell to remove excess CellFECTIN, 30 ml of fresh Ex-Cell media is added and the cells are incubated for 3 days at 28°C. The supernatant is harvested and the expression of the sequence in the baculovirus expression vector is determined by batch binding of 1 ml of supernatant to 25 ml of Ni $^{2+}$-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The conditioned media from the transfected cells (0.5 to 3 L) is harvested by centrifugation to remove the cells and filtered through 0.22 micron filters. For the poly-His tagged constructs, the protein comprising the sequence is purified using a Ni $^{2+}$-NTA column (Qiagen). Before purification, imidazole is added to the conditioned media to a concentration of 5 mM. The conditioned media is pumped onto a 6 ml Ni $^{2+}$-NTA column equilibrated in 20 mM Hepes, pH 7.4, buffer containing 0.3 M NaCl and 5 mM imidazole at a flow rate of 4-5 ml/min. at 48°C. After loading, the column is washed with additional equilibration buffer and the protein eluted with equilibration buffer containing 0.25 M imidazole. The highly purified protein is then subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of proteins are purified from the conditioned media as follows. The conditioned media is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 ml of 1 M Tris buffer, pH 9. The highly purified protein is subsequently desalted into storage buffer as described above for the poly-His tagged proteins. The homogeneity of the sequence is assessed by SDS polyacrylamide gels and by N-terminal amino acid sequencing by Edman degradation and otheranalytical procedures as desired or necessary.

PRO381, PRO1410 and PRO4354 were successfully expressed by the above modified baculovirus procedure incorporating high 5 cells.

EXAMPLE 26

Preparation of Antibodies that Bind PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262

This example illustrates preparation of monoclonal antibodies which can specifically bind PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262.

Techniques for producing the monoclonal antibodies are known in the art and are described, for instance, in Goding, *supra*. Immunogens that may be employed include purified PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 fusion proteins containing PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 and cells expressing recombinant PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 on the cell surface. Selection of the immunogen can be made by the skilled artisan without undue experimentation.

Mice, such as Balb/c, are immunized with the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testingin ELISA assays to detect anti-PRO381, anti-PRO1269, anti-PRO1410, anti-PRO1755, anti-PRO1780,anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibodies.

After a suitable antibody titer has been detected, the animals "positive" for antibodies can be injected with a final intravenous injection of PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262. Determination of "positive" hybridoma cells secreting the desired monoclonal antibodies against PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 is within the skill in the art.

The positive hybridoma cells can be injected intraperitoneally into syngeneic Balb/c mice to produce ascitescontainingthe anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293,anti-PRO1303,anti-PRO4344, anti-PRO4354, anti-PRO4397, anti-PRO4407, anti-PRO1555, anti-PRO1096, anti-PRO2038 or anti-PRO2262 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

Deposit of Material:

The following materials have been deposited with the American Type Culture Collection, 10801 University Blvd., Manassas, VA 20110-2209, USA (ATCC):

| Material | ATCC Deposit No.: | Deposit Date |
|---|---|---|
| DNA44194-1317 | 209808 | April 28, 1998 |
| DNA66520-1536 | 203226 | September 15, 1998 |
| DNA68874-1622 | 203277 | September 22, 1998 |
| DNA76396-1698 | 203471 | November 17, 1998 |
| DNA71169-1709 | 203467 | November 17, 1998 |
| DNA77652-2505 | 203480 | November 17, 1998 |
| DNA77631-2537 | 203651 | February 9, 1999 |
| DNA82307-2531 | 203537 | December 15, 1998 |
| DNA56049-2543 | 203662 | February 9, 1999 |
| DNA59218-1559 | 203287 | September 29, 1998 |
| DNA60618-1557 | 203292 | September 29, 1998 |
| DNA65409-1566 | 203232 | September 15, 1998 |
| DNA84927-2585 | 203865 | March 23, 1999 |
| DNA92256-2596 | 203891 | March 30, 1999 |
| DNA83505-2606 | 132-PTA | May 25, 1999 |
| DNA92264-2616 | 203969 | April 27, 1999 |
| DNA73744-1665 | 203322 | October 6, 1998 |

These deposits were made under the provisions of the Budapest Treaty on the International Recognition of the Deposit of Microorganisms for the Purpose of Patent Procedure and the Regulations thereunder (Budapest Treaty). This assures the maintenance of a viable culture of the deposit for 30 years from the date of deposit. The deposit will be made available by the ATCC under the terms of the Budapest Treaty, and subject to an agreement between Genentech, Inc., and the ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 U.S.C. § 122 and the Commissioner's rules pursuant thereto (including 37 C.F.R. § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

The following numbered paragraphs (paras.) contain further statements of various aspects of the present invention:-

1. An isolated antibody that binds to a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.
2. The antibody of Para. 1 which specifically binds to said polypeptide.
3. The antibody of Para. 1 which induces the death of a cell that expresses said polypeptide.

4. The antibody of Para. 3, wherein said cell is a cancer cell that overexpresses said polypeptide as compared to a normal cell of the same tissue type.

5. The antibody of Para. 1 which is a monoclonal antibody.

6. The antibody of Para. 5 which comprises a non-human complementarity determining region (CDR) or a human framework region (FR).

7. The antibody of Para. 1 which is labeled.

8. The antibody of Para. 1 which is an antibody fragment or a single-chain antibody.

9. A composition of matter which comprises an antibody of Para. 1 in admixture with a pharmaceutically acceptable carrier.

10. The composition of matter of Para. 9 which comprises a therapeutically effective amount of said antibody.

11. The composition of matter of Para. 9 which further comprises a cytotoxic or a chemotherapeutic agent.

12. An isolated nucleic acid molecule that encodes the antibody of Para. 1.

13. A vector comprising the nucleic acid molecule of Para. 12.

14. A host cell comprising the vector of Para. 13.

15. A method for producing an antibody that binds to a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, said method comprising culturing the host cell of Para. 14 under conditions sufficient to allow expression of said antibody and recovering said antibody from the cell culture.

16. An antagonist of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

17. The antagonist of Para. 16, wherein said antagonist inhibits tumor cell growth.

18. An isolated nucleic acid molecule that hybridizes to a nucleic acid sequence that encodes a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, or the complement thereof.

19. The isolated nucleic acid molecule of Para. 18, wherein said hybridization is under stringent hybridization and wash conditions.

20. A method for determining the presence of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in a sample suspected of containing said polypeptide, said method comprising exposing the sample to an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody and determining binding of said antibody to said polypeptide in said sample.

21. The method of Para. 20, wherein said sample comprises a cell suspected of containing a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

22. The method of Para. 21, wherein said cell is a cancer cell.

23. A method of diagnosing tumor in a mammal, said method comprising detecting the level of expression of a gene encoding a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide (a) in a test sample of tissue cells obtained from the mammal, and (b) in a control sample of known normal tissue cells of the same cell type, wherein a higher expression level in the test sample, as compared to the control sample, is indicative of the presence of tumor in the mammal from which the test tissue cells were obtained.

24. A method of diagnosing tumor in a mammal, said method comprising (a) contacting an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody with a test sample of tissue cells obtained from the mammal, and (b) detecting the formation of a complex between said antibody and a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in the test sample, wherein the formation of a complex is indicative of the presence of a tumor in said mammal.

25. The method of Para. 24, wherein said antibody is detectably labeled.

26. The method of Para. 24, wherein said test sample of tissue cells is obtained from an individual suspected of having neoplastic cell growth or proliferation.

27. A cancer diagnostic kit comprising an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody and a carrier in suitable packaging.

28. The kit of Para. 27 which further comprises instructions for using said antibody to detect the presence of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in a sample suspected of containing the same.

29. A method for inhibiting the growth of tumor cells, said method comprising exposing tumor cells that express a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide to an effective amount of an agent that inhibits a biological activity of said polypeptide, wherein growth of said tumor cells is thereby inhibited.

30. The method of Para. 29, wherein said tumor cells overexpress said polypeptide as compared to normal cells of the same tissue type.

31. The method of Para. 29, wherein said agent is an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody.

32. The method of Para. 31, wherein said anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody induces cell death.

33. The method of Para. 29, wherein said tumor cells are further exposed to radiation treatment, a cytotoxic agent or a chemotherapeutic agent.

34. A method for inhibiting the growth of tumor cells, said method comprising exposing tumor cells that express a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide to an effective amount of an agent that inhibits the expression of said polypeptide, wherein growth of said tumor cells is thereby inhibited.

35. The method of Para. 34, wherein said tumor cells overexpress said polypeptide as compared to normal cells of the same tissue type.

36. The method of Para. 34, wherein said agent is an antisense oligonucleotide that hybridizes to a nucleic acid which encodes the PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide or the complement thereof.

37. The method of Para. 36, wherein said tumor cells are further exposed to radiation treatment, a cytotoxic agent or a chemotherapeutic agent.

38. An article of manufacture, comprising:

a container;
a label on the container; and
a composition comprising an active agent contained within the container, wherein the composition is effective for inhibiting the growth of tumor cells and wherein the label on the container indicates that the composition is effective for treating conditions characterized by overexpression of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in said tumor cells as compared to in normal cells of the same tissue type.

39. The article of manufacture of Para. 38, wherein said active agent inhibits a biological activity of and/or the expression of said PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide.

40. The article of manufacture of Para. 39, wherein said active agent is an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody.

41. The article of manufacture of Para. 39, wherein said active agent is an antisense oligonucleotide.

42. A method of identifying a compound that inhibits a biological or immunological activity of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, said method comprising contacting a candidate compound with said polypeptide under conditions and for a time sufficient to allow the two components to interact and determining whether a biological or immunological activity of said polypeptide is inhibited.

43. The method of Para. 42, wherein said candidate compound is an anti-PRO381, anti-PRO1269, anti-PRO1410, anti-PRO1755, anti-PRO1780, anti-PRO1788, anti-PRO3434, anti-PRO1927, anti-PRO3567, anti-PRO1295, anti-PRO1293, anti-PRO1303, anti-PRO4344, anti-PRO4354, anti-PRO4397, anti-PRO4407, anti-PRO1555, anti-PRO1096, anti-PRO2038 or anti-PRO2262 antibody.

44. The method of Para. 42, wherein said candidate compound or said PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide is immobilized on a solid support.

45. The method of Para. 44, wherein the non-immobilized component is detectably labeled.

46. A method of identifying a compound that inhibits an activity of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide, said method comprising the steps of (a) contacting cells and a candidate compound to be screened in the presence of said polypeptide under conditions suitable for the induction of a cellular response normally induced by said polypeptide and (b) determining the induction of said cellular response to determine if the test compound is an effective antagonist, wherein the lack of induction of said cellular response is indicative of said compound being an effective antagonist.

47. A method for identifying a compound that inhibits the expression of a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide in cells that express said polypeptide, wherein said method comprises contacting said cells with a candidate compound and determining whether expression of said polypeptide is inhibited.

48. The method of Para. 47, wherein said candidate compound is an antisense oligonucleotide.

49. Isolated nucleic acid having at least 80% nucleic acid sequence identity to a nucleotide sequence that encodes an amino acid sequence selected from the group consisting of the amino acid sequence shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), and Figure 40 (SEQ ID NO:55).

50. Isolated nucleic acid having at least 80% nucleic acid sequence identity to a nucleotide sequence selected from the group consisting of the nucleotide sequence shown in Figure 1 (SEQ ID NO:1), Figure 3 (SEQ ID NO:6), Figure 5 (SEQ ID NO:8), Figure 7 (SEQ ID NO:10), Figure 9 (SEQ ID NO:12), Figure 11 (SEQ ID NO:17), Figure 13 (SEQ ID NO:22), Figure 15 (SEQ ID NO:24), Figure 17 (SEQ ID NO:26), Figure 19 (SEQ ID NO:28), Figure 21 (SEQ ID NO:30), Figure 23 (SEQ ID NO:32), Figure 25 (SEQ ID NO:34), Figure 27 (SEQ ID NO:39), Figure 29 (SEQ ID NO: 41), Figure 31 (SEQ ID NO:46), Figure 33 (SEQ ID NO:48), Figure 35 (SEQ ID NO:50), Figure 37 (SEQ ID NO:52), and Figure 39 (SEQ ID NO:54).

51. Isolated nucleic acid having at least 80% nucleic acid sequence identity to a nucleotide sequence selected from the group consisting of the full-length coding sequence of the nucleotide sequence shown in Figure 1 (SEQ ID NO: 1), Figure 3 (SEQ ID NO:6), Figure 5 (SEQ ID NO:8), Figure 7 (SEQ ID NO:10), Figure 9 (SEQ ID NO:12), Figure 11 (SEQ ID NO:17), Figure 13 (SEQ ID NO:22), Figure 15 (SEQ ID NO:24), Figure 17 (SEQ ID NO:26), Figure 19 (SEQ ID NO:28), Figure 21 (SEQ ID NO:30), Figure 23 (SEQ ID NO:32), Figure 25 (SEQ ID NO:34), Figure 27 (SEQ ID NO:39), Figure 29 (SEQ ID NO:41), Figure 31 (SEQ ID NO:46), Figure 33 (SEQ ID NO:48), Figure 35 (SEQ ID NO:50), Figure 37 (SEQ ID NO:52), and Figure 39 (SEQ ID NO:54).

52. Isolated nucleic acid having at least 80% nucleic acid sequence identity to the full-length coding sequence of the DNA deposited under ATCC accession number 209808, 203226, 203277, 203471, 203467, 203480, 203651, 203537, 203662, 203287, 203292, 203232, 203865, 203891, 132-PTA, 203969, or 203322.

53. A vector comprising the nucleic acid of any one of Paras. 49 to 52.

54. The vector of Para. 53 operably linked to control sequences recognized by a host cell transformed with the vector.

55. A host cell comprising the vector of Para. 53.

56. The host cell of Para. 55, wherein said cell is a CHO cell.

57. The host cell of Para. 55, wherein said cell is an *E. coli*.

58. The host cell of Para. 55, wherein said cell is a yeast cell.

59. The host cell of Para. 55, wherein said cell is a Baculovirus-infected insect cell.

60. A process for producing a PRO381, PRO1269, PRO1410, PRO1755, PRO1780, PRO1788, PRO3434, PRO1927, PRO3567, PRO1295, PRO1293, PRO1303, PRO4344, PRO4354, PRO4397, PRO4407, PRO1555, PRO1096, PRO2038 or PRO2262 polypeptide comprising culturing the host cell of Para. 55 under conditions suitable for expression of said polypeptide and recovering said polypeptide from the cell culture.
61. An isolated polypeptide having at least 80% amino acid sequence identity to an amino acid sequence selected from the group consisting of the amino acid sequence shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), and Figure 40 (SEQ ID NO:55).
62. An isolated polypeptide scoring at least 80% positives when compared to an amino acid sequence selected from the group consisting of the amino acid sequence shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), and Figure 40 (SEQ ID NO:55).
63. An isolated polypeptide having at least 80% amino acid sequence identity to an amino acid sequence encoded by the full-length coding sequence of the DNA deposited under ATCC accession number 209808, 203226, 203277, 203471, 203467, 203480, 203651, 203537, 203662, 203287, 203292, 203232, 203865, 203891, 132-PTA, 203969, or 203322.
64. A chimeric molecule comprising a polypeptide according to any one of Paras. 61 to 63 fused to a heterologous amino acid sequence.
65. The chimeric molecule of Para. 64, wherein said heterologous amino acid sequence is an epitope tag sequence.
66. The chimeric molecule of Para. 64, wherein said heterologous amino acid sequence is a Fc region of an immunoglobulin.
67. An antibody which specifically binds to a polypeptide according to any one of Paras. 61 to 63.
68. The antibody of Para. 67, wherein said antibody is a monoclonal antibody, a humanized antibody or a single-chain antibody.
69. Isolated nucleic acid having at least 80% nucleic acid sequence identity to:

(a) a nucleotide sequence encoding the polypeptide shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), lacking its associated signal peptide;
(b) a nucleotide sequence encoding an extracellular domain of the polypeptide shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), with its associated signal peptide; or
(c) a nucleotide sequence encoding an extracellular domain of the polypeptide shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), lacking its associated signal peptide.

70. An isolated polypeptide having at least 80% amino acid sequence identity to:

(a) the polypeptide shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), lacking its associated signal peptide;

(b) an extracellular domain of the polypeptide shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), with its associated signal peptide; or

(c) an extracellular domain of the polypeptide shown in Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 18 (SEQ ID NO:27), Figure 20 (SEQ ID NO:29), Figure 22 (SEQ ID NO:31), Figure 24 (SEQ ID NO:33), Figure 26 (SEQ ID NO:35), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), lacking its associated signal peptide.

NOTICE

The content of the present application is not to be construed as limited to that of the claims as filed, but includes all subject matter disclosed in the specification.

The applicant maintains the right to file amendments and/or further divisional applications from this one, directed to any subject matter that is disclosed in the present specification, irrespective of whether that subject matter is in the claims of this application as filed, and irrespective of the fate of any European patent application from which this application is divided.

## Claims

1. An isolated antibody that binds to a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide, for use as a medicament.

2. The antibody of Claim 1 which specifically binds to said polypeptide.

3. The antibody of Claim 1 which induces the death of a cell that expresses said polypeptide.

4. The antibody of Claim 3, wherein said cell is a cancer cell that overexpresses said polypeptide as compared to a normal cell of the same tissue type.

5. The antibody of Claim 1 which is a monoclonal antibody.

6. The antibody of Claim 5 which comprises a non-human complementarity determining region (CDR) or a human framework region (FR).

7. The antibody of Claim 1 which is labeled.

8. The antibody of Claim 1 which is an antibody fragment or a single-chain antibody.

9. A composition of matter which comprises an antibody of Claim 1 in admixture with a pharmaceutically acceptable carrier.

10. The composition of matter of Claim 9 which comprises a therapeutically effective amount of said antibody.

11. The composition of matter of Claim 9 which further comprises a cytotoxic or a chemotherapeutic agent.

12. A method for determining the presence of a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide in a sample suspected of containing said polypeptide, said method comprising exposing the sample to an anti-PRO4344, anti-PRO3567, anti-PRO381, anti-PRO1269, anti-PRO1410, anti-PRO1755, anti-PRO1780, anti-PRO1788, anti-PRO3434, anti-PRO1927, anti-PRO1295, anti-PRO1293, anti-PRO1303, anti-PRO4354, anti-PRO4397, anti-PRO4407, anti-PRO1555, anti-PRO1096, anti-PRO2038 or anti-PRO2262 antibody and determining binding of said antibody to said polypeptide in said sample, wherein said sample comprises a cancer cell suspected of containing a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide.

13. A method of diagnosing tumor in a mammal, said method comprising detecting the level of expression of a gene encoding a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide (a) in a test sample of tissue cells obtained from the mammal, and (b) in a control sample of known normal tissue cells of the same cell type, wherein a higher expression level in the test sample, as compared to the control sample, is indicative of the presence of tumor in the mammal from which the test tissue cells were obtained.

**14.** A method of diagnosing tumor in a mammal, said method comprising (a) contacting an anti-PRO4344, anti-PRO3567, anti-PRO381, anti-PRO1269, anti-PRO1410, anti-PRO1755, anti-PRO1780, anti-PRO1788, anti-PRO3434, anti-PRO1927, anti-PRO1295, anti-PRO1293, anti-PRO1303, anti-PRO4354, anti-PRO4397, anti-PRO4407, anti-PRO1555, anti-PRO1096, anti-PRO2038 or anti-PRO2262 antibody with a test sample of tissue cells obtained from the mammal, and (b) detecting the formation of a complex between said antibody and a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide in the test sample, wherein the formation of a complex is indicative of the presence of a tumor in said mammal.

**15.** The method of Claim 14, wherein said antibody is detectably labeled.

**16.** The method of Claim 14, wherein said test sample of tissue cells is obtained from an individual suspected of having neoplastic cell growth or proliferation.

**17.** A cancer diagnostic kit comprising an anti-PRO4344, anti-PRO3567, anti-PRO381, anti-PRO1269, anti-PRO1410, anti-PRO1755, anti-PRO1780, anti-PRO1788, anti-PRO3434, anti-PRO1927, anti-PRO1295, anti-PRO1293, anti-PRO1303, anti-PRO4354, anti-PRO4397, anti-PRO4407, anti-PRO1555, anti-PRO1096, anti-PRO2038 or anti-PRO2262 antibody and a carrier in suitable packaging, and instructions for using said antibody to detect the presence of a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide in a sample suspected of containing the same.

**18.** Use of an agent in the manufacture of a medicament for the inhibition of growth of tumour cells, wherein the tumour cells express a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide, and wherein the agent inhibits a biological activity of said polypeptide.

**19.** The use of Claim 18, wherein said tumor cells overexpress said polypeptide as compared to normal cells of the same tissue type.

**20.** The use of Claim 18, wherein said agent is an anti-PRO4344, anti-PRO3567, anti-PRO381, anti-PRO1269, anti-PRO1410, anti-PRO1755, anti-PRO1780, anti-PRO1788, anti-PRO3434, anti-PRO1927, anti-PRO1295, anti-PRO1293, anti-PRO1303, anti-PRO4354, anti-PRO4397, anti-PRO4407, anti-PRO1555, anti-PRO1096, anti-PRO2038 or anti-PRO2262 antibody.

**21.** The use of Claim 20, wherein said anti-PRO4344, anti-PRO3567, anti-PRO381, anti-PRO1269, anti-PRO1410, anti-PRO1755, anti-PRO1780, anti-PRO1788, anti-PRO3434, anti-PRO1927, anti-PRO1295, anti-PRO1293, anti-PRO1303, anti-PRO4354, anti-PRO4397, anti-PRO4407, anti-PRO1555, anti-PRO1096, anti-PRO2038 or anti-PRO2262 antibody induces cell death.

**22.** The use of Claim 18, wherein said tumor cells are further exposed to radiation treatment, a cytotoxic agent or a chemotherapeutic agent.

**23.** Use of an agent in the manufacture of a medicament for inhibiting the growth of tumor cells, wherein the tumor cells express a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide, and wherein the agent inhibits the expression of said polypeptide.

**24.** The use of Claim 23, wherein said tumor cells overexpress said polypeptide as compared to normal cells of the same tissue type.

**25.** The use of Claim 23, wherein said agent is an antisense oligonucleotide that hybridizes to a nucleic acid which encodes the PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide or the complement thereof.

**26.** The use of Claim 23, wherein said tumor cells are further exposed to radiation treatment, a cytotoxic agent or a chemotherapeutic agent.

**27.** An article of manufacture, comprising:

a container;
a label on the container; and
a composition comprising an active agent contained within the container, wherein the composition is effective for inhibiting the growth of tumor cells and wherein the label on the container indicates that the composition is effective for treating conditions **characterized by** overexpression of a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide in said tumor cells as compared to in normal cells of the same tissue type.

**28.** The article of manufacture of Claim 27, wherein said active agent inhibits a biological activity of and/or the expression of said PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide.

**29.** The article of manufacture of Claim 28, wherein said active agent is an anti-PRO4344, anti-PRO3567, anti-PRO381, anti-PRO1269, anti-PRO1410, anti-PRO1755, anti-PRO1780, anti-PRO1788, anti-PRO3434, anti-PRO1927, anti-PRO1295, anti-PRO1293, anti-PRO1303, anti-PRO4354, anti-PRO4397, anti-PRO4407, anti-PRO1555, anti-PRO1096, anti-PRO2038 or anti-PRO2262 antibody.

**30.** The article of manufacture of Claim 28, wherein said active agent is an antisense oligonucleotide.

**31.** Isolated nucleic acid having at least 80% nucleic acid sequence identity to:

(a) a nucleotide sequence that encodes an amino acid sequence selected from the group consisting of the amino acid sequence shown in Figure 26 (SEQ ID NO:35), Figure 18 (SEQ ID NO:27), Figure 2 (SEQ ID NO: 2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 20 (SEQ ID NO:29), Figure 22 (SEQ ID NO:31), Figure 24 (SEQ ID NO:33), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), and Figure 40 (SEQ ID NO:55);
(b) a nucleotide sequence selected from the group consisting of the nucleotide sequence shown in Figure 25 (SEQ ID NO:34), Figure 17 (SEQ ID NO:26), Figure 1 (SEQ ID NO:1), Figure 3 (SEQ ID NO:6), Figure 5 (SEQ ID NO:8), Figure 7 (SEQ ID NO:10), Figure 9 (SEQ ID NO:12), Figure 11 (SEQ ID NO:17), Figure 13 (SEQ ID NO:22), Figure 15 (SEQ ID NO:24), Figure 19 (SEQ ID NO:28), Figure 21 (SEQ ID NO:30), Figure 23 (SEQ ID NO:32), Figure 27 (SEQ ID NO:39), Figure 29 (SEQ ID NO:41), Figure 31 (SEQ ID NO:46), Figure 33 (SEQ ID NO:48), Figure 35 (SEQ ID NO:50), Figure 37 (SEQ ID NO:52), and Figure 39 (SEQ ID NO:54);
(c) a nucleotide sequence selected from the group consisting of the full-length coding sequence of the nucleotide sequence shown in Figure 25 (SEQ ID NO:34), Figure 17 (SEQ ID NO:26), Figure 1 (SEQ ID NO:1), Figure 3 (SEQ ID NO:6), Figure 5 (SEQ ID NO:8), Figure 7 (SEQ ID NO:10), Figure 9 (SEQ ID NO:12), Figure 11 (SEO ID NO:17), Figure 13 (SEQ ID NO:22), Figure 15 (SEQ ID NO:24), Figure 19 (SEQ ID NO:28), Figure 21 (SEQ ID NO:30), Figure 23 (SEQ ID NO:32), Figure 27 (SEQ ID NO:39), Figure 29 (SEQ ID NO:41), Figure 31 (SEQ ID NO:46), Figure 33 (SEQ ID NO:48), Figure 35 (SEQ ID NO:50), Figure 37 (SEQ ID NO:52), and Figure 39 (SEQ ID NO:54);
(d) the full-length coding sequence of the DNA deposited under ATCC accession number 203865, 203662, 209808, 203226, 203277, 203471, 203467, 203480, 203651, 203537, 203287, 203292, 203232, 203891, 132-PTA, 203969, or 203322;
(e) a nucleotide sequence encoding the polypeptide shown in Figure 26 (SEQ ID NO:35), Figure 18 (SEQ ID NO:27), Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 20 (SEQ ID NO:29), Figure 22 (SEQ ID NO:31), Figure 24 (SEQ ID NO:33), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), lacking its associated signal peptide;
(f) a nucleotide sequence encoding an extracellular domain of the polypeptide shown in Figure 26 (SEQ ID NO: 35), Figure 18 (SEQ ID NO:27), Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 20 (SEQ ID NO:29), Figure 22 (SEQ ID NO:31), Figure 24 (SEQ ID NO:33), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), with its associated signal peptide; or
(g) a nucleotide sequence encoding an extracellular domain of the polypeptide shown in Figure 26 (SEQ ID NO:35), Figure 18 (SEQ ID NO:27), Figure 2 (SEQ ID NO:2), Figure 4 (SEQ ID NO:7), Figure 6 (SEQ ID NO: 9), Figure 8 (SEQ ID NO:11), Figure 10 (SEQ ID NO:13), Figure 12 (SEQ ID NO:18), Figure 14 (SEQ ID NO: 23), Figure 16 (SEQ ID NO:25), Figure 20 (SEQ ID NO:29), Figure 22 (SEQ ID NO:31), Figure 24 (SEQ ID NO: 33), Figure 28 (SEQ ID NO:40), Figure 30 (SEQ ID NO:42), Figure 32 (SEQ ID NO:47), Figure 34 (SEQ ID NO: 49), Figure 36 (SEQ ID NO:51), Figure 38 (SEQ ID NO:53), or Figure 40 (SEQ ID NO:55), lacking its associated signal peptide.

**32.** A vector comprising the nucleic acid of Claim 31, or a host cell comprising said vector.

**33.** A process for producing a PRO4344, PRO381, PRO1269, PRO1410, PRO1755, PRO1780, PRO1788, PRO3434, PRO1927, PRO3567, PRO1295, PRO1293, PRO1303, PRO4354, PRO4397, PRO4407, PRO1555, PRO1096, PRO2038 or PRO2262 polypeptide comprising culturing the host cell of Claim 32 under conditions suitable for expression of said polypeptide and recovering said polypeptide from the cell culture.

**34.** An isolated polypeptide:

(a) having at least 80% amino acid sequence identity to an amino acid sequence selected from the group consisting of the amino acid sequence shown in Figure 26 (SEQ ID NO:35), Figure 18 (SEQ ID NO:27), Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 20 (SEQ ID NO:29), Figure 22 (SEQ ID NO:31), Figure 24 (SEQ ID NO:33), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), and Figure 40 (SEQ ID NO:55);
(b) scoring at least 80% positives when compared to an amino acid sequence selected from the group consisting of the amino acid sequence shown in Figure 26 (SEQ ID NO:35), Figure 18 (SEQ ID NO:27), Figure 2 (SEQ ID NO:2), Figure 4 (SEQ ID NO:7), Figure 6 (SEQ ID NO:9), Figure 8 (SEQ ID NO:11), Figure 10 (SEQ ID NO: 13), Figure 12 (SEQ ID NO:18), Figure 14 (SEQ ID NO:23), Figure 16 (SEQ ID NO:25), Figure 20 (SEQ ID NO: 29), Figure 22 (SEQ ID NO:31), Figure 24 (SEQ ID NO:33), Figure 28 (SEQ ID NO:40), Figure 30 (SEQ ID NO: 42), Figure 32 (SEQ ID NO:47), Figure 34 (SEQ ID NO:49), Figure 36 (SEQ ID NO:51), Figure 38 (SEQ ID NO: 53), and Figure 40 (SEQ ID NO:55);
(c) having at least 80% amino acid sequence identity to an amino acid sequence encoded by the full-length coding sequence of the DNA deposited under ATCC accession number 203865, 203662, 209808, 203226, 203277, 203471, 203467, 203480, 203651, 203537, 203287, 203292, 203232, 203891, 132-PTA, 203969, or 203322;
(d) having at least 80% amino acid sequence identity to the polypeptide shown in Figure 26 (SEQ ID NO:35), Figure 18 (SEQ ID NO:27), Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 20 (SEQ ID NO:29), Figure 22 (SEQ ID NO:31), Figure 24 (SEQ ID NO:33), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), lacking its associated signal peptide;
(e) having at least 80% amino acid sequence identity to an extracellular domain of the polypeptide shown in Figure 26 (SEQ ID NO:35), Figure 18 (SEQ ID NO:27), Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 20 (SEQ ID NO:29), Figure 22 (SEQ ID NO:31), Figure 24 (SEQ ID NO:33), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), with its associated signal peptide; or
(f) having at least 80% amino acid sequence identity to an extracellular domain of the polypeptide shown in Figure 26 (SEQ ID NO:35), Figure 18 (SEQ ID NO:27), Figure 2 (SEQ ID NO:2), Figure 4 (SEQ ID NO:7), Figure 6 (SEQ ID NO:9), Figure 8 (SEQ ID NO:11), Figure 10 (SEQ ID NO:13), Figure 12 (SEQ ID NO:18), Figure 14 (SEQ ID NO:23), Figure 16 (SEQ ID NO:25), Figure 20 (SEQ ID NO:29), Figure 22 (SEQ ID NO:31), Figure 24 (SEQ ID NO:33), Figure 28 (SEQ ID NO:40), Figure 30 (SEQ ID NO:42), Figure 32 (SEQ ID NO:47), Figure 34 (SEQ ID NO:49), Figure 36 (SEQ ID NO:51), Figure 38 (SEQ ID NO:53), or Figure 40 (SEQ ID NO:55), lacking its associated signal peptide.

**35.** A chimeric molecule comprising a polypeptide according to Claim 34 fused to a heterologous amino acid sequence.

**36.** An antibody which specifically binds to a polypeptide according to Claim 34.

**37.** An isolated nucleic acid molecule that hybridizes to a nucleic acid sequence that encodes a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide, or the complement thereof.

**38.** An isolated nucleic acid molecule that encodes the antibody of Claim 1.

**39.** A vector comprising the nucleic acid molecule of Claim 38, or a host cell comprising said vector.

**40.** A method for producing an antibody that binds to a PRO4344, PRO3567, PRO381, PRO1269, PRO1410, PRO1755, PRO1780, PRO1788, PRO3434, PRO1927, PRO1295, PRO1293, PRO1303, PRO4354, PRO4397, PRO4407, PRO1555, PRO1096, PRO2038 or PRO2262 polypeptide, said method comprising culturing the host cell of Claim 39 under conditions sufficient to allow expression of said antibody and recovering said antibody from the cell culture.

<u>FIGURE 1</u>

TTCGTGACCCTTGAGAAAAGAGTTGGTGGTAAATGTGCCACGTCTTCTAAGAAGGGGGAGTCCTGAACTTGTCTG
AAGCCCTTGTCCGTAAGCCTTGAACTACGTTCTTAAATCTATGAAGTCGAGGGACCTTTCGCTGCTTTTGTAGGG
ACTTCTTTCCTTGCTTCAGCAAC**<u>ATG</u>**AGGCTTTTCTTGTGGAACGCGGTCTTGACTCTGTTCGTCACTTCTTTGA
TTGGGGCTTTGATCCCTGAACCAGAAGTGAAAATTGAAGTTCTCCAGAAGCCATTCATCTGCCATCGCAAGACCA
AAGGAGGGGATTTGATGTTGGTCCACTATGAAGGCTACTTAGAAAAGGACGGCTCCTTATTTCACTCCACTCACA
AACATAACAATGGTCAGCCCATTTGGTTTACCCTGGGCATCCTGGAGGCTCTCAAAGGTTGGGACCAGGGCTTGA
AAGGAATGTGTGTAGGAGAGAAGAGAAAGCTCATCATTCCTCCTGCTCTGGGCTATGGAAAAGAAGGAAAAGGTA
AAATTCCCCCAGAAAGTACACTGATATTTAATATTGATCTCCTGGAGATTCGAAATGGACCAAGATCCCATGAAT
CATTCCAAGAAATGGATCTTAATGATGACTGGAAACTCTCTAAAGATGAGGTTAAAGCATATTTAAAGAAGGAGT
TTGAAAAACATGGTGCGGTGGTGAATGAAAGTCATCATGATGCTTTGGTGGAGGATATTTTTGATAAAGAAGATG
AAGACAAAGATGGGTTTATATCTGCCAGAGAATTTACATATAAACACGATGAGTTA**<u>TAG</u>**AGATACATCTACCCTT
TTAATATAGCACTCATCTTTCAAGAGAGGGCAGTCATCTTTAAAGAACATTTTATTTTTATACAATGTTCTTTCT
TGCTTTGTTTTTTATTTTTATATATTTTTTCTGACTCCTATTTAAAGAACCCCTTAGGTTTCTAAGTACCCATTT
CTTTCTGATAAGTTATTGGGAAGAAAAAGCTAATTGGTCTTTGAATAGAAGACTTCTGGACAATTTTTCACTTTC
ACAGATATGAAGCTTTGTTTTACTTTCTCACTTATAAATTTAAAATGTTGCAACTGGGAATATACCACGACATGA
GACCAGGTTATAGCACAAATTAGCACCCTATATTTCTGCTTCCCTCTATTTTCTCCAAGTTAGAGGTCAACATTT
GAAAAGCCTTTTGCAATAGCCCAAGGCTTGCTATTTTCATGTTATAATGAAATAGTTTATGTGTAACTGGCTCTG
AGTCTCTGCTTGAGGACCAGAGGAAAATGGTTGTTGGACCTGACTTGTTAATGGCTACTGCTTTACTAAGGAGAT
GTGCAATGCTGAAGTTAGAAACAAGGTTAATAGCCAGGCATGGTGGCTCATGCCTGTAATCCCAGCACTTTGGGA
GGCTGAGGCGGGCGGATCACCTGAGGTTGGGAGTTCGAGACCAGCCTGACCAACACGGAGAAACCCTATCTCTAC
TAAAAATACAAAGTAGCCCGGCGTGGTGATGCGTGCCTGTAATCCCAGCTACCCAGGAAGGCTGAGGCGGCAGAATC
ACTTGAACCCGAGGCCGAGGTTGCGGTAAGCCGAGATCACCTNCAGCCTGGACACTCTGTCTCGAAAAAAGAAAA
GAACACGGTTAATACCATATNAATATGTATGCATTGAGACATGCTACCTAGGACTTAAGCTGATGAAGCTTGGCT
CCTAGTGATTGGTGGCCTATTATGATAAATAGGACAAATCATTTATGTGTGAGTTTCTTTGTAATAAAATGTATC
AATATGTTATAGATGAGGTAGAAAGTTATATTTATATTCAATATTTACTTCTTAAGGCTAGCGGAATATCCTTCC
TGGTTCTTTAATGGGTAGTCTATAGTATATTATACTACAATAACATTGTATCATAAGATAAAGTAGTAAACCAGT
CTACATTTTCCCATTTCTGTCTCATCAAAAACTGAAGTTAGCTGGGTGTGGTGGCTCATGCCTGTAATCCCAGCA
CTTTGGGGGCCAAGGAGGGTGGATCACTTGAGATCAGGAGTTCAAGACCAGCCTGGCCAACATGGTGAAACCTTG
TCTCTACTAAAAATACAAAAATTAGCCAGGCGTGGTGGTGCACACCTGTAGTCCCAGCTACTCGGGAGGCTGAGA
CAGGAGATTTGCTTGAACCCGGGAGGCGGAGGTTGCAGTGAGCCAAGATTGTGCCACTGCACTCCAGCCTGGGTG
ACAGAGCAAGACTCCATCTCAAAAAAAAAAAAAAAGAAGCAGACCTACAGCAGCTACTATTGAATAAATACCTATC
CTGGATTTT

# FIGURE 2

| | |
|---|---|
| Signal peptide: | Amino acids 1-20 |
| N-glycosylation site: | Amino acids 176-180 |
| Endoplasmic reticulum targeting sequence: | Amino acids 208-212 |
| FKBP-type peptidyl-prolyl cis-trans isomerase sites: | Amino acids 78-115; 118-132 |
| EF-hand calcium binding domains: | Amino acids 191-204; 184-204; 140-160 |
| S-100/ICaBP type calcium binding domain: | Amino acids 183-204 |

MRLFLWNAVLTLFVTSLIGALIPEPEVKIEVLQKPFICHRKTKGGDLMLVHYEGYLEKDGSLFHSTHKHNNGQPI
WFTLGILEALKGWDQGLKGMCVGEKRKLIIPPALGYGKEGKGKIPPESTLIFNIDLLEIRNGPRSHESFQEMDLN
DDWKLSKDEVKAYLKKEFEKHGAVVNESHHDALVEDIFDKEDEDKDGFISAREFTYKHDEL

## FIGURE 3

TCCCGGACCCTGCCGCCCTGCCACT**<u>ATG</u>**TCCCGCCGCTCTATGCTGCTTGCCTGGGCTCTCCCCAGCCTCCTTCG
ACTCGGAGCGGCTCAGGAGACAGAAGACCCGGCCTGCTGCAGCCCCATAGTGCCCCGGAACGAGTGGAAGGCCCT
GGCATCAGAGTGCGCCCAGCACCTGAGCCTGCCCTTACGCTATGTGGTGGTATCGCACACGGCGGGCAGCAGCTG
CAACACCCCCGCCTCGTGCCAGCAGCAGGCCCGGAATGTGCAGCACTACCACATGAAGACACTGGGCTGGTGCGA
CGTGGGCTACAACTTCCTGATTGGAGAAGACGGGCTCGTATACGAGGGCCGTGGCTGGAACTTCACGGGTGCCCA
CTCAGGTCACTTATGGAACCCCATGTCCATTGGCATCAGCTTCATGGGCAACTACATGGATCGGGTGCCCACACC
CCAGGCCATCCGGGCAGCCCAGGGTCTACTGGCCTGCGGTGTGGCTCAGGGAGCCCTGAGGTCCAACTATGTGCT
CAAAGGACACCGGGATGTGCAGCGTACACTCTCTCCAGGCAACCAGCTCTACCACCTCATCCAGAATTGGCCACA
CTACCGCTCCCCC**<u>TGA</u>**GGCCCTGCTGATCCGCACCCCATTCCTCCCCTCCCATGGCCAAAAACCCCACTGTCTCC
TTCTCCAATAAAGATGTAGCTC

## FIGURE 4

Signal peptide: Amino acids 1-20

N-glycosylation site: Amino acids 112-116

MSRRSMLLAWALPSLLRLGAAQETEDPACCSPIVPRNEWKALASECAQHLSLPLRYVVVSHTAGSSCNTPASCQQ
QARNVQHYHMKTLGWCDVGYNFLIGEDGLVYEGRGWNFTGAHSGHLWNPMSIGISFMGNYMDRVPTPQAIRAAQG
LLACGVAQGALRSNYVLKGHRDVQRTLSPGNQLYHLIQNWPHYRSP

## FIGURE 5

GTCGAAGGTTATAAAAGCTTCCAGCCAAACGGCATTGAAGTTGAAGATACAACCTGACAGCACAGCCTGAGATCT
TGGGGATCCCTCAGCCTAACACCCACAGACGTCAGCTGGTGGATTCCCGCTGCATCAAGGCCTACCCACTGTCTCC
**ATG**CTGGGCTCTCCCTGCCTTCTGTGGCTCCTGGCCGTGACCTTCTTGGTTCCCAGAGCTCAGCCCTTGGCCCCT
CAAGACTTTGAAGAAGAGGAGGCAGATGAGACTGAGACGGCGTGGCCGCCTTTGCCGGCTGTCCCCTGCGACTAC
GACCACTGCCGACACCTGCAGGTGCCCTGCAAGGAGCTACAGAGGGTCGGGCCGGCGGCCTGCCTGTGCCCAGGA
CTCTCCAGCCCCGCCCAGCCGCCCGACCCGCCGCGCATGGGAGAAGTGCGCATTGCGGCCGAAGAGGGCCGCGCA
GTGGTCCACTGGTGTGCCCCCTTCTCCCCGGTCCTCCACTACTGGCTGCTGCTTTGGGACGGCAGCGAGGCTGCG
CAGAAGGGGCCCCCGCTGAACGCTACGGTCCGCAGAGCCGAACTGAAGGGGCTGAAGCCAGGGGGCATTTATGTC
GTTTGCGTAGTGGCCGCTAACGAGGCCGGGGCAAGCCGCGTGCCCCAGGCTGGAGGAGAGGGCCTCGAGGGGGCC
GACATCCCTGCCTTCGGGCCTTGCAGCCGCCTTGCGGTGCCGCCCAACCCCCGCACTCTGGTCCACGCGGCCGTC
GGGGTGGGCACGGCCCTGGCCCTGCTAAGCTGTGCCGCCCTGGTGTGGCACTTCTGCCTGCGCGATCGCTGGGGC
TGCCCGCGCCGAGCCGCCGCCCGAGCCGCAGGGGCGCTC**TGA**AAGGGGCCTGGGGGCATCTCGGGCACAGACAGC
CCCACCTGGGGCGCTCAGCCTGGCCCCCGGGAAAGAGGAAAACCCGCTGCCTCCAGGGAGGGCTGGACGGCGAGC
TGGGAGCCAGCCCCAGGCTCCAGGGCCACGGCGGAGTCATGGTTCTCAGGACTGAGCGCTTGTTTAGGTCCGGTA
CTTGGCGCTTTGTTTCCTGGCTGAGGTCTGGGAAGGAATAGAAAGGGGCCCCCAATTTTTTTTAAGCGGCCAGA
TAATAAATAATGTAACCTTTGCGGTTAAAAAAAAAAAAAAAAAA

## FIGURE 6

| | |
|---|---|
| Signal peptide: | Amino acids 1-20 |
| Transmembrane domain: | Amino acids 194-220 |
| N-glycosylation site: | Amino acids 132-136 |

MLGSPCLLWLLAVTFLVPRAQPLAPQDFEEEEADETETAWPPLPAVPCDYDHCRHLQVPCKELQRVGPAACLCPG
LSSPAQPPDPPRMGEVRIAAEEGRAVVHWCAPFSPVLHYWLLLWDGSEAAQKGPPLNATVRRAELKGLKPGGIYV
VCVVAANEAGASRVPQAGGEGLEGADIPAFGPCSRLAVPPNPRTLVHAAVGVGTALALLSCAALVWHFCLRDRWG
CPRRAAARAAGAL

FIGURE 7

CGCCAAGCATGCAGTAAAGGCTGAAAATCTGGGTCACAGCTGAGGAAGACCTCAGAC**ATG**GAGTCCAGGATGTGG
CCTGCGCTGCTGCTGTCCCACCTCCTCCCTCTCTGGCCACTGCTGTTGCTGCCCCTCCCACCGCCTGCTCAGGGC
TCTTCATCCTCCCCTCGAACCCCACCAGCCCCAGCCCGCCCCCCGTGTGCCAGGGGAGGCCCCTCGGCCCCACGT
CATGTGTGCGTGTGGGAGCGAGCACCTCCACCAAGCCGATCTCCTCGGGTCCCAAGATCACGTCGGCAAGTCCTG
CCTGGCACTGCACCCCCAGCCACCCCATCAGGCTTTGAGGAGGGGCCGCCCTCATCCCAATACCCCTGGCTATC
GTGTGGGGTCCCACCGTGTCTCGAGAGGATGGAGGGGACCCCAACTCTGCCAATCCCGGATTTCTGGACTATGGT
TTTGCAGCCCCTCATGGGCTCGCAACCCCACACCCCAACTCAGACTCCATGCGAGGTGATGGAGATGGGCTTATC
CTTGGAGAGGCACCTGCCACCCTGCGGCCATTCCTGTTCGGGGGCCGTGGGGAAGGTGTGGACCCCCAGCTCTAT
GTCACAATTACCATCTCCATCATCATTGTTCTCGTGGCCACTGGCATCATCTTCAAGTTCTGCTGGGACCGCAGC
CAGAAGCGACGCAGACCCTCAGGGCAGCAAGGTGCCCTGAGGCAGGAGGAGAGCCAGCAGCCACTGACAGACCTG
TCCCCGGCTGGAGTCACTGTGCTGGGGGCCTTCGGGGACTCACCTACCCCCACCCCTGACCATGAGGAGCCCCGA
GGGGGACCCCGGCCTGGGATGCCCCACCCCAAGGGGGCTCCAGCCTTCCAGTTGAACCGG**TGA**GGGCAGGGGCAA
TGGGATGGGAGGGCAAAGAGGGAAGGCAACTTAGGTCTTCAGAGCTGGGGTGGGGGTGCCCTCTGGATGGGTAGT
GAGGAGGCAGGCGTGGCCTCCCACAGCCCCTGGCCCTCCCAAGGGGGCTGGACCAGCTCCTCTCTGGGAGGCACC
CTTCCTTCTCCCAGTCTCTCAGGATCTGTGTCCTATTCTCTGCTGCCCATAACTCCAACTCTGCCCTCTTTGGTT
TTTTCTCATGCCACCTTGTCTAAGACAACTCTGCCCTCTTAACCTTGATTCCCCCTCTTTGTCTTGAACTTCCCC
TTCTATTCTGGCCTACCCCTTGGTTCCTGACTGTGCCCTTTCCCTCTTCCTCTCAGGATTCCCCTGGTGAATCTG
TGATGCCCCCAATGTTGGGGTGCAGCCAAGCAGGAGGCCAAGGGGCCGGCACAGCCCCCATCCCACTGAGGGTGG
GGCAGCTGTGGGGAGCTGGGGCCACAGGGGCTCCTGGCTCCTGCCCCTTGCACACCACCCGGAACACTCCCCAGC
CCCACGGGCAATCCTATCTGCTCGCCCTCCTGCAGGTGGGGGCCTCACATATCTGTGACTTCGGGTCCCTGTCCC
CACCCTTGTGCACTCACATGAAAGCCTTGCACACTCACCTCCACCTTCACAGGCCATTTGCACACGCTCCTGCAC
CCTCTCCCCGTCCATACCGCTCCGCTCAGCTGACTCTCATGTTCTCTCGTCTCACATTTGCACTCTCTCCTTCCC
ACATTCTGTGCTCAGCTCACTCAGTGGTCAGCGTTTCCTGCACACTTTACCTCTCATGTGCGTTTCCCGGCCTGA
TGTTGTGGTGGTGTGCGGCGTGCTCACTCTCTCCCTCATGAACACCCACCCACCTCGTTTCCGCAGCCCCTGCGT
GCTGCTCCAGAGGTGGGTGGGAGGTGAGCTGGGGGCTCCTTGGGCCCTCATCGGTCATGGTCTCGTCCCATTCCA
CACCATTTGTTTCTCTGTCTCCCCATCCTACTCCAAGGATGCCGGCATCACCCTGAGGGCTCCCCCTTGGGAATG
GGGTAGTGAGGCCCCAGACTTCACCCCCAGCCCACTGCTAAAATCTGTTTTCTGACAGATGGGTTTTGGGGAGTC
GCCTGCTGCACTACATGAGAAAGGGACTCCCATTTGCCCTTCCCTTTCTCCTACAGTCCCTTTTGTCTTGTCTGT
CCTGGCTGTCTGTGTGTGTGCCATTCTCTGGACTTCAGAGCCCCCTGAGCCAGTCCTCCCTTCCCAGCCTCCCTT
TGGGCCTCCCTAACTCCACCTAGGCTGCCAGGGACCGGAGTCAGCTGGTTCAAGGCCATCGGGAGCTCTGCCTCC
AAGTCTACCCTTCCCTTCCCGGACTCCCTCCTGTCCCCTCCTTTCCTCCCTCCTTCCTTCCACTCTCCTTCCTTT
TGCTTCCCTGCCCTTTCCCCCTCCTCAGGTTCTTCCCTCCTTCTCACTGGTTTTTCCACCTTCCTCCTTCCCTTC
TTCCCTGGCTCCTAGGCTGTGATATATATTTTTGTATTATCTCTTTCTTCTTCTTGTGGTGATCATCTTGAATTA
CTGTGGGATGTAAGTTTCAAAATTTTCAAATAAAGCCTTTGCAAGATAA

## FIGURE 8

| | |
|---|---|
| Signal peptide: | Amino acids 1-33 |
| Transmembrane domain: | Amino acids 178-198 |
| cAMP- and cGMP-dependent protein kinase phosphorylation site: | Amino acids 210-214 |
| N-myristoylation sites: | Amino acids 117-123; 154-160; 214-220 |
| Cell attachment sequence: | Amino acids 149-152 |

MESRMWPALLLSHLLPLWPLLLLPLPPPAQGSSSSPRTPPAPARPPCARGGPSAPRHVCVWERAPPPSRSPRVPR
SRRQVLPGTAPPATPSGFEEGPPSSQYPWAIVWGPTVSREDGGDPNSANPGFLDYGFAAPHGLATPHPNSDSMRG
DGDGLILGEAPATLRPFLFGGRGEGVDPQLYVTITISIIIVLVATGIIFKFCWDRSQKRRRPSGQQGALRQEESQ
QPLTDLSPAGVTVLGAFGDSPTPTPDHEEPRGGPRPGMPHPKGAPAFQLNR

## FIGURE 9

AGGGTCCCTTAGCCGGGCGCAGGGCGCGCAGCCCAGGCTGAGATCCGCGGCTTCCGTAGAAGTGAGC**ATG**GCTGG
GCAGCGAGTGCTTCTTCTAGTGGGCTTCCTTCTCCCTGGGGTCCTGCTCTCAGAGGCTGCCAAAATCCTGACAAT
ATCTACAGTAGGTGGAAGCCATTATCTACTGATGGACCGGGTTTCTCAGATTCTTCAAGATCACGGTCATAATGT
CACCATGCTTAACCACAAAAGAGGTCCTTTTATGCCAGATTTTAAAAAGGAAGAAAAATCATATCAAGTTATCAG
TTGGCTTGCACCTGAAGATCATCAAAGAGAATTTAAAAAGAGTTTTGATTTCTTTCTGGAAGAAACTTTAGGTGG
CAGAGGAAAATTTGAAAACTTATTAAATGTTCTAGAATACTTGGCGTTGCAGTGCAGTCATTTTTTAAATAGAAA
GGATATCATGGATTCCTTAAAGAATGAGAACTTCGACATGGTGATAGTTGAAACTTTTGACTACTGTCCTTTCCT
GATTGCTGAGAAGCTTGGGAAGCCATTTGTGGCCATTCTTTCCACTTCATTCGGCTCTTTGGAATTTGGGCTACC
AATCCCCTTGTCTTATGTTCCAGTATTCCGTTCCTTGCTGACTGATCACATGGACTTCTGGGGCCGAGTGAAGAA
TTTTCTGATGTTCTTTAGTTTCTGCAGGAGGCAACAGCACATGCAGTCTACATTTGACAACACCATCAAGGAACA
TTTCACAGAAGGCTCTAGGCCAGTTTTGTCTCATCTTCTACTGAAAGCAGAGTTGTGGTTCATTAACTCTGACTT
TGCCTTTGATTTTGCTCGACCTCTGCTTCCCAACACTGTTTATGTTGGAGGCTTGATGGAAAAACCTATTAAACC
AGTACCACAAGACTTGGAGAACTTCATTGCCAAGTTTGGGGACTCTGGTTTTGTCCTTGTGACCTTGGGCTCCAT
GGTGAACACCTGTCAGAATCCGGAAATCTTCAAGGAGATGAACAATGCCTTTGCTCACCTACCCCAAGGGGTGAT
ATGGAAGTGTCAGTGTTCTCATTGGCCCAAAGATGTCCACCTGGCTGCAAATGTGAAAATTGTGGACTGGCTTCC
TCAGAGTGACCTCCTGGCTCACCCAAGCATCCGTCTGTTTGTCACCCACGGCGGGCAGAATAGCATAATGGAGGC
CATCCAGCATGGTGTGCCCATGGTGGGGATCCCTCTCTTTGGAGACCAGCCTGAAAACATGGTCCGAGTAGAAGC
CAAAAAGTTTGGTGTTTCTATTCAGTTAAAGAAGCTCAAGGCAGAGACATTGGCTCTTAAGATGAAACAAATCAT
GGAAGACAAGAGATACAAGTCCGCGGCAGTGGCTGCCAGTGTCATCCTGCGCTCCCACCCGCTCAGCCCCACACA
GCGGCTGGTGGGCTGGATTGACCACGTCCTCCAGACAGGGGGCGCGACGCACCTCAAGCCCTATGTCTTTCAGCA
GCCCTGGCATGAGCAGTACCTGTTCGACGTTTTTGTGTTTCTGCTGGGGCTCACTCTGGGGACTCTATGGCTTTG
TGGGAAGCTGCTGGGCATGGCTGTCTGGTGGCTGCGTGGGGCCAGAAAGGTGAAGGAGACA**TAA**GGCCAGGTGCA
GCCTTGGCGGGGTCTGTTTGGTGGGCGATGTCACCATTTCTAGGGAGCTTCCCACTAGTTCTGGCAGCCCCATTC
TCTAGTCCTTCTAGTTATCTCCTGTTTTCTTGAAGAACAGGAAAAATGGCCAAAAATCATCCTTTCCACTTGCTA
ATTTTGCTACAAATTCATCCTTACTAGCTCCTGCCTGCTAGCAGAAATCTTTCCAGTCCTCTTGTCCTCCTTTGT
TTGCCATCAGCAAGGGCTATGCTGTGATTCTGTCTCTGAGTGACTTGGACCACTGACCCTCAGATTTCCAGCCTT
AAAATCCACCTTCCTTCTCATGCGCCTCTCCGAATCACACCCTGACTCTTCCAGCCTCCATGTCCAGACCTAGTC
AGCCTCTCTCACTCCTGCCCCTACTATCTATCATGGAATAACATCCAAGAAAGACACCTTGCATATTCTTTCAGT
TTCTGTTTTGTTCTCCCACATATTCTCTTCAATGCTCAGGAAGCCTGCCCTGTGCTTGAGAGTTCAGGGCCGGAC
ACAGGCTCACAGGTCTCCACATTGGGTCCCTGTCTCTGGTGCCCACAGTGAGCTCCTTCTTGGCTGAGCAGGCAT
GGAGACTGTAGGTTTCCAGATTTCCTGAAAAATAAAAGTTTACAGCGTTATCTCTCCCCAACCTCACTAA

## FIGURE 10

Signal peptide: Amino acids 1-19

Transmembrane domain: Amino acids 483-504

N-glycosylation site: Amino acids 52-56

Tyrosine kinase phosphorylation sites: Amino acids 68-75; 425-434

N-myristoylation sites: Amino acids 16-22; 301-307; 370-376; 494-500

Leucine zipper pattern: Amino acids 493-515

UDP-glucoronosyl site: Amino acids 241-294

MAGQRVLLLVGFLLPGVLLSEAAKILTISTVGGSHYLLMDRVSQILQDHGHNVTMLNHKRGPFMPDFKKEEKSYQ
VISWLAPEDHQREFKKSFDFFLEETLGGRGKFENLLNVLEYLALQCSHFLNRKDIMDSLKNENFDMVIVETFDYC
PFLIAEKLGKPFVAILSTSFGSLEFGLPIPLSYVPVFRSLLTDHMDFWGRVKNFLMFFSFCRRQQHMQSTFDNTI
KEHFTEGSRPVLSHLLLKAELWFINSDFAFDFARPLLPNTVYVGGLMEKPIKPVPQDLENFIAKFGDSGFVLVTL
GSMVNTCQNPEIFKEMNNAFAHLPQGVIWKCQCSHWPKDVHLAANVKIVDWLPQSDLLAHPSIRLFVTHGGQNSI
MEAIQHGVPMVGIPLFGDQPENMVRVEAKKFGVSIQLKKLKAETLALKMKQIMEDKRYKSAAVAASVILRSHPLS
PTQRLVGWIDHVLQTGGATHLKPYVFQQPWHEQYLFDVFVFLLGLTLGTLWLCGKLLGMAVWWLRGARKVKET

# FIGURE 11

CGCGGCCGGGCCGCCGGGGTGAGCGTGCCGAGGCGGCTGTGGCGCAGGCTTCCAGCCCCCACC**ATG**CCGTGGCCC
CTGCTGCTGCTGCTGGCCGTGAGTGGGGCCCAGACAACCCGGCCATGCTTCCCCGGGTGCCAATGCGAGGTGGAG
ACCTTCGGCCTTTTCGACAGCTTCAGCCTGACTCGGGTGGATTGTAGCGGCCTGGGCCCCCACATCATGCCGGTG
CCCATCCCTCTGGACACAGCCCACTTGGACCTGTCCTCCAACCGGCTGGAGATGGTGAATGAGTCGGTGTTGGCGGGG
CCGGGCTACACGACGTTGGCTGGCCTGGATCTCAGCCACAACCTGCTCACCAGCATCTCACCCACTGCCTTCTCC
CGCCTTCGCTACCTGGAGTCGCTTGACCTCAGCCACAATGGCCTGACAGCCCTGCCAGCCGAGAGCTTCACCAGC
TCACCCCTGAGCGACGTGAACCTTAGCCACAACCAGCTCCGGGAGGTCTCAGTGTCTGCCTTCACGACGCACAGT
CAGGGCCGGGCACTACACGTGGACCTCTCCCACAACCTCATTCACCGCCTCGTGCCCCACCCCACGAGGGCCGGC
CTGCCTGCGCCCACCATTCAGAGCCTGAACCTGGCCTGGAACCGGCTCCATGCCGTGCCCAACCTCCGAGACTTG
CCCCTGCGCTACCTGAGCCTGGATGGGAACCCTCTAGCTGTCATTGGTCCGGGTGCCTTCGCGGGGCTGGAGGC
CTTACACACCTGTCTCTGGCCAGCCTGCAGAGGCTCCCTGAGCTGGCGCCCAGTGGCTTCCGTGAGCTACCGGGC
CTGCAGGTCCTGGACCTGTCGGGCAACCCCAAGCTTAACTGGGCAGGAGCTGAGGTGTTTTCAGGCCTGAGCTCC
CTGCAGGAGCTGGACCTTTCGGGCACCAACCTGGTGCCCCTGCCTGAGGCGCTGCTCCTCCACCTCCCGGCACTG
CAGAGCGTCAGCGTGGGCCAGGATGTGCGGTGCCGGCGCCTGGTGCGGGAGGGCACCTACCCCCGGAGGCCTGGC
TCCAGCCCCAAGGTGCCCCTGCACTGCGTAGACACCCGGGAATCTGCTGCCAGGGGCCCCACCATCTTG**TGA**CAA
ATGGTGTGGCCCAGGGCCACATAACAGACTGCTGTCCTGGGCTGCCTCAGGTCCCGAGTAACTTATGTTCAATGT
GCCAACACCAGTGGGGAGCCCGCAGGCCTATGTGGCAGCGTCACCACAGGAGTTGTGGGCCTAGGAGAGGCTTTG
GACCTGGGAGCCACACCTAGGAGCAAAGTCTCACCCCTTTGTCTACGTTGCTTCCCCAAACCATGAGCAGAGGGA
CTTCGATGCCAAACCAGACTCGGGTCCCCTCCTGCTTCCCTTCCCCACTTATCCCCCAAGTGCCTTCCCTCATGC
CTGGGCCGGCCTGACCCGCAATGGGCAGAGGGTGGGTGGGACCCCCTGCTGCAGGGCAGAGTTCAGGTCCACTGG
GCTGAGTGTCCCCTTGGGCCCATGGCCCAGTCACTCAGGGGCGAGTTTCTTTTCTAACATAGCCCTTTCTTTGCC
ATGAGGCCATGAGGCCCGCTTCATCCTTTTCTATTTCCCTAGAACCTTAATGGTAGAAGGAATTGCAAAGAATCA
AGTCCACCCTTCTCATGTGACAGATGGGGAAACTGAGGCCTTGAGAAGGAAAAAGGCTAATCTAAGTTCCTGCGG
GCAGTGGCATGACTGGAGCACAGCCTCCTGCCTCCCAGCCCGGACCCAATGCACTTTCTTGTCTCCTCTAATAAG
CCCCACCCTCCCCGCCTGGGCTCCCCTTGCTGCCCTTGCCTGTTCCCCATTAGCACAGGAGTAGCAGCAGCAGGA
CAGGCAAGAGCCTCACAAGTGGGACTCTGGGCCTCTGACCAGCTGTGCGGCATGGGCTAAGTCACTCTGCCCTTC
GGAGCCTCTGGAAGCTTAGGGCACATTGGTTCCAGCCTAGCCAGTTTCTCACCCTGGGTTGGGGTCCCCCAGCAT
CCAGACTGGAAACCTACCCATTTTCCCCTGAGCATCCTCTAGATGCTGCCCCAAGGAGTTGCTGCAGTTCTGGAG
CCTCATCTGGCTGGGATCTCCAAGGGGCCTCCTGGATTCAGTCCCCACTGGCCCTGAGCACGACAGCCCTTCTTA
CCCTCCCAGGAATGCCGTGAAAGGAGACAAGGTCTGCCCGACCCATGTCTATGCTCTACCCCCAGGGCAGCATCT
CAGCTTCCGAACCCTGGGCTGTTTCCTTAGTCTTCATTTTATAAAAGTTGTTGCCTTTTTAACGGAGTGTCACTT
TCAACCGGCCTCCCCTACCCCTGCTGGCCGGGGATGGAGACATGTCATTTGTAAAAGCAGAAAAAGGTTGCATTT
GTTCACTTTTGTAATATTGTCCTGGGCCTGTGTTGGGGTGTTGGGGGAAGCTGGGCATCAGTGGCCACATGGGCA
TCAGGGGCTGGCCCCACAGAGACCCCACAGGGCAGTGAGCTCTGTCTTCCCCCACCTGCCTAGCCCATCATCTAT
CTAACCGGTCCTTGATTTAATAAACACTATAAAAGGTTTAAAAAAAAAAAAAAAAAAAAAAAAAAAAAAAAAAAA
AAAAAAAAAAA

## FIGURE 12

| | |
|---|---|
| Signal peptide: | Amino acids 1-16 |
| Transmembrane domains: | Amino acids 215-232; 287-304 |
| N-glycosylation sites: | Amino acids 74-78; 137-141 |
| Glycosaminoglycan attachment site: | Amino acids 45-49 |
| Tyrosine kinase phosphorylation site: | Amino acids 318-326 |
| N-myristoylation sites: | Amino acids 13-19; 32-38; 88-94; 214-220; 223-229 |
| Leucine zipper pattern: | Amino acids 284-306 |

MPWPLLLLLAVSGAQTTRPCFPGCQCEVETFGLFDSFSLTRVDCSGLGPHIMPVPIPLDTAHLDLSSNRLEMVNE
SVLAGPGYTTLAGLDLSHNLLTSISPTAFSRLRYLESLDLSHNGLTALPAESFTSSPLSDVNLSHNQLREVSVSA
FTTHSQGRALHVDLSHNLIHRLVPHPTRAGLPAPTIQSLNLAWNRLHAVPNLRDLPLRYLSLDGNPLAVIGPGAF
AGLGGLTHLSLASLQRLPELAPSGFRELPGLQVLDLSGNPKLNWAGAEVFSGLSSLQELDLSGTNLVPLPEALLL
HLPALQSVSVGQDVRCRRLVREGTYPRRPGSSPKVPLHCVDTRESAARGPTIL

FIGURE 13

CAGGACCAGGTCTTCCTACGCTGGAGCAGCGGGGAGACAGCCACC**ATG**CACATCCTCGTGGTCCATGCCATGGTG
ATCCTGCTGACGCTGGGCCCGCCTCGAGCCGACGACAGCGAGTTCCAGGCGCTGCTGGACATCTGGTTTCCGGAG
GAGAAGCCACTGCCCACCGCCTTCCTGGTGGACACATCGGAGGAGGCGCTGCTGCTTCCTGACTGGCTGAAGCTG
CGCATGATCCGTTCTGAGGTGCTCCGCCTGGTGGACGCCGCCCTGCAGGACCTGGAGCCGCAGCAGCTGCTGCTG
TTCGTGCAGTCGTTTGGCATCCCCGTGTCCAGCATGAGCAAACTCCTCCAGTTCCTGGACCAGGCAGTGGCCCAC
GACCCCCAGACTCTGGAGCAGAACATCATGGACAAGAATTACATGGCCCACCTGGTGGAGGTCCAGCATGAGCGC
GGCGCCTCCGGAGGCCAGACTTTCCACTCCTTGCTCACAGCCTCCCTGCCGCCCCGCCGAGACAGCACAGAGGCA
CCCAAACCAAAGAGCAGCCCAGAGCAGCCCATAGGCCAGGGCCGGATTCGGGTGGGGACCCAGCTCCGGGTGCTG
GGCCCTGAGGACGACCTGGCTGGCATGTTCCTCCAGATTTTCCCGCTCAGCCCGGACCCTCGGTGGCAGAGCTCC
AGTCCCCGCCCCGTGGCCCTCGCCCTGCAGCAGGCCCTGGGCCAGGAGCTGGCCCGCGTCGTCCAGGGCAGCCCC
GAGGTGCCGGGCATCACGGTGCGTGTCCTGCAGGCCCTCGCCACCCTGCTCAGCTCCCCACACGGCGGTGCCCTG
GTGATGTCCATGCACCGTAGCCACTTCCTGGCCTGCCCGCTGCTGCGCCAGCTCTGCCAGTACCAGCGCTGTGTG
CCACAGGACACCGGCTTCTCCTCGCTCTTCCTGAAGGTGCTCCTGCAGATGCTGCAGTGGCTGGACAGCCCTGGC
GTGGAGGGCGGGCCCCTGCGGGCACAGCTCAGGATGCTTGCCAGCCAGGCCTCAGCCGGGCGCAGGCTCAGTGAT
GTGCGAGGGGGGCTCCTGCGCCTGGCCGAGGCCCTGGCCTTCCGTCAGGACCTGGAGGTGGTCAGCTCCACCGTC
CGTGCCGTCATCGCCACCCTGAGGTCTGGGGAGCAGTGCAGCGTGGAGCCGGACCTGATCAGCAAAGTCCTCCAG
GGGCTGATCGAGGTGAGGTCCCCCCACCTGGAGGAGCTGCTGACTGCATTCTTCTCTGCCACTGCGGATGCTGCC
TCCCCGTTTCCAGCCTGTAAGCCCGTTGTGGTGGTGAGCTCCCTGCTGCTGCAGGAGGAGGAGCCCCTGGCTGGG
GGGAAGCCGGGTGCGGACGGTGGCAGCCTGGAGGCCGTGCGGCTGGGGCCCTCGTCAGGCCTCCTAGTGGACTGG
CTGGAAATGCTGGACCCCGAGGTGGTCAGCAGCTGCCCCGACCTGCAGCTCAGGCTGCTCTTCTCCCGGAGGAAG
GGCAAAGGTCAGGCCCAGGTGCCCTCGTTCCGTCCCTACCTCCTGACCCTCTTCACGCATCAGTCCAGCTGGCCC
ACACTGCACCAGTGCATCCGAGTCCTGCTGGGCAAGAGCCGGGAACAGAGGTTCGACCCCTCTGCCTCTCTGGAC
TTCCTCTGGGCCTGCATCCATGTTCCTCGCATCTGGCAGGGGCGGGACCAGCGCACCCCGCAGAAGCGGCGGGAG
GAGCTGGTGCTGCGGGTCCAGGGCCCGGAGCTCATCAGCCTGGTGGAGCTGATCCTGGCCGAGGCGGAGACGCGG
AGCCAGGACGGGGACACAGCCGCCTGCAGCCTCATCCAGGCCCGGCTGCCCCTGCTGCTCAGCTGCTGCTGTGGG
GACGATGAGAGTGTCAGGAAGGTGACGGAGCACCTGTCAGGCTGCATCCAGCAGTGGGGAGACAGCGTGCTGGGA
AGGCGCTGCCGAGACCTTCTCCTGCAGCTCTACCTACAGCGGCCGGAGCTGCGGGTGCCCGTGCCTGAGGTCCTA
CTGCACAGCGAAGGGGCTGCCAGCAGCAGCGTCTGCAAGCTGGACGGACTCATCCACCGCTTCATCACGCTCCTT
GCGGACACCAGCGACTCCCGGGCGTTGGAGAACCGAGGGGCGGATGCCAGCATGGCCTGCCGGAAGCTGGCGGTG
GCGCACCCGCTGCTGCTGCTCAGGCACCTGCCCATGATCGCGGCGCTCCTGCACGGCCGCACCCACCTCAACTTC
CAGGAGTTCCGGCAGCAGAACCACCTGAGCTGCTTCCTGCACGTGCTGGGCCTGCTGGAGCTGCTGCAGCCGCAC
GTGTTCCGCAGCGAGCACCAGGGGGCGCTGTGGGACTGCCTTCTGTCCTTCATCCGCCTGCTGCTGAATTACAGG
AAGTCCTCCCGCCATCTGGCTGCCTTCATCAACAAGTTTGTGCAGTTCATCCATAAGTACATTACCTACAATGCC
CCAGCAGCCATCTCCTTCCTGCAGAAGCACGCCGACCCGCTCCACGACCTGTCCTTCGACAACAGTGACCTGGTG
ATGCTGAAATCCCTCCTTGCAGGGCTCAGCCTGCCCAGCAGGGACGACAGGACCGACCGAGGCCTGGACGAAGAG
GGCGAGGAGGAGAGCTCAGCCGGCTCCTTGCCCCTGGTCAGCGTCTCCCTGTTCACCCCTCTGACCGCGGCCGAG
ATGGCCCCCTACATGAAACGGCTTTCCCGGGGCCAAACGGTGGAGGATCTGCTGGAGGTTCTGAGTGACATAGAC
GAGATGTCCCGGCGGAGACCCGAGATCCTGAGCTTCTTCTCGACCAACCTGCAGCGGCTGATGAGCTCGGCCGAG
GAGTGTTGCCGCAACCTCGCCTTCAGCCTGGCCCTGCGCTCCATGCAGAACAGCCCCAGCATTGCAGCCGCTTTC
CTGCCCACGTTCATGTACTGCCTGGGCAGCCAGGACTTTGAGGTGGTGCAGACGGCCCTCCGGAACCTGCCTGAG
TACGCTCTCCTGTGCCAAGAGCACGCGGCTGTGCTGCTCCACCGGGCCTTCCTGGTGGGCATGTACGGCCAGATG
GACCCCAGCGCGCAGATCTCCGAGGCCCTGAGGATCCTGCATATGGAGGCCGTGATG**TGA**GCCTGTGGCAGCCGA
CCCCCCTCCAAGCCCCGGCCCGTCCCGTCCCCGGGATCCTCGAGGCAAAGCCCAGGAAGCGTGGGCGTTGCTGG
TCTGTCCGAGGAGGTGAGGGCGCCGAGCCCTGAGGCCAGGCAGGCCCAGGAGCAATACTCCGAGCCCTGGGGTGG
CTCCGGGCCGGCCGCTGGCATCAGGGGCCGTCCAGCAAGCCCTCATTCACCTTCTGGGCCACAGCCCTGCCGCGG
AGCGGCGGATCCCCCCGGGCATGGCCTGGGCTGGTTTTGAATGAAACGACCTGAACTGTCAA

## FIGURE 14

Signal peptide: Amino acids 1-16

cAMP- and cGMP-dependent protein kinase phosphorylation sites: Amino acids 154-158; 331-335; 616-620; 785-789; 891-895

N-myristoylation sites: Amino acids 91-97; 136-142; 224-230; 435-441; 439-445, 443-449; 665-671; 698-704

Amidation sites: Amino acids 329-333; 634-638

Oligoadenylate synthetase site: Amino acids 96-135

MHILVVHAMVILLTLGPPRADDSEFQALLDIWFPEEKPLPTAFLVDTSEEALLLPDWLKLRMIRSEVLRLVDAAL
QDLEPQQLLLFVQSFGIPVSSMSKLLQFLDQAVAHDPQTLEQNIMDKNYMAHLVEVQHERGASGGQTFHSLLTAS
LPPRRDSTEAPKPKSSPEQPIGQGRIRVGTQLRVLGPEDDLAGMFLQIFPLSPDPRWQSSSPRPVALALQQALGQ
ELARVVQGSPEVPGITVRVLQALATLLSSPHGGALVMSMHRSHFLACPLLRQLCQYQRCVPQDTGFSSLFLKVLL
QMLQWLDSPGVEGGPLRAQLRMLASQASAGRRLSDVRGGLLRLAEALAFRQDLEVVSSTVRAVIATLRSGEQCSV
EPDLISKVLQGLIEVRSPHLEELLTAFFSATADAASPFPACKPVVVVSSLLLQEEEPLAGGKPGADGGSLEAVRL
GPSSGLLVDWLEMLDPEVVSSCPDLQLRLLFSRRKGKGQAQVPSFRPYLLTLFTHQSSWPTLHQCIRVLLGKSRE
QRFDPSASLDFLWACIHVPRIWQGRDQRTPQKRREELVLRVQGPELISLVELILAEAETRSQDGDTAACSLIQAR
LPLLLSCCCGDDESVRKVTEHLSGCIQQWGDSVLGRRCRDLLLQLYLQRPELRVPVPEVLLHSEGAASSSVCKLD
GLIHRFITLLADTSDSRALENRGADASMACRKLAVAHPLLLLRHLPMIAALLHGRTHLNFQEFRQQNHLSCFLHV
LGLLELLQPHVFRSEHQGALWDCLLSFIRLLLNYRKSSRHLAAFINKFVQFIHKYITYNAPAAISFLQKHADPLH
DLSFDNSDLVMLKSLLAGLSLPSRDDRTDRGLDEEGEEESSAGSLPLVSVSLFTPLTAAEMAPYMKRLSRGQTVE
DLLEVLSDIDEMSRRRPEILSFFSTNLQRLMSSAEECCRNLAFSLALRSMQNSPSIAAAFLPTFMYCLGSQDFEV
VQTALRNLPEYALLCQEHAAVLLHRAFLVGMYGQMDPSAQISEALRILHMEAVM

FIGURE 15

CCGGGCCATGCAGCCTCGGCCCCGCGGGCGCCCGCCGCGCACCCGAGGAG**ATG**AGGCTCCGCAATGGCACCTTCC
TGACGCTGCTGCTCTTCTGCCTGTGCGCCTTCCTCTCGCTGTCCTGGTACGCGGCACTCAGCGGCCAGAAAGGCG
ACGTTGTGGACGTTTACCAGCGGGAGTTCCTGGCGCTGCGCGATCGGTTGCACGCAGCTGAGCAGGAGAGCCTCA
AGCGCTCCAAGGAGCTCAACCTGGTGCTGGACGAGATCAAGAGGGCCGTGTCAGAAAGGCAGGCGCTGCGAGACG
GAGACGGCAATCGCACCTGGGGCCGCCTAACAGAGGACCCCCGATTGAAGCCGTGGAACGGCTCACACCGGCACG
TGCTGCACCTGCCCACCGTCTTCCATCACCTGCCACACCTGCTGGCCAAGGAGAGCAGTCTGCAGCCCGCGGTGC
GCGTGGGCCAGGGCCGCACCGGAGTGTCGGTGGTGATGGGCATCCCGAGCGTGCGGCGCGAGGTGCACTCGTACC
TGACTGACACTCTGCACTCGCTCATCTCCGAGCTGAGCCCGCAGGAGAAGGAGGACTCGGTCATCGTGGTGCTGA
TCGCCGAGACTGACTCACAGTACACTTCGGCAGTGACAGAGAACATCAAGGCCTTGTTCCCCACGGAGATCCATT
CTGGGCTCCTGGAGGTCATCTCACCCTCCCCCCACTTCTACCCTGACTTCTCCCGCCTCCGAGAGTCCTTTGGGG
ACCCCAAGGAGAGAGTCAGGTGGAGGACCAAACAGAACCTCGATTACTGCTTCCTCATGATGTACGCGCAGTCCA
AAGGCATCTACTACGTGCAGCTGGAGGATGACATCGTGGCCAAGCCCAACTACCTGAGCACCATGAAGAACTTTG
CACTGCAGCAGCCTTCAGAGGACTGGATGATCCTGGAGTTCTCCCAGCTGGGCTTCATTGGTAAGATGTTCAAGT
CGCTGGACCTGAGCCTGATTGTAGAGTTCATTCTCATGTTCTACCGGGACAAGCCCATCGACTGGCTCCTGGACC
ATATTCTGTGGGTGAAAGTCTGCAACCCCGAGAAGGATGCGAAGCACTGTGACCGGCAGAAAGCCAACCTGCGGA
TCCGCTTCAAACCGTCCCTCTTCCAGCACGTGGGCACTCACTCCTCGCTGGCTGGCAAGATCCAGAAACTGAAGG
ACAAAGACTTTGGAAAGCAGGCGCTGCGGAAGGAGCATGTGAACCCGCCAGCAGAGGTGAGCACGAGCCTGAAGA
CATACCAGCACTTCACCCTGGAGAAAGCCTACCTGCGCGAGGACTTCTTCTGGGCCTTCACCCCTGCCGCGGGGG
ACTTCATCCGCTTCCGCTTCTTCCAACCTCTAAGACTGGAGCGGTTCTTCTTCCGCAGTGGGAACATCGAGCACC
CGGAGGACAAGCTCTTCAACACGTCTGTGGAGGTGCTGCCCTTCGACAACCCTCAGTCAGACAAGGAGGCCCTGC
AGGAGGGCCGCACCGCCACCCTCCGGTACCCTCGGAGCCCCGACGGCTACCTCCAGATCGGCTCCTTCTACAAGG
GAGTGGCAGAGGGAGAGGTGGACCCAGCCTTCGGCCCTCTGGAAGCACTGCGCCTCTCGATCCAGACGGACTCCC
CTGTGTGGGTGATTCTGAGCGAGATCTTCCTGAAAAAGGCCGAC**TAA**GCTGCGGGCTTCTGAGGGTACCCTGTGG
CCAGCCCTGAAGCCCACATTTCTGGGGGTGTCGTCACTGCCGTCCCCGGAGGGCCAGATACGGCCCCGCCCAAAG
GGTTCTGCCTGGCGTCGGGCTTGGGCCGGCCTGGGGTCCGCCGCTGGCCCGGAGGCCCTAGGAGCTGGTGCTGCC
CCCGCCCGCCGGGCCGCGGAGGAGGCAGGCGGCCCCCACACTGTGCCTGAGGCCCGGAACCGTTCGCACCCGGCC
TGCCCCAGTCAGGCCGTTTTAGAAGAGCTTTTACTTGGGCGCCCGCCGTCTCTGGCGCGAACACTGGAATGCATA
TACTACTTTATGTGCTGTGTTTTTTATTCTTGGATACATTTGATTTTTTCACGTAAGTCCACATATACTTCTATA
AGAGCGTGACTTGTAATAAAGGGTTAATGAAGAAAAAAAAAAAAAAAAAAAAAAAAAAAAAAAAAAAAAAAAAAA
AAAAAAAAAAA

## FIGURE 16

| | |
|---|---|
| Signal peptide: | Amino acids 1-23 |
| N-glycosylation sites: | Amino acids 5-9; 87-91; 103-107; 465-469 |
| N-myristoylation sites: | Amino acids 6-12; 136-142; 370-376; 509-515 |

MRLRNGTFLTLLLFCLCAFLSLSWYAALSGQKGDVVDVYQREFLALRDRLHAAEQESLKRSKELNLVLDEIKRAV
SERQALRDGDGNRTWGRLTEDPRLKPWNGSHRHVLHLPTVFHHLPHLLAKESSLQPAVRVGQGRTGVSVVMGIPS
VRREVHSYLTDTLHSLISELSPQEKEDSVIVVLIAETDSQYTSAVTENIKALFPTEIHSGLLEVISPSPHFYPDF
SRLRESFGDPKERVRWRTKQNLDYCFLMMYAQSKGIYYVQLEDDIVAKPNYLSTMKNFALQQPSEDWMILEFSQL
GFIGKMFKSLDLSLIVEFILMFYRDKPIDWLLDHILWVKVCNPEKDAKHCDRQKANLRIRFKPSLFQHVGTHSSL
AGKIQKLKDKDFGKQALRKEHVNPPAEVSTSLKTYQHFTLEKAYLREDFFWAFTPAAGDFIRFRFFQPLRLERFF
FRSGNIEHPEDKLFNTSVEVLPFDNPQSDKEALQEGRTATLRYPRSPDGYLQIGSFYKGVAEGEVDPAFGPLEAL
RLSIQTDSPVWVILSEIFLKKAD

## FIGURE 17

GCACCGCAGACGGCGCGGATCGCAGGGAGCCGGTCCGCCGCCGGAACGGGAGCCTGGGTGTGCGTGTGGAGTCCG
GACTCGTGGGAGACGATCGCG**ATG**AACACGGTGCTGTCGCGGGCGAACTCACTGTTCGCCTTCTCGCTGAGCGTG
ATGGCGGCGCTCACCTTCGGCTGCTTCATCACCACCGCCTTCAAAGACAGGAGCGTCCCGGTGCGGCTGCACGTC
TCGCGGATCATGCTAAAAAATGTAGAAGATTTCACTGGACCTAGAGAAAGAAGTGATCTGGGATTTATCACATTT
GATATAACTGCTGATCTAGAGAATATATTTGATTGGAATGTTAAGCAGTTGTTTCTTTATTTATCAGCAGAATAT
TCAACAAAAAATAATGCTCTGAACCAAGTTGTCCTATGGGACAAGATTGTTTTGAGAGGTGATAATCCGAAGCTG
CTGCTGAAAGATATGAAAACAAAATATTTTTTCTTTGACGATGGAAATGGTCTCAAGGGAAACAGGAATGTCACT
TTGACCCTGTCTTGGAACGTCGTACCAAATGCTGGAATTCTACCTCTTGTGACAGGATCAGGACACGTATCTGTC
CCATTTCCAGATACATATGAAATAACGAAGAGTTAT**TAA**ATTATTCTGAATTTGAAACAAAAA

## FIGURE 18

| | |
|---|---|
| Signal peptide: | Amino acids 1-25 |
| Transmembrane domain: | Amino acids 149-164 |
| N-glycosylation site: | Amino acids 141-145 |
| N-myristoylation sites: | Amino acids 25-31; 135-141 |
| Prokaryotic membrane lipoprotein lipid attachment site: | Amino acids 16-27 |
| Cell attachment sequence: | Amino acids 112-115 |
| TonB-dependent receptor protein signature 1: | Amino acids 1-21 |

MNTVLSRANSLFAFSLSVMAALTFGCFITTAFKDRSVPVRLHVSRIMLKNVEDFTGPRERSDLGFITFDITADLE
NIFDWNVKQLFLYLSAEYSTKNNALNQVVLWDKIVLRGDNPKLLLKDMKTKYFFFDDGNGLKGNRNVTLTLSWNV
VPNAGILPLVTGSGHVSVPFPDTYEITKSY

## FIGURE 19

GACCGGTCCCTCCGGTCCTGGATGTGCGGACTCTGCTGCAGCGAGGGCTGCAGGCCCGCCGGGCGGTGCTCACCG
TGCCCTGGCTGGTGGAGTTTCTCTCCTTTGCTGACCATGTTGTTCCCTTGCTGGAATATTACCGGGACATCTTCA
CTCTCCTGCTGCGCCTGCACCGGAGCTTGGTGTTGTCGCAGGAGAGTGAGGGGAAG**ATG**TGTTTCCTGAACAAGC
TGCTGCTACTTGCTGTCCTGGGCTGGCTTTTCCAGATTCCCACAGTCCCTGAGGACTTGTTCTTTCTGGAAGAGG
GTCCCTCATATGCCTTTGAGGTGGACACAGTAGCCCCAGAGCATGGCTTGGACAATGCGCCTGTGGTGGACCAGC
AGCTGCTCTACACCTGCTGCCCCTACATCGGAGAGCTCCGGAAACTGCTCGCTTCGTGGGTGTCAGGCAGTAGTG
GACGGAGTGGGGGCTTCATGAGGAAAATCACCCCCACCACTACCACCAGCCTGGGAGCCCAGCCTTCCCAGACCA
GCCAGGGGCTGCAGGCACAGCTCGCCCAGGCCTTTTTCCACAACCAGCCGCCCTCCTTGCGCCGGACCGTAGAGT
TCGTGGCAGAAAGAATTGGATCAAACTGTGTCAAACATATCAAGGCTACACTGGTGGCAGATCTGGTGCGCCAGG
CAGAGTCACTTCTCCAAGAGCAGCTGGTGACACAGGGAGAGGAAGGGGGAGACCCAGCCCAGCTGTTGGAGATCT
TGTGTTCCCAGCTGTGCCCTCACGGGGCCCAGGCATTGGCCCTGGGGCGGGAGTTCTGTCAAAGGAAGAGCCCTG
GGGCTGTGCGGGCGCTGCTTCCAGAGGAGACCCCGGCAGCCGTTCTGAGCAGTGCAGAGAACATTGCTGTGGGGC
TTGCAACAGAGAAAGCCTGTGCTTGGCTGTCAGCCAACATCACAGCACTGATCAGGAGGGAGGTGAAAGCAGCAG
TGAGTCGCACACTTCGAGCCCAGGGTCCTGAACCTGCTGCCCGGGGGGAGCGGAGGGGCTGCTCCCGCGCC**TGA**C
GTGCTCTCCTTGGCCGTGGGGCCACGGACCCTGACGAGGGAGTCTCCCCAGAGCATCTGGAACAGCTCCTAGGC
CAGCTGGGCCAGACGCTGCGGTGCCGCCAGTTCCTGTGCCCACCTGCTGAGCAGCATCTGGCAAAGTGCTCTGTG
GAGTTAGCTTCCCTCCTCGTTGCAGATCAAATTCCTATCCTAGGGCCCCCGGCACAGTACAGGCTGGAGAGAGGG
CAGGCTCGAAGGCTTCTGCACATGCTGCTTTCCTTGTGGAAGGAAGACTTTCAGGGGCCGGTTCCGCTGCAGCTG
CTGCTGAGCCCAAGAAATGTGGGGCTTCTGGCAGACACAAGGCCAAGGGAGTGGGACTTGCTGCTATTCTTGCTA
CGGGAGCTGGTGGAGAAGGGTCTGATGGGACGGATGGAGATAGAGGCCTGCCTGGGCAGCCTCCACCAGGCCCAG
TGGCCAGGGGACTTTGCTGAAGAATTAGCAACACTGTCTAATCTGTTTCTAGCCGAGCCCCACCTGCCAGAACCC
CAGCTAAGAGCCTGTGAGTTGGTGCAGCCAAACCGGGGCACTGTGCTGGCCCAGAGCTAGGGCTGAGAAGTGGCC
CTGCCTTGGGCATTGCACCAGAACCCTGGACCCCCGCCTCACGAGGAGGCCCAAGTGCCCAATGCAGACCCTCAC
TGGTTGGGGTGTAGCTGGGTCTACAGTCAGACTTCCTGCTCTAAGGGTGTCACTGCCTGGCATCCCACCACGCGA
ATCCTAGAGGAAGGAGAGTTGGCCTGATTTGGGATTATGGCAGAAAAGTCCAGAGATGCCAGTCCTGGAGTAGAA
GAGGTGGTGTTTGTTTATCTCTTGGATACTAAATGAAATGAGGTGTGTGGGCTTGTCAACACAGAATTCAAGCCT
CATTTGCTATCCCAGCATCTCTTAAAACTTTGTAGTCTTGGAATTCATGACAGAGGCAAATGACTCCTGCTTAAC
TTATGAAGAAAGTTAAAACATGAATCTTGGGAGTCTACATTTTCTTATCACCAGGAGCTGGACTGCCATCTCCTT
ATAAATGCCTAACACAGGCCGGGTCTGGTGGCTCATGCCTGTAATCCCAGCACTTTGAGAGGCCTGAGGTCGGCGG
ACTGCCTGAGGTCAGGAATTCAAGACCAGCCTGGCCAACATGGCAAAACCCCATCTCTACTAAAAATAAAAAAAT
TATTAGCTGGGCATGGTGGTGTGTGCCTGTAATCCCAGCTACTCAGGAGGATGAGGCAGGAGACCTGCTTGAACC
TGGAGGTGGAGGTTGCAGTGAGCCGAGGTCGCACCACTGCACTCCAGTCTGGGTAACAGAGCGAGACTTTCTAGA
AAAAGCCTAACAAACAGATAAGGTAGGACTCAACCAACTGAAACCTGACTTTCCCCCTGTACCTTCAGCCCCTGT
GCAGGTAGTAACCTCTTGAGACCTCTCCCTGACCAGGGACCAAGCACAGGGCATTTAGAGCTTTTAGAATAAAC
TGGTTTTCTTTAAAAAAAAAAAAAAAAAAAGGGCGGCCGCCCTTTTTTTTTTTTTTTTTTTTTTTTTTTTT
TTTTTTTTTTTTTTAAAAAGGGCTTTTATTAAAATTCTCCCCACACGATGGCTCCTGCAATCTGCCACAGCTCT
GGGGCGTGTCCTGTAGGGAAAGGCCCTGTTTTCCCTGAGGCGGGGCTGGGCTTGTCCATGGGTCCGCGGAGCTGG
CCGTGCTTGGCGCCCTGGCGTGTGTCTAGCTGCTTCTTGCCGGGCACAGAGCTGCGGGGTCTGGGGGCACCGGGA
GCTAAGAGCAGGCTCTGGTGCAGGGGTGGAGGCCTGTCTCTTAACCGACACCCTGAGGTGCTCCTGAGATGCTGG
GTCCACCCTGAGTGGCACGGGGAGCAGCTGTGGCCGGTGCTCCTTCYTAGGCCAGTCCTGGGGAAACTAAGCTCG
GGCCCTTCTTTGCAAAGACCGAGGATGGGGTGGGTGTGGGGGACTCATGGGGAATGGCCTGAGGAGCTACGTGTG
AAGAGGGCGCCGGTTTGTTGGCTGCAGCGGCCTGGAGCGCCTCTCTCCTGAGCCTCAGTTTCCCTTTCCGTCTAA
TGAAGAACATGCCGTCTCGGTGTCTCAGGGCTATTAGGACTTGCCCTCAGGAAGTGGCCTTGGACGAGCGTCATG
TTATTTTCACAACTGTCCTGCGACGTTGGCCTGGGCACGTCATGGAATGGCCCATGTCCCTCTGCTGCGTGGACG
TCGCGGTCGGGAGTGCGCAGCCAGAGGCGGGGCCAGACGTGCGCCTGGGGGTGAGGGGAGGCGCCCCGGGAGGGC
CTCACAGGAAGTTGGGCTCCCGCACCACCAGGCAGGGCGGGCTCCCGCCGCCGCCGCCGCCACCACCGTCCAGGG
GCCGGTAGACAAAGTGGAAGTCGCGCTTGGGCTCGCTGCGCAGCAGGTAGCCCTTGATGCAGTGCGGCAGCGCGT
CGTCCGCCAGCTGGAAGCAGCGCCCGTCCACCAGCACGAACAGCCGGTGCGCCT

## FIGURE 20

Signal peptide: Amino acids 1-18

N-glycosylation site: Amino acids 244-248

Microbodies C-terminal targeting signal: Amino acids 278-282

MCFLNKLLLLAVLGWLFQIPTVPEDLFFLEEGPSYAFEVDTVAPEHGLDNAPVVDQQLLYTCCPYIGELRKLLAS
WVSGSSGRSGGFMRKITPTTTTSLGAQPSQTSQGLQAQLAQAFFHNQPPSLRRTVEFVAERIGSNCVKHIKATLV
ADLVRQAESLLQEQLVTQGEEGGDPAQLLEILCSQLCPHGAQALALGREFCQRKSPGAVRALLPEETPAAVLSSA
ENIAVGLATEKACAWLSANITALIRREVKAAVSRTLRAQGPEPAARGERRGCSRA

FIGURE 21

GCCGAGTGGGACAAAGCCTGGGGCTGGGCGGGGGCC**ATG**GCGCTGCCATCCCGAATCCTGCTTTGGAAACTTGTG
CTTCTGCAGAGCTCTGCTGTTCTCCTGCACTCAGCGGTGGAGGAGACGGACGCGGGGCTGTACACCTGCAACCTGCAC
CATCACTACTGCCACCTCTACGAGAGCCTGGCCGTCCGCCTGGAGGTCACCGACGGCCCCCCGGCCACCCCCGCC
TACTGGGACGGCGAGAAGGAGGTGCTGGCGGTGGCGCGCGGCGCACCCGCGCTTCTGACCTGCGTGAACCGCGGG
CACGTGTGGACCGACCGGCACGTGGAGGAGGCTCAACAGGTGGTGCACTGGGACCGGCAGCCGCCCGGGGTCCCG
CACGACCGCGCGGACCGCCTGCTGGACCTCTACGCGTCGGGCGAGCGCCGCGCCTACGGGCCCCTTTTTCTGCGC
GACCGCGTGGCTGTGGGCGCGGATGCCTTTGAGCGCGGTGACTTCTCACTGCGTATCGAGCCGCTGGAGGTCGCC
GACGAGGGCACCTACTCCTGCCACCTGCACCACCATTACTGTGGCCTGCACGAACGCCGCGTCTTCCACCTGACG
GTCGCCGAACCCCACGCGGAGCCGCCCCCCCGGGGCTCTCCGGGCAACGGCTCCAGCCACAGCGGCGCCCCAGGC
CCAGACCCCACACTGGCGCGCGGCCACAACGTCATCAATGTCATCGTCCCCGAGAGCCGAGCCCACTTCTTCCAGCAG
CTGGGCTACGTGCTGGCCACGCTGCTGCTCTTCATCCTGCTACTGGTCACTGTCCTCCTGGCCGCCCGCAGGCGC
CGCGGAGGCTACGAATACTCGGACCAGAAGTCGGGAAAGTCAAAGGGGAAGGATGTTAACTTGGCGGAGTTCGCT
GTGGCTGCAGGGGACCAGATGCTTTACAGGAGTGAGGACATCCAGCTAGATTACAAAAACAACATCCTGAAGGAG
AGGGCGGAGCTGGCCCACAGCCCCCTGCCTGCCAAGTACATCGACCTAGACAAAGGGTTCCGGAAGGAGAACTGC
AAA**TAG**GGAGGCCCTGGGCTCCTGGCTGGGCCAGCAGCTGCACCTCTCCTGTCTGTGCTCCTCGGGGCATCTCCT
GATGCTCCGGGGCTCACCCCCCTTCCAGCGGCTGGTCCCGCTTTCCTGGAATTTGGCCTGGGCGTATGCAGAGGC
CGCCTCCACACCCCTCCCCCAGGGGCTTGGTGGCAGCATAGCCCCCACCCCTGCGGCCTTTGCTCACGGGTGGCC
CTGCCCACCCCTGGCACAACCAAAATCCCACTGATGCCCATCATGCCCTCAGACCCTTCTGGGCTCTGCCCGCTG
GGGGCCTGAAGACATTCCTGGAGGACACTCCCATCAGAACCTGGCAGCCCCAAAACTGGGGTCAGCCTCAGGGCA
GGAGTCCCACTCCTCCAGGGCTCTGCTCGTCCGGGGCTGGGAGATGTTCCTGGAGGAGGACACTCCCATCAGAAC
TTGGCAGCCTTGAAGTTGGGGTCAGCCTCGGCAGGAGTCCCACTCCTCCTGGGGTGCTGCCTGCCACCAAGAGCT
CCCCCACCTGTACCACCATGTGGGACTCCAGGCACCATCTGTTCTCCCCAGGGACCTGCTGACTTGAATGCCAGC
CCTTGCTCCTCTGTGTTGCTTTGGCCACCTGGGGCTGCACCCCCTGCCCTTTCTCTGCCCCATCCCTACCCTAG
CCTTGCTCTCAGCCACCTTGATAGTCACTGGGCTCCCTGTGACTTCTGACCCTGACACCCCTCCCTTGGACTCTG
CCTGGGCTGGAGTCTAGGGCTGGGGCTACATTTGGCTTCTGTACTGGCTGAGGACAGGGGAGGGAGTGAAGTTGG
TTTGGGGTGGCCTGTGTTGCCACTCTCAGCACCCCACATTTGCATCTGCTGGTGGACCTGCCACCATCACAATAA
AGTCCCCATCTGATTTTTAAAAAAAAAAAAAAAA

## FIGURE 22

Signal peptide: Amino acids 1-19

Transmembrane domain: Amino acids 237-262

N-glycosylation site: Amino acids 205-209

Cell attachment sequence: Amino acids 151-154

Coproporphyrinogen III oxidase proteins:
Amino acids 115-141

MALPSRILLWKLVLLQSSAVLLHSAVEETDAGLYTCNLHHHYCHLYESLAVRLEVTDGPPATPAYWDGEKEVLAV
ARGAPALLTCVNRGHVWTDRHVEEAQQVVHWDRQPPGVPHDRADRLLDLYASGERRAYGPLFLRDRVAVGADAFE
RGDFSLRIEPLEVADEGTYSCHLHHHYCGLHERRVFHLTVAEPHAEPPPRGSPGNGSSHSGAPGPDPTLARGHNV
INVIVPESRAHFFQQLGYVLATLLLFILLLVTVLLAARRRRGGYEYSDQKSGKSKGKDVNLAEFAVAAGDQMLYR
SEDIQLDYKNNILKERAELAHSPLPAKYIDLDKGFRKENCK

FIGURE 23

CAAGCAGGTCATCCCCTTGGTGACCTTCAAAGAGAAGCAGAGAGGGCAGAGGTGGGGGCACAGGGAAAGGGTGA
CCTCTGAGATTCCCCTTTTCCCCCAGACTTTGGAAGTGACCCACC**ATG**GGGCTCAGCATCTTTTTGCTCCTGTGT
GTTCTTGGGCTCAGCCAGGCAGCCACACCGAAGATTTTCAATGGCACTGAGTGTGGGCGTAACTCACAGCCGTGG
CAGGTGGGGCTGTTTGAGGGCACCAGCCTGCGCTGCGGGGGTGTCCTTATTGACCACAGGTGGGTCCTCACAGCG
GCTCACTGCAGCGGCAGCAGGTACTGGGTGCGCCTGGGGGAACACAGCCTCAGCCAGCTCGACTGGACCGAGCAG
ATCCGGCACAGCGGCTTCTCTGTGACCCATCCCGGCTACCTGGGAGCCTCGACGAGCCACGAGCACGACCTCCGG
CTGCTGCGGCTGCGCCTGCCCGTCCGCGTAACCAGCAGCGTTCAACCCCTGCCCCTGCCCAATGACTGTGCAACC
GCTGGCACCGAGTGCCACGTCTCAGGCTGGGGCATCACCAACCACCCACGGAACCCATTCCCGGATCTGCTCCAG
TGCCTCAACCTCTCCATCGTCTCCCATGCCACCTGCCATGGTGTGTATCCCGGGAGAATCACGAGCAACATGGTG
TGTGCAGGCGGCGTCCCGGGGCAGGATGCCTGCCAGGGTGATTCTGGGGGCCCCCTGGTGTGTGGGGGAGTCCTT
CAAGGTCTGGTGTCCTGGGGGTCTGTGGGGCCCTGTGGACAAGATGGCATCCCTGGAGTCTACACCTATATTTGC
AAGTATGTGGACTGGATCCGGATGATCATGAGGAACAAC**TGA**CCTGTTTCCTCCACCTCCACCCCCACCCCTTAA
CTTGGGTACCCCTCTGGCCCTCAGAGCACCAATATCTCCTCCATCACTTCCCCTAGCTCCACTCTTGTTGGCCTG
GGAACTTCTTGGAACTTTAACTCCTGCCAGCCCTTCTAAGACCCACGAGCGGGGTGAGAGAAGTGTGCAATAGTC
TGGAATAAATATAAATGAAGGAGGGGCAAAAAAAAAAAAAA

## FIGURE 24

Signal peptide: Amino acids 1-17

N-glycosylation sites: Amino acids 24-28; 163-167

Serine proteases, trypsin family, histidine active site:
Amino acids 58-64

Serine proteases, trypsin family, histidine protein domains:
Amino acids 47-64; 196-207; 218-242

Kringle domain protein sites: Amino acids 47-65; 194-207

Apple domain protein site: Amino acids 220-248

MGLSIFLLLCVLGLSQAATPKIFNGTECGRNSQPWQVGLFEGTSLRCGGVLIDHRWVLTAAHCSGSRYWVRLGEH
SLSQLDWTEQIRHSGFSVTHPGYLGASTSHEHDLRLLRLRLPVRVTSSVQPLPLPNDCATAGTECHVSGWGITNH
PRNPFPDLLQCLNLSIVSHATCHGVYPGRITSNMVCAGGVPGQDACQGDSGGPLVCGGVLQGLVSWGSVGPCGQD
GIPGVYTYICKYVDWIRMIMRNN

FIGURE 25

AGCAGCCTCTGCCCGACCCGGCTCGTGCGGACCCCAGGACCGGGCGCGGACGCGTGCGTCCAGCCTCCGGCGCT
GCGGAGACCCGCGGCTGGGTCCGGGGAGGCCCCAAACCCGCCCCCGCCAGAACCCCGCCCCAAATTCCCACCTCC
TCCAGAAGCCCCGCCCACTCCCGAGCCCCGAGAGCTCCGCGCACCTGGGCGCCATCCGCCCTGGCTCCGCTGCAC
GAGCTCCACGCCCGTACCCCGGCGTCACGCTCAGCCCGCGGTGCTCGCACACCTGAGACTCATCTCGCTTCGACC
CCGCCGCCGCCGCCGCCCGGCATCCTGAGCACGGAGACAGTCTCCAGCTGCCGTTC**ATG**CTTCCTCCCCAGCCTT
CCGCAGCCCACCAGGGAAGGGGCGGTAGGAGTGGCCTTTTACCAAAGGACCGGCGATGCTCTGCAGGCTGTGCT
GGCTGGTCTCGTACAGCTTGGCTGTGCTGTTGCTCGGCTGCCTGCTCTTCCTGAGGAAGGCGGCCAAGCCCGCAG
GAGACCCCACGGCCCACCAGCCTTTCTGGGCTCCCCCAACACCCCGTCACAGCCGGTGTCCACCCAACCACACAG
TGTCTAGCGCCTCTCTGTCCCTGCCTAGCCGTCACCGTCTCTTCTTGACCTATCGTCACTGCCGAAATTTCTCTA
TCTTGCTGGAGCCTTCAGGCTGTTCCAAGGATACCTTCTTGCTCCTGGCCATCAAGTCACAGCCTGGTCACGTGG
AGCGACGTGCGGCTATCCGCAGCACGTGGGGCAGGGTGGGGGGATGGGCTAGGGGCCGGCAGCTGAAGCTGGTGT
TCCTCCTAGGGGTGGCAGGATCCGCTCCCCCAGCCCAGCTGCTGGCCTATGAGAGTAGGGAGTTTGATGACATCC
TCCAGTGGGACTTCACTGAGGACTTCTTCAACCTGACGCTCAAGGAGCTGCACCTGCAGCGCTGGGTGGTGGCTG
CCTGCCCCCAGGCCCATTTCATGCTAAAGGGAGATGACGATGTCTTTGTCCACGTCCCCAACGTGTTAGAGTTCC
TGGATGGCTGGGACCCAGCCCAGGACCTCCTGGTGGGAGATGTCATCCGCCAAGCCCTGCCCAACAGGAACACTA
AGGTCAAATACTTCATCCCACCCTCAATGTACAGGGCCACCCACTACCCACCCTATGCTGGTGGGGGAGGATATG
TCATGTCCAGAGCCACAGTGCGGCGCCTCCAGGCTATCATGGAAGATGCTGAACTCTTCCCCATTGATGATGTCT
TTGTGGGTATGTGCCTGAGGAGGCTGGGGCTGAGCCCTATGCACCATGCTGGCTTCAAGACATTTGGAATCCGGC
GGCCCCTGGACCCCTTAGACCCCTGCCTGTATAGGGGGCTCCTGCTGGTTCACCGCCTCAGCCCCCTCGAGATGT
GGACCATGTGGGCACTGGTGACAGATGAGGGGCTCAAGTGTGCAGCTGGCCCCATACCCCAGCGC**TGA**AGGGTGG
GTTGGGCAACAGCCTGAGAGTGGACTCAGTGTTGATTCTCTATCGTGATGCGAAATTGATGCCTGCTGCTCTACA
GAAAATGCCAACTTGGTTTTTTAACTCCTCTCACCCTGTTAGCTCTGATTAAAAACACTGCAACCCAA

## FIGURE 26

Signal peptide: Amino acids 1-39

Type II transmembrane domain: Amino acids 30-49

N-glycosylation sites: Amino acids 79-83; 104-108; 192-196

Casein kinase II phosphorylation sites: Amino acids 194-198; 352-356

N-myristoylation sites: Amino acids 14-20; 160-166; 367-373

Prokaryotic membrane lipoprotein lipid attachment site:
Amino acids 35-46

MLPPQPSAAHQGRGGRSGLLPKGPAMLCRLCWLVSYSLAVLLLGCLLFLRKAAKPAGDPTAHQPFWAPPTPRHSR
CPPNHTVSSASLSLPSRHRLFLTYRHCRNFSILLEPSGCSKDTFLLLAIKSQPGHVERRAAIRSTWGRVGGWARG
RQLKLVFLLGVAGSAPPAQLLAYESREFDDILQWDFTEDFFNLTLKELHLQRWVVAACPQAHFMLKGDDDVFVHV
PNVLEFLDGWDPAQDLLVGDVIRQALPNRNTKVKYFIPPSMYRATHYPPYAGGGGYVMSRATVRRLQAIMEDAEL
FPIDDVFVGMCLRRLGLSPMHHAGFKTFGIRRPLDPLDPCLYRGLLLVHRLSPLEMWTMWALVTDEGLKCAAGPIPQR

## FIGURE 27

CTCGGCTGGATTTAAGGTTGCCGCTAGCCGCCTGGGAATTTAAGGGACCCACACTACCTTCCCGAAGTTGAAGGC
AAGCGGTGATTGTTTGTAGACGGCGCTTTGTC**ATG**GGACCTGTGCGGTTGGGAATATTGCTTTTCCTTTTTTTGG
CCGTGCACGAGGCTTGGGCTGGGATGTTGAAGGAGGAGGACGATGACACAGAACGCTTGCCCAGCAAATGCGAAG
TGTGTAAGCTGCTGAGCACAGAGCTACAGGCGGAACTGAGTCGCACCGGTCGATCTCGAGAGGTGCTGGAGCTGG
GGCAGGTGCTGGATACAGGCAAGAGGAAGAGACACGTGCCTTACAGCGTTTCAGAGACAAGGCTGGAAGAGGCCT
TAGAGAATTTATGTGAGCGGATCCTGGACTATAGTGTTCACGCTGAGCGCAAGGGCTCACTGAGATATGCCAAGG
GTCAGAGTCAGACCATGGCAACACTGAAAGGCCTAGTGCAGAAGGGGGTGAAGGTGGATCTGGGGATCCCTCTGG
AGCTTTGGGATGAGCCCAGCGTGGAGGTCACATACCTCAAGAAGCAGTGTGAGACCATGTTGGAGGAGTTTGAAG
ACATTGTGGGAGACTGGTACTTCCACCATCAGGAGCAGCCCCTACAAAATTTTCTCTGTGAAGGTCATGTGCTCC
CAGCTGCTGAAACTGCATGTCTACAGGAAACTTGGACTGGAAAGGAGATCACAGATGGGGAAGAGAAAACAGAAG
GGGAGGAAGAGCAGGAGGAGGAGGAGGAAGAGGAGGAAGAGGAAGGGGGAGACAAGATGACCAAGACAGGAAGCC
ACCCCAAACTTGACCGAGAAGATCTT**TGA**CCCTTGCCTTTGAGCCCCCAGGAGGGGAAGGGATCATGGAGAGCCC
TCTAAAGCCTGCACTCTCCCTGCTCCACAGCTTTCAGGGTGTGTTTATGAGTGACTCCACCCAAGCTTGTAGCTG
TTCTCTCCCATCTAACCTCAGGCAAGATCCTGGTGAAACAGCATGACATGGCTTCTGGGGTGGAGGGTGGGGGTG
GAGGTCCTGCTCCTAGAGATGAACTCTATCCAGCCCCTTAATTGGCAGGTGTATGTGCTGACAGTACTGAAAGCT
TTCCTCTTTAACTGATCCCACCCCCACCCAAAAGTCAGCAGTGGCACTGGAGCTGTGGGCTTTGGGGAAGTCACT
TAGCTCCTTAAGGTCTGTTTTTAGACCCTTCCAAGGAAGAGGCCAGAACGGACATTCTCTGCGATCTATATACAT
TGCCTGTATCCAGGAGGCTACACACCAGCAAACCGTGAAGGAGAATGGGACACTGGGTCATGGCCTGGAGTTGCT
GATAATTTAGGTGGGATAGATACTTGGTCTACTTAAGCTCAATGTAACCCAGAGCCCACCATATAGTTTTATAGG
TGCTCAACTTTCTATATCGCTATTAAACTTTTTCTTTTTTTCTA

## FIGURE 28

| | |
|---|---|
| Signal peptide: | Amino acids 1-21 |
| cAMP- and cGMP-dependent protein kinase phosphorylation site: | Amino acids 106-110 |
| Casein kinase II phosphorylation sites: | Amino acids 36-40; 80-84; 84-88; 158-162; 202-206; 207-211; 213-217 |
| N-myristoylation site: | Amino acids 115-121 |
| Amidation site: | Amino acids 70-74 |

MGPVRLGILLFLFLAVHEAWAGMLKEEDDDTERLPSKCEVCKLLSTELQAELSRTGRSREVLELGQVLDTGKRKR
HVPYSVSETRLEEALENLCERILDYSVHAERKGSLRYAKGQSQTMATLKGLVQKGVKVDLGIPLELWDEPSVEVT
YLKKQCETMLEEFEDIVGDWYFHHQEQPLQNFLCEGHVLPAAETACLQETWTGKEITDGEEKTEGEEEQEEEEEE
EEEEGGDKMTKTGSHPKLDREDL

FIGURE 29

CGGACGCGTGGGCGCTGAGCCCCGGAGGCCAGGGCGTCCGGGGCTGCGCCACTTCCGAGGGCCGAGCGCTGCCGG
TCCCGGCGGTGCGACACGGCCGGGAGGAGGAGAACAACGCAAGGGGCTCAACCGTCGGTCGCTGGAGCCCCCCC
GGGGCGTGGCCTCCCGCCCCCTCAGCTGGGGAGGGCGGGGCTCGCTGCCCCCTGCTGCCGACTGCGACCCTTACA
GGGGAGGGAGGGCGCAGGCCGCGCGGAG**ATG**AGGAGGAGGCTGCGCCTACGCAGGGACGCATTGCTCACGCTGCT
CCTTGGCGCCTCCCTGGGCCTCTTACTCTATGCGCAGCGCGACGGCGCGGCCCCGACGGCGAGCGCGCCGCGAGG
GCGAGGGAGGGCGGCACCGAGGCCCACCCCCGGACCCCGCGCGTTCCAGTTACCCGACGCGGGTGCAGCCCCGCC
GGCCTACGAAGGGGACACACCGGCGCCGCCCACGCCTACGGGACCCTTTGACTTCGCCCGCTATTTGCGCGCCAA
GGACCAGCGGCGGTTTCCACTGCTCATTAACCAGCCGCACAAGTGCCGCGGCGACGGCGCACCCGGTGGCCGCCC
GGACCTGCTTATTGCTGTCAAGTCGGTGGCAGAGGACTTCGAGCGGCGCCAAGCCGTGCGCCAGACGTGGGGCGC
GGAGGGTCGCGTGCAGGGGGCGCTGGTGCGCCGCGTGTTCTTGCTGGGCGTGCCCAGGGGCGCAGGCTCGGGCGG
GGCCGACGAAGTTGGGGAGGGCGCGCGAACCCACTGGCGCGCCCTGCTGCGGGCCGAGAGCCTTGCGTATGCGGA
CATCCTGCTCTGGGCCTTCGACGACACCTTTTTTAACCTAACGCTCAAGGAGATCCACTTTCTAGCCTGGGCCTC
AGCTTTCTGCCCCGACGTGCGCTTCGTTTTTAAGGGCGACGCAGATGTGTTCGTGAACGTGGGAAATCTCCTGGA
GTTCCTGGCGCCGCGGGACCCGGCGCAAGACCTGCTTGCTGGTGACGTAATTGTGCATGCGCGGCCCATCCGCAC
GCGGGCTAGCAAGTACTACATCCCCGAGGCCGTGTACGGCCTGCCCGCCTATCCGGCCTACGCGGGCGGCGGTGG
CTTTGTGCTTTCCGGGGCCACGCTGCACCGCCTGGCTGGCGCCTGTGCGCAGGTCGAGCTCTTCCCCATCGACGA
CGTCTTTCTGGGCATGTGTCTGCAGCGCCTGCGGCTCACGCCCGAGCCTCACCCTGCCTTCCGCACCTTTGGCAT
CCCCCAGCCTTCAGCCGCGCCGCATTTGAGCACCTTCGACCCCTGCTTTTACCGTGAGCTGGTTGTAGTGCACGG
GCTCTCGGCCGCTGACATCTGGCTTATGTGGCGCCTGCTGCACGGGCCGCATGGGCCAGCCTGTGCGCATCCACA
GCCTGTCGCTGCAGGCCCCTTCCAATGGGACTCC**TAG**CTCCCCACTACAGCCCCAAGCTCCTAACTCAGACCCAG
AATGGAGCCGGTTTCCCAGATTATTGCCGTGTATGTGGTTCTTCCCTGATCACCAGGTGCCTGTCTCCACAGGATCCC
AGGGGATGGGGGTTAAGCTTGGCTCCTGGCGGTCCACCCTGCTGGAACCAGTTGAAACCCGTGTAATGGTGACCC
TTTGAGCGAGCCAAGGCTGGGTGGTAGATGACCATCTCTTGTCCAACAGGTCCCAGAGCAGTGGATATGTCTGGT
CCTCCTAGTAGCACAGAGGTGTGTTCTGGTGTGGTGGCAGGGACTTAGGGAATCCTACCACTCTGCTGGATTTGG
AACCCCCTAGGCTGACGCGGACGTATGCAGAGGCTCTCAAGGCCAGGCCCCACAGGGAGGTGGAGGGGCTCCGGC
CGCCACAGCCTGAATTCATGAACCTGGCAGGCACTTTGCCATAGCTCATCTGAAAACAGATATTATGCTTCCCAC
AACCTCTCCTGGGCCCAGGTGTGGCTGAGCACCAGGGATGGAGCCACACATAAGGGACAAATGAGTGCACGGTCC
TACCTAGTCTTTCCTCACCTCCTGAACTCACACAACAATGCCAGTCTCCCACTGGAGGCTGTATCCCCTCAGAGG
AGCCAAGGAATGTCTTCCCCTGAGATGCCACCACTATTAATTTCCCCATATGCTTCAACCACCCCCTTGCTCAAA
AAACCAATACCCACACTTACCTTAATACAAACATCCCAGCAACAGCACATGGCAGGCCATTGCTGAGGGCACAGG
TGCTTTATTGGAGAGGGGATGTGGGCAGGGGATAAGGAAGGTTCCCCCATTCCAGGAGGATGGGAACAGTCCTGG
CTGCCCCTGACAGTGGGGATATGCAAGGGGCTCTGGCCAGGCCACAGTCCAAATGGGAAGACACCAGTCAGTCAC
AAAAGTCGGGAGCGCCACACAAACCTGGCTATAAGGCCCAGGAACCATATAGGAGCCTGAGACAGGTCCCCTGCA
CATTCATCATTAAACTATACAGGATGAGGCTGTACATGAGTTAATTACAAAAGAGTCATATTTACAAAAATCTGT
ACACACATTTGAAAAACTCACAAAATTGTCATCTATGTATCACAAGTTGCTAGACCCAAAATATTAAAAATGGGA
TAAAATTNNTTAAAAAAAAAAAAAAAAAAAAAAAAAAAAAAAAAAAAAAAAA

## FIGURE 30

| | |
|---|---|
| Signal peptide: | Amino acids 1-27 |
| N-glycosylation site: | Amino acids 203-207 |
| Casein kinase II phosphorylation sites: | Amino acids 124-128; 205-209; 351-355; 368-372 |
| N-myristoylation sites: | Amino acids 18-24; 31-37; 110-116; 157-163; 161-167; 163-169; 366-372 |
| Cell attachment sequence: | Amino acids 107-110 |

MRRRLRLRRDALLTLLLGASLGLLLYAQRDGAAPTASAPRGRGRAAPRPTPGPRAFQLPDAGAAPPAYEGDTPAP
PTPTGPFDFARYLRAKDQRRFPLLINQPHKCRGDGAPGGRPDLLIAVKSVAEDFERRQAVRQTWGAEGRVQGALV
RRVFLLGVPRGAGSGGADEVGEGARTHWRALLRAESLAYADILLWAFDDTFFNLTLKEIHFLAWASAFCPDVRFV
FKGDADVFVNVGNLLEFLAPRDPAQDLLAGDVIVHARPIRTRASKYYIPEAVYGLPAYPAYAGGGGFVLSGATLH
RLAGACAQVELFPIDDVFLGMCLQRLRLTPEPHPAFRTFGIPQPSAAPHLSTFDPCFYRELVVVHGLSAADIWLM
WRLLHGPHGPACAHPQPVAAGPFQWDS

## FIGURE 31

ATGAAAGTGATAATCAGGCAGCCCAAATGATTGTTAATAAGGATCAAATGAGATCGTGTATGTGGGTCCAATCAA
TTGATTCTACACAAAGGAGCCTGGGGAGGGGCC**ATG**GTGCCAATGCACTTACTGGGGAGACTGGAGAAGCCGCTT
CTCCTCCTGTGCTGCGCCTCCTTCCTACTGGGGCTGGCTTTGCTGGGCATAAAGACGGACATCACCCCCGTTGCT
TATTTCTTTCTCACATTGGGTGGCTTCTTCTTGTTTGCCTATCTCCTGGTCCGGTTTCTGGAATGGGGGCTTCGGTCC
CAGCTCCAATCAATGCAGACTGAGAGCCCAGGGCCCTCAGGCAATGCACGGGACAATGAAGCCTTTGAAGTGCCA
GTCTATGAAGAGGCCGTGGTGGGACTAGAATCCCAGTGCCGCCCCCAAGAGTTGGACCAACCACCCCCCTACAGC
ACTGTTGTGATACCCCCAGCACCTGAGGAGGAACAACCTAGCCATCCAGAGGGGTCCAGGAGAGCCAAACTGGAA
CAGAGGCGAATGGCCTCAGAGGGGTCCATGGCCCAGGAAGGAAGCCCTGGAAGAGCTCCAATCAACCTTCGGCTT
CGGGGACCACGGGCTGTGTCCACTGCTCCTGATCTGCAGAGCTTGGCGGCAGTCCCCACATTAGAGCCTCTGACT
CCACCCCCTGCCTATGATGTCTGCTTTGGTCACCCTGATGATGATAGTGTTTTTTATGAGGACAACTGGGCACCCCC
TTAAATGACTCTCCCAAGATTTCTCTTCTCTCCACACCAGACCTCGTTCATTTGACTAACATTTTCCAGCGCCTA
CTATGTGTCAGAAACAAGTGTTTCTGCCTGGACATCATAAATGGGGACTTGGACCCTGAGGAGAGTCAGGCCACG
GTAAGCCCTTCCCAGCTGAGATATGGGTGGCATAATTTGAGTCTTCTGGCAACATTTGGTGACCTACCCCATATC
CAATATTTCCAGCGTTAGATTGAGGATGAGGTAGGGAGGTGATCCAGAGAAGGCGGAGAAGGAAGAAGTAACCTCTGA
GTGGCGGCTATTGCTTCTGTTCCAGGTGCTGTTCGAGCTGTTAGAACCCTTAGGCTTGACAGCTTTGTGAGTTAT
TATTGAAAAATGAGGATTCCAAGAGTCAGAGGAGTTTGATAATGTGCACGAGGGCACACTGCTAGTAAATAACAT
TAAAATAACTGGAATGAA

## FIGURE 32

| | |
|---|---|
| Signal peptide: | Amino acids 1-25 |
| Transmembrane domain: | Amino acids 41-59 |
| Casein kinase II phosphorylation sites: | Amino acids 129-133; 173-177 |
| N-myristoylation site: | Amino acids 133-139 |

MVPMHLLGRLEKPLLLLCCASFLLGLALLGIKTDITPVAYFFLTLGGFFLFAYLLVRFLEWGLRSQLQSMQTESP
GPSGNARDNEAFEVPVYEEAVVGLESQCRPQELDQPPPYSTVVIPPAPEEEQPSHPEGSRRAKLEQRRMASEGSM
AQEGSPGRAPINLRLRGPRAVSTAPDLQSLAAVPTLEPLTPPPAYDVCFGHPDDDSVFYEDNWAPP

FIGURE 33

CGGCTCGAGCCCGCCCGGAAGTGCCCGAGGGGCCGCGATGGAGCTGGGGGAGCCGGGCGCTCGGTAGCGCGGCGG
GCAAGGCAGGCGCC**ATG**ACCCTGATTGAAGGGGTGGGTGATGAGGTGACCGTCCTTTTCTCGGTGCTTGCCTGCC
TTCTGGTGCTGGCCCTTGCCTGGGTCTCAACGCACACCGCTGAGGGCGGGGACCCACTGCCCCAGCCGTCAGGGA
CCCCAACGCCATCCCAGCCCAGCGCAGCCATGGCAGCTACCGACAGCATGAGAGGGGAGGCCCCAGGGGCAGAGACC
CCCAGCCTGAGACACAGAGGTCAAGCTGCACAGCCAGAGCCCAGCACGGGGTTCACAGCAACACCGCCAGCCCCG
GACTCCCCGCAGGAGCCCCTCGTGCTACGGCTGAAATTCCTCAATGATTCAGAGCAGGTGGCCAGGGCCTGGCCC
CACGACACCATTGGCTCCTTGAAAAGGACCCAGTTTCCCGGCCGGGAACAGCAGGTGCGACTCATCTACCAAGGG
CAGCTGCTAGGCGACGACACCCAGACCCTGGGCAGCCTTCACCTCCCTCCCAACTGCGTTCTCCACTGCCACGTG
TCCACGAGAGTCGGTCCCCCAAATCCCCCCTGCCCGCCGGGGTCCGAGCCCGGCCCCTCCGGGCTGGAAATCGGCAG
CCTGCTGCTGCCCCTGCTGCTCCTGCTGTTGCTGCTGCTCTGGTACTGCCAGATCCAGTACCGGCCCTTCTTTCC
CCTGACCGCCACTCTGGGCCTGGCCGGCTTCACCCTGCTCCTCAGTCTCCTGGCCTTTGCCATGTACCGCCCG**TA**
**G**TGCCTCCGCGGGCGCTTGGCAGCGTCGCCGGCCCCTCCGGACCTTGCTCCCCGCGCCGCGGCGGGAGCTGCTGC
CTGCCCAGGCCCGCCTCTCCGGCCTGCCTCTTCCCGCTGCCCTGGAGCCCAGCCCTGCGCCGCAGAGGACTCCCG
GGACTGGCGGAGGCCCCGCCCTGCGACCGCCGGGGCTCGGGGCCACCTCCCGGGGCTGCTGAACCTCAGCCCGCA
CTGGGAGTGGGCTCCTCGGGGTCGGGCATCTGCTGTCGCTGCCTCGGCCCCGGGCAGAGCCGGGCCGCCCCGGGG
GCCCGTCTTAGTGTTCTGCCGGAGGACCCAGCCGCCTCCAATCCCTGACAGCTCCTTGGGCTGAGTTGGGACGC
CAGGTCGGTGGGAGGCTGGTGAAGGGGAGCGGGGAGGGGCAGAGGAGTTCCCCGGAACCCGTGCAGATTAAAGTA
ACTGTGAAGTTTTAAAAAAAAAAAAAAAAAA

## FIGURE 34

| | |
|---|---|
| Signal peptide: | Amino acids 1-31 |
| Transmembrane domain: | Amino acids 11-32; 195-217 |
| N-glycosylation site: | Amino acids 111-115 |
| Casein kinase II phosphorylation sites: | Amino acids 2-6; 98-102; 191-195 |
| N-myristoylation sites: | Amino acids 146-152; 192-198 |

MTLIEGVGDEVTVLFSVLACLLVLALAWVSTHTAEGGDPLPQPSGTPTPSQPSAAMAATDSMRGEAPGAETPSLR
HRGQAAQPEPSTGFTATPPAPDSPQEPLVLRLKFLNDSEQVARAWPHDTIGSLKRTQFPGREQQVRLIYQGQLLG
DDTQTLGSLHLPPNCVLHCHVSTRVGPPNPPCPPGSEPGPSGLEIGSLLLPLLLLLLLLWYCQIQYRPFFPLTA
TLGLAGFTLLLSLLAFAMYRP

## FIGURE 35

TTTCCTCACTGACTATAAAAGAATAGAGAAGGAAGGGCTTCAGTGACCGGCTGCCTGGCTGACTTACAGCAGTCA
GACTCTGACAGGATC**ATG**GCTATGATGGAGGTCCAGGGGGGACCCAGCCTGGGACAGACCTGCGTGCTGATCGTG
ATCTTCACAGTGCTCCTGCAGTCTCTCTGTGTGGCTGTAACTTACGTGTACTTTACCAACGAGCTGAAGCAGATG
CAGGACAAGTACTCCAAAAGTGGCATTGCTTGTTTCTTAAAAGAAGATGACAGTTATTGGGACCCCAATGACGAA
GAGAGTATGAACAGCCCCTGCTGGCAAGTCAAGTGGCAACTCCGTCAGCTCGTTAGAAAGATGATTTTGAGAACC
TCTGAGGAAACCATTTCTACAGTTCAAGAAAAGCAACAAAATATTTCTCCCCTAGTGAGAGAAAGAGGTCCTCAG
AGAGTAGCAGCTCACATAACTGGGACCAGAGGAAGAAGCAACACATTGTCTTCTCCAAACTCCAAGAATGAAAAG
GCTCTGGGCCGCAAAATAAACTCCTGGGAATCATCAAGGAGTGGGCATTCATTCCTGAGCAACTTGCACTTGAGG
AATGGTGAACTGGTCATCCATGAAAAAGGGTTTTACTACATCTATTCCCAAACATACTTTCGATTTCAGGAGGAA
ATAAAAGAAAACACAAAGAACGACAAACAAATGGTCCAATATATTTACAAATACACAAGTTATCCTGACCCTATA
TTGTTGATGAAAAGTGCTAGAAATAGTTGTTGGTCTAAAGATGCAGAATATGGACTCTATTCCATCTATCAAGGG
GGAATATTTGAGCTTAAGGAAAATGACAGAATTTTTGTTTCTGTAACAAATGAGCACTTGATAGACATGGACCAT
GAAGCCAGTTTTTTCGGGGCCTTTTTAGTTGGC**TAA**CTGACCTGGAAAGAAAAAGCAATAACCTCAAAGTGACTATTC
AGTTTTCAGGATGATACACTATGAAGATGTTTCAAAAAATCTGACCAAAACAAACAAACAGAAA

## FIGURE 36

| | |
|---|---|
| Signal sequence: | Amino acids 1-32 |
| Tyrosine kinase phosphorylation site: | Amino acids 233-241 |
| Amidation site: | Amino acids 147-151 |

MAMMEVQGGPSLGQTCVLIVIFTVLLQSLCVAVTYVYFTNELKQMQDKYSKSGIACFLKEDDSYWDPNDEESMNS
PCWQVKWQLRQLVRKMILRTSEETISTVQEKQQNISPLVRERGPQRVAAHITGTRGRSNTLSSPNSKNEKALGRK
INSWESSRSGHSFLSNLHLRNGELVIHEKGFYYIYSQTYFRFQEEIKENTKNDKQMVQYIYKYTSYPDPILLMKS
ARNSCWSKDAEYGLYSIYQGGIFELKENDRIFVSVTNEHLIDMDHEASFFGAFLVG

## FIGURE 37

ATCACTTCTGAGCACGGAGCA**ATG**GCCTCTCGCTGGGCTGTGCAGCTGCTGCTCGTGGCAGCCTGGAGCATGGGC
TGTGGTGAGGCCCTCAAGTGCTACACCTGCAAGGAGCCCATGACCAGTGCTTCCTGCAGGACCATTACCCGCTGC
AAGCCAGAGGACACAGCCTGCATGACCACGCTGGTGACGGTGGAGGCAGAGTACCCCTTCAACCAGAGCCCCGTG
GTGACCCGCTCCTGCTCCAGCTCCTGTGTGGCCACCGACCCCGACAGCATCGGGGCCGCCCACCTGATCTTCTGC
TGCTTCCGAGACCTCTGCAACTCGGAACTC**TGA**ACCCAGGGCGGCAGGGCGGAAGGTGCTCCTCAGGCACCTCCT
CTCTGACGGGGCCTGGCTCCACCTGTGATCACCTCCCCCTGCTTCCTGCTGCTGTGGCACAGCTCACTCATGGGG
TCTGAGGGGAGAGAAGCACACCAGGGGCGCCCTCTGCCTTCCATACCCCACGCTTATAAAACATAACTAAGCCAA
AAAAAAAAAAAAAAA

# FIGURE 38

MASRWAVQLLLVAAWSMGCGEALKCYTCKEPMTSASCRTITRCKPEDTACMTTLVTVEAEYPFNQSPVVTRSCSS
SCVATDPDSIGAAHLIFCCFRDLCNSEL

FIGURE 39

GCGGCCGCGGAGGGAAGGGGTGGGTCGGTGGGTCTGACAGCGGGTCTGCGTAGGCGGCAGCGTCTGTCCCTCCCA
GCCTCTCGCTCCGCGCC**ATG**GGCGGGCCCCGGGCTCTGCTGGCCGCACTCTGGGCGCTGGAAGCCGCCGGGACCGCC
GCGCTTCGCATCGGAGCCTTCAACATTCAGAGCTTCGGTGACAGCAAAGTGTCGGACCCCGCTTGCGGCAGCATC
ATCGCGAAGATCCTGGCTGGCTATGACCTCGCGCTGGTGCAGGAGGTGCGAGACCCAGACCTCAGCGCCGTGTCC
GCGCTCATGGAGCAGATCAACAGCGTGTCCGAGCACGAGTACAGCTTTGTGAGCAGCCAGCCCCTGGGCCGGGAC
CAGTACAAGGAGATGTACCTGTTCGTGTACAGGAAAGACGCGGTGTCGGTCGTGGACACCTACCTGTACCCAGAC
CCCGAGGACGTCTTCAGCCGCGAGCCCTTCGTGGTCAAGTTCTCGGCCCCCGGCACCGGTGAGCGGGCCCCGCCC
CTCCCCTCCCGCCGAGCTCTGACGCCCCCACCCCTTCCCGCAGCAGCACAGAACCTGGTGCTGATCCCGCTGCAC
GCGGCGCCGCATCAAGCCGTGGCGGAGATCGACGCGCTCTACGACGTGTACCTGGACGTGATCGACAAGTGGGGC
ACCGACGACATGCTGTTCCTGGGCGACTTCAACGCCGACTGCAGCTATGTGCGGGCGCAGGACTGGGCCGCCATC
CGTCTGAGGAGCAGTGAGGTCTTCAAGTGGCTCATCCCTGACAGCGCCGACACCACGGTGGGCAACTCAGACTGC
GCCTACGACCGCATTGTGGCCTGTGGCGCCCGCCTGCGCCGGAGCCTGAAGCCCCAGTCGGCCACCGTGCACGAC
TTCCAGGAGGAATTCGGCCTGGACCAGACTCAGGCTCTTGCCATCAGCGACCACTTTCCAGTGGAGGTGACCCTC
AAGTTCCACCGA**TGA**CTCGAGGCCTGACTGGGGCATGCCACCTGCAGACCCTGGCTCTGAGGAATGGCCCAACAG
TGGCCCCTTCAGGGTGGCAGCCACCCTTCAGTGAGGCCCCAAGGCAGAGTCGGCTGGGCGTGGACCAGGGGCATGGAC
ACGTGATGTGCTGCTCTGTACCTCCGTTCCCCATCTGTGGGACGGGCTGGATC

FIGURE 40

MGGPRALLAALWALEAAGTAALRIGAFNIQSFGDSKVSDPACGSIIAKILAGYDLALVQEVRDPDLSAVSALMEQ
INSVSEHEYSFVSSQPLGRDQYKEMYLFVYRKDAVSVVDTYLYPDPEDVFSREPFVVKFSAPGTGERAPPLPSRR
ALTPPPLPAAAQNLVLIPLHAAPHQAVAEIDALYDVYLDVIDKWGTDDMLFLGDFNADCSYVRAQDWAAIRLRSS
EVFKWLIPDSADTTVGNSDCAYDRIVACGARLRRSLKPQSATVHDFQEEFGLDQTQALAISDHFPVEVTLKFHR

Sequence Listing

<110> Genentech, Inc.

<120> COMPOSITONS AND METHODS FOR THE TREATMENT OF TUMOR

<130> CMD/FP6321947

<140> EP 05019846.4
<141> 1999-12-02

<150> EP 99965090.6
<151> 1999-12-02

<150> PCT/US99/28551
<151> 1999-12-02

<150> PCT/US99/05028
<151> 1999-03-08

<150> PCT/US99/20111
<151> 1999-09-01

<150> US 60/162,506
<151> 1999-10-29

<150> PCT/US99/28313
<151> 1999-11-30

<150> PCT/US99/28634
<151> 1999-12-01

<160> 124

<210> 1
<211> 2336
<212> DNA
<213> Homo Sapien

<220>
<221> unsure
<222> 1620, 1673
<223> unknown base

<400> 1
ttcgtgaccc ttgagaaaag agttggtggt aaatgtgcca cgtcttctaa 50

gaagggggag tcctgaactt gtctgaagcc cttgtccgta agccttgaac 100

tacgttctta aatctatgaa gtcgagggac ctttcgctgc ttttgtaggg 150

acttctttcc ttgcttcagc aacatgaggc ttttcttgtg gaacgcggtc 200

ttgactctgt tcgtcacttc tttgattggg gctttgatcc ctgaaccaga 250

agtgaaaatt gaagttctcc agaagccatt catctgccat cgcaagacca 300

aaggagggga tttgatgttg gtccactatg aaggctactt agaaaaggac 350

ggctccttat ttcactccac tcacaaacat aacaatggtc agcccatttg 400

gtttaccctg ggcatcctgg aggctctcaa aggttgggac cagggcttga 450

```
aaggaatgtg tgtaggagag aagagaaagc tcatcattcc tcctgctctg 500
ggctatggaa aagaaggaaa aggtaaaatt cccccagaaa gtacactgat 550
atttaatatt gatctcctgg agattcgaaa tggaccaaga tcccatgaat 600
cattccaaga aatggatctt aatgatgact ggaaactctc taaagatgag 650
gttaaagcat atttaaagaa ggagtttgaa aaacatggtg cggtggtgaa 700
tgaaagtcat catgatgctt tggtggagga tatttttgat aaagaagatg 750
aagacaaaga tgggtttata tctgccagag aatttacata taaacacgat 800
gagttataga gatacatcta ccctttaat atagcactca tctttcaaga 850
gagggcagtc atctttaaag aacattttat ttttatacaa tgttctttct 900
tgctttgttt tttattttta tatatttttt ctgactccta tttaaagaac 950
cccttaggtt tctaagtacc catttctttc tgataagtta ttgggaagaa 1000
aaagctaatt ggtctttgaa tagaagactt ctggacaatt tttcactttc 1050
acagatatga agctttgttt tactttctca cttataaatt taaaatgttg 1100
caactgggaa tataccacga catgagacca ggttatagca caaattagca 1150
ccctatattt ctgcttccct ctattttctc caagttagag gtcaacattt 1200
gaaaagcctt ttgcaatagc ccaaggcttg ctattttcat gttataatga 1250
aatagtttat gtgtaactgg ctctgagtct ctgcttgagg accagaggaa 1300
aatggttgtt ggacctgact tgttaatggc tactgcttta ctaaggagat 1350
gtgcaatgct gaagttagaa acaaggttaa tagccaggca tggtggctca 1400
tgcctgtaat cccagcactt tgggaggctg aggcgggcgg atcacctgag 1450
gttgggagtt cgagaccagc ctgaccaaca cggagaaacc ctatctctac 1500
taaaaataca aagtagcccg gcgtggtgat gcgtgcctgt aatcccagct 1550
acccaggaag gctgaggcgg cagaatcact tgaacccgag gccgaggttg 1600
cggtaagccg agatcacctn cagcctggac actctgtctc gaaaaaagaa 1650
aagaacacgg ttaataccat atnaatatgt atgcattgag acatgctacc 1700
taggacttaa gctgatgaag cttggctcct agtgattggt ggcctattat 1750
gataaatagg acaaatcatt tatgtgtgag tttctttgta ataaaatgta 1800
tcaatatgtt atagatgagg tagaaagtta tatttatatt caatatttac 1850
ttcttaaggc tagcggaata tccttcctgg ttctttaatg ggtagtctat 1900
agtatattat actacaataa cattgtatca taagataaag tagtaaacca 1950
gtctacattt tcccatttct gtctcatcaa aaactgaagt tagctgggtg 2000
tggtggctca tgcctgtaat cccagcactt tgggggccaa ggagggtgga 2050
```

tcacttgaga tcaggagttc aagaccagcc tggccaacat ggtgaaacct 2100

tgtctctact aaaaatacaa aaattagcca ggcgtggtgg tgcacacctg 2150

tagtcccagc tactcgggag gctgagacag gagatttgct tgaacccggg 2200

aggcggaggt tgcagtgagc caagattgtg ccactgcact ccagcctggg 2250

tgacagagca agactccatc tcaaaaaaaa aaaaaagaag cagacctaca 2300

gcagctacta ttgaataaat acctatcctg gatttt 2336

<210> 2
<211> 211
<212> PRT
<213> Homo Sapien

<400> 2
```
Met Arg Leu Phe Leu Trp Asn Ala Val Leu Thr Leu Phe Val Thr
  1               5                  10                  15

Ser Leu Ile Gly Ala Leu Ile Pro Glu Pro Glu Val Lys Ile Glu
                 20                  25                  30

Val Leu Gln Lys Pro Phe Ile Cys His Arg Lys Thr Lys Gly Gly
                 35                  40                  45

Asp Leu Met Leu Val His Tyr Glu Gly Tyr Leu Glu Lys Asp Gly
                 50                  55                  60

Ser Leu Phe His Ser Thr His Lys His Asn Asn Gly Gln Pro Ile
                 65                  70                  75

Trp Phe Thr Leu Gly Ile Leu Glu Ala Leu Lys Gly Trp Asp Gln
                 80                  85                  90

Gly Leu Lys Gly Met Cys Val Gly Glu Lys Arg Lys Leu Ile Ile
                 95                 100                 105

Pro Pro Ala Leu Gly Tyr Gly Lys Glu Gly Lys Gly Lys Ile Pro
                110                 115                 120

Pro Glu Ser Thr Leu Ile Phe Asn Ile Asp Leu Leu Glu Ile Arg
                125                 130                 135

Asn Gly Pro Arg Ser His Glu Ser Phe Gln Glu Met Asp Leu Asn
                140                 145                 150

Asp Asp Trp Lys Leu Ser Lys Asp Glu Val Lys Ala Tyr Leu Lys
                155                 160                 165

Lys Glu Phe Glu Lys His Gly Ala Val Val Asn Glu Ser His His
                170                 175                 180

Asp Ala Leu Val Glu Asp Ile Phe Asp Lys Glu Asp Glu Asp Lys
                185                 190                 195

Asp Gly Phe Ile Ser Ala Arg Glu Phe Thr Tyr Lys His Asp Glu
                200                 205                 210

Leu
211
```

<210> 3
<211> 26
<212> DNA
<213> Artificial Sequence

<220>
<223> Synthetic Oligonucleotide Probe

<400> 3
ctttccttgc ttcagcaaca tgaggc 26

<210> 4
<211> 25
<212> DNA
<213> Artificial Sequence

<220>
<223> Synthetic Oligonucleotide Probe

<400> 4
gcccagagca ggaggaatga tgagc 25

<210> 5
<211> 49
<212> DNA
<213> Artificial Sequence

<220>
<223> Synthetic Oligonucleotide Probe

<400> 5
gtggaacgcg gtcttgactc tgttcgtcac ttctttgatt ggggctttg 49

<210> 6
<211> 697
<212> DNA
<213> Homo Sapien

<400> 6
tcccggaccc tgccgccctg ccactatgtc ccgccgctct atgctgcttg 50

cctgggctct ccccagcctc cttcgactcg gagcggctca ggagacagaa 100

gacccggcct gctgcagccc catagtgccc cggaacgagt ggaaggccct 150

ggcatcagag tgcgcccagc acctgagcct gcccttacgc tatgtggtgg 200

tatcgcacac ggcgggcagc agctgcaaca cccccgcctc gtgccagcag 250

caggcccgga atgtgcagca ctaccacatg aagacactgg gctggtgcga 300

cgtgggctac aacttcctga ttggagaaga cgggctcgta tacgagggcc 350

gtggctggaa cttcacgggt gcccactcag gtcacttatg gaaccccatg 400

tccattggca tcagcttcat gggcaactac atggatcggg tgcccacacc 450

ccaggccatc cgggcagccc agggtctact ggcctgcggt gtggctcagg 500

gagccctgag gtccaactat gtgctcaaag gacaccggga tgtgcagcgt 550

acactctctc caggcaacca gctctaccac ctcatccaga attggccaca 600

ctaccgctcc ccctgaggcc ctgctgatcc gcaccccatt cctcccctcc 650

catggccaaa aaccccactg tctccttctc caataaagat gtagctc 697

<210> 7
<211> 196
<212> PRT
<213> Homo Sapien

<400> 7
Met Ser Arg Arg Ser Met Leu Leu Ala Trp Ala Leu Pro Ser Leu
1 5 10 15

Leu Arg Leu Gly Ala Ala Gln Glu Thr Glu Asp Pro Ala Cys Cys
20 25 30

Ser Pro Ile Val Pro Arg Asn Glu Trp Lys Ala Leu Ala Ser Glu
35 40 45

Cys Ala Gln His Leu Ser Leu Pro Leu Arg Tyr Val Val Val Ser
50 55 60

His Thr Ala Gly Ser Ser Cys Asn Thr Pro Ala Ser Cys Gln Gln
65 70 75

Gln Ala Arg Asn Val Gln His Tyr His Met Lys Thr Leu Gly Trp
80 85 90

Cys Asp Val Gly Tyr Asn Phe Leu Ile Gly Glu Asp Gly Leu Val
95 100 105

Tyr Glu Gly Arg Gly Trp Asn Phe Thr Gly Ala His Ser Gly His
110 115 120

Leu Trp Asn Pro Met Ser Ile Gly Ile Ser Phe Met Gly Asn Tyr
125 130 135

Met Asp Arg Val Pro Thr Pro Gln Ala Ile Arg Ala Ala Gln Gly
140 145 150

Leu Leu Ala Cys Gly Val Ala Gln Gly Ala Leu Arg Ser Asn Tyr
155 160 165

Val Leu Lys Gly His Arg Asp Val Gln Arg Thr Leu Ser Pro Gly
170 175 180

Asn Gln Leu Tyr His Leu Ile Gln Asn Trp Pro His Tyr Arg Ser
185 190 195

Pro
196

<210> 8
<211> 1170
<212> DNA
<213> Homo Sapien

<400> 8
gtcgaaggtt ataaaagctt ccagccaaac ggcattgaag ttgaagatac 50

aacctgacag cacagcctga gatcttgggg atccctcagc ctaacaccca 100

cagacgtcag ctggtggatt cccgctgcat caaggcctac ccactgtctc 150

catgctgggc tctccctgcc ttctgtggct cctggccgtg accttcttgg 200

ttcccagagc tcagcccttg gcccctcaag actttgaaga agaggaggca 250

gatgagactg agacggcgtg gccgcctttg ccggctgtcc cctgcgacta 300

cgaccactgc cgacacctgc aggtgccctg caaggagcta cagagggtcg 350

ggccggcggc ctgcctgtgc ccaggactct ccagccccgc ccagccgccc 400

gacccgccgc gcatgggaga agtgcgcatt gcggccgaag agggccgcgc 450

agtggtccac tggtgtgccc ccttctcccc ggtcctccac tactggctgc 500

tgctttggga cggcagcgag gctgcgcaga aggggccccc gctgaacgct 550

acggtccgca gagccgaact gaaggggctg aagccagggg gcatttatgt 600

cgtttgcgta gtggccgcta acgaggccgg ggcaagccgc gtgccccagg 650

ctggaggaga gggcctcgag gggccgaca tccctgcctt cgggccttgc 700

agccgccttg cggtgccgcc caaccccgc actctggtcc acgcggccgt 750

cggggtgggc acggccctgg ccctgctaag ctgtgccgcc ctggtgtggc 800

acttctgcct gcgcgatcgc tggggctgcc cgcgccgagc cgccgcccga 850

gccgcagggg cgctctgaaa ggggcctggg ggcatctcgg gcacagacag 900

ccccacctgg ggcgctcagc ctggcccccg ggaaagagga aaacccgctg 950

cctccaggga gggctggacg gcgagctggg agccagcccc aggctccagg 1000

gccacggcgg agtcatggtt ctcaggactg agcgcttgtt taggtccggt 1050

acttggcgct ttgtttcctg gctgaggtct gggaaggaat agaaaggggc 1100

ccccaatttt tttttaagcg gccagataat aaataatgta acctttgcgg 1150

ttaaaaaaaa aaaaaaaaaa 1170

<210> 9
<211> 238
<212> PRT
<213> Homo Sapien

<400> 9

```
Met Leu Gly Ser Pro Cys Leu Leu Trp Leu Leu Ala Val Thr Phe
  1               5                  10                  15

Leu Val Pro Arg Ala Gln Pro Leu Ala Pro Gln Asp Phe Glu Glu
                 20                  25                  30

Glu Glu Ala Asp Glu Thr Glu Thr Ala Trp Pro Pro Leu Pro Ala
                 35                  40                  45

Val Pro Cys Asp Tyr Asp His Cys Arg His Leu Gln Val Pro Cys
                 50                  55                  60

Lys Glu Leu Gln Arg Val Gly Pro Ala Ala Cys Leu Cys Pro Gly
                 65                  70                  75
```

```
Leu Ser Ser Pro Ala Gln Pro Pro Asp Pro Pro Arg Met Gly Glu
                80                  85                  90

Val Arg Ile Ala Ala Glu Glu Gly Arg Ala Val Val His Trp Cys
                95                  100                 105

Ala Pro Phe Ser Pro Val Leu His Tyr Trp Leu Leu Leu Trp Asp
                110                 115                 120

Gly Ser Glu Ala Ala Gln Lys Gly Pro Pro Leu Asn Ala Thr Val
                125                 130                 135

Arg Arg Ala Glu Leu Lys Gly Leu Lys Pro Gly Gly Ile Tyr Val
                140                 145                 150

Val Cys Val Val Ala Ala Asn Glu Ala Gly Ala Ser Arg Val Pro
                155                 160                 165

Gln Ala Gly Gly Glu Gly Leu Glu Gly Ala Asp Ile Pro Ala Phe
                170                 175                 180

Gly Pro Cys Ser Arg Leu Ala Val Pro Pro Asn Pro Arg Thr Leu
                185                 190                 195

Val His Ala Ala Val Gly Val Gly Thr Ala Leu Ala Leu Leu Ser
                200                 205                 210

Cys Ala Ala Leu Val Trp His Phe Cys Leu Arg Asp Arg Trp Gly
                215                 220                 225

Cys Pro Arg Arg Ala Ala Ala Arg Ala Ala Gly Ala Leu
                230                 235         238
```

<210> 10
<211> 2524
<212> DNA
<213> Homo Sapien

<400> 10

```
cgccaagcat gcagtaaagg ctgaaaatct gggtcacagc tgaggaagac 50
ctcagacatg gagtccagga tgtggcctgc gctgctgctg tcccacctcc 100
tccctctctg gccactgctg ttgctgcccc tcccaccgcc tgctcagggc 150
tcttcatcct cccctcgaac cccaccagcc ccagcccgcc ccccgtgtgc 200
caggggaggc ccctcggccc cacgtcatgt gtgcgtgtgg gagcgagcac 250
ctccaccaag ccgatctcct cgggtcccaa gatcacgtcg gcaagtcctg 300
cctggcactg cacccccagc caccccatca ggctttgagg aggggccgcc 350
ctcatcccaa tacccctggg ctatcgtgtg ggtcccacc gtgtctcgag 400
aggatggagg ggaccccaac tctgccaatc ccggatttct ggactatggt 450
tttgcagccc ctcatgggct cgcaacccca caccccaact cagactccat 500
gcgaggtgat ggagatgggc ttatccttgg agaggcacct gccaccctgc 550
ggccattcct gttcgggggc cgtggggaag gtgtggaccc ccagctctat 600
```

gtcacaatta ccatctccat catcattgtt ctcgtggcca ctggcatcat 650

cttcaagttc tgctgggacc gcagccagaa gcgacgcaga ccctcagggc 700

agcaaggtgc cctgaggcag gaggagagcc agcagccact gacagacctg 750

tccccggctg gagtcactgt gctgggggcc ttcggggact cacctacccc 800

cacccctgac catgaggagc cccgaggggg accccggcct gggatgcccc 850

accccaaggg ggctccagcc ttccagttga accggtgagg gcaggggcaa 900

tgggatggga gggcaaagag ggaaggcaac ttaggtcttc agagctgggg 950

tgggggtgcc ctctggatgg gtagtgagga ggcaggcgtg gcctcccaca 1000

gcccctggcc ctcccaaggg ggctggacca gctcctctct gggaggcacc 1050

cttccttctc ccagtctctc aggatctgtg tcctattctc tgctgcccat 1100

aactccaact ctgccctctt tggtttttttc tcatgccacc ttgtctaaga 1150

caactctgcc ctcttaacct tgattccccc tctttgtctt gaacttcccc 1200

ttctattctg gcctacccct tggttcctga ctgtgccctt tccctcttcc 1250

tctcaggatt cccctggtga atctgtgatg cccccaatgt tggggtgcag 1300

ccaagcagga ggccaagggg ccggcacagc cccatccca ctgagggtgg 1350

ggcagctgtg gggagctggg gccacagggg ctcctggctc ctgccccttg 1400

cacaccaccc ggaacactcc ccagccccac gggcaatcct atctgctcgc 1450

cctcctgcag gtgggggcct cacatatctg tgacttcggg tccctgtccc 1500

cacccttgtg cactcacatg aaagccttgc acactcacct ccaccttcac 1550

aggccatttg cacacgctcc tgcaccctct ccccgtccat accgctccgc 1600

tcagctgact ctcatgttct ctcgtctcac atttgcactc tctccttccc 1650

acattctgtg ctcagctcac tcagtggtca gcgtttcctg cacactttac 1700

ctctcatgtg cgtttcccgg cctgatgttg tggtggtgtg cggcgtgctc 1750

actctctccc tcatgaacac ccacccacct cgtttccgca gcccctgcgt 1800

gctgctccag aggtgggtgg gaggtgagct gggggctcct tgggccctca 1850

tcggtcatgg tctcgtccca ttccacacca tttgtttctc tgtctcccca 1900

tcctactcca aggatgccgg catcaccctg agggctcccc cttgggaatg 1950

gggtagtgag gccccagact tcacccccag cccactgcta aaatctgttt 2000

tctgacagat gggttttggg gagtcgcctg ctgcactaca tgagaaaggg 2050

actcccattt gcccttccct ttctcctaca gtcccttttg tcttgtctgt 2100

cctggctgtc tgtgtgtgtg ccattctctg gacttcagag ccccctgagc 2150

cagtcctccc ttcccagcct ccctttgggc ctccctaact ccacctaggc 2200

tgccagggac cggagtcagc tggttcaagg ccatcgggag ctctgcctcc 2250

aagtctaccc ttcccttccc ggactccctc ctgtcccctc ctttcctccc 2300

tccttccttc cactctcctt ccttttgctt ccctgccctt tccccctcct 2350

caggttcttc cctccttctc actggttttt ccaccttcct ccttcccttc 2400

ttccctggct cctaggctgt gatatatatt tttgtattat ctctttcttc 2450

ttcttgtggt gatcatcttg aattactgtg ggatgtaagt ttcaaaattt 2500

tcaaataaag cctttgcaag ataa 2524

<210> 11
<211> 276
<212> PRT
<213> Homo Sapien

<400> 11
Met Glu Ser Arg Met Trp Pro Ala Leu Leu Leu Ser His Leu Leu
1 5 10 15

Pro Leu Trp Pro Leu Leu Leu Leu Pro Leu Pro Pro Pro Ala Gln
20 25 30

Gly Ser Ser Ser Ser Pro Arg Thr Pro Pro Ala Pro Ala Arg Pro
35 40 45

Pro Cys Ala Arg Gly Gly Pro Ser Ala Pro Arg His Val Cys Val
50 55 60

Trp Glu Arg Ala Pro Pro Pro Ser Arg Ser Pro Arg Val Pro Arg
65 70 75

Ser Arg Arg Gln Val Leu Pro Gly Thr Ala Pro Pro Ala Thr Pro
80 85 90

Ser Gly Phe Glu Glu Gly Pro Pro Ser Ser Gln Tyr Pro Trp Ala
95 100 105

Ile Val Trp Gly Pro Thr Val Ser Arg Glu Asp Gly Gly Asp Pro
110 115 120

Asn Ser Ala Asn Pro Gly Phe Leu Asp Tyr Gly Phe Ala Ala Pro
125 130 135

His Gly Leu Ala Thr Pro His Pro Asn Ser Asp Ser Met Arg Gly
140 145 150

Asp Gly Asp Gly Leu Ile Leu Gly Glu Ala Pro Ala Thr Leu Arg
155 160 165

Pro Phe Leu Phe Gly Gly Arg Gly Glu Gly Val Asp Pro Gln Leu
170 175 180

Tyr Val Thr Ile Thr Ile Ser Ile Ile Ile Val Leu Val Ala Thr
185 190 195

Gly Ile Ile Phe Lys Phe Cys Trp Asp Arg Ser Gln Lys Arg Arg
200 205 210

Arg Pro Ser Gly Gln Gln Gly Ala Leu Arg Gln Glu Glu Ser Gln
215 220 225

Gln Pro Leu Thr Asp Leu Ser Pro Ala Gly Val Thr Val Leu Gly
230 235 240

Ala Phe Gly Asp Ser Pro Thr Pro Thr Pro Asp His Glu Glu Pro
245 250 255

Arg Gly Gly Pro Arg Pro Gly Met Pro His Pro Lys Gly Ala Pro
260 265 270

Ala Phe Gln Leu Asn Arg
275 276

<210> 12
<211> 2320
<212> DNA
<213> Homo Sapien

<400> 12
agggtccctt agccgggcgc agggcgcgca gcccaggctg agatccgcgg 50

cttccgtaga agtgagcatg gctgggcagc gagtgcttct tctagtgggc 100

ttccttctcc ctggggtcct gctctcagag gctgccaaaa tcctgacaat 150

atctacagta ggtggaagcc attatctact gatggaccgg gtttctcaga 200

ttcttcaaga tcacggtcat aatgtcacca tgcttaacca caaaagaggt 250

ccttttatgc cagattttaa aaaggaagaa aaatcatatc aagttatcag 300

ttggcttgca cctgaagatc atcaaagaga atttaaaaag agttttgatt 350

tctttctgga agaaacttta ggtggcagag gaaaatttga aaacttatta 400

aatgttctag aatacttggc gttgcagtgc agtcattttt taaatagaaa 450

ggatatcatg gattccttaa agaatgagaa cttcgacatg gtgatagttg 500

aaacttttga ctactgtcct ttcctgattg ctgagaagct tgggaagcca 550

tttgtggcca ttctttccac ttcattcggc tctttggaat ttgggctacc 600

aatccccttg tcttatgttc cagtattccg ttccttgctg actgatcaca 650

tggacttctg ggggccgagtg aagaattttc tgatgttctt tagtttctgc 700

aggaggcaac agcacatgca gtctacattt gacaacacca tcaaggaaca 750

tttcacagaa ggctctaggc cagttttgtc tcatcttcta ctgaaagcag 800

agttgtggtt cattaactct gactttgcct ttgattttgc tcgacctctg 850

cttcccaaca ctgtttatgt tggaggcttg atggaaaaac ctattaaacc 900

agtaccacaa gacttggaga acttcattgc caagtttggg gactctggtt 950

ttgtccttgt gaccttgggc tccatggtga acacctgtca gaatccggaa 1000

atcttcaagg agatgaacaa tgcctttgct cacctacccc aaggggtgat 1050

atggaagtgt cagtgttctc attggcccaa agatgtccac ctggctgcaa 1100

atgtgaaaat tgtggactgg cttcctcaga gtgacctcct ggctcaccca 1150

agcatccgtc tgtttgtcac ccacggcggg cagaatagca taatggaggc 1200

catccagcat ggtgtgccca tggtggggat ccctctcttt ggagaccagc 1250

ctgaaaacat ggtccgagta gaagccaaaa agtttggtgt ttctattcag 1300

ttaaagaagc tcaaggcaga gacattggct cttaagatga aacaaatcat 1350

ggaagacaag agatacaagt ccgcggcagt ggctgccagt gtcatcctgc 1400

gctcccaccc gctcagcccc acacagcggc tggtgggctg gattgaccac 1450

gtcctccaga caggggggcgc gacgcacctc aagccctatg tctttcagca 1500

gccctggcat gagcagtacc tgttcgacgt ttttgtgttt ctgctggggc 1550

tcactctggg gactctatgg ctttgtggga agctgctggg catggctgtc 1600

tggtggctgc gtggggccag aaaggtgaag gagacataag gccaggtgca 1650

gccttggcgg ggtctgtttg gtgggcgatg tcaccatttc tagggagctt 1700

cccactagtt ctggcagccc cattctctag tccttctagt tatctcctgt 1750

tttcttgaag aacaggaaaa atggccaaaa atcatccttt ccacttgcta 1800

attttgctac aaattcatcc ttactagctc ctgcctgcta gcagaaatct 1850

ttccagtcct cttgtcctcc tttgtttgcc atcagcaagg gctatgctgt 1900

gattctgtct ctgagtgact tggaccactg accctcagat ttccagcctt 1950

aaaatccacc ttccttctca tgcgcctctc cgaatcacac cctgactctt 2000

ccagcctcca tgtccagacc tagtcagcct ctctcactcc tgcccctact 2050

atctatcatg gaataacatc caagaaagac accttgcata ttctttcagt 2100

ttctgttttg ttctcccaca tattctcttc aatgctcagg aagcctgccc 2150

tgtgcttgag agttcagggc cggacacagg ctcacaggtc tccacattgg 2200

gtccctgtct ctggtgccca cagtgagctc cttcttggct gagcaggcat 2250

ggagactgta ggtttccaga tttcctgaaa aataaaagtt tacagcgtta 2300

tctctcccca acctcactaa 2320

<210> 13
<211> 523
<212> PRT
<213> Homo Sapien

<400> 13

```
Met Ala Gly Gln Arg Val Leu Leu Leu Val Gly Phe Leu Leu Pro
 1               5                   10                  15

Gly Val Leu Leu Ser Glu Ala Ala Lys Ile Leu Thr Ile Ser Thr
                20                   25                  30
```

```
Val Gly Gly Ser His Tyr Leu Leu Met Asp Arg Val Ser Gln Ile
                35                  40                  45

Leu Gln Asp His Gly His Asn Val Thr Met Leu Asn His Lys Arg
                50                  55                  60

Gly Pro Phe Met Pro Asp Phe Lys Lys Glu Glu Lys Ser Tyr Gln
                65                  70                  75

Val Ile Ser Trp Leu Ala Pro Glu Asp His Gln Arg Glu Phe Lys
                80                  85                  90

Lys Ser Phe Asp Phe Phe Leu Glu Glu Thr Leu Gly Gly Arg Gly
                95                  100                 105

Lys Phe Glu Asn Leu Leu Asn Val Leu Glu Tyr Leu Ala Leu Gln
                110                 115                 120

Cys Ser His Phe Leu Asn Arg Lys Asp Ile Met Asp Ser Leu Lys
                125                 130                 135

Asn Glu Asn Phe Asp Met Val Ile Val Glu Thr Phe Asp Tyr Cys
                140                 145                 150

Pro Phe Leu Ile Ala Glu Lys Leu Gly Lys Pro Phe Val Ala Ile
                155                 160                 165

Leu Ser Thr Ser Phe Gly Ser Leu Glu Phe Gly Leu Pro Ile Pro
                170                 175                 180

Leu Ser Tyr Val Pro Val Phe Arg Ser Leu Leu Thr Asp His Met
                185                 190                 195

Asp Phe Trp Gly Arg Val Lys Asn Phe Leu Met Phe Phe Ser Phe
                200                 205                 210

Cys Arg Arg Gln Gln His Met Gln Ser Thr Phe Asp Asn Thr Ile
                215                 220                 225

Lys Glu His Phe Thr Glu Gly Ser Arg Pro Val Leu Ser His Leu
                230                 235                 240

Leu Leu Lys Ala Glu Leu Trp Phe Ile Asn Ser Asp Phe Ala Phe
                245                 250                 255

Asp Phe Ala Arg Pro Leu Leu Pro Asn Thr Val Tyr Val Gly Gly
                260                 265                 270

Leu Met Glu Lys Pro Ile Lys Pro Val Pro Gln Asp Leu Glu Asn
                275                 280                 285

Phe Ile Ala Lys Phe Gly Asp Ser Gly Phe Val Leu Val Thr Leu
                290                 295                 300

Gly Ser Met Val Asn Thr Cys Gln Asn Pro Glu Ile Phe Lys Glu
                305                 310                 315

Met Asn Asn Ala Phe Ala His Leu Pro Gln Gly Val Ile Trp Lys
                320                 325                 330

Cys Gln Cys Ser His Trp Pro Lys Asp Val His Leu Ala Ala Asn
                335                 340                 345
```

```
Val Lys Ile Val Asp Trp Leu Pro Gln Ser Asp Leu Leu Ala His
                350                 355                 360

Pro Ser Ile Arg Leu Phe Val Thr His Gly Gly Gln Asn Ser Ile
                365                 370                 375

Met Glu Ala Ile Gln His Gly Val Pro Met Val Gly Ile Pro Leu
                380                 385                 390

Phe Gly Asp Gln Pro Glu Asn Met Val Arg Val Glu Ala Lys Lys
                395                 400                 405

Phe Gly Val Ser Ile Gln Leu Lys Lys Leu Lys Ala Glu Thr Leu
                410                 415                 420

Ala Leu Lys Met Lys Gln Ile Met Glu Asp Lys Arg Tyr Lys Ser
                425                 430                 435

Ala Ala Val Ala Ala Ser Val Ile Leu Arg Ser His Pro Leu Ser
                440                 445                 450

Pro Thr Gln Arg Leu Val Gly Trp Ile Asp His Val Leu Gln Thr
                455                 460                 465

Gly Gly Ala Thr His Leu Lys Pro Tyr Val Phe Gln Gln Pro Trp
                470                 475                 480

His Glu Gln Tyr Leu Phe Asp Val Phe Val Phe Leu Leu Gly Leu
                485                 490                 495

Thr Leu Gly Thr Leu Trp Leu Cys Gly Lys Leu Leu Gly Met Ala
                500                 505                 510

Val Trp Trp Leu Arg Gly Ala Arg Lys Val Lys Glu Thr
                515                 520         523
```

<210> 14
<211> 24
<212> DNA
<213> Artificial Sequence

<220>
<223> Synthetic Oligonucleotide Probe

<400> 14
tgcctttgct cacctacccc aagg 24

<210> 15
<211> 24
<212> DNA
<213> Artificial Sequence

<220>
<223> Synthetic Oligonucleotide Probe

<400> 15
tcaggctggt ctccaaagag aggg 24

<210> 16
<211> 45
<212> DNA
<213> Artificial Sequence

<220>
<223> Synthetic Oligonucleotide Probe

<400> 16
cccaaagatg tccacctggc tgcaaatgtg aaaattgtgg actgg 45

<210> 17
<211> 2639
<212> DNA
<213> Homo Sapien

<400> 17
cgcggccggg ccgccggggt gagcgtgccg aggcggctgt ggcgcaggct 50

tccagccccc accatgccgt ggcccctgct gctgctgctg gccgtgagtg 100

gggcccagac aacccggcca tgcttccccg ggtgccaatg cgaggtggag 150

accttcggcc ttttcgacag cttcagcctg actcgggtgg attgtagcgg 200

cctgggcccc cacatcatgc cggtgcccat ccctctggac acagcccact 250

tggacctgtc ctccaaccgg ctggagatgg tgaatgagtc ggtgttggcg 300

gggccgggct acacgacgtt ggctggcctg gatctcagcc acaacctgct 350

caccagcatc tcacccactg ccttctcccg ccttcgctac ctggagtcgc 400

ttgacctcag ccacaatggc ctgacagccc tgccagccga gagcttcacc 450

agctcacccc tgagcgacgt gaaccttagc cacaaccagc tccgggaggt 500

ctcagtgtct gccttcacga cgcacagtca gggccgggca ctacacgtgg 550

acctctccca caacctcatt caccgcctcg tgccccaccc cacgagggcc 600

ggcctgcctg cgcccaccat tcagagcctg aacctggcct ggaaccggct 650

ccatgccgtg cccaacctcc gagacttgcc cctgcgctac ctgagcctgg 700

atgggaaccc tctagctgtc attggtccgg gtgccttcgc ggggctggga 750

ggccttacac acctgtctct ggccagcctg cagaggctcc ctgagctggc 800

gcccagtggc ttccgtgagc taccgggcct gcaggtcctg gacctgtcgg 850

gcaaccccaa gcttaactgg gcaggagctg aggtgttttc aggcctgagc 900

tccctgcagg agctggacct ttcgggcacc aacctggtgc ccctgcctga 950

ggcgctgctc ctccacctcc cggcactgca gagcgtcagc gtgggccagg 1000

atgtgcggtg ccggcgcctg gtgcgggagg gcacctaccc ccggaggcct 1050

ggctccagcc ccaaggtgcc cctgcactgc gtagacaccc gggaatctgc 1100

tgccaggggc cccaccatct tgtgacaaat ggtgtggccc agggccacat 1150

aacagactgc tgtcctgggc tgcctcaggt cccgagtaac ttatgttcaa 1200

tgtgccaaca ccagtgggga gcccgcaggc ctatgtggca gcgtcaccac 1250

aggagttgtg ggcctaggag aggctttgga cctgggagcc acacctagga 1300

gcaaagtctc accccttttgt ctacgttgct tccccaaacc atgagcagag 1350

ggacttcgat gccaaaccag actcgggtcc cctcctgctt cccttcccca 1400

cttatccccc aagtgccttc cctcatgcct gggccggcct gacccgcaat 1450

gggcagaggg tgggtgggac cccctgctgc agggcagagt tcaggtccac 1500

tgggctgagt gtccccttgg gcccatggcc cagtcactca ggggcgagtt 1550

tcttttctaa catagccctt tctttgccat gaggccatga ggcccgcttc 1600

atccttttct atttccctag aaccttaatg gtagaaggaa ttgcaaagaa 1650

tcaagtccac ccttctcatg tgacagatgg ggaaactgag gccttgagaa 1700

ggaaaaaggc taatctaagt tcctgcgggc agtggcatga ctggagcaca 1750

gcctcctgcc tcccagcccg gacccaatgc actttcttgt ctcctctaat 1800

aagccccacc ctccccgcct gggctcccct tgctgccctt gcctgttccc 1850

cattagcaca ggagtagcag cagcaggaca ggcaagagcc tcacaagtgg 1900

gactctgggc ctctgaccag ctgtgcggca tgggctaagt cactctgccc 1950

ttcggagcct ctggaagctt agggcacatt ggttccagcc tagccagttt 2000

ctcaccctgg gttggggtcc cccagcatcc agactggaaa cctacccatt 2050

ttcccctgag catcctctag atgctgcccc aaggagttgc tgcagttctg 2100

gagcctcatc tggctgggat ctccaagggg cctcctggat tcagtcccca 2150

ctggccctga gcacgacagc ccttcttacc ctcccaggaa tgccgtgaaa 2200

ggagacaagg tctgcccgac ccatgtctat gctctacccc cagggcagca 2250

tctcagcttc cgaaccctgg gctgtttcct tagtcttcat tttataaaag 2300

ttgttgcctt tttaacggag tgtcactttc aaccggcctc ccctacccct 2350

gctggccggg gatggagaca tgtcatttgt aaaagcagaa aaaggttgca 2400

tttgttcact tttgtaatat tgtcctgggc ctgtgttggg gtgttggggg 2450

aagctgggca tcagtggcca catgggcatc aggggctggc cccacagaga 2500

ccccacaggg cagtgagctc tgtcttcccc cacctgccta gcccatcatc 2550

tatctaaccg gtccttgatt taataaacac tataaaaggt ttaaaaaaaa 2600

aaaaaaaaaa aaaaaaaaaa aaaaaaaaaa aaaaaaaaa 2639

<210> 18
<211> 353
<212> PRT
<213> Homo Sapien

<400> 18
Met Pro Trp Pro Leu Leu Leu Leu Leu Ala Val Ser Gly Ala Gln
1 5 10 15

Thr Thr Arg Pro Cys Phe Pro Gly Cys Gln Cys Glu Val Glu Thr
20 25 30

Phe Gly Leu Phe Asp Ser Phe Ser Leu Thr Arg Val Asp Cys Ser
35 40 45

Gly Leu Gly Pro His Ile Met Pro Val Pro Ile Pro Leu Asp Thr
50 55 60

Ala His Leu Asp Leu Ser Ser Asn Arg Leu Glu Met Val Asn Glu
65 70 75

Ser Val Leu Ala Gly Pro Gly Tyr Thr Thr Leu Ala Gly Leu Asp
80 85 90

Leu Ser His Asn Leu Leu Thr Ser Ile Ser Pro Thr Ala Phe Ser
95 100 105

Arg Leu Arg Tyr Leu Glu Ser Leu Asp Leu Ser His Asn Gly Leu
110 115 120

Thr Ala Leu Pro Ala Glu Ser Phe Thr Ser Ser Pro Leu Ser Asp
125 130 135

Val Asn Leu Ser His Asn Gln Leu Arg Glu Val Ser Val Ser Ala
140 145 150

Phe Thr Thr His Ser Gln Gly Arg Ala Leu His Val Asp Leu Ser
155 160 165

His Asn Leu Ile His Arg Leu Val Pro His Pro Thr Arg Ala Gly
170 175 180

Leu Pro Ala Pro Thr Ile Gln Ser Leu Asn Leu Ala Trp Asn Arg
185 190 195

Leu His Ala Val Pro Asn Leu Arg Asp Leu Pro Leu Arg Tyr Leu
200 205 210

Ser Leu Asp Gly Asn Pro Leu Ala Val Ile Gly Pro Gly Ala Phe
215 220 225

Ala Gly Leu Gly Gly Leu Thr His Leu Ser Leu Ala Ser Leu Gln
230 235 240

Arg Leu Pro Glu Leu Ala Pro Ser Gly Phe Arg Glu Leu Pro Gly
245 250 255

Leu Gln Val Leu Asp Leu Ser Gly Asn Pro Lys Leu Asn Trp Ala
260 265 270

Gly Ala Glu Val Phe Ser Gly Leu Ser Ser Leu Gln Glu Leu Asp
275 280 285

Leu Ser Gly Thr Asn Leu Val Pro Leu Pro Glu Ala Leu Leu Leu
290 295 300

His Leu Pro Ala Leu Gln Ser Val Ser Val Gly Gln Asp Val Arg
305 310 315

Cys Arg Arg Leu Val Arg Glu Gly Thr Tyr Pro Arg Arg Pro Gly
320 325 330

```
Ser Ser Pro Lys Val Pro Leu His Cys Val Asp Thr Arg Glu Ser
                335                 340                 345

Ala Ala Arg Gly Pro Thr Ile Leu
                350         353
```

<210> 19
<211> 23
<212> DNA
<213> Artificial Sequence

<220>
<223> Synthetic Oligonucleotide Probe

<400> 19
ccctgccagc cgagagcttc acc 23

<210> 20
<211> 23
<212> DNA
<213> Artificial Sequence

<220>
<223> Synthetic Oligonucleotide Probe

<400> 20
ggttggtgcc cgaaaggtcc agc 23

<210> 21
<211> 44
<212> DNA
<213> Artificial Sequence

<220>
<223> Synthetic Oligonucleotide Probe

<400> 21
caaccccaag cttaactggg caggagctga ggtgttttca ggcc 44

<210> 22
<211> 3437
<212> DNA
<213> Homo Sapien

<400> 22
caggaccagg tcttcctacg ctggagcagc ggggagacag ccaccatgca 50

catcctcgtg gtccatgcca tggtgatcct gctgacgctg ggcccgcctc 100

gagccgacga cagcgagttc caggcgctgc tggacatctg gtttccggag 150

gagaagccac tgcccaccgc cttcctggtg gacacatcgg aggaggcgct 200

gctgcttcct gactggctga agctgcgcat gatccgttct gaggtgctcc 250

gcctggtgga cgccgccctg caggacctgg agccgcagca gctgctgctg 300

ttcgtgcagt cgtttggcat ccccgtgtcc agcatgagca aactcctcca 350

gttcctggac caggcagtgg cccacgaccc ccagactctg gagcagaaca 400

tcatggacaa gaattacatg gcccacctgg tggaggtcca gcatgagcgc 450

ggcgcctccg gaggccagac tttccactcc ttgctcacag cctccctgcc 500
gccccgccga gacagcacag aggcacccaa accaaagagc agcccagagc 550
agcccatagg ccagggccgg attcgggtgg ggacccagct ccgggtgctg 600
ggccctgagg acgacctggc tggcatgttc ctccagattt tcccgctcag 650
cccggaccct cggtggcaga gctccagtcc ccgccccgtg gccctcgccc 700
tgcagcaggc cctgggccag gagctggccc gcgtcgtcca gggcagcccc 750
gaggtgccgg gcatcacggt gcgtgtcctg caggccctcg ccaccctgct 800
cagctcccca cacggcggtg ccctggtgat gtccatgcac cgtagccact 850
tcctggcctg cccgctgctg cgccagctct gccagtacca gcgctgtgtg 900
ccacaggaca ccggcttctc ctcgctcttc ctgaaggtgc tcctgcagat 950
gctgcagtgg ctggacagcc ctggcgtgga gggcgggccc ctgcgggcac 1000
agctcaggat gcttgccagc caggcctcag ccgggcgcag gctcagtgat 1050
gtgcgagggg ggctcctgcg cctggccgag gccctggcct tccgtcagga 1100
cctggaggtg gtcagctcca ccgtccgtgc cgtcatcgcc accctgaggt 1150
ctggggagca gtgcagcgtg gagccggacc tgatcagcaa agtcctccag 1200
gggctgatcg aggtgaggtc cccccacctg gaggagctgc tgactgcatt 1250
cttctctgcc actgcggatg ctgcctcccc gtttccagcc tgtaagcccg 1300
ttgtggtggt gagctccctg ctgctgcagg aggaggagcc cctggctggg 1350
gggaagccgg gtgcggacgg tggcagcctg gaggccgtgc ggctggggcc 1400
ctcgtcaggc ctcctagtgg actggctgga aatgctggac cccgaggtgg 1450
tcagcagctg ccccgacctg cagctcaggc tgctcttctc ccggaggaag 1500
ggcaaaggtc aggcccaggt gccctcgttc cgtccctacc tcctgaccct 1550
cttcacgcat cagtccagct ggcccacact gcaccagtgc atccgagtcc 1600
tgctgggcaa gagccgggaa cagaggttcg acccctctgc ctctctggac 1650
ttcctctggg cctgcatcca tgttcctcgc atctggcagg ggcgggacca 1700
gcgcaccccg cagaagcggc gggaggagct ggtgctgcgg gtccagggcc 1750
cggagctcat cagcctggtg gagctgatcc tggccgaggc ggagacgcgg 1800
agccaggacg gggacacagc cgcctgcagc ctcatccagg cccggctgcc 1850
cctgctgctc agctgctgct gtggggacga tgagagtgtc aggaaggtga 1900
cggagcacct gtcaggctgc atccagcagt ggggagacag cgtgctggga 1950
aggcgctgcc gagaccttct cctgcagctc tacctacagc ggccggagct 2000
gcgggtgccc gtgcctgagg tcctactgca cagcgaaggg gctgccagca 2050

gcagcgtctg caagctggac ggactcatcc accgcttcat cacgctcctt 2100
gcggacacca gcgactcccg ggcgttggag aaccgagggg cggatgccag 2150
catggcctgc cggaagctgg cggtggcgca cccgctgctg ctgctcaggc 2200
acctgcccat gatcgcggcg ctcctgcacg gccgcaccca cctcaacttc 2250
caggagttcc ggcagcagaa ccacctgagc tgcttcctgc acgtgctggg 2300
cctgctggag ctgctgcagc cgcacgtgtt ccgcagcgag caccaggggg 2350
cgctgtggga ctgccttctg tccttcatcc gcctgctgct gaattacagg 2400
aagtcctccc gccatctggc tgccttcatc aacaagtttg tgcagttcat 2450
ccataagtac attacctaca atgccccagc agccatctcc ttcctgcaga 2500
agcacgccga cccgctccac gacctgtcct tcgacaacag tgacctggtg 2550
atgctgaaat ccctccttgc agggctcagc ctgcccagca gggacgacag 2600
gaccgaccga ggcctggacg aagagggcga ggaggagagc tcagccggct 2650
ccttgcccct ggtcagcgtc tccctgttca cccctctgac cgcggccgag 2700
atggccccct acatgaaacg gctttcccgg ggccaaacgg tggaggatct 2750
gctggaggtt ctgagtgaca tagacgagat gtcccggcgg agacccgaga 2800
tcctgagctt cttctcgacc aacctgcagc ggctgatgag ctcggccgag 2850
gagtgttgcc gcaacctcgc cttcagcctg gccctgcgct ccatgcagaa 2900
cagccccagc attgcagccg ctttcctgcc cacgttcatg tactgcctgg 2950
gcagccagga ctttgaggtg gtgcagacgg ccctccggaa cctgcctgag 3000
tacgctctcc tgtgccaaga gcacgcggct gtgctgctcc accgggcctt 3050
cctggtgggc atgtacggcc agatggaccc cagcgcgcag atctccgagg 3100
ccctgaggat cctgcatatg gaggccgtga tgtgagcctg tggcagccga 3150
ccccccctcca agccccggcc cgtcccgtcc ccggggatcc tcgaggcaaa 3200
gcccaggaag cgtgggcgtt gctggtctgt ccgaggaggt gagggcgccg 3250
agccctgagg ccaggcaggc ccaggagcaa tactccgagc cctggggtgg 3300
ctccgggccg gccgctggca tcaggggccg tccagcaagc cctcattcac 3350
cttctgggcc acagccctgc cgcggagcgg cggatccccc cgggcatggc 3400
ctgggctggt tttgaatgaa acgacctgaa ctgtcaa 3437

<210> 23
<211> 1029
<212> PRT
<213> Homo Sapien

<400> 23
```
Met His Ile Leu Val Val His Ala Met Val Ile Leu Leu Thr Leu
1               5                   10                  15

Gly Pro Pro Arg Ala Asp Asp Ser Glu Phe Gln Ala Leu Leu Asp
                20                  25                  30

Ile Trp Phe Pro Glu Glu Lys Pro Leu Pro Thr Ala Phe Leu Val
                35                  40                  45

Asp Thr Ser Glu Glu Ala Leu Leu Leu Pro Asp Trp Leu Lys Leu
                50                  55                  60

Arg Met Ile Arg Ser Glu Val Leu Arg Leu Val Asp Ala Ala Leu
                65                  70                  75

Gln Asp Leu Glu Pro Gln Gln Leu Leu Leu Phe Val Gln Ser Phe
                80                  85                  90

Gly Ile Pro Val Ser Ser Met Ser Lys Leu Leu Gln Phe Leu Asp
                95                  100                 105

Gln Ala Val Ala His Asp Pro Gln Thr Leu Glu Gln Asn Ile Met
                110                 115                 120

Asp Lys Asn Tyr Met Ala His Leu Val Glu Val Gln His Glu Arg
                125                 130                 135

Gly Ala Ser Gly Gly Gln Thr Phe His Ser Leu Leu Thr Ala Ser
                140                 145                 150

Leu Pro Pro Arg Arg Asp Ser Thr Glu Ala Pro Lys Pro Lys Ser
                155                 160                 165

Ser Pro Glu Gln Pro Ile Gly Gln Gly Arg Ile Arg Val Gly Thr
                170                 175                 180

Gln Leu Arg Val Leu Gly Pro Glu Asp Asp Leu Ala Gly Met Phe
                185                 190                 195

Leu Gln Ile Phe Pro Leu Ser Pro Asp Pro Arg Trp Gln Ser Ser
                200                 205                 210

Ser Pro Arg Pro Val Ala Leu Ala Leu Gln Gln Ala Leu Gly Gln
                215                 220                 225

Glu Leu Ala Arg Val Val Gln Gly Ser Pro Glu Val Pro Gly Ile
                230                 235                 240

Thr Val Arg Val Leu Gln Ala Leu Ala Thr Leu Leu Ser Ser Pro
                245                 250                 255

His Gly Gly Ala Leu Val Met Ser Met His Arg Ser His Phe Leu
                260                 265                 270

Ala Cys Pro Leu Leu Arg Gln Leu Cys Gln Tyr Gln Arg Cys Val
                275                 280                 285

Pro Gln Asp Thr Gly Phe Ser Ser Leu Phe Leu Lys Val Leu Leu
                290                 295                 300

Gln Met Leu Gln Trp Leu Asp Ser Pro Gly Val Glu Gly Gly Pro
                305                 310                 315
```

Leu Arg Ala Gln Leu Arg Met Leu Ala Ser Gln Ala Ser Ala Gly
320 325 330

Arg Arg Leu Ser Asp Val Arg Gly Gly Leu Leu Arg Leu Ala Glu
335 340 345

Ala Leu Ala Phe Arg Gln Asp Leu Glu Val Val Ser Ser Thr Val
350 355 360

Arg Ala Val Ile Ala Thr Leu Arg Ser Gly Glu Gln Cys Ser Val
365 370 375

Glu Pro Asp Leu Ile Ser Lys Val Leu Gln Gly Leu Ile Glu Val
380 385 390

Arg Ser Pro His Leu Glu Glu Leu Leu Thr Ala Phe Phe Ser Ala
395 400 405

Thr Ala Asp Ala Ala Ser Pro Phe Pro Ala Cys Lys Pro Val Val
410 415 420

Val Val Ser Ser Leu Leu Leu Gln Glu Glu Glu Pro Leu Ala Gly
425 430 435

Gly Lys Pro Gly Ala Asp Gly Gly Ser Leu Glu Ala Val Arg Leu
440 445 450

Gly Pro Ser Ser Gly Leu Leu Val Asp Trp Leu Glu Met Leu Asp
455 460 465

Pro Glu Val Val Ser Ser Cys Pro Asp Leu Gln Leu Arg Leu Leu
470 475 480

Phe Ser Arg Arg Lys Gly Lys Gly Gln Ala Gln Val Pro Ser Phe
485 490 495

Arg Pro Tyr Leu Leu Thr Leu Phe Thr His Gln Ser Ser Trp Pro
500 505 510

Thr Leu His Gln Cys Ile Arg Val Leu Leu Gly Lys Ser Arg Glu
515 520 525

Gln Arg Phe Asp Pro Ser Ala Ser Leu Asp Phe Leu Trp Ala Cys
530 535 540

Ile His Val Pro Arg Ile Trp Gln Gly Arg Asp Gln Arg Thr Pro
545 550 555

Gln Lys Arg Arg Glu Glu Leu Val Leu Arg Val Gln Gly Pro Glu
560 565 570

Leu Ile Ser Leu Val Glu Leu Ile Leu Ala Glu Ala Glu Thr Arg
575 580 585

Ser Gln Asp Gly Asp Thr Ala Ala Cys Ser Leu Ile Gln Ala Arg
590 595 600

Leu Pro Leu Leu Leu Ser Cys Cys Cys Gly Asp Asp Glu Ser Val
605 610 615

Arg Lys Val Thr Glu His Leu Ser Gly Cys Ile Gln Gln Trp Gly
620 625 630

```
Asp Ser Val Leu Gly Arg Arg Cys Arg Asp Leu Leu Leu Gln Leu
                635                 640                 645

Tyr Leu Gln Arg Pro Glu Leu Arg Val Pro Val Pro Glu Val Leu
                650                 655                 660

Leu His Ser Glu Gly Ala Ala Ser Ser Ser Val Cys Lys Leu Asp
                665                 670                 675

Gly Leu Ile His Arg Phe Ile Thr Leu Leu Ala Asp Thr Ser Asp
                680                 685                 690

Ser Arg Ala Leu Glu Asn Arg Gly Ala Asp Ala Ser Met Ala Cys
                695                 700                 705

Arg Lys Leu Ala Val Ala His Pro Leu Leu Leu Leu Arg His Leu
                710                 715                 720

Pro Met Ile Ala Ala Leu Leu His Gly Arg Thr His Leu Asn Phe
                725                 730                 735

Gln Glu Phe Arg Gln Gln Asn His Leu Ser Cys Phe Leu His Val
                740                 745                 750

Leu Gly Leu Leu Glu Leu Leu Gln Pro His Val Phe Arg Ser Glu
                755                 760                 765

His Gln Gly Ala Leu Trp Asp Cys Leu Leu Ser Phe Ile Arg Leu
                770                 775                 780

Leu Leu Asn Tyr Arg Lys Ser Ser Arg His Leu Ala Ala Phe Ile
                785                 790                 795

Asn Lys Phe Val Gln Phe Ile His Lys Tyr Ile Thr Tyr Asn Ala
                800                 805                 810

Pro Ala Ala Ile Ser Phe Leu Gln Lys His Ala Asp Pro Leu His
                815                 820                 825

Asp Leu Ser Phe Asp Asn Ser Asp Leu Val Met Leu Lys Ser Leu
                830                 835                 840

Leu Ala Gly Leu Ser Leu Pro Ser Arg Asp Asp Arg Thr Asp Arg
                845                 850                 855

Gly Leu Asp Glu Glu Gly Glu Glu Glu Ser Ser Ala Gly Ser Leu
                860                 865                 870

Pro Leu Val Ser Val Ser Leu Phe Thr Pro Leu Thr Ala Ala Glu
                875                 880                 885

Met Ala Pro Tyr Met Lys Arg Leu Ser Arg Gly Gln Thr Val Glu
                890                 895                 900

Asp Leu Leu Glu Val Leu Ser Asp Ile Asp Glu Met Ser Arg Arg
                905                 910                 915

Arg Pro Glu Ile Leu Ser Phe Phe Ser Thr Asn Leu Gln Arg Leu
                920                 925                 930

Met Ser Ser Ala Glu Glu Cys Cys Arg Asn Leu Ala Phe Ser Leu
                935                 940                 945
```

```
Ala Leu Arg Ser Met Gln Asn Ser Pro Ser Ile Ala Ala Ala Phe
                950                 955                 960

Leu Pro Thr Phe Met Tyr Cys Leu Gly Ser Gln Asp Phe Glu Val
                965                 970                 975

Val Gln Thr Ala Leu Arg Asn Leu Pro Glu Tyr Ala Leu Leu Cys
                980                 985                 990

Gln Glu His Ala Ala Val Leu Leu His Arg Ala Phe Leu Val Gly
                995                 1000                1005

Met Tyr Gly Gln Met Asp Pro Ser Ala Gln Ile Ser Glu Ala Leu
                1010                1015                1020

Arg Ile Leu His Met Glu Ala Val Met
                1025            1029
```

<210> 24
<211> 2186
<212> DNA
<213> Homo Sapien

<400> 24

```
ccgggccatg cagcctcggc cccgcgggcg cccgccgcgc acccgaggag 50
atgaggctcc gcaatggcac cttcctgacg ctgctgctct tctgcctgtg 100
cgccttcctc tcgctgtcct ggtacgcggc actcagcggc cagaaaggcg 150
acgttgtgga cgtttaccag cgggagttcc tggcgctgcg cgatcggttg 200
cacgcagctg agcaggagag cctcaagcgc tccaaggagc tcaacctggt 250
gctggacgag atcaagaggg ccgtgtcaga aaggcaggcg ctgcgagacg 300
gagacggcaa tcgcacctgg ggccgcctaa cagaggaccc ccgattgaag 350
ccgtggaacg gctcacaccg gcacgtgctg cacctgccca ccgtcttcca 400
tcacctgcca cacctgctgg ccaaggagag cagtctgcag cccgcggtgc 450
gcgtgggcca gggccgcacc ggagtgtcgg tggtgatggg catcccgagc 500
gtgcggcgcg aggtgcactc gtacctgact gacactctgc actcgctcat 550
ctccgagctg agcccgcagg agaaggagga ctcggtcatc gtggtgctga 600
tcgccgagac tgactcacag tacacttcgg cagtgacaga gaacatcaag 650
gccttgttcc ccacggagat ccattctggg ctcctggagg tcatctcacc 700
ctccccccac ttctaccctg acttctcccg cctccgagag tcctttgggg 750
accccaagga gagagtcagg tggaggacca aacagaacct cgattactgc 800
ttcctcatga tgtacgcgca gtccaaaggc atctactacg tgcagctgga 850
ggatgacatc gtggccaagc ccaactacct gagcaccatg aagaactttg 900
cactgcagca gccttcagag gactggatga tcctggagtt ctcccagctg 950
ggcttcattg gtaagatgtt caagtcgctg gacctgagcc tgattgtaga 1000
```

gttcattctc atgttctacc gggacaagcc catcgactgg ctcctggacc 1050

atattctgtg ggtgaaagtc tgcaaccccg agaaggatgc gaagcactgt 1100

gaccggcaga aagccaacct gcggatccgc ttcaaaccgt ccctcttcca 1150

gcacgtgggc actcactcct cgctggctgg caagatccag aaactgaagg 1200

acaaagactt tggaaagcag gcgctgcgga aggagcatgt gaacccgcca 1250

gcagaggtga gcacgagcct gaagacatac cagcacttca ccctggagaa 1300

agcctacctg cgcgaggact tcttctgggc cttcacccct gccgcggggg 1350

acttcatccg cttccgcttc ttccaacctc taagactgga gcggttcttc 1400

ttccgcagtg ggaacatcga gcacccggag gacaagctct tcaacacgtc 1450

tgtggaggtg ctgcccttcg acaaccctca gtcagacaag gaggccctgc 1500

aggagggccg caccgccacc ctccggtacc ctcggagccc cgacggctac 1550

ctccagatcg gctccttcta caagggagtg gcagagggag aggtggaccc 1600

agccttcggc cctctggaag cactgcgcct ctcgatccag acggactccc 1650

ctgtgtgggt gattctgagc gagatcttcc tgaaaaaggc cgactaagct 1700

gcgggcttct gagggtaccc tgtggccagc cctgaagccc acatttctgg 1750

gggtgtcgtc actgccgtcc ccggagggcc agatacggcc ccgcccaaag 1800

ggttctgcct ggcgtcgggc ttgggccggc ctggggtccg ccgctggccc 1850

ggaggcccta ggagctggtg ctgcccccgc ccgccgggcc gcggaggagg 1900

caggcggccc ccacactgtg cctgaggccc ggaaccgttc gcacccggcc 1950

tgccccagtc aggccgtttt agaagagctt ttacttgggc gcccgccgtc 2000

tctggcgcga acactggaat gcatatacta ctttatgtgc tgtgtttttt 2050

attcttggat acatttgatt ttttcacgta agtccacata tacttctata 2100

agagcgtgac ttgtaataaa gggttaatga agaaaaaaaa aaaaaaaaaa 2150

aaaaaaaaaa aaaaaaaaaa aaaaaaaaaa aaaaaa 2186

<210> 25
<211> 548
<212> PRT
<213> Homo Sapien

<400> 25

```
Met Arg Leu Arg Asn Gly Thr Phe Leu Thr Leu Leu Leu Phe Cys
1               5                   10                  15

Leu Cys Ala Phe Leu Ser Leu Ser Trp Tyr Ala Ala Leu Ser Gly
                20                  25                  30

Gln Lys Gly Asp Val Val Asp Val Tyr Gln Arg Glu Phe Leu Ala
                35                  40                  45
```

```
Leu Arg Asp Arg Leu His Ala Ala Glu Gln Glu Ser Leu Lys Arg
                50                  55                  60

Ser Lys Glu Leu Asn Leu Val Leu Asp Glu Ile Lys Arg Ala Val
                65                  70                  75

Ser Glu Arg Gln Ala Leu Arg Asp Gly Asp Gly Asn Arg Thr Trp
                80                  85                  90

Gly Arg Leu Thr Glu Asp Pro Arg Leu Lys Pro Trp Asn Gly Ser
                95                  100                 105

His Arg His Val Leu His Leu Pro Thr Val Phe His His Leu Pro
                110                 115                 120

His Leu Leu Ala Lys Glu Ser Ser Leu Gln Pro Ala Val Arg Val
                125                 130                 135

Gly Gln Gly Arg Thr Gly Val Ser Val Val Met Gly Ile Pro Ser
                140                 145                 150

Val Arg Arg Glu Val His Ser Tyr Leu Thr Asp Thr Leu His Ser
                155                 160                 165

Leu Ile Ser Glu Leu Ser Pro Gln Glu Lys Glu Asp Ser Val Ile
                170                 175                 180

Val Val Leu Ile Ala Glu Thr Asp Ser Gln Tyr Thr Ser Ala Val
                185                 190                 195

Thr Glu Asn Ile Lys Ala Leu Phe Pro Thr Glu Ile His Ser Gly
                200                 205                 210

Leu Leu Glu Val Ile Ser Pro Ser Pro His Phe Tyr Pro Asp Phe
                215                 220                 225

Ser Arg Leu Arg Glu Ser Phe Gly Asp Pro Lys Glu Arg Val Arg
                230                 235                 240

Trp Arg Thr Lys Gln Asn Leu Asp Tyr Cys Phe Leu Met Met Tyr
                245                 250                 255

Ala Gln Ser Lys Gly Ile Tyr Tyr Val Gln Leu Glu Asp Asp Ile
                260                 265                 270

Val Ala Lys Pro Asn Tyr Leu Ser Thr Met Lys Asn Phe Ala Leu
                275                 280                 285

Gln Gln Pro Ser Glu Asp Trp Met Ile Leu Glu Phe Ser Gln Leu
                290                 295                 300

Gly Phe Ile Gly Lys Met Phe Lys Ser Leu Asp Leu Ser Leu Ile
                305                 310                 315

Val Glu Phe Ile Leu Met Phe Tyr Arg Asp Lys Pro Ile Asp Trp
                320                 325                 330

Leu Leu Asp His Ile Leu Trp Val Lys Val Cys Asn Pro Glu Lys
                335                 340                 345

Asp Ala Lys His Cys Asp Arg Gln Lys Ala Asn Leu Arg Ile Arg
                350                 355                 360
```

Phe Lys Pro Ser Leu Phe Gln His Val Gly Thr His Ser Ser Leu
365 370 375

Ala Gly Lys Ile Gln Lys Leu Lys Asp Lys Asp Phe Gly Lys Gln
380 385 390

Ala Leu Arg Lys Glu His Val Asn Pro Pro Ala Glu Val Ser Thr
395 400 405

Ser Leu Lys Thr Tyr Gln His Phe Thr Leu Glu Lys Ala Tyr Leu
410 415 420

Arg Glu Asp Phe Phe Trp Ala Phe Thr Pro Ala Ala Gly Asp Phe
425 430 435

Ile Arg Phe Arg Phe Phe Gln Pro Leu Arg Leu Glu Arg Phe Phe
440 445 450

Phe Arg Ser Gly Asn Ile Glu His Pro Glu Asp Lys Leu Phe Asn
455 460 465

Thr Ser Val Glu Val Leu Pro Phe Asp Asn Pro Gln Ser Asp Lys
470 475 480

Glu Ala Leu Gln Glu Gly Arg Thr Ala Thr Leu Arg Tyr Pro Arg
485 490 495

Ser Pro Asp Gly Tyr Leu Gln Ile Gly Ser Phe Tyr Lys Gly Val
500 505 510

Ala Glu Gly Glu Val Asp Pro Ala Phe Gly Pro Leu Glu Ala Leu
515 520 525

Arg Leu Ser Ile Gln Thr Asp Ser Pro Val Trp Val Ile Leu Ser
530 535 540

Glu Ile Phe Leu Lys Lys Ala Asp
545 548

<210> 26
<211> 663
<212> DNA
<213> Homo Sapien

<400> 26
gcaccgcaga cggcgcggat cgcagggagc cggtccgccg ccggaacggg 50

agcctgggtg tgcgtgtgga gtccggactc gtgggagacg atcgcgatga 100

acacggtgct gtcgcgggcg aactcactgt tcgccttctc gctgagcgtg 150

atggcggcgc tcaccttcgg ctgcttcatc accaccgcct tcaaagacag 200

gagcgtcccg gtgcggctgc acgtctcgcg gatcatgcta aaaaatgtag 250

aagatttcac tggacctaga gaaagaagtg atctgggatt tatcacattt 300

gatataactg ctgatctaga gaatatattt gattggaatg ttaagcagtt 350

gtttctttat ttatcagcag aatattcaac aaaaaataat gctctgaacc 400

aagttgtcct atgggacaag attgttttga gaggtgataa tccgaagctg 450

ctgctgaaag atatgaaaac aaaatatttt ttctttgacg atggaaatgg 500

tctcaaggga aacaggaatg tcactttgac cctgtcttgg aacgtcgtac 550

caaatgctgg aattctacct cttgtgacag gatcaggaca cgtatctgtc 600

ccatttccag atacatatga aataacgaag agttattaaa ttattctgaa 650

tttgaaacaa aaa 663

<210> 27
<211> 180
<212> PRT
<213> Homo Sapien

<400> 27

```
Met Asn Thr Val Leu Ser Arg Ala Asn Ser Leu Phe Ala Phe Ser
 1               5                  10                  15

Leu Ser Val Met Ala Ala Leu Thr Phe Gly Cys Phe Ile Thr Thr
                20                  25                  30

Ala Phe Lys Asp Arg Ser Val Pro Val Arg Leu His Val Ser Arg
                35                  40                  45

Ile Met Leu Lys Asn Val Glu Asp Phe Thr Gly Pro Arg Glu Arg
                50                  55                  60

Ser Asp Leu Gly Phe Ile Thr Phe Asp Ile Thr Ala Asp Leu Glu
                65                  70                  75

Asn Ile Phe Asp Trp Asn Val Lys Gln Leu Phe Leu Tyr Leu Ser
                80                  85                  90

Ala Glu Tyr Ser Thr Lys Asn Asn Ala Leu Asn Gln Val Val Leu
                95                 100                 105

Trp Asp Lys Ile Val Leu Arg Gly Asp Asn Pro Lys Leu Leu Leu
               110                 115                 120

Lys Asp Met Lys Thr Lys Tyr Phe Phe Phe Asp Asp Gly Asn Gly
               125                 130                 135

Leu Lys Gly Asn Arg Asn Val Thr Leu Thr Leu Ser Trp Asn Val
               140                 145                 150

Val Pro Asn Ala Gly Ile Leu Pro Leu Val Thr Gly Ser Gly His
               155                 160                 165

Val Ser Val Pro Phe Pro Asp Thr Tyr Glu Ile Thr Lys Ser Tyr
               170                 175                 180
```

<210> 28
<211> 3580
<212> DNA
<213> Homo Sapien

<400> 28

gaccggtccc tccggtcctg gatgtgcgga ctctgctgca gcgagggctg 50

caggcccgcc gggcggtgct caccgtgccc tggctggtgg agtttctctc 100

ctttgctgac catgttgttc ccttgctgga atattaccgg gacatcttca 150

ctctcctgct gcgcctgcac cggagcttgg tgttgtcgca ggagagtgag 200
gggaagatgt gtttcctgaa caagctgctg ctacttgctg tcctgggctg 250
gcttttccag attcccacag tccctgagga cttgttcttt ctggaagagg 300
gtccctcata tgcctttgag gtggacacag tagccccaga gcatggcttg 350
gacaatgcgc ctgtggtgga ccagcagctg ctctacacct gctgccccta 400
catcggagag ctccggaaac tgctcgcttc gtgggtgtca ggcagtagtg 450
gacggagtgg gggcttcatg aggaaaatca cccccaccac taccaccagc 500
ctgggagccc agccttccca gaccagccag gggctgcagg cacagctcgc 550
ccaggccttt ttccacaacc agccgccctc cttgcgccgg accgtagagt 600
tcgtggcaga aagaattgga tcaaactgtg tcaaacatat caaggctaca 650
ctggtggcag atctggtgcg ccaggcagag tcacttctcc aagagcagct 700
ggtgacacag ggagaggaag gggagaccc agcccagctg ttggagatct 750
tgtgttccca gctgtgccct cacggggccc aggcattggc cctggggcgg 800
gagttctgtc aaaggaagag ccctggggct gtgcgggcgc tgcttccaga 850
ggagaccccg gcagccgttc tgagcagtgc agagaacatt gctgtggggc 900
ttgcaacaga gaaagcctgt gcttggctgt cagccaacat cacagcactg 950
atcaggaggg aggtgaaagc agcagtgagt cgcacacttc gagcccaggg 1000
tcctgaacct gctgcccggg gggagcggag ggctgctcc cgcgcctgac 1050
gtgctctcct tggccgtggg gccacgggac cctgacgagg gagtctcccc 1100
agagcatctg gaacagctcc taggccagct gggccagacg ctgcggtgcc 1150
gccagttcct gtgcccacct gctgagcagc atctggcaaa gtgctctgtg 1200
gagttagctt ccctcctcgt tgcagatcaa attcctatcc tagggccccc 1250
ggcacagtac aggctggaga gagggcaggc tcgaaggctt ctgcacatgc 1300
tgctttcctt gtggaaggaa gactttcagg ggccggttcc gctgcagctg 1350
ctgctgagcc caagaaatgt ggggcttctg gcagacacaa ggccaaggga 1400
gtgggacttg ctgctattct tgctacggga gctggtggag aagggtctga 1450
tgggacggat ggagatagag gcctgcctgg gcagcctcca ccaggcccag 1500
tggccagggg actttgctga agaattagca acactgtcta atctgtttct 1550
agccgagccc cacctgccag aaccccagct aagagcctgt gagttggtgc 1600
agccaaaccg ggcactgtg ctggcccaga gctagggctg agaagtggcc 1650
ctgccttggg cattgcacca gaaccctgga cccccgcctc acgaggaggc 1700
ccaagtgccc aatgcagacc ctcactggtt ggggtgtagc tgggtctaca 1750

gtcagacttc ctgctctaag ggtgtcactg cctggcatcc caccacgcga 1800
atcctagagg aaggagagtt ggcctgattt gggattatgg cagaaaagtc 1850
cagagatgcc agtcctggag tagaagaggt ggtgtttgtt tatctcttgg 1900
atactaaatg aaatgaggtg tgtgggcttg tcaacacaga attcaagcct 1950
catttgctat cccagcatct cttaaaactt tgtagtcttg gaattcatga 2000
cagaggcaaa tgactcctgc ttaacttatg aagaaagtta aaacatgaat 2050
cttgggagtc tacattttct tatcaccagg agctggactg ccatctcctt 2100
ataaatgcct aacacaggcc gggtctggtg gctcatgcct gtaatcccag 2150
cactttgaga ggcctgaggt cggcggactg cctgaggtca ggaattcaag 2200
accagcctgg ccaacatggc aaaaccccat ctctactaaa aataaaaaaa 2250
ttattagctg ggcatggtgg tgtgtgcctg taatcccagc tactcaggag 2300
gatgaggcag gagacctgct tgaacctgga ggtggaggtt gcagtgagcc 2350
gaggtcgcac cactgcactc cagtctgggt aacagagcga gactttctag 2400
aaaaagccta acaaacagat aaggtaggac tcaaccaact gaaacctgac 2450
tttccccctg taccttcagc ccctgtgcag gtagtaacct cttgagacct 2500
ctccctgacc agggaccaag cacagggcat ttagagcttt ttagaataaa 2550
ctggttttct ttaaaaaaaa aaaaaaaaaa agggcggccg cccttttttt 2600
tttttttttt tttttttttt tttttttttt tttttttttt taaaaagggc 2650
ttttattaaa attctcccca cacgatggct cctgcaatct gccacagctc 2700
tggggcgtgt cctgtaggga aaggccctgt tttccctgag gcggggctgg 2750
gcttgtccat gggtccgcgg agctggccgt gcttggcgcc ctggcgtgtg 2800
tctagctgct tcttgccggg cacagagctg cggggtctgg gggcaccggg 2850
agctaagagc aggctctggt gcaggggtgg aggcctgtct cttaaccgac 2900
accctgaggt gctcctgaga tgctgggtcc accctgagtg gcacggggag 2950
cagctgtggc cggtgctcct tcytaggcca gtcctgggga aactaagctc 3000
gggcccttct ttgcaaagac cgaggatggg gtgggtgtgg gggactcatg 3050
gggaatggcc tgaggagcta cgtgtgaaga gggcgccggt ttgttggctg 3100
cagcggcctg gagcgcctct ctcctgagcc tcagtttccc tttccgtcta 3150
atgaagaaca tgccgtctcg gtgtctcagg gctattagga cttgccctca 3200
ggaagtggcc ttggacgagc gtcatgttat tttcacaact gtcctgcgac 3250
gttggcctgg gcacgtcatg gaatggccca tgtccctctg ctgcgtggac 3300
gtcgcggtcg ggagtgcgca gccagaggcg ggccagacgg tgcgcctggg 3350

ggtgagggga ggcgccccgg gagggcctca caggaagttg ggctcccgca 3400

ccaccaggca gggcgggctc ccgccgccgc cgccgccacc accgtccagg 3450

ggccggtaga caaagtggaa gtcgcgcttg ggctcgctgc gcagcaggta 3500

gcccttgatg cagtgcggca gcgcgtcgtc cgccagctgg aagcagcgcc 3550

cgtccaccag cacgaacagc cggtgcgcct 3580

<210> 29
<211> 280
<212> PRT
<213> Homo Sapien

<400> 29
Met Cys Phe Leu Asn Lys Leu Leu Leu Leu Ala Val Leu Gly Trp
1 5 10 15

Leu Phe Gln Ile Pro Thr Val Pro Glu Asp Leu Phe Phe Leu Glu
20 25 30

Glu Gly Pro Ser Tyr Ala Phe Glu Val Asp Thr Val Ala Pro Glu
35 40 45

His Gly Leu Asp Asn Ala Pro Val Val Asp Gln Gln Leu Leu Tyr
50 55 60

Thr Cys Cys Pro Tyr Ile Gly Glu Leu Arg Lys Leu Leu Ala Ser
65 70 75

Trp Val Ser Gly Ser Ser Gly Arg Ser Gly Gly Phe Met Arg Lys
80 85 90

Ile Thr Pro Thr Thr Thr Thr Ser Leu Gly Ala Gln Pro Ser Gln
95 100 105

Thr Ser Gln Gly Leu Gln Ala Gln Leu Ala Gln Ala Phe Phe His
110 115 120

Asn Gln Pro Pro Ser Leu Arg Arg Thr Val Glu Phe Val Ala Glu
125 130 135

Arg Ile Gly Ser Asn Cys Val Lys His Ile Lys Ala Thr Leu Val
140 145 150

Ala Asp Leu Val Arg Gln Ala Glu Ser Leu Leu Gln Glu Gln Leu
155 160 165

Val Thr Gln Gly Glu Glu Gly Gly Asp Pro Ala Gln Leu Leu Glu
170 175 180

Ile Leu Cys Ser Gln Leu Cys Pro His Gly Ala Gln Ala Leu Ala
185 190 195

Leu Gly Arg Glu Phe Cys Gln Arg Lys Ser Pro Gly Ala Val Arg
200 205 210

Ala Leu Leu Pro Glu Glu Thr Pro Ala Ala Val Leu Ser Ser Ala
215 220 225

Glu Asn Ile Ala Val Gly Leu Ala Thr Glu Lys Ala Cys Ala Trp
230 235 240

```
Leu Ser Ala Asn Ile Thr Ala Leu Ile Arg Arg Glu Val Lys Ala
                245                 250                 255

Ala Val Ser Arg Thr Leu Arg Ala Gln Gly Pro Glu Pro Ala Ala
                260                 265                 270

Arg Gly Glu Arg Arg Gly Cys Ser Arg Ala
                275                 280
```

<210> 30
<211> 1990
<212> DNA
<213> Homo Sapien

<400> 30

```
gccgagtggg acaaagcctg ggctgggcg ggggccatgg cgctgccatc 50

ccgaatcctg ctttggaaac ttgtgcttct gcagagctct gctgttctcc 100

tgcactcagc ggtggaggag acggacgcgg ggctgtacac ctgcaacctg 150

caccatcact actgccacct ctacgagagc ctggccgtcc gcctggaggt 200

caccgacggc cccccggcca cccccgccta ctgggacggc gagaaggagg 250

tgctggcggt ggcgcgcggc gcacccgcgc ttctgacctg cgtgaaccgc 300

gggcacgtgt ggaccgaccg gcacgtggag gaggctcaac aggtggtgca 350

ctgggaccgg cagccgcccg gggtcccgca cgaccgcgcg gaccgcctgc 400

tggacctcta cgcgtcgggc gagcgccgcg cctacgggcc cctttttctg 450

cgcgaccgcg tggctgtggg cgcggatgcc tttgagcgcg gtgacttctc 500

actgcgtatc gagccgctgg aggtcgccga cgagggcacc tactcctgcc 550

acctgcacca ccattactgt ggcctgcacg aacgccgcgt cttccacctg 600

acggtcgccg aaccccacgc ggagccgccc ccccggggct ctccgggcaa 650

cggctccagc cacagcggcg ccccaggccc agaccccaca ctggcgcgcg 700

gccacaacgt catcaatgtc atcgtccccg agagccgagc ccacttcttc 750

cagcagctgg gctacgtgct ggccacgctg ctgctcttca tcctgctact 800

ggtcactgtc ctcctggccg cccgcaggcg ccgcggaggc tacgaatact 850

cggaccagaa gtcgggaaag tcaaagggga aggatgttaa cttggcggag 900

ttcgctgtgg ctgcagggga ccagatgctt tacaggagtg aggacatcca 950

gctagattac aaaaacaaca tcctgaagga gagggcggag ctggcccaca 1000

gccccctgcc tgccaagtac atcgacctag acaaagggtt ccggaaggag 1050

aactgcaaat agggaggccc tgggctcctg gctgggccag cagctgcacc 1100

tctcctgtct gtgctcctcg gggcatctcc tgatgctccg gggctcaccc 1150

cccttccagc ggctggtccc gctttcctgg aatttggcct gggcgtatgc 1200
```

agaggccgcc tccacacccc tcccccaggg gcttggtggc agcatagccc 1250

ccacccctgc ggcctttgct cacgggtggc cctgcccacc cctggcacaa 1300

ccaaaatccc actgatgccc atcatgccct cagacccttc tgggctctgc 1350

ccgctggggg cctgaagaca ttcctggagg acactcccat cagaacctgg 1400

cagccccaaa actggggtca gcctcagggc aggagtccca ctcctccagg 1450

gctctgctcg tccggggctg ggagatgttc ctggaggagg acactcccat 1500

cagaacttgg cagccttgaa gttggggtca gcctcggcag gagtcccact 1550

cctcctgggg tgctgcctgc caccaagagc tcccccacct gtaccaccat 1600

gtgggactcc aggcaccatc tgttctcccc agggacctgc tgacttgaat 1650

gccagccctt gctcctctgt gttgctttgg gccacctggg gctgcacccc 1700

ctgccctttc tctgccccat ccctacccta gccttgctct cagccacctt 1750

gatagtcact gggctccctg tgacttctga ccctgacacc cctcccttgg 1800

actctgcctg ggctggagtc tagggctggg gctacatttg gcttctgtac 1850

tggctgagga caggggaggg agtgaagttg gtttggggtg gcctgtgttg 1900

ccactctcag caccccacat ttgcatctgc tggtggacct gccaccatca 1950

caataaagtc cccatctgat tttaaaaaaa aaaaaaaaaa 1990

<210> 31
<211> 341
<212> PRT
<213> Homo Sapien

<400> 31
Met Ala Leu Pro Ser Arg Ile Leu Leu Trp Lys Leu Val Leu Leu
1 5 10 15

Gln Ser Ser Ala Val Leu Leu His Ser Ala Val Glu Glu Thr Asp
20 25 30

Ala Gly Leu Tyr Thr Cys Asn Leu His His His Tyr Cys His Leu
35 40 45

Tyr Glu Ser Leu Ala Val Arg Leu Glu Val Thr Asp Gly Pro Pro
50 55 60

Ala Thr Pro Ala Tyr Trp Asp Gly Glu Lys Glu Val Leu Ala Val
65 70 75

Ala Arg Gly Ala Pro Ala Leu Leu Thr Cys Val Asn Arg Gly His
80 85 90

Val Trp Thr Asp Arg His Val Glu Glu Ala Gln Gln Val Val His
95 100 105

Trp Asp Arg Gln Pro Pro Gly Val Pro His Asp Arg Ala Asp Arg
110 115 120

Leu Leu Asp Leu Tyr Ala Ser Gly Glu Arg Arg Ala Tyr Gly Pro
125 130 135

Leu Phe Leu Arg Asp Arg Val Ala Val Gly Ala Asp Ala Phe Glu
140 145 150

Arg Gly Asp Phe Ser Leu Arg Ile Glu Pro Leu Glu Val Ala Asp
155 160 165

Glu Gly Thr Tyr Ser Cys His Leu His His His Tyr Cys Gly Leu
170 175 180

His Glu Arg Arg Val Phe His Leu Thr Val Ala Glu Pro His Ala
185 190 195

Glu Pro Pro Pro Arg Gly Ser Pro Gly Asn Gly Ser Ser His Ser
200 205 210

Gly Ala Pro Gly Pro Asp Pro Thr Leu Ala Arg Gly His Asn Val
215 220 225

Ile Asn Val Ile Val Pro Glu Ser Arg Ala His Phe Phe Gln Gln
230 235 240

Leu Gly Tyr Val Leu Ala Thr Leu Leu Leu Phe Ile Leu Leu Leu
245 250 255

Val Thr Val Leu Leu Ala Ala Arg Arg Arg Arg Gly Gly Tyr Glu
260 265 270

Tyr Ser Asp Gln Lys Ser Gly Lys Ser Lys Gly Lys Asp Val Asn
275 280 285

Leu Ala Glu Phe Ala Val Ala Ala Gly Asp Gln Met Leu Tyr Arg
290 295 300

Ser Glu Asp Ile Gln Leu Asp Tyr Lys Asn Asn Ile Leu Lys Glu
305 310 315

Arg Ala Glu Leu Ala His Ser Pro Leu Pro Ala Lys Tyr Ile Asp
320 325 330

Leu Asp Lys Gly Phe Arg Lys Glu Asn Cys Lys
335 340 341

<210> 32
<211> 1091
<212> DNA
<213> Homo Sapien

<400> 32
caagcaggtc atccccttgg tgaccttcaa agagaagcag agagggcaga 50

ggtgggggc acagggaaag ggtgacctct gagattcccc ttttccccca 100

gactttggaa gtgacccacc atggggctca gcatcttttt gctcctgtgt 150

gttcttgggc tcagccaggc agccacaccg aagattttca atggcactga 200

gtgtgggcgt aactcacagc cgtggcaggt ggggctgttt gagggcacca 250

gcctgcgctg cgggggtgtc cttattgacc acaggtgggt cctcacagcg 300

gctcactgca gcggcagcag gtactgggtg cgcctggggg aacacagcct 350

cagccagctc gactggaccg agcagatccg gcacagcggc ttctctgtga 400

cccatcccgg ctacctggga gcctcgacga gccacgagca cgacctccgg 450

ctgctgcggc tgcgcctgcc cgtccgcgta accagcagcg ttcaacccct 500

gcccctgccc aatgactgtg caaccgctgg caccgagtgc cacgtctcag 550

gctggggcat caccaaccac ccacggaacc cattcccgga tctgctccag 600

tgcctcaacc tctccatcgt ctcccatgcc acctgccatg gtgtgtatcc 650

cgggagaatc acgagcaaca tggtgtgtgc aggcggcgtc ccggggcagg 700

atgcctgcca gggtgattct gggggccccc tggtgtgtgg gggagtcctt 750

caaggtctgg tgtcctgggg gtctgtgggg ccctgtggac aagatggcat 800

ccctggagtc tacacctata tttgcaagta tgtggactgg atccggatga 850

tcatgaggaa caactgacct gtttcctcca cctccacccc caccccttaa 900

cttgggtacc cctctggccc tcagagcacc aatatctcct ccatcacttc 950

ccctagctcc actcttgttg gcctgggaac ttcttggaac tttaactcct 1000

gccagccctt ctaagaccca cgagcggggt gagagaagtg tgcaatagtc 1050

tggaataaat ataaatgaag gaggggcaaa aaaaaaaaaa a 1091

<210> 33
<211> 248
<212> PRT
<213> Homo Sapien

<400> 33

```
Met Gly Leu Ser Ile Phe Leu Leu Leu Cys Val Leu Gly Leu Ser
1               5                   10                  15

Gln Ala Ala Thr Pro Lys Ile Phe Asn Gly Thr Glu Cys Gly Arg
                20                  25                  30

Asn Ser Gln Pro Trp Gln Val Gly Leu Phe Glu Gly Thr Ser Leu
                35                  40                  45

Arg Cys Gly Gly Val Leu Ile Asp His Arg Trp Val Leu Thr Ala
                50                  55                  60

Ala His Cys Ser Gly Ser Arg Tyr Trp Val Arg Leu Gly Glu His
                65                  70                  75

Ser Leu Ser Gln Leu Asp Trp Thr Glu Gln Ile Arg His Ser Gly
                80                  85                  90

Phe Ser Val Thr His Pro Gly Tyr Leu Gly Ala Ser Thr Ser His
                95                  100                 105

Glu His Asp Leu Arg Leu Leu Arg Leu Arg Leu Pro Val Arg Val
                110                 115                 120
```

```
Thr Ser Ser Val Gln Pro Leu Pro Leu Pro Asn Asp Cys Ala Thr
                125                 130                 135

Ala Gly Thr Glu Cys His Val Ser Gly Trp Gly Ile Thr Asn His
                140                 145                 150

Pro Arg Asn Pro Phe Pro Asp Leu Leu Gln Cys Leu Asn Leu Ser
                155                 160                 165

Ile Val Ser His Ala Thr Cys His Gly Val Tyr Pro Gly Arg Ile
                170                 175                 180

Thr Ser Asn Met Val Cys Ala Gly Gly Val Pro Gly Gln Asp Ala
                185                 190                 195

Cys Gln Gly Asp Ser Gly Gly Pro Leu Val Cys Gly Gly Val Leu
                200                 205                 210

Gln Gly Leu Val Ser Trp Gly Ser Val Gly Pro Cys Gly Gln Asp
                215                 220                 225

Gly Ile Pro Gly Val Tyr Thr Tyr Ile Cys Lys Tyr Val Asp Trp
                230                 235                 240

Ile Arg Met Ile Met Arg Asn Asn
                245         248
```

<210> 34
<211> 1643
<212> DNA
<213> Homo Sapien

<400> 34

```
agcagcctct gcccgacccg gctcgtgcgg accccaggac cgggcgcggg 50
acgcgtgcgt ccagcctccg gcgctgcgga gacccgcggc tgggtccggg 100
gaggccccaa acccgccccc gccagaaccc cgccccaaat tcccacctcc 150
tccagaagcc ccgcccactc ccgagccccg agagctccgc gcacctgggc 200
gccatccgcc ctggctccgc tgcacgagct ccacgcccgt accccggcgt 250
cacgctcagc ccgcggtgct cgcacacctg agactcatct cgcttcgacc 300
ccgccgccgc cgccgcccgg catcctgagc acggagacag tctccagctg 350
ccgttcatgc ttcctcccca gccttccgca gcccaccagg gaaggggcgg 400
taggagtggc cttttaccaa agggaccggc gatgctctgc aggctgtgct 450
ggctggtctc gtacagcttg gctgtgctgt tgctcggctg cctgctcttc 500
ctgaggaagg cggccaagcc cgcaggagac cccacggccc accagccttt 550
ctgggctccc ccaacacccc gtcacagccg gtgtccaccc aaccacacag 600
tgtctagcgc ctctctgtcc ctgcctagcc gtcaccgtct cttcttgacc 650
tatcgtcact gccgaaattt ctctatcttg ctggagcctt caggctgttc 700
caaggatacc ttcttgctcc tggccatcaa gtcacagcct ggtcacgtgg 750
```

agcgacgtgc ggctatccgc agcacgtggg gcagggtggg gggatgggct 800

aggggccggc agctgaagct ggtgttcctc ctaggggtgg caggatccgc 850

tcccccagcc cagctgctgg cctatgagag tagggagttt gatgacatcc 900

tccagtggga cttcactgag gacttcttca acctgacgct caaggagctg 950

cacctgcagc gctgggtggt ggctgcctgc ccccaggccc atttcatgct 1000

aaagggagat gacgatgtct ttgtccacgt ccccaacgtg ttagagttcc 1050

tggatggctg ggacccagcc caggacctcc tggtgggaga tgtcatccgc 1100

caagccctgc ccaacaggaa cactaaggtc aaatacttca tcccaccctc 1150

aatgtacagg gccacccact acccacccta tgctggtggg ggaggatatg 1200

tcatgtccag agccacagtg cggcgcctcc aggctatcat ggaagatgct 1250

gaactcttcc ccattgatga tgtctttgtg ggtatgtgcc tgaggaggct 1300

ggggctgagc cctatgcacc atgctggctt caagacattt ggaatccggc 1350

ggcccctgga ccccttagac ccctgcctgt ataggggggct cctgctggtt 1400

caccgcctca gcccccctcga gatgtggacc atgtgggcac tggtgacaga 1450

tgagggggctc aagtgtgcag ctggccccat accccagcgc tgaagggtgg 1500

gttgggcaac agcctgagag tggactcagt gttgattctc tatcgtgatg 1550

cgaaattgat gcctgctgct ctacagaaaa tgccaacttg gtttttttaac 1600

tcctctcacc ctgttagctc tgattaaaaa cactgcaacc caa 1643

<210> 35
<211> 378
<212> PRT
<213> Homo Sapien

<400> 35

```
Met Leu Pro Pro Gln Pro Ser Ala Ala His Gln Gly Arg Gly Gly
1               5                   10                  15

Arg Ser Gly Leu Leu Pro Lys Gly Pro Ala Met Leu Cys Arg Leu
                20                  25                  30

Cys Trp Leu Val Ser Tyr Ser Leu Ala Val Leu Leu Leu Gly Cys
                35                  40                  45

Leu Leu Phe Leu Arg Lys Ala Ala Lys Pro Ala Gly Asp Pro Thr
                50                  55                  60

Ala His Gln Pro Phe Trp Ala Pro Pro Thr Pro Arg His Ser Arg
                65                  70                  75

Cys Pro Pro Asn His Thr Val Ser Ser Ala Ser Leu Ser Leu Pro
                80                  85                  90

Ser Arg His Arg Leu Phe Leu Thr Tyr Arg His Cys Arg Asn Phe
                95                  100                 105
```

```
Ser Ile Leu Leu Glu Pro Ser Gly Cys Ser Lys Asp Thr Phe Leu
                110                 115                 120

Leu Leu Ala Ile Lys Ser Gln Pro Gly His Val Glu Arg Arg Ala
                125                 130                 135

Ala Ile Arg Ser Thr Trp Gly Arg Val Gly Gly Trp Ala Arg Gly
                140                 145                 150

Arg Gln Leu Lys Leu Val Phe Leu Leu Gly Val Ala Gly Ser Ala
                155                 160                 165

Pro Pro Ala Gln Leu Leu Ala Tyr Glu Ser Arg Glu Phe Asp Asp
                170                 175                 180

Ile Leu Gln Trp Asp Phe Thr Glu Asp Phe Phe Asn Leu Thr Leu
                185                 190                 195

Lys Glu Leu His Leu Gln Arg Trp Val Val Ala Ala Cys Pro Gln
                200                 205                 210

Ala His Phe Met Leu Lys Gly Asp Asp Asp Val Phe Val His Val
                215                 220                 225

Pro Asn Val Leu Glu Phe Leu Asp Gly Trp Asp Pro Ala Gln Asp
                230                 235                 240

Leu Leu Val Gly Asp Val Ile Arg Gln Ala Leu Pro Asn Arg Asn
                245                 250                 255

Thr Lys Val Lys Tyr Phe Ile Pro Pro Ser Met Tyr Arg Ala Thr
                260                 265                 270

His Tyr Pro Pro Tyr Ala Gly Gly Gly Gly Tyr Val Met Ser Arg
                275                 280                 285

Ala Thr Val Arg Arg Leu Gln Ala Ile Met Glu Asp Ala Glu Leu
                290                 295                 300

Phe Pro Ile Asp Asp Val Phe Val Gly Met Cys Leu Arg Arg Leu
                305                 310                 315

Gly Leu Ser Pro Met His His Ala Gly Phe Lys Thr Phe Gly Ile
                320                 325                 330

Arg Arg Pro Leu Asp Pro Leu Asp Pro Cys Leu Tyr Arg Gly Leu
                335                 340                 345

Leu Leu Val His Arg Leu Ser Pro Leu Glu Met Trp Thr Met Trp
                350                 355                 360

Ala Leu Val Thr Asp Glu Gly Leu Lys Cys Ala Ala Gly Pro Ile
                365                 370                 375

Pro Gln Arg
        378
```

<210> 36
<211> 23
<212> DNA
<213> Artificial Sequence

<220>

<223> Synthetic Oligonucleotide Probe

<400> 36
cttgctcctg gccatcaagt cac 23

<210> 37
<211> 28
<212> DNA
<213> Artificial Sequence

<220>
<223> Synthetic Oligonucleotide Probe

<400> 37
gttgaagaag tcctcagtga agtcccac 28

<210> 38
<211> 40
<212> DNA
<213> Artificial Sequence

<220>
<223> Synthetic Oligonucleotide Probe

<400> 38
cagctgaagc tggtgttcct cctaggggtg gcaggatccg 40

<210> 39
<211> 1470
<212> DNA
<213> Homo Sapien

<400> 39
ctcggctgga tttaaggttg ccgctagccg cctgggaatt taagggaccc 50

acactacctt cccgaagttg aaggcaagcg gtgattgttt gtagacggcg 100

ctttgtcatg ggacctgtgc ggttgggaat attgcttttc cttttttttgg 150

ccgtgcacga ggcttgggct gggatgttga aggaggagga cgatgacaca 200

gaacgcttgc ccagcaaatg cgaagtgtgt aagctgctga gcacagagct 250

acaggcggaa ctgagtcgca ccggtcgatc tcgagaggtg ctggagctgg 300

ggcaggtgct ggatacaggc aagaggaaga gacacgtgcc ttacagcgtt 350

tcagagacaa ggctggaaga ggccttagag aatttatgtg agcggatcct 400

ggactatagt gttcacgctg agcgcaaggg ctcactgaga tatgccaagg 450

gtcagagtca gaccatggca acactgaaag gcctagtgca gaagggggtg 500

aaggtggatc tggggatccc tctggagctt tgggatgagc ccagcgtgga 550

ggtcacatac ctcaagaagc agtgtgagac catgttggag gagtttgaag 600

acattgtggg agactggtac ttccaccatc aggagcagcc cctacaaaat 650

tttctctgtg aaggtcatgt gctcccagct gctgaaactg catgtctaca 700

ggaaacttgg actggaaagg agatcacaga tggggaagag aaaacagaag 750

gggaggaaga gcaggaggag gaggaggaag aggaggaaga ggaaggggga 800

gacaagatga ccaagacagg aagccacccc aaacttgacc gagaagatct 850

ttgacccttg cctttgagcc cccaggaggg gaagggatca tggagagccc 900

tctaaagcct gcactctccc tgctccacag ctttcagggt gtgtttatga 950

gtgactccac ccaagcttgt agctgttctc tcccatctaa cctcaggcaa 1000

gatcctggtg aaacagcatg acatggcttc tggggtggag ggtgggggtg 1050

gaggtcctgc tcctagagat gaactctatc cagcccctta attggcaggt 1100

gtatgtgctg acagtactga aagctttcct ctttaactga tcccaccccc 1150

acccaaaagt cagcagtggc actggagctg tgggctttgg ggaagtcact 1200

tagctcctta aggtctgttt ttagaccctt ccaaggaaga ggccagaacg 1250

gacattctct gcgatctata tacattgcct gtatccagga ggctacacac 1300

cagcaaaccg tgaaggagaa tgggacactg ggtcatggcc tggagttgct 1350

gataatttag gtgggataga tacttggtct acttaagctc aatgtaaccc 1400

agagcccacc atatagtttt ataggtgctc aactttctat atcgctatta 1450

aactttttc tttttttcta 1470

<210> 40
<211> 248
<212> PRT
<213> Homo Sapien

<400> 40
Met Gly Pro Val Arg Leu Gly Ile Leu Leu Phe Leu Phe Leu Ala
1 5 10 15

Val His Glu Ala Trp Ala Gly Met Leu Lys Glu Glu Asp Asp Asp
20 25 30

Thr Glu Arg Leu Pro Ser Lys Cys Glu Val Cys Lys Leu Leu Ser
35 40 45

Thr Glu Leu Gln Ala Glu Leu Ser Arg Thr Gly Arg Ser Arg Glu
50 55 60

Val Leu Glu Leu Gly Gln Val Leu Asp Thr Gly Lys Arg Lys Arg
65 70 75

His Val Pro Tyr Ser Val Ser Glu Thr Arg Leu Glu Glu Ala Leu
80 85 90

Glu Asn Leu Cys Glu Arg Ile Leu Asp Tyr Ser Val His Ala Glu
95 100 105

Arg Lys Gly Ser Leu Arg Tyr Ala Lys Gly Gln Ser Gln Thr Met
110 115 120

Ala Thr Leu Lys Gly Leu Val Gln Lys Gly Val Lys Val Asp Leu
125 130 135

```
Gly Ile Pro Leu Glu Leu Trp Asp Glu Pro Ser Val Glu Val Thr
                140                 145                 150

Tyr Leu Lys Lys Gln Cys Glu Thr Met Leu Glu Glu Phe Glu Asp
                155                 160                 165

Ile Val Gly Asp Trp Tyr Phe His His Gln Glu Gln Pro Leu Gln
                170                 175                 180

Asn Phe Leu Cys Glu Gly His Val Leu Pro Ala Ala Glu Thr Ala
                185                 190                 195

Cys Leu Gln Glu Thr Trp Thr Gly Lys Glu Ile Thr Asp Gly Glu
                200                 205                 210

Glu Lys Thr Glu Gly Glu Glu Glu Gln Glu Glu Glu Glu Glu Glu
                215                 220                 225

Glu Glu Glu Glu Gly Gly Asp Lys Met Thr Lys Thr Gly Ser His
                230                 235                 240

Pro Lys Leu Asp Arg Glu Asp Leu
                245         248
```

<210> 41
<211> 2684
<212> DNA
<213> Homo Sapien

<220>
<221> unsure
<222> 2636-2637
<223> unknown base

<400> 41
```
cggacgcgtg ggcgctgagc cccggaggcc agggcgtccg gggctgcgcc 50

acttccgagg gccgagcgct gccggtcccg gcggtgcgac acggccggga 100

ggaggagaac aacgcaaggg gctcaaccgt cggtcgctgg agcccccccc 150

ggggcgtggc ctcccgcccc ctcagctggg gagggcgggg ctcgctgccc 200

cctgctgccg actgcgaccc ttacagggga gggagggcgc aggccgcgcg 250

gagatgagga ggaggctgcg cctacgcagg gacgcattgc tcacgctgct 300

ccttggcgcc tccctgggcc tcttactcta tgcgcagcgc gacggcgcgg 350

ccccgacggc gagcgcgccg cgagggcgag ggagggcggc accgaggccc 400

acccccggac cccgcgcgtt ccagttaccc gacgcgggtg cagccccgcc 450

ggcctacgaa ggggacacac cggcgccgcc cacgcctacg ggaccctttg 500

acttcgcccg ctatttgcgc gccaaggacc agcggcggtt tccactgctc 550

attaaccagc cgcacaagtg ccgcggcgac ggcgcacccg gtggccgccc 600

ggacctgctt attgctgtca agtcggtggc agaggacttc gagcggcgcc 650

aagccgtgcg ccagacgtgg ggcgcggagg gtcgcgtgca gggggcgctg 700
```

gtgcgccgcg tgttcttgct gggcgtgccc aggggcgcag gctcgggcgg 750
ggccgacgaa gttggggagg gcgcgcgaac ccactggcgc gccctgctgc 800
gggccgagag ccttgcgtat gcggacatcc tgctctgggc cttcgacgac 850
acctttttta acctaacgct caaggagatc cactttctag cctgggcctc 900
agctttctgc cccgacgtgc gcttcgtttt taagggcgac gcagatgtgt 950
tcgtgaacgt gggaaatctc ctggagttcc tggcgccgcg ggacccggcg 1000
caagacctgc ttgctggtga cgtaattgtg catgcgcggc ccatccgcac 1050
gcgggctagc aagtactaca tccccgaggc cgtgtacggc ctgcccgcct 1100
atccggccta cgcgggcggc ggtggctttg tgctttccgg ggccacgctg 1150
caccgcctgg ctggcgcctg tgcgcaggtc gagctcttcc ccatcgacga 1200
cgtctttctg ggcatgtgtc tgcagcgcct gcggctcacg cccgagcctc 1250
accctgcctt ccgcaccttt ggcatccccc agccttcagc cgcgccgcat 1300
ttgagcacct tcgacccctg cttttaccgt gagctggttg tagtgcacgg 1350
gctctcggcc gctgacatct ggcttatgtg gcgcctgctg cacgggccgc 1400
atgggccagc ctgtgcgcat ccacagcctg tcgctgcagg ccccttccaa 1450
tgggactcct agctccccac tacagcccca agctcctaac tcagacccag 1500
aatggagccg gtttcccaga ttattgccgt gtatgtggtt cttccctgat 1550
caccaggtgc ctgtctccac aggatcccag gggatggggg ttaagcttgg 1600
ctcctggcgg tccaccctgc tggaaccagt tgaaacccgt gtaatggtga 1650
ccctttgagc gagccaaggc tgggtggtag atgaccatct cttgtccaac 1700
aggtcccaga gcagtggata tgtctggtcc tcctagtagc acagaggtgt 1750
gttctggtgt ggtggcaggg acttagggaa tcctaccact ctgctggatt 1800
tggaaccccc taggctgacg cggacgtatg cagaggctct caaggccagg 1850
ccccacaggg aggtggaggg gctccggccg ccacagcctg aattcatgaa 1900
cctggcaggc actttgccat agctcatctg aaaacagata ttatgcttcc 1950
cacaacctct cctgggccca ggtgtggctg agcaccaggg atggagccac 2000
acataaggga caaatgagtg cacggtccta cctagtcttt cctcacctcc 2050
tgaactcaca caacaatgcc agtctcccac tggaggctgt atcccctcag 2100
aggagccaag gaatgtcttc ccctgagatg ccaccactat taatttcccc 2150
atatgcttca accacccccct tgctcaaaaa accaataccc acacttacct 2200
taatacaaac atcccagcaa cagcacatgg caggccattg ctgagggcac 2250
aggtgcttta ttggagaggg gatgtgggca ggggataagg aaggttcccc 2300

cattccagga ggatgggaac agtcctggct gcccctgaca gtggggatat 2350

gcaaggggct ctggccaggc cacagtccaa atgggaagac accagtcagt 2400

cacaaaagtc gggagcgcca cacaaacctg gctataaggc ccaggaacca 2450

tataggagcc tgagacaggt cccctgcaca ttcatcatta aactatacag 2500

gatgaggctg tacatgagtt aattacaaaa gagtcatatt tacaaaaatc 2550

tgtacacaca tttgaaaaac tcacaaaatt gtcatctatg tatcacaagt 2600

tgctagaccc aaaatattaa aaatgggata aaattnnttt aaaaaaaaaa 2650

aaaaaaaaaa aaaaaaaaaa aaaaaaaaaa aaaa 2684

<210> 42
<211> 402
<212> PRT
<213> Homo Sapien

<400> 42
Met Arg Arg Arg Leu Arg Leu Arg Arg Asp Ala Leu Leu Thr Leu
1 5 10 15

Leu Leu Gly Ala Ser Leu Gly Leu Leu Leu Tyr Ala Gln Arg Asp
20 25 30

Gly Ala Ala Pro Thr Ala Ser Ala Pro Arg Gly Arg Gly Arg Ala
35 40 45

Ala Pro Arg Pro Thr Pro Gly Pro Arg Ala Phe Gln Leu Pro Asp
50 55 60

Ala Gly Ala Ala Pro Pro Ala Tyr Glu Gly Asp Thr Pro Ala Pro
65 70 75

Pro Thr Pro Thr Gly Pro Phe Asp Phe Ala Arg Tyr Leu Arg Ala
80 85 90

Lys Asp Gln Arg Arg Phe Pro Leu Leu Ile Asn Gln Pro His Lys
95 100 105

Cys Arg Gly Asp Gly Ala Pro Gly Gly Arg Pro Asp Leu Leu Ile
110 115 120

Ala Val Lys Ser Val Ala Glu Asp Phe Glu Arg Arg Gln Ala Val
125 130 135

Arg Gln Thr Trp Gly Ala Glu Gly Arg Val Gln Gly Ala Leu Val
140 145 150

Arg Arg Val Phe Leu Leu Gly Val Pro Arg Gly Ala Gly Ser Gly
155 160 165

Gly Ala Asp Glu Val Gly Glu Gly Ala Arg Thr His Trp Arg Ala
170 175 180

Leu Leu Arg Ala Glu Ser Leu Ala Tyr Ala Asp Ile Leu Leu Trp
185 190 195

Ala Phe Asp Asp Thr Phe Phe Asn Leu Thr Leu Lys Glu Ile His
200 205 210

Phe Leu Ala Trp Ala Ser Ala Phe Cys Pro Asp Val Arg Phe Val
215 220 225

Phe Lys Gly Asp Ala Asp Val Phe Val Asn Val Gly Asn Leu Leu
230 235 240

Glu Phe Leu Ala Pro Arg Asp Pro Ala Gln Asp Leu Leu Ala Gly
245 250 255

Asp Val Ile Val His Ala Arg Pro Ile Arg Thr Arg Ala Ser Lys
260 265 270

Tyr Tyr Ile Pro Glu Ala Val Tyr Gly Leu Pro Ala Tyr Pro Ala
275 280 285

Tyr Ala Gly Gly Gly Gly Phe Val Leu Ser Gly Ala Thr Leu His
290 295 300

Arg Leu Ala Gly Ala Cys Ala Gln Val Glu Leu Phe Pro Ile Asp
305 310 315

Asp Val Phe Leu Gly Met Cys Leu Gln Arg Leu Arg Leu Thr Pro
320 325 330

Glu Pro His Pro Ala Phe Arg Thr Phe Gly Ile Pro Gln Pro Ser
335 340 345

Ala Ala Pro His Leu Ser Thr Phe Asp Pro Cys Phe Tyr Arg Glu
350 355 360

Leu Val Val Val His Gly Leu Ser Ala Ala Asp Ile Trp Leu Met
365 370 375

Trp Arg Leu Leu His Gly Pro His Gly Pro Ala Cys Ala His Pro
380 385 390

Gln Pro Val Ala Ala Gly Pro Phe Gln Trp Asp Ser
395 400 402

<210> 43
<211> 27
<212> DNA
<213> Artificial Sequence

<220>
<223> Synthetic Oligonucleotide Probe

<400> 43
acctaacgct caaggagatc cactttc 27

<210> 44
<211> 25
<212> DNA
<213> Artificial Sequence

<220>
<223> Synthetic Oligonucleotide Probe

<400> 44
ggctccattc tgggtctgag ttagg 25

<210> 45
<211> 40

<212> DNA
<213> Artificial Sequence

<220>
<223> Synthetic Oligonucleotide Probe

<400> 45
gcctcagctt tctgccccga cgtgcgcttc gtttttaagg 40

<210> 46
<211> 1226
<212> DNA
<213> Homo Sapien

<400> 46
atgaaagtga taatcaggca gcccaaatga ttgttaataa ggatcaaatg 50

agatcgtgta tgtgggtcca atcaattgat tctacacaaa ggagcctggg 100

gaggggccat ggtgccaatg cacttactgg ggagactgga gaagccgctt 150

ctcctcctgt gctgcgcctc cttcctactg gggctggctt tgctgggcat 200

aaagacggac atcacccccg ttgcttattt ctttctcaca ttgggtggct 250

tcttcttgtt tgcctatctc ctggtccggt ttctggaatg gggcttcgg 300

tcccagctcc aatcaatgca gactgagagc ccagggccct caggcaatgc 350

acgggacaat gaagcctttg aagtgccagt ctatgaagag gccgtggtgg 400

gactagaatc ccagtgccgc ccccaagagt tggaccaacc accccccctac 450

agcactgttg tgataccccc agcacctgag gaggaacaac ctagccatcc 500

agaggggtcc aggagagcca aactggaaca gaggcgaatg gcctcagagg 550

ggtccatggc ccaggaagga agccctggaa gagctccaat caaccttcgg 600

cttcggggac cacgggctgt gtccactgct cctgatctgc agagcttggc 650

ggcagtcccc acattagagc ctctgactcc acccccctgcc tatgatgtct 700

gctttggtca ccctgatgat gatagtgttt tttatgagga caactgggca 750

ccccccttaaa tgactctccc aagatttctc ttctctccac accagacctc 800

gttcatttga ctaacatttt ccagcgccta ctatgtgtca gaaacaagtg 850

tttctgcctg gacatcataa atggggactt ggaccctgag gagagtcagg 900

ccacggtaag cccttcccag ctgagatatg ggtggcataa tttgagtctt 950

ctggcaacat ttggtgacct accccatatc caatatttcc agcgttagat 1000

tgaggatgag gtagggaggt gatccagaga aggcggagaa ggaagaagta 1050

acctctgagt ggcggctatt gcttctgttc caggtgctgt tcgagctgtt 1100

agaaccctta ggcttgacag ctttgtgagt tattattgaa aaatgaggat 1150

tccaagagtc agaggagttt gataatgtgc acgagggcac actgctagta 1200

aataacatta aaataactgg aatgaa 1226

<210> 47
<211> 216
<212> PRT
<213> Homo Sapien

<400> 47
Met Val Pro Met His Leu Leu Gly Arg Leu Glu Lys Pro Leu Leu
1 5 10 15

Leu Leu Cys Cys Ala Ser Phe Leu Leu Gly Leu Ala Leu Leu Gly
20 25 30

Ile Lys Thr Asp Ile Thr Pro Val Ala Tyr Phe Phe Leu Thr Leu
35 40 45

Gly Gly Phe Phe Leu Phe Ala Tyr Leu Leu Val Arg Phe Leu Glu
50 55 60

Trp Gly Leu Arg Ser Gln Leu Gln Ser Met Gln Thr Glu Ser Pro
65 70 75

Gly Pro Ser Gly Asn Ala Arg Asp Asn Glu Ala Phe Glu Val Pro
80 85 90

Val Tyr Glu Glu Ala Val Val Gly Leu Glu Ser Gln Cys Arg Pro
95 100 105

Gln Glu Leu Asp Gln Pro Pro Pro Tyr Ser Thr Val Val Ile Pro
110 115 120

Pro Ala Pro Glu Glu Glu Gln Pro Ser His Pro Glu Gly Ser Arg
125 130 135

Arg Ala Lys Leu Glu Gln Arg Arg Met Ala Ser Glu Gly Ser Met
140 145 150

Ala Gln Glu Gly Ser Pro Gly Arg Ala Pro Ile Asn Leu Arg Leu
155 160 165

Arg Gly Pro Arg Ala Val Ser Thr Ala Pro Asp Leu Gln Ser Leu
170 175 180

Ala Ala Val Pro Thr Leu Glu Pro Leu Thr Pro Pro Pro Ala Tyr
185 190 195

Asp Val Cys Phe Gly His Pro Asp Asp Asp Ser Val Phe Tyr Glu
200 205 210

Asp Asn Trp Ala Pro Pro
215 216

<210> 48
<211> 1310
<212> DNA
<213> Homo Sapien

<400> 48
cggctcgagc ccgcccggaa gtgcccgagg ggccgcgatg gagctggggg 50

agccgggcgc tcggtagcgc ggcgggcaag gcaggcgcca tgaccctgat 100

tgaaggggtg ggtgatgagg tgaccgtcct tttctcggtg cttgcctgcc 150

ttctggtgct ggcccttgcc tgggtctcaa cgcacaccgc tgagggcggg 200

gacccactgc cccagccgtc agggacccca acgccatccc agcccagcgc 250

agccatggca gctaccgaca gcatgagagg ggaggcccca ggggcagaga 300

cccccagcct gagacacaga ggtcaagctg cacagccaga gcccagcacg 350

gggttcacag caacaccgcc agccccggac tccccgcagg agcccctcgt 400

gctacggctg aaattcctca atgattcaga gcaggtggcc agggcctggc 450

cccacgacac cattggctcc ttgaaaagga cccagtttcc cggccgggaa 500

cagcaggtgc gactcatcta ccaagggcag ctgctaggcg acgacaccca 550

gaccctgggc agccttcacc tccctcccaa ctgcgttctc cactgccacg 600

tgtccacgag agtcggtccc ccaaatcccc cctgcccgcc ggggtccgag 650

cccggcccct ccgggctgga aatcggcagc ctgctgctgc ccctgctgct 700

cctgctgttg ctgctgctct ggtactgcca gatccagtac cggcccttct 750

ttcccctgac cgccactctg ggcctggccg gcttcaccct gctcctcagt 800

ctcctggcct ttgccatgta ccgcccgtag tgcctccgcg ggcgcttggc 850

agcgtcgccg gcccctccgg accttgctcc ccgcgccgcg gcgggagctg 900

ctgcctgccc aggcccgcct ctccggcctg cctcttcccg ctgccctgga 950

gcccagccct gcgccgcaga ggactcccgg gactggcgga ggccccgccc 1000

tgcgaccgcc ggggctcggg gccacctccc ggggctgctg aacctcagcc 1050

cgcactggga gtgggctcct cggggtcggg catctgctgt cgctgcctcg 1100

gccccgggca gagccgggcc gccccggggg cccgtcttag tgttctgccg 1150

gaggacccag ccgcctccaa tccctgacag ctccttgggc tgagttgggg 1200

acgccaggtc ggtgggaggc tggtgaaggg gagcggggag gggcagagga 1250

gttccccgga acccgtgcag attaaagtaa ctgtgaagtt ttaaaaaaaa 1300

aaaaaaaaaa 1310

<210> 49
<211> 246
<212> PRT
<213> Homo Sapien

<400> 49

Met Thr Leu Ile Glu Gly Val Gly Asp Glu Val Thr Val Leu Phe
1 5 10 15

Ser Val Leu Ala Cys Leu Leu Val Leu Ala Leu Ala Trp Val Ser
20 25 30

Thr His Thr Ala Glu Gly Gly Asp Pro Leu Pro Gln Pro Ser Gly
35 40 45

Thr Pro Thr Pro Ser Gln Pro Ser Ala Ala Met Ala Ala Thr Asp
50 55 60

Ser Met Arg Gly Glu Ala Pro Gly Ala Glu Thr Pro Ser Leu Arg
65 70 75

His Arg Gly Gln Ala Ala Gln Pro Glu Pro Ser Thr Gly Phe Thr
80 85 90

Ala Thr Pro Pro Ala Pro Asp Ser Pro Gln Glu Pro Leu Val Leu
95 100 105

Arg Leu Lys Phe Leu Asn Asp Ser Glu Gln Val Ala Arg Ala Trp
110 115 120

Pro His Asp Thr Ile Gly Ser Leu Lys Arg Thr Gln Phe Pro Gly
125 130 135

Arg Glu Gln Gln Val Arg Leu Ile Tyr Gln Gly Gln Leu Leu Gly
140 145 150

Asp Asp Thr Gln Thr Leu Gly Ser Leu His Leu Pro Pro Asn Cys
155 160 165

Val Leu His Cys His Val Ser Thr Arg Val Gly Pro Pro Asn Pro
170 175 180

Pro Cys Pro Pro Gly Ser Glu Pro Gly Pro Ser Gly Leu Glu Ile
185 190 195

Gly Ser Leu Leu Leu Pro Leu Leu Leu Leu Leu Leu Leu Leu Leu
200 205 210

Trp Tyr Cys Gln Ile Gln Tyr Arg Pro Phe Phe Pro Leu Thr Ala
215 220 225

Thr Leu Gly Leu Ala Gly Phe Thr Leu Leu Leu Ser Leu Leu Ala
230 235 240

Phe Ala Met Tyr Arg Pro
245 246

<210> 50
<211> 1042
<212> DNA
<213> Homo Sapien

<400> 50
tttcctcact gactataaaa gaatagagaa ggaagggctt cagtgaccgg 50

ctgcctggct gacttacagc agtcagactc tgacaggatc atggctatga 100

tggaggtcca ggggggaccc agcctgggac agacctgcgt gctgatcgtg 150

atcttcacag tgctcctgca gtctctctgt gtggctgtaa cttacgtgta 200

ctttaccaac gagctgaagc agatgcagga caagtactcc aaaagtggca 250

ttgcttgttt cttaaaagaa gatgacagtt attgggaccc caatgacgaa 300

gagagtatga acagcccctg ctggcaagtc aagtggcaac tccgtcagct 350

cgttagaaag atgattttga gaacctctga ggaaaccatt tctacagttc 400

aagaaaagca acaaaatatt tctcccctag tgagagaaag aggtcctcag 450

agagtagcag ctcacataac tgggaccaga ggaagaagca acacattgtc 500

ttctccaaac tccaagaatg aaaaggctct gggccgcaaa ataaactcct 550

gggaatcatc aaggagtggg cattcattcc tgagcaactt gcacttgagg 600

aatggtgaac tggtcatcca tgaaaaaggg ttttactaca tctattccca 650

aacatacttt cgatttcagg aggaaataaa agaaaacaca aagaacgaca 700

aacaaatggt ccaatatatt tacaaataca caagttatcc tgaccctata 750

ttgttgatga aaagtgctag aaatagttgt tggtctaaag atgcagaata 800

tggactctat tccatctatc aagggggaat atttgagctt aaggaaaatg 850

acagaatttt tgtttctgta acaaatgagc acttgataga catggaccat 900

gaagccagtt ttttcggggc ctttttagtt ggctaactga cctggaaaga 950

aaaagcaata acctcaaagt gactattcag ttttcaggat gatacactat 1000

gaagatgttt caaaaaatct gaccaaaaca aacaaacaga aa 1042

<210> 51
<211> 281
<212> PRT
<213> Homo Sapien

<400> 51
Met Ala Met Met Glu Val Gln Gly Gly Pro Ser Leu Gly Gln Thr
1 5 10 15

Cys Val Leu Ile Val Ile Phe Thr Val Leu Leu Gln Ser Leu Cys
20 25 30

Val Ala Val Thr Tyr Val Tyr Phe Thr Asn Glu Leu Lys Gln Met
35 40 45

Gln Asp Lys Tyr Ser Lys Ser Gly Ile Ala Cys Phe Leu Lys Glu
50 55 60

Asp Asp Ser Tyr Trp Asp Pro Asn Asp Glu Glu Ser Met Asn Ser
65 70 75

Pro Cys Trp Gln Val Lys Trp Gln Leu Arg Gln Leu Val Arg Lys
80 85 90

Met Ile Leu Arg Thr Ser Glu Glu Thr Ile Ser Thr Val Gln Glu
95 100 105

Lys Gln Gln Asn Ile Ser Pro Leu Val Arg Glu Arg Gly Pro Gln
110 115 120

Arg Val Ala Ala His Ile Thr Gly Thr Arg Gly Arg Ser Asn Thr
125 130 135

```
Leu Ser Ser Pro Asn Ser Lys Asn Glu Lys Ala Leu Gly Arg Lys
                140                 145                 150

Ile Asn Ser Trp Glu Ser Ser Arg Ser Gly His Ser Phe Leu Ser
                155                 160                 165

Asn Leu His Leu Arg Asn Gly Glu Leu Val Ile His Glu Lys Gly
                170                 175                 180

Phe Tyr Tyr Ile Tyr Ser Gln Thr Tyr Phe Arg Phe Gln Glu Glu
                185                 190                 195

Ile Lys Glu Asn Thr Lys Asn Asp Lys Gln Met Val Gln Tyr Ile
                200                 205                 210

Tyr Lys Tyr Thr Ser Tyr Pro Asp Pro Ile Leu Leu Met Lys Ser
                215                 220                 225

Ala Arg Asn Ser Cys Trp Ser Lys Asp Ala Glu Tyr Gly Leu Tyr
                230                 235                 240

Ser Ile Tyr Gln Gly Gly Ile Phe Glu Leu Lys Glu Asn Asp Arg
                245                 250                 255

Ile Phe Val Ser Val Thr Asn Glu His Leu Ile Asp Met Asp His
                260                 265                 270

Glu Ala Ser Phe Phe Gly Ala Phe Leu Val Gly
                275                 280 281
```

```
<210> 52
<211> 540
<212> DNA
<213> Homo Sapien

<400> 52
atcacttctg agcacggagc aatggcctct cgctgggctg tgcagctgct 50

gctcgtggca gcctggagca tgggctgtgg tgaggccctc aagtgctaca 100

cctgcaagga gcccatgacc agtgcttcct gcaggaccat tacccgctgc 150

aagccagagg acacagcctg catgaccacg ctggtgacgg tggaggcaga 200

gtaccccttc aaccagagcc ccgtggtgac ccgctcctgc tccagctcct 250

gtgtggccac cgaccccgac agcatcgggg ccgcccacct gatcttctgc 300

tgcttccgag acctctgcaa ctcggaactc tgaacccagg gcggcagggc 350

ggaaggtgct cctcaggcac ctcctctctg acggggcctg gctccacctg 400

tgatcacctc cccctgcttc ctgctgctgt ggcacagctc actcatgggg 450

tctgagggga gagaagcaca ccaggggcgc cctctgcctt ccatacccca 500

cgcttataaa acataactaa gccaaaaaaa aaaaaaaaaa 540

<210> 53
<211> 103
<212> PRT
<213> Homo Sapien
```

<400> 53
Met Ala Ser Arg Trp Ala Val Gln Leu Leu Leu Val Ala Ala Trp
1 5 10 15

Ser Met Gly Cys Gly Glu Ala Leu Lys Cys Tyr Thr Cys Lys Glu
20 25 30

Pro Met Thr Ser Ala Ser Cys Arg Thr Ile Thr Arg Cys Lys Pro
35 40 45

Glu Asp Thr Ala Cys Met Thr Thr Leu Val Thr Val Glu Ala Glu
50 55 60

Tyr Pro Phe Asn Gln Ser Pro Val Val Thr Arg Ser Cys Ser Ser
65 70 75

Ser Cys Val Ala Thr Asp Pro Asp Ser Ile Gly Ala Ala His Leu
80 85 90

Ile Phe Cys Cys Phe Arg Asp Leu Cys Asn Ser Glu Leu
95 100 103

<210> 54
<211> 1183
<212> DNA
<213> Homo Sapien

<400> 54
gcggccgcgg agggaagggg tgggtcggtg ggtctgacag cgggtctgcg 50

taggcggcag cgtctgtccc tcccagcctc tcgctccgcg ccatgggcgg 100

gccccgggct ctgctggccg cactctgggc gctggaagcc gccgggaccg 150

ccgcgcttcg catcggagcc ttcaacattc agagcttcgg tgacagcaaa 200

gtgtcggacc ccgcttgcgg cagcatcatc gcgaagatcc tggctggcta 250

tgacctcgcg ctggtgcagg aggtgcgaga cccagacctc agcgccgtgt 300

ccgcgctcat ggagcagatc aacagcgtgt ccgagcacga gtacagcttt 350

gtgagcagcc agcccctggg ccgggaccag tacaaggaga tgtacctgtt 400

cgtgtacagg aaagacgcgg tgtcggtcgt ggacacctac ctgtacccag 450

accccgagga cgtcttcagc cgcgagccct tcgtggtcaa gttctcggcc 500

cccggcaccg gtgagcgggc cccgcccctc ccctcccgcc gagctctgac 550

gcccccaccc cttcccgcag cagcacagaa cctggtgctg atcccgctgc 600

acgcggcgcc gcatcaagcc gtggcggaga tcgacgcgct ctacgacgtg 650

tacctggacg tgatcgacaa gtggggcacc gacgacatgc tgttcctggg 700

cgacttcaac gccgactgca gctatgtgcg ggcgcaggac tgggccgcca 750

tccgtctgag gagcagtgag gtcttcaagt ggctcatccc tgacagcgcc 800

gacaccacgg tgggcaactc agactgcgcc tacgaccgca ttgtggcctg 850

tggcgcccgc ctgcgccgga gcctgaagcc ccagtcggcc accgtgcacg 900

acttccagga ggaattcggc ctggaccaga ctcaggctct tgccatcagc 950

gaccactttc cagtggaggt gaccctcaag ttccaccgat gactcgaggc 1000

ctgactgggg catgccacct gcagaccctg gctctgagga atggcccaac 1050

agtggcccct tcagggtggc agccaccctt cagtgaggcc ccaaggcaga 1100

gtcggctggg cgtggaccag gggcatggac acgtgatgtg ctgctctgta 1150

cctccgttcc ccatctgtgg gacgggctgg atc 1183

<210> 55
<211> 299
<212> PRT
<213> Homo Sapien

<400> 55
Met Gly Gly Pro Arg Ala Leu Leu Ala Ala Leu Trp Ala Leu Glu
1 5 10 15

Ala Ala Gly Thr Ala Ala Leu Arg Ile Gly Ala Phe Asn Ile Gln
20 25 30

Ser Phe Gly Asp Ser Lys Val Ser Asp Pro Ala Cys Gly Ser Ile
35 40 45

Ile Ala Lys Ile Leu Ala Gly Tyr Asp Leu Ala Leu Val Gln Glu
50 55 60

Val Arg Asp Pro Asp Leu Ser Ala Val Ser Ala Leu Met Glu Gln
65 70 75

Ile Asn Ser Val Ser Glu His Glu Tyr Ser Phe Val Ser Ser Gln
80 85 90

Pro Leu Gly Arg Asp Gln Tyr Lys Glu Met Tyr Leu Phe Val Tyr
95 100 105

Arg Lys Asp Ala Val Ser Val Val Asp Thr Tyr Leu Tyr Pro Asp
110 115 120

Pro Glu Asp Val Phe Ser Arg Glu Pro Phe Val Val Lys Phe Ser
125 130 135

Ala Pro Gly Thr Gly Glu Arg Ala Pro Pro Leu Pro Ser Arg Arg
140 145 150

Ala Leu Thr Pro Pro Pro Leu Pro Ala Ala Ala Gln Asn Leu Val
155 160 165

Leu Ile Pro Leu His Ala Ala Pro His Gln Ala Val Ala Glu Ile
170 175 180

Asp Ala Leu Tyr Asp Val Tyr Leu Asp Val Ile Asp Lys Trp Gly
185 190 195

Thr Asp Asp Met Leu Phe Leu Gly Asp Phe Asn Ala Asp Cys Ser
200 205 210

Tyr Val Arg Ala Gln Asp Trp Ala Ala Ile Arg Leu Arg Ser Ser
215 220 225

```
Glu Val Phe Lys Trp Leu Ile Pro Asp Ser Ala Asp Thr Thr Val
                230                 235                 240

Gly Asn Ser Asp Cys Ala Tyr Asp Arg Ile Val Ala Cys Gly Ala
                245                 250                 255

Arg Leu Arg Arg Ser Leu Lys Pro Gln Ser Ala Thr Val His Asp
                260                 265                 270

Phe Gln Glu Glu Phe Gly Leu Asp Gln Thr Gln Ala Leu Ala Ile
                275                 280                 285

Ser Asp His Phe Pro Val Glu Val Thr Leu Lys Phe His Arg
                290                 295             299
```

<210> 56
<211> 28
<212> DNA
<213> Artificial Sequence

<220>
<223> Synthetic Oligonucleotide Probe

<400> 56
ctttgaatag aagacttctg gacaattt 28

<210> 57
<211> 30
<212> DNA
<213> Artificial Sequence

<220>
<223> Synthetic Oligonucleotide Probe

<400> 57
ttgcaactgg gaatatacca cgacatgaga 30

<210> 58
<211> 26
<212> DNA
<213> Artificial Sequence

<220>
<223> Synthetic Oligonucleotide Probe

<400> 58
tagggtgcta atttgtgcta taacct 26

<210> 59
<211> 20
<212> DNA
<213> Artificial Sequence

<220>
<223> Synthetic Oligonucleotide Probe

<400> 59
ggctctgagt ctctgcttga 20

<210> 60
<211> 25
<212> DNA
<213> Artificial Sequence

<220>
<223> Synthetic Oligonucleotide Probe

<400> 60
tccaacaacc attttcctct ggtcc 25

<210> 61
<211> 23
<212> DNA
<213> Artificial Sequence

<220>
<223> Synthetic Oligonucleotide Probe

<400> 61
aagcagtagc cattaacaag tca 23

<210> 62
<211> 18
<212> DNA
<213> Artificial Sequence

<220>
<223> Synthetic Oligonucleotide Probe

<400> 62
aaaggacacc gggatgtg 18

<210> 63
<211> 26
<212> DNA
<213> Artificial Sequence

<220>
<223> Synthetic Oligonucleotide Probe

<400> 63
agcgtacact ctctccaggc aaccag 26

<210> 64
<211> 22
<212> DNA
<213> Artificial Sequence

<220>
<223> Synthetic Oligonucleotide Probe

<400> 64
caattctgga tgaggtggta ga 22

<210> 65
<211> 20
<212> DNA
<213> Artificial Sequence

<220>
<223> Synthetic Oligonucleotide Probe

<400> 65
caggactgag cgcttgttta 20

<210> 66
<211> 21

<212> DNA
<213> Artificial Sequence

<220>
<223> Synthetic Oligonucleotide Probe

<400> 66
caaagcgcca agtaccggac c 21

<210> 67
<211> 18
<212> DNA
<213> Artificial Sequence

<220>
<223> Synthetic Oligonucleotide Probe

<400> 67
ccagacctca gccaggaa 18

<210> 68
<211> 19
<212> DNA
<213> Artificial Sequence

<220>
<223> Synthetic Oligonucleotide Probe

<400> 68
tcatggtctc gtcccattc 19

<210> 69
<211> 27
<212> DNA
<213> Artificial Sequence

<220>
<223> Synthetic Oligonucleotide Probe

<400> 69
caccatttgt ttctctgtct ccccatc 27

<210> 70
<211> 18
<212> DNA
<213> Artificial Sequence

<220>
<223> Synthetic Oligonucleotide Probe

<400> 70
ccggcatcct tggagtag 18

<210> 71
<211> 18
<212> DNA
<213> Artificial Sequence

<220>
<223> Synthetic Oligonucleotide Probe

<400> 71
ctctggtgcc cacagtga 18

<210> 72
<211> 21
<212> DNA
<213> Artificial Sequence

<220>
<223> Synthetic Oligonucleotide Probe

<400> 72
ccatgcctgc tcagccaaga a 21

<210> 73
<211> 23
<212> DNA
<213> Artificial Sequence

<220>
<223> Synthetic Oligonucleotide Probe

<400> 73
caggaaatct ggaaacctac agt 23

<210> 74
<211> 20
<212> DNA
<213> Artificial Sequence

<220>
<223> Synthetic Oligonucleotide Probe

<400> 74
tccccattag cacaggagta 20

<210> 75
<211> 23
<212> DNA
<213> Artificial Sequence

<220>
<223> Synthetic Oligonucleotide Probe

<400> 75
aggctcttgc ctgtcctgct gct 23

<210> 76
<211> 18
<212> DNA
<213> Artificial Sequence

<220>
<223> Synthetic Oligonucleotide Probe

<400> 76
gcccagagtc ccacttgt 18

<210> 77
<211> 18
<212> DNA
<213> Artificial Sequence

<220>
<223> Synthetic Oligonucleotide Probe

<400> 77
gtccagcaag ccctcatt 18

<210> 78
<211> 20
<212> DNA
<213> Artificial Sequence

<220>
<223> Synthetic Oligonucleotide Probe

<400> 78
cttctgggcc acagccctgc 20

<210> 79
<211> 21
<212> DNA
<213> Artificial Sequence

<220>
<223> Synthetic Oligonucleotide Probe

<400> 79
cagttcaggt cgtttcattc a 21

<210> 80
<211> 19
<212> DNA
<213> Artificial Sequence

<220>
<223> Synthetic Oligonucleotide Probe

<400> 80
ccagtcaggc cgttttaga 19

<210> 81
<211> 21
<212> DNA
<213> Artificial Sequence

<220>
<223> Synthetic Oligonucleotide Probe

<400> 81
cgggcgccca agtaaaagct c 21

<210> 82
<211> 28
<212> DNA
<213> Artificial Sequence

<220>
<223> Synthetic Oligonucleotide Probe

<400> 82
cataaagtag tatatgcatt ccagtgtt 28

<210> 83
<211> 20
<212> DNA
<213> Artificial Sequence

<220>
<223> Synthetic Oligonucleotide Probe

<400> 83
ggaaatggtc tcaagggaaa 20

<210> 84
<211> 26
<212> DNA
<213> Artificial Sequence

<220>
<223> Synthetic Oligonucleotide Probe

<400> 84
tcactttgac cctgtcttgg aacgtc 26

<210> 85
<211> 22
<212> DNA
<213> Artificial Sequence

<220>
<223> Synthetic Oligonucleotide Probe

<400> 85
ggtagaattc cagcatttgg ta 22

<210> 86
<211> 18
<212> DNA
<213> Artificial Sequence

<220>
<223> Synthetic Oligonucleotide Probe

<400> 86
aggacttgcc ctcaggaa 18

<210> 87
<211> 21
<212> DNA
<213> Artificial Sequence

<220>
<223> Synthetic Oligonucleotide Probe

<400> 87
cgcaggacag ttgtgaaaat a 21

<210> 88
<211> 21
<212> DNA
<213> Artificial Sequence

<220>
<223> Synthetic Oligonucleotide Probe

<400> 88
atgacgctcg tccaaggcca c 21

<210> 89
<211> 19

<212> DNA
<213> Artificial Sequence

<220>
<223> Synthetic Oligonucleotide Probe

<400> 89
cccacctgta ccaccatgt 19

<210> 90
<211> 24
<212> DNA
<213> Artificial Sequence

<220>
<223> Synthetic Oligonucleotide Probe

<400> 90
actccaggca ccatctgttc tccc 24

<210> 91
<211> 19
<212> DNA
<213> Artificial Sequence

<220>
<223> Synthetic Oligonucleotide Probe

<400> 91
aagggctggc attcaagtu 19

<210> 92
<211> 20
<212> DNA
<213> Artificial Sequence

<220>
<223> Synthetic Oligonucleotide Probe

<400> 92
ctggccctca gagcaccaat 20

<210> 93
<211> 25
<212> DNA
<213> Artificial Sequence

<220>
<223> Synthetic Oligonucleotide Probe

<400> 93
tcctccatca cttcccctag ctcca 25

<210> 94
<211> 24
<212> DNA
<213> Artificial Sequence

<220>
<223> Synthetic Oligonucleotide Probe

<400> 94
ctggcaggag ttaaagttcc aaga 24

<210> 95
<211> 18
<212> DNA
<213> Artificial Sequence

<220>
<223> Synthetic Oligonucleotide Probe

<400> 95
gggcaacagc ctgagagt 18

<210> 96
<211> 29
<212> DNA
<213> Artificial Sequence

<220>
<223> Synthetic Oligonucleotide Probe

<400> 96
actcagtgtt gattctctat cgtgatgcg 29

<210> 97
<211> 19
<212> DNA
<213> Artificial Sequence

<220>
<223> Synthetic Oligonucleotide Probe

<400> 97
gagcagcagg catcaattt 19

<210> 98
<211> 19
<212> DNA
<213> Artificial Sequence

<220>
<223> Synthetic Oligonucleotide Probe

<400> 98
ggcctggagt tgctgataa 19

<210> 99
<211> 37
<212> DNA
<213> Artificial Sequence

<220>
<223> Synthetic Oligonucleotide Probe

<400> 99
ttgagcttaa gtagaccaag tatctatccc acctaaa 37

<210> 100
<211> 18
<212> DNA
<213> Artificial Sequence

<220>
<223> Synthetic Oligonucleotide Probe

<400> 100
ggtgggctct gggttaca 18

<210> 101
<211> 19
<212> DNA
<213> Artificial Sequence

<220>
<223> Synthetic Oligonucleotide Probe

<400> 101
agccggtttc ccagattat 19

<210> 102
<211> 24
<212> DNA
<213> Artificial Sequence

<220>
<223> Synthetic Oligonucleotide Probe

<400> 102
tgccgtgtat gtggttcttc cctg 24

<210> 103
<211> 19
<212> DNA
<213> Artificial Sequence

<220>
<223> Synthetic Oligonucleotide Probe

<400> 103
gagacaggca cctggtgat 19

<210> 104
<211> 19
<212> DNA
<213> Artificial Sequence

<220>
<223> Synthetic Oligonucleotide Probe

<400> 104
tgtttctgcc tggacatca 19

<210> 105
<211> 18
<212> DNA
<213> Artificial Sequence

<220>
<223> Synthetic Oligonucleotide Probe

<400> 105
gcttaccgtg gcctgact 18

<210> 106
<211> 22
<212> DNA
<213> Artificial Sequence

<220>
<223> Synthetic Oligonucleotide Probe

<400> 106
tcctcagggt ccaagtcccc at 22

<210> 107
<211> 20
<212> DNA
<213> Artificial Sequence

<220>
<223> Synthetic Oligonucleotide Probe

<400> 107
ccttgaaaag gacccagttt 20

<210> 108
<211> 22
<212> DNA
<213> Artificial Sequence

<220>
<223> Synthetic Oligonucleotide Probe

<400> 108
atgagtcgca cctgctgttc cc 22

<210> 109
<211> 18
<212> DNA
<213> Artificial Sequence

<220>
<223> Synthetic Oligonucleotide Probe

<400> 109
tagcagctgc ccttggta 18

<210> 110
<211> 22
<212> DNA
<213> Artificial Sequence

<220>
<223> Synthetic Oligonucleotide Probe

<400> 110
aacagcaggt gcgactcatc ta 22

<210> 111
<211> 23
<212> DNA
<213> Artificial Sequence

<220>
<223> Synthetic Oligonucleotide Probe

<400> 111
tgctaggcga cgacacccag acc 23

<210> 112
<211> 18

<212> DNA
<213> Artificial Sequence

<220>
<223> Synthetic Oligonucleotide Probe

<400> 112
tggacacgtg gcagtgga 18

<210> 113
<211> 19
<212> DNA
<213> Artificial Sequence

<220>
<223> Synthetic Oligonucleotide Probe

<400> 113
tggaccatga agccagttt 19

<210> 114
<211> 32
<212> DNA
<213> Artificial Sequence

<220>
<223> Synthetic Oligonucleotide Probe

<400> 114
ccttttagt tggctaactg acctggaaag aa 32

<210> 115
<211> 25
<212> DNA
<213> Artificial Sequence

<220>
<223> Synthetic Oligonucleotide Probe

<400> 115
tgaatagtca ctttgaggtt attgc 25

<210> 116
<211> 18
<212> DNA
<213> Artificial Sequence

<220>
<223> Synthetic Oligonucleotide Probe

<400> 116
cctggctcca cctgtgat 18

<210> 117
<211> 21
<212> DNA
<213> Artificial Sequence

<220>
<223> Synthetic Oligonucleotide Probe

<400> 117
acctccccct gcttcctgct g 21

<210> 118
<211> 19
<212> DNA
<213> Artificial Sequence

<220>
<223> Synthetic Oligonucleotide Probe

<400> 118
cctcagaccc catgagtga 19

<210> 119
<211> 18
<212> DNA
<213> Artificial Sequence

<220>
<223> Synthetic Oligonucleotide Probe

<400> 119
gaggaatggc ccaacagt 18

<210> 120
<211> 21
<212> DNA
<213> Artificial Sequence

<220>
<223> Synthetic Oligonucleotide Probe

<400> 120
tggcagccac ccttcagtga g 21

<210> 121
<211> 18
<212> DNA
<213> Artificial Sequence

<220>
<223> Synthetic Oligonucleotide Probe

<400> 121
cagcacatca cgtgtcca 18

<210> 122
<211> 18
<212> DNA
<213> Artificial Sequence

<220>
<223> Synthetic Oligonucleotide Probe

<400> 122
gaggaatggc ccaacagt 18

<210> 123
<211> 20
<212> DNA
<213> Artificial Sequence

<220>
<223> Synthetic Oligonucleotide Probe

<400> 123
tgtccatgcc cctggtccac 20

<210> 124
<211> 21
<212> DNA
<213> Artificial Sequence

<220>
<223> Synthetic Oligonucleotide Probe

<400> 124
gaggtacaga gcagcacatc a 21